# EUROPEAN PATENT APPLICATION

(11) **EP 1 621 621 A1**
(43) Date of publication of application: **01.02.2006**
(21) Application number: 05020875.0
(22) Date of filing: 08.09.2000
(51) Int. Cl.: C12N 15/12, C07K 14/705, C07K 16/28, A01K 67/027, C12N 1/21

(54) **Human 7tm protein receptors and polynucleotides encoding the same**

(30) Priority: 10.09.1999 US 153366 P; 15.11.1999 US 165510 P
(62) Divisional of application: 00964952.6
(71) Applicant: Lexicon Genetics Incorporated, The Woodlands, TX 77381-1160 (US)
(72) Inventor: Turner, C. Alexander, The Woodlands, TX 77381 (US); Nehls, Michael, 82131 Stockdorf (DE); Friedrich, Glenn, Houston, TX 77004 (US); Scoville, John, Houston, TX 77024 (US); Zambrowicz, Brian, The Woodlands, TX 77382 (US); Sands, Arthur, T., The Woodlands, TX 77382 (US)
(74) Representative: Silveston, Judith

(57) **Abstract**

The nucleotide and amino acid sequences of several novel human proteins sharing homology with human G protein coupled receptors are described.

## Description

The present invention claims priority to U.S. Provisional Applications Ser. Nos. 60/153,366, filed 9/10/99 and 60/165,510, filed 11/15/99 which are herein incorporated by reference in their entirety.

### 1. INTRODUCTION

The present invention relates to the discovery, identification and characterization of novel human polynucleotides that encode proteins and membrane associated receptors. The invention encompasses the described polynucleotides, host cell expression systems, the encoded proteins, fusion proteins, polypeptides and peptides, antibodies to the encoded proteins and peptides, and genetically engineered animals that lack the disclosed genes, or over express the disclosed sequences, or antagonists and agonists of the proteins, and other compounds that modulate the expression or activity of the proteins encoded by the disclosed genes that can be used for diagnosis, drug screening, clinical trial monitoring, and/or the treatment of physiological or behavioral disorders.

Membrane receptor proteins are integral components of the mechanism through which cells sense their surroundings, regulate cellular functions, and maintain physiological homeostasis. Accordingly, membrane receptor proteins are often involved in signal transduction pathways that control cell physiology, chemical communication, and gene expression. A particularly relevant class of membrane receptors are those typically characterized by the presence of 7 conserved transmembrane domains that are interconnected by nonconserved hydrophilic loops. Such, "7TM receptors" include a superfamily of receptors known as G-protein coupled receptors (GPCRs). GPCRs are typically involved in signal transduction pathways involving G-proteins. As such, the GPCR family includes many receptors that are known to serve as drug targets for therapeutic agents.

### 3. SUMMARY OF THE INVENTION

The present invention relates to the discovery, identification and characterization of nucleotides that encode novel GPCRs, and the corresponding amino acid sequences of the novel GPCRs. The GPCRs described for the first time herein, are transmembrane proteins that span the cellular membrane and are involved in signal transduction after ligand binding. The described GPCRs have structural motifs found in the 7TM receptor family. The GPCR mRNA transcripts are expressed, *inter alia*, in placenta, lung, kidney, liver, and pancreas, cells, among others. The novel human GPCRs described herein, encode proteins of 1,250, 1,221, 718, 1,112, 1,249, 1,220, 717, 1,111, 1,250, 1,221, 718, 1,112, 541, 512, 8, 403, 1,222, 1,193, 690, 1,084, 1,221, 1,192, 689, 1,083, 1,222, 1,193, 690, 1,084, and 1,192 amino acids in length (see SEQ ID NOS: 2, 4, 6, 10, 12, 14, 16, 18, 20, 22, 24, 26, 28, 30, 32, 34, 36, 38, 40, 42, 44, 46, 48, 50, 52, 54, 56, and 58 respectively). The described GPCRs have the characteristic seven transmembrane regions (of about 20-30 amino acids), as well as several predicted cytoplasmic domains. As evidenced by the alternative 5' regions, and splice junctions of the open reading frames presented in the Sequence Listing, the transcription of the described novel GPCRs can apparently initiate from one of several different promoters present within the genome. Depending on which promoter is used, the described novel GPCRs (NGPCRs) can have one of several distinct amino acid sequences at or near the amino terminal region of the protein.

Additionally contemplated are murine homologs of the described NGPCRs and corresponding "knockout" ES cells that are produced using conventional methods (see, for example, PCT Applic. No. PCT/US98/03243, filed February 20, 1998, herein incorporated by reference). Accordingly, an additional aspect of the present invention includes knockout cells and animals having genetically engineered mutations in the gene(s) encoding the presently described NGPCRs.

The invention encompasses the nucleotides presented in the Sequence Listing, host cells expressing such nucleotides, the expression products of such nucleotides, and: (a) nucleotides that encode mammalian homologs of the described NGPCRs, including the specifically described human NGPCRs, and the human NGPCR gene products; (b) nucleotides that encode one or more portions of the NGPCRs that correspond to functional domains, and the polypeptide products specified by such nucleotide sequences, including but not limited to the novel regions of the described extracellular domain(s) (ECD), one or more transmembrane domain(s) (TM) first disclosed herein, and the cytoplasmic domain(s) (CD); (c) isolated nucleotides that encode mutants, engineered or naturally occurring, of the described NGPCRs in which all or a part of at least one of the domains is deleted or altered, and the polypeptide products specified by such nucleotide sequences, including but not limited to soluble receptors in which all or a portion of the TM is deleted, and nonfunctional receptors in which all or a portion of the CD is deleted; (d) nucleotides that encode fusion proteins containing the coding region from an NGPCR, or one of its domains (*e.g*., an extracellular domain) fused to another peptide or polypeptide.

The invention also encompasses agonists and antagonists of the NGPCRs, including small molecules, large molecules, mutant NGPCR proteins, or portions thereof that compete with the native NGPCR, and antibodies, as well as nucleotide sequences that can be used to inhibit the expression of the described NGPCR (e.g., antisense and ribozyme molecules, and gene or regulatory sequence replacement constructs) or to enhance the expression of the described NGPCR gene (e.g., expression constructs that place the described gene under the control of a strong promoter system), and transgenic animals that express a NGPCR transgene or "knock-outs" that do not express a functional NGPCR.

Further, the present invention also relates to methods for the use of the described NGPCR gene and/or NGPCR gene products for the identification of compounds that modulate, i.e., act as agonists or antagonists, of NGPCR gene expression and or NGPCR gene product activity. Such compounds can be used as therapeutic agents for the treatment of various symptomatic representations of biological disorders or imbalances.

### 4. DESCRIPTION OF THE SEQUENCE LISTING AND FIGURES

The Sequence Listing provides the polynucleotide sequences of the described NGPCRs, and the amino acid sequences encoded thereby.

### 5. DETAILED DESCRIPTION OF THE INVENTION

The human NGPCRs described for the first time herein are novel proteins that are expressed, *inter alia,* in placenta, lung, liver, pancreas, spinal cord, spleen, thymus, lymph node, adrenal gland, stomach, salivary gland, stomach, mammary gland, thyroid, heart, brain, testis, kidney, adipose, esophagus, rectum, pericardium, trachea, and gene trapped human cells. In view of the tissues that may express the NGPCRs, the described human NGPCRs may be important targets for the therapeutic treatment of, *inter alia,* diabetes, abnormal body weight or obesity, atherosclerosis, heart disease, abnormal blood pressure, cancer, and any associated symptoms.

The described NGPCRs are transmembrane proteins that fall within the 7TM family of receptors. As with other GPCRs, signal transduction is triggered when a ligand binds to the receptor. Interfering with the binding of the natural ligand, or neutralizing or removing the ligand, or interference with its binding to a NGPCR will effect NGPCR mediated signal transduction. Because of their biological significance, 7TM proteins, and particularly GPCRs, have been subjected to intense scientific and commercial scrutiny (see, for example, U.S. Applic. Ser. Nos. 08/820,521, filed March 19, 1997, and 08/833,226, filed April 17, 1997 both of which are herein incorporated by reference in their entirety).

The invention encompasses the use of the described NGPCR nucleotides, NGPCR proteins and peptides as antagonists that inhibit receptor activity or expression, or agonists that activate receptor activity or increase its expression in the diagnosis and treatment of disease. The invention also encompasses the use of antibodies and anti-idiotypic antibodies (including Fab fragments), preferably humanized monoclonal antibodies, or binding fragments, domains, or fusion proteins thereof which bind to NGPCR nucleotides, proteins or peptides and act as therapeutic NGPCR agonists or antagonists.

In particular, the invention described in the subsections below encompasses NGPCR polypeptides or peptides corresponding to functional domains of NGPCR (*e.g*., ECD, TM or CD), mutated, truncated or deleted NGPCRs (*e.g.*, NGPCRs missing one or more functional domains or portions thereof, such as, ΔECD, ΔTM and/or ΔCD), NGPCR fusion proteins (*e.g*., a NGPCR or a functional domain of a NGPCR, such as the ECD, fused to an unrelated protein or peptide such as an immunoglobulin constant region, *i.e*., IgFc), nucleotide sequences encoding such products, and host cell expression systems that can produce such NGPCR products.

The invention also encompasses compounds or nucleotide constructs that inhibit expression of a NGPCR gene (transcription factor inhibitors, antisense and ribozyme molecules, or gene or regulatory sequence replacement constructs), or promote expression of a NGPCR *(e.g.,* expression constructs in which NGPCR coding sequences are operatively associated with expression control elements such as promoters, promoter/enhancers, etc.). The invention also relates to host cells and animals genetically engineered to express the human NGPCRs (or mutants thereof) or to inhibit or "knockout" expression of the animal's endogenous NGPCR genes.

The NGPCR proteins or peptides, NGPCR fusion proteins, NGPCR nucleotide sequences, antibodies, antagonists and agonists can be useful for the detection of mutant NGPCRs or inappropriately expressed NGPCRs for the diagnosis of disease. The NGPCR proteins or peptides, NGPCR fusion proteins, NGPCR nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations resulting from perturbation of the normal function of a NGPCR in the body. The use of engineered host cells and/or animals can offer an advantage in that such systems allow not only for the identification of compounds that bind to an ECD of a NGPCR, but can also identify compounds that affect the signal transduced by an activated NGPCR.

One feature of the described NGPCR proteins is that the 7TM region of the protein is typically present at the C-terminal portion of a protein (for example, beginning at about amino acid 710 of SEQ ID NO: 2) that can incorporate a large upstream open reading frame that is substantially similar to a variety of other proteins such as bone morphogenic protein, several proteases, etc. Given that, in lower organisms, ORFs encoding receptor ligands can be linked to the ORF encoding the cognate receptor, it is possible that the presently described upstream ORF encodes a product (or cleavage product) that can serve as a receptor ligand in the body. Accordingly, an additional embodiment of the present invention includes soluble protein products or ligands that are encoded by at least a portion of the described novel polynucleotide sequences.

Finally, the NGPCR protein products (especially soluble derivatives such as peptides corresponding to a NGPCR ECD, the upstream ORF, or truncated polypeptides lacking one or more TM domains) and fusion protein products (especially NGPCR-Ig fusion proteins, i.e., fusions of a NGPCR, or a domain of a NGPCR, e.g., ECD, ΔTM to an IgFc), antibodies and anti-idiotypic antibodies (including Fab fragments), antagonists or agonists (including compounds that modulate signal transduction which may act on downstream targets in a NGPCR-mediated signal transduction pathway) can be used for therapy of such diseases. For example, the administration of an effective amount of soluble NGPCR ECD, ΔTM, or an ECD-IgFc fusion protein or an anti-idiotypic antibody (or its Fab) that mimics the NGPCR ECD would "mop up" or "neutralize" the endogenous NGPCR ligand, and prevent or reduce binding and receptor activation. Nucleotide constructs encoding such NGPCR products can be used to genetically engineer host cells to express such products *in vivo;* these genetically engineered cells function as "bioreactors" in the body delivering a continuous supply of a NGPCR, a NGPCR peptide, soluble ECD or ΔTM or a NGPCR fusion protein that will "mop up" or neutralize a NGPCR ligand. Nucleotide constructs encoding functional NGPCRs, mutant NGPCRs, as well as antisense and ribozyme molecules can be used in "gene therapy" approaches for the modulation of NGPCR expression. Thus, the invention also encompasses pharmaceutical formulations and methods for treating biological disorders.

Various aspects of the invention are described in greater detail in the subsections below.

### 5.1 NGPCR POLYNUCLEOTIDES

The cDNA sequences and deduced amino acid sequences of the described human proteins are presented in the Sequence Listing.
The NGPCR polynucleotides of the present invention include: (a) the human DNA sequences presented in the Sequence Listing and additionally contemplate any nucleotide sequence encoding a contiguous and functional NGPCR open reading frame (ORF) that hybridizes to a complement of the DNA sequences presented in the Sequence Listing under highly stringent conditions, e.g., hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. et *al*., eds., 1989, Current Protocols in Molecular Biology, Vol. I, Green Publishing Associates, Inc., and John Wiley & sons, Inc., New York, at p. 2.10.3) and encodes a functionally equivalent gene product. Additionally contemplated are any nucleotide sequences that hybridize to the complement of the DNA sequences that encode and express an amino acid sequence presented in the Sequence Listing under moderately stringent conditions, e.g., washing in 0.2xSSC/0.1% SDS at 42°C (Ausubel et al., 1989, supra), yet which still encode a functionally equivalent NGPCR gene product. Functional equivalents of NGPCR include naturally occurring NGPCRs present in other species, and mutant NGPCRs whether naturally occurring or engineered (by site directed mutagenesis, gene shuffling, directed evolution as described in, for example, U.S. Patent No. 5,837,458, herein incorporated by reference). The invention also includes degenerate variants of the disclosed sequences.

Additionally contemplated are polynucleotides encoding NGPCR ORFs, or their functional equivalents, encoded by polynucleotide sequences that are about 99, 95, 90, or about 85 percent similar or identical to corresponding regions of the nucleotide sequences of the Sequence Listing (as measured by BLAST sequence comparison analysis using, for example, the GCG sequence analysis package (Madison, Wisconsin) using standard default settings).

The invention also includes nucleic acid molecules, preferably DNA molecules, that hybridize to, and are therefore the complements of, the described NGPCR nucleotide sequences. Such hybridization conditions may be highly stringent or less highly stringent, as described above. In instances wherein the nucleic acid molecules are deoxyoligonucleotides ("DNA oligos"), such molecules (and particularly about 16 to about 100 base long, about 20 to about 80, or about 34 to about 45 base long, or any variation or combination of sizes represented therein incorporating a contiguous region of sequence first disclosed in the present Sequence Listing, can be used in conjunction with the polymerase chain reaction (PCR) to screen libraries, isolate clones, and prepare cloning and sequencing templates, etc. Alternatively, such NGPCR oligonucleotides can be used as hybridization probes for screening libraries, and assessing gene expression patterns (particularly using a micro array or high-throughput "chip" format). Additionally, a series of the described NGPCR oligonucleotide sequences, or the complements thereof, can be used to represent all or a portion of the described NGPCR encoding nucleotides. The oligonucleotides, typically between about 16 to about 40 (or any whole number within the stated range) nucleotides in length can partially overlap each other and/or a NGPCR sequence may be represented using oligonucleotides that do not overlap. Accordingly, the described NGPCR polynucleotide sequences shall typically comprise at least about two or three distinct oligonucleotide sequences of at least about 18, and preferably about 25, nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

For oligonucleotides probes, highly stringent conditions may refer, e.g., to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NGPCR antisense molecules, useful, for example, in NGPCR gene regulation (for and/or as antisense primers in amplification reactions of NGPCR gene nucleic acid sequences). With respect to NGPCR gene regulation, such techniques can be used to regulate biological functions. Further, such sequences may be used as part of ribozyme and/or triple helix sequences, also useful for NGPCR gene regulation.

Additionally, the antisense oligonucleotides may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641). The oligonucleotide is a 2'-0-methylribonucleotide (Inoue *et al.,* 1987, Nucl. Acids Res. 15:6131-6148), or a chimeric RNA-DNA analogue (Inoue et al., 1987, FEBS Lett. 215:327-330).

Oligonucleotides of the invention may be synthesized by standard methods known in the art, e.g. by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16:3209), methylphosphonate oligonucleotides can be prepared by use of controlled pore glass polymer supports (Sarin et *al.,* 1988, Proc. Natl. Acad. Sci. U.S.A. 85:7448-7451), etc.

Low stringency conditions are well known to those of skill in the art, and will vary predictably depending on the specific organisms from which the library and the labeled sequences are derived. For guidance regarding such conditions see, for example, Sambrook et al., 1989, Molecular Cloning, A Laboratory Manual (and periodic updates thereof), Cold Springs Harbor Press, N.Y.; and Ausubel et al., 1989, Current Protocols in Molecular Biology, Green Publishing Associates and Wiley Interscience, N.Y. Alternatively, suitably labeled NGPCR nucleotide probes can be used to screen a human genomic library using appropriately stringent conditions or by PCR. The identification and characterization of human genomic clones is helpful for identifying polymorphisms, determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (e.g., splice acceptor and/or donor sites), etc., that can be used in diagnostics and pharmacogenomics.

Further, a NGPCR gene homolog may be isolated from nucleic acid from the organism of interest by performing PCR using two degenerate oligonucleotide primer pools designed on the basis of amino acid sequences within the NGPCR gene product disclosed herein. The template for the reaction may be total RNA, mRNA, and/or cDNA obtained by reverse transcription of mRNA prepared from, for example, human or non-human cell lines or tissue, such as brain, known or suspected to express a NGPCR gene allele.

The PCR product can be subcloned and sequenced to ensure that the amplified sequences represent the sequence of the desired NGPCR gene. The PCR fragment can then be used to isolate a full length cDNA clone by a variety of methods. For example, the amplified fragment may be labeled and used to screen a cDNA library, such as a bacteriophage cDNA library. Alternatively, the labeled fragment may be used to isolate genomic clones via the screening of a genomic library.

PCR technology can also be utilized to isolate full length cDNA sequences. For example, RNA may be isolated, following standard procedures, from an appropriate cellular or tissue source (*i.e.*, one known, or suspected, to express a NGPCR gene, such as, for example, brain tissue). A reverse transcription (RT) reaction may be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment may easily be isolated. For a review of cloning strategies which may be used, see e.g., Sambrook et *al*., 1989, *supra.*

A cDNA of a mutant NGPCR gene can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NGPCR allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NGPCR allele to that of the normal NGPCR allele, the mutation(s) responsible for the loss or alteration of function of the mutant NGPCR gene product can be ascertained.

Alternatively, a genomic library can be constructed using DNA obtained from an individual suspected of or known to carry the mutant NGPCR allele, or a cDNA library can be constructed using RNA from a tissue known, or suspected, to express the mutant NGPCR allele. A normal NGPCR gene, or any suitable fragment thereof, can then be labeled and used as a probe to identify the corresponding mutant NGPCR allele in such libraries. Clones containing the mutant NGPCR gene sequences may then be purified and subjected to sequence analysis according to methods well known to those of skill in the art.

Additionally, an expression library can be constructed utilizing cDNA synthesized from, for example, RNA isolated from a tissue known, or suspected, to express a mutant NGPCR allele in an individual suspected of or known to carry such a mutant allele. In this manner, gene products made by the putatively mutant tissue may be expressed and screened using standard antibody screening techniques in conjunction with antibodies raised against the normal NGPCR gene product, as described, below, in Section 5.3. (For screening techniques, see, for example, Harlow, E. and Lane, eds., 1988, "Antibodies: A Laboratory Manual", Cold Spring Harbor Press, Cold Spring Harbor.)

Additionally, screening can be accomplished by screening with labeled NGPCR fusion proteins, such as, for example, alkaline phosphatase-NGPCR (AP-NGPCR) or NGPCR-AP fusion proteins. In cases where a NGPCR mutation results in an expressed gene product with altered function (e.g., as a result of a missense or a frameshift mutation), a polyclonal set of antibodies to NGPCR are likely to cross-react with the mutant NGPCR gene product. Library clones detected via their reaction with such labeled antibodies can be purified and subjected to sequence analysis according to methods well known to those of skill in the art.

The invention also encompasses nucleotide sequences that encode mutant NGPCRs, peptide fragments of the NGPCRs, truncated NGPCRs, and NGPCR fusion proteins. These include, but are not limited to nucleotide sequences encoding mutant NGPCRs described in section 5.2 infra; polypeptides or peptides corresponding to one or more ECD, TM and/or CD domains of the NGPCR or portions of these domains; truncated NGPCRs in which one or two of the domains is deleted, *e.g*., a soluble NGPCR lacking the TM or both the TM and CD regions, or a truncated, nonfunctional NGPCR lacking all or a portion of a, for example, CD region. Nucleotides encoding fusion proteins may include, but are not limited to, full length NGPCR sequences, truncated NGPCRs, or nucleotides encoding peptide fragments of NGPCR fused to an unrelated protein or peptide, such as for example, a transmembrane sequence, which anchors the NGPCR ECD to the cell membrane; an Ig Fc domain which increases the stability and half life of the resulting fusion protein (e.g., NGPCR-Ig) in the bloodstream; or an enzyme, fluorescent protein, luminescent protein which can be used as a marker.

The invention also encompasses (a) DNA vectors that contain any of the foregoing NGPCR coding sequences and/or their complements (*i.e*., antisense); (b) DNA expression vectors that contain any of the foregoing NGPCR coding sequences operatively associated with a regulatory element that directs the expression of the coding sequences; and (c) genetically engineered host cells that contain any of the foregoing NGPCR coding sequences operatively associated with a regulatory element that directs the expression of the coding sequences in the host cell. As used herein, regulatory elements include but are not limited to inducible and non-inducible promoters, enhancers, operators and other elements known to those skilled in the art that drive and regulate expression. Such regulatory elements include but are not limited to the human cytomegalovirus (hCMV) immediate early gene, regulatable, viral promoters (particularly retroviral LTR promoters), the early or late promoters of SV40 adenovirus, the lac system, the trp system, the TAC system, the TRC system, the major operator and promoter regions of phage A, the control regions of fd coat protein, the promoter for 3-phosphoglycerate kinase (PGK), the promoters of acid phosphatase, and the promoters of the yeast α-mating factors. Vectors incorporating foreign regulatory elements can also be used in conjunction with gene targeting technology to induce or activate the expression of endogenous NGPCRs by gene activation of the introduction of suitable transcription factors.

Additionally contemplated uses for the described sequences include the engineering of constitutively "on" variants for use in cell assays and genetically engineered animals using the methods and applications described in U.S. Patent Applications Ser Nos. 60/110,906, 60/106,300, 60/094,879, and 60/121,851 all of which are herein incorporated by reference in their entirety.

### 5.2 NGPCR PROTEINS AND POLYPEPTIDES

NGPCR proteins, polypeptides and peptide fragments, mutated, truncated or deleted forms of the NGPCR and/or NGPCR fusion proteins can be prepared for a variety of uses, including but not limited to use as agonist or antagonist, for the generation of antibodies, as reagents in diagnostic assays, the identification of other cellular gene products related to a NGPCR, as reagents in assays for screening for compounds that can be as pharmaceutical reagents useful in the therapeutic treatment of mental, biological, or medical disorders and disease.

The Sequence Listing discloses the amino acid sequences encoded by the described NGPCR genes. The NGPCRs have initiator methionines in DNA sequence contexts consistent with translation initiation sites, followed by hydrophobic signal sequences typical of membrane associated proteins. The sequence data presented herein indicate that alternatively spliced forms of the NGPCRs exist (which may or may not be tissue specific).

The NGPCR amino acid sequences of the invention include the nucleotide and amino acid sequences presented in the Sequence Listing as well as analogues and derivatives thereof. Further, corresponding NGPCR homologues from other species are encompassed by the invention. In fact, any NGPCR protein encoded by the NGPCR nucleotides described in Section 5.1, above, is within the scope of the invention, as are any novel polynucleotide sequences encoding all or any novel portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell et al. eds., Scientific American Books, New York, NY, herein incorporated by reference) are generically representative of all the various permutations and combinations of nucleic acid sequences that can encode such amino acid sequences.

The invention also encompasses proteins that are functionally equivalent to the NGPCR encoded by the nucleotide sequences described in Section 5.1, as judged by any of a number of criteria, including but not limited to the ability to bind a ligand for NGPCR, the ability to effect an identical or complementary signal transduction pathway, a change in cellular metabolism (*e.g*., ion flux, tyrosine phosphorylation, etc.) or change in phenotype when the NGPCR equivalent is present in an appropriate cell type (such as the amelioration, prevention or delay of a biochemical, biophysical, or overt phenotype. Such functionally equivalent NGPCR proteins include but are not limited to additions or substitutions of amino acid residues within the amino acid sequence encoded by the NGPCR nucleotide sequences described above, in Section 5.1, but which result in a silent change, thus producing a functionally equivalent gene product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

While random mutations can be made in NGPCR DNA (using random mutagenesis techniques well known to those skilled in the art) and the resulting mutant NGPCRs tested for activity, site-directed mutations of the NGPCR coding sequence can be engineered (using site-directed mutagenesis techniques well known to those skilled in the art) to generate mutant NGPCRs with increased function, *e.g*., higher binding affinity for the target ligand, and/or greater signaling capacity; or decreased function, and/or decreased signal transduction capacity. One starting point for such analysis is by aligning the disclosed human sequences with corresponding gene/protein sequences from, for example, other mammals in order to identify amino acid sequence motifs that are conserved between different species. Non-conservative changes can be engineered at variable positions to alter function, signal transduction capability, or both. Alternatively, where alteration of function is desired, deletion or non-conservative alterations of the conserved regions (*i.e*., identical amino acids) can be engineered. For example, deletion or non-conservative alterations (substitutions or insertions) of the various conserved transmembrane domains.

Other mutations to the NGPCR coding sequence can be made to generate NGPCRs that are better suited for expression, scale up, etc. in the host cells chosen. For example, cysteine residues can be deleted or substituted with another amino acid in order to eliminate disulfide bridges; N-linked glycosylation sites can be altered or eliminated to achieve, for example, expression of a homogeneous product that is more easily recovered and purified from yeast hosts which are known to hyperglycosylate N-linked sites. To this end, a variety of amino acid substitutions at one or both of the first or third amino acid positions of any one or more of the glycosylation recognition sequences which occur in the ECD (N-X-S or N-X-T), and/or an amino acid deletion at the second position of any one or more such recognition sequences in the ECD will prevent glycosylation of the NGPCR at the modified tripeptide sequence. (See, *e.g*., Miyajima et *al*., 1986, EMBO J. 5(6):1193-1197).

Peptides corresponding to one or more domains of the NGPCR (e.g., ECD, TM, CD, etc.), truncated or deleted NGPCRs (e.g., NGPCR in which a ECD, TM and/or CD is deleted) as well as fusion proteins in which a full length NGPCR, a NGPCR peptide, or truncated NGPCR is fused to an unrelated protein, are also within the scope of the invention and can be designed on the basis of the presently disclosed NGPCR nucleotide and NGPCR amino acid sequences. Such fusion proteins include but are not limited to IgFc fusions which stabilize the NGPCR protein or peptide and prolong half-life in *vivo;* or fusions to any amino acid sequence that allows the fusion protein to be anchored to the cell membrane, allowing an ECD to be exhibited on the cell surface; or fusions to an enzyme, fluorescent protein, or luminescent protein which provide a marker function.

While the NGPCR polypeptides and peptides can be chemically synthesized (*e.g*., see Creighton, 1983, Proteins: Structures and Molecular Principles, W.H. Freeman & Co., N.Y.), large polypeptides derived from a NGPCR and full length NGPCRs can be advantageously produced by recombinant DNA technology using techniques well known in the art for expressing nucleic acid containing NGPCR gene sequences and/or coding sequences. Such methods can be used to construct expression vectors containing a NGPCR nucleotide sequences described in Section 5.1 and appropriate transcriptional and translational control signals. These methods include, for example, *in vitro* recombinant DNA techniques, synthetic techniques, and *in vivo* genetic recombination. See, for example, the techniques described in Sambrook et al., 1989, supra, and Ausubel et *al.,* 1989, supra. Alternatively, RNA corresponding to all or a portion of a transcript encoded by a NGPCR nucleotide sequence may be chemically synthesized using, for example, synthesizers. See, for example, the techniques described in "Oligonucleotide Synthesis", 1984, Gait, M.J. ed., IRL Press, Oxford, which is incorporated by reference herein in its entirety.

A variety of host-expression vector systems may be utilized to express a NGPCR nucleotide sequences of the invention. Where the NGPCR peptide or polypeptide is a soluble derivative *(e.g.,* NGPCR peptides corresponding to an ECD; truncated or deleted NGPCR in which a TM and/or CD are deleted) the peptide or polypeptide can be recovered from the culture, i.e., from the host cell in cases where the NGPCR peptide or polypeptide is not secreted, and from the culture media in cases where the NGPCR peptide or polypeptide is secreted by the cells. However, such expression systems also encompass engineered host cells that express a NGPCR, or functional equivalent, *in situ, i.e*., anchored in the cell membrane. Purification or enrichment of NGPCR from such expression systems can be accomplished using appropriate detergents and lipid micelles and methods well known to those skilled in the art. However, such engineered host cells themselves may be used in situations where it is important not only to retain the structural and functional characteristics of the NGPCR, but to assess biological activity, *e.g*., in drug screening assays.

Expression systems that can be used for purposes of the invention include, but are not limited to, microorganisms such as bacteria *(e.g., E. coli, B. subtilis)* transformed with recombinant bacteriophage DNA, plasmid DNA or cosmid DNA expression vectors containing NGPCR nucleotide sequences; yeast *(e.g., Saccharomyces, Pichia)* transformed with recombinant yeast expression vectors containing NGPCR nucleotide sequences; insect cell systems infected with recombinant virus expression vectors (*e.g*., baculovirus) containing NGPCR sequences; plant cell systems infected with recombinant virus expression vectors *(e.g.,* cauliflower mosaic virus, CaMV; tobacco mosaic virus, TMV) or transformed with recombinant plasmid expression vectors *(e.g.,* Ti plasmid) containing NGPCR nucleotide sequences; or mammalian cell systems (*e.g*., COS, CHO, BHK, 293, 3T3) harboring recombinant expression constructs containing promoters derived from the genome of mammalian cells *(e.g.,* metallothionein promoter) or from mammalian viruses (*e.g*., the adenovirus late promoter; the vaccinia virus 7.5K promoter).

In bacterial systems, a number of expression vectors may be advantageously selected depending upon the use intended for the NGPCR gene product being expressed. For example, when a large quantity of such a protein is to be produced, for the generation of pharmaceutical compositions of NGPCR protein or for raising antibodies to a NGPCR protein, for example, vectors that direct the expression of high levels of fusion protein products that are readily purified may be desirable. Such vectors include, but are not limited, to the E. *coli* expression vector pUR278 (Ruther *et al.,* 1983, EMBO J. 2:1791), in which a NGPCR coding sequence may be ligated individually into the vector in frame with the *lacZ* coding region so that a fusion protein is produced; pIN vectors (Inouye & Inouye, 1985, Nucleic Acids Res. 13:3101-3109; Van Heeke & Schuster, 1989, J. Biol. Chem. 264:5503-5509); and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, *Autographa californica* nuclear polyhidrosis virus (AcNPV) is used as a vector to express foreign genes. The virus grows in *Spodoptera frugiperda* cells. A NGPCR gene coding sequence may be cloned individually into non-essential regions (for example the polyhedrin gene) of the virus and placed under control of an AcNPV promoter (for example the polyhedrin promoter). Successful insertion of NGPCR gene coding sequence will result in inactivation of the polyhedrin gene and production of non-occluded recombinant virus (*i.e*., virus lacking the proteinaceous coat coded for by the polyhedrin gene). These recombinant viruses are then used to infect Spodoptera frugiperda cells in which the inserted gene is expressed (*e.g*., see Smith et *al.,* 1983, J. Virol. 46: 584; Smith, U.S. Patent No. 4,215,051).

In mammalian host cells, a number of viral-based expression systems may be utilized. In cases where an adenovirus is used as an expression vector, the NGPCR nucleotide sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing a NGPCR gene product in infected hosts *(e.g.,* See Logan & Shenk, 1984, Proc. Natl. Acad. Sci. USA 81:3655-3659). Specific initiation signals may also be required for efficient translation of inserted NGPCR nucleotide sequences. These signals include the ATG initiation codon and adjacent sequences. In cases where an entire NGPCR gene or cDNA, including its own initiation codon and adjacent sequences, is inserted into the appropriate expression vector, no additional translational control signals may be needed. However, in cases where only a portion of a NGPCR coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bittner et *al*., 1987, Methods in Enzymol. 153:516-544).

In addition, a host cell strain may be chosen that modulates the expression of the inserted sequences, or modifies and processes the gene product in the specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, WI38, and in particular, choroid plexus cell lines.

For long-term, high-yield production of recombinant proteins, stable expression is preferred. For example, cell lines which stably express the NGPCR sequences described above may be engineered. Rather than using expression vectors which contain viral origins of replication, host cells can be transformed with DNA controlled by appropriate expression control elements (*e.g*., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the NGPCR gene product. Such engineered cell lines may be particularly useful in screening and evaluation of compounds that affect the endogenous activity of the NGPCR gene product.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et *al.,* 1980, Cell 22:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, et al., 1980, Natl. Acad. Sci. USA 77:3567; O'Hare, et *al.,* 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et *al.,* 1981, J. Mol. Biol. 150:1); and hygro, which confers resistance to hygromycin (Santerre, et *al*., 1984, Gene 30:147).

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht, et *al.,* 1991, Proc. Natl. Acad. Sci. USA 88: 8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺ nitriloacetic acid-agarose columns and histidine-tagged proteins are selectively eluted with imidazole-containing buffers. NGPCR gene products can also be expressed in transgenic animals. Animals of any species, including, but not limited to, worms, mice, rats, rabbits, guinea pigs, pigs, micro-pigs, birds, goats, and non-human primates, *e.g*., baboons, monkeys, and chimpanzees may be used to generate NGPCR transgenic animals.

Any technique known in the art may be used to introduce a NGPCR transgene into animals to produce the founder lines of transgenic animals. Such techniques include, but are not limited to pronuclear microinjection (Hoppe, P.C. and Wagner, T.E., 1989, U.S. Pat. No. 4,873,191); retrovirus mediated gene transfer into germ lines (Van der Putten et al., 1985, Proc. Natl. Acad. Sci., USA 82:6148-6152); gene targeting in embryonic stem cells (Thompson et *al.*, 1989, Cell 56:313-321); electroporation of embryos (Lo, 1983, Mol Cell. Biol. 3:1803-1814); and sperm-mediated gene transfer (Lavitrano et *al.,* 1989, Cell 57:717-723); etc. For a review of such techniques, see Gordon, 1989, Transgenic Animals, Intl. Rev. Cytol. 115:171-229, which is incorporated by reference herein in its entirety.

The present invention provides for transgenic animals that carry the NGPCR transgene in all their cells, as well as animals which carry the transgene in some, but not all their cells, i.e., mosaic animals or somatic cell transgenic animals. The transgene may be integrated as a single transgene or in concatamers, *e.g*., head-to-head tandems or head-to-tail tandems. The transgene may also be selectively introduced into and activated in a particular cell type by following, for example, the teaching of Lasko et al., 1992, Proc. Natl. Acad. Sci. USA 89:6232-6236. The regulatory sequences required for such a cell-type specific activation will depend upon the particular cell type of interest, and will be apparent to those of skill in the art.

When it is desired that the NGPCR gene transgene be integrated into the chromosomal site of the endogenous NGPCR gene, gene targeting is preferred. Briefly, when such a technique is to be utilized, vectors containing some nucleotide sequences homologous to the endogenous NGPCR gene are designed for the purpose of integrating, via homologous recombination with chromosomal sequences, into and disrupting the function of the nucleotide sequence of the endogenous NGPCR gene (i.e., "knockout" animals).

The transgene may also be selectively introduced into a particular cell type, thus inactivating the endogenous NGPCR gene in only that cell type, by following, for example, the teaching of Gu et *al*., 1994, Science, 265:103-106. The regulatory sequences required for such a cell-type specific inactivation will depend upon the particular cell type of interest, and will be apparent to those of skill in the art.

Once transgenic animals have been generated, the expression of the recombinant NGPCR gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques which include but are not limited to Northern blot analysis of tissue samples obtained from the animal, *in situ* hybridization analysis, and RT-PCR. Samples of NGPCR gene-expressing tissue, may also be evaluated immunocytochemically using antibodies specific for the NGPCR transgene product.

### 5.3 ANTIBODIES TO NGPCR PROTEINS

Antibodies that specifically recognize one or more epitopes of a NGPCR, or epitopes of conserved variants of a NGPCR, or peptide fragments of a NGPCR are also encompassed by the invention. Such antibodies include but are not limited to polyclonal antibodies, monoclonal antibodies (mAbs), humanized or chimeric antibodies, single chain antibodies, Fab fragments, F (ab') ₂ fragments, fragments produced by a Fab expression library, anti-idiotypic (anti-Id) antibodies, and epitope-binding fragments of any of the above.

The antibodies of the invention may be used, for example, in the detection of NGPCR in a biological sample and may, therefore, be utilized as part of a diagnostic or prognostic technique whereby patients may be tested for abnormal amounts of NGPCR. Such antibodies may also be utilized in conjunction with, for example, compound screening schemes, as described, below, in Section 5.5, for the evaluation of the effect of test compounds on expression and/or activity of a NGPCR gene product. Additionally, such antibodies can be used in conjunction gene therapy to, for example, evaluate the normal and/or engineered NGPCR-expressing cells prior to their introduction into the patient. Such antibodies may additionally be used as a method for the inhibition of abnormal NGPCR activity. Thus, such antibodies may, therefore, be utilized as part of weight disorder treatment methods.

For the production of antibodies, various host animals may be immunized by injection with the NGPCR, an NGPCR peptide (*e.g*., one corresponding the a functional domain of the receptor, such as an ECD, TM or CD), truncated NGPCR polypeptides (NGPCR in which one or more domains, *e.g*., a TM or CD, has been deleted), functional equivalents of the NGPCR or mutants of the NGPCR. Such host animals may include but are not limited to rabbits, mice, and rats, to name but a few. Various adjuvants may be used to increase the immunological response, depending on the host species, including but not limited to Freund's adjuvant (complete and incomplete), mineral salts such as aluminum hydroxide or aluminum phosphate, surface active substances such as lysolecithin, pluronic polyols, polyanions, peptides, oil emulsions, and potentially useful human adjuvants such as BCG (bacille Calmette-Guerin) and *Corynebacterium parvum.* Alternatively, the immune response could be enhanced by combination and or coupling with molecules such as keyhole limpet hemocyanin, tetanus toxoid, diptheria toxoid, ovalbumin, cholera toxoin or fragments thereof. Polyclonal antibodies are heterogeneous populations of antibody molecules derived from the sera of the immunized animals.

Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler and Milstein, (1975, Nature 256:495-497; and U.S. Patent No. 4,376,110), the human B-cell hybridoma technique (Kosbor et al., 1983, Immunology Today 4:72; Cole et al., 1983, Proc. Natl. Acad. Sci. USA 80:2026-2030), and the EBV-hybridoma technique (Cole et al., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or in vivo. Production of high titers of mAbs in vivo makes this the presently preferred method of production.

In addition, techniques developed for the production of "chimeric antibodies" (Morrison et al., 1984, Proc. Natl. Acad. Sci., 81:6851-6855; Neuberger et al., 1984, Nature, 312:604-608; Takeda et al., 1985, Nature, 314:452-454) by splicing the genes from a mouse antibody molecule of appropriate antigen specificity together with genes from a human antibody molecule of appropriate biological activity can be used (see U.S. Patents Nos. 6,075,181 and 5,877,397 which are herein incorporated by reference in their entirety). A chimeric antibody is a molecule in which different portions are derived from different animal species, such as those having a variable region derived from a murine mAb and a human immunoglobulin constant region.

Alternatively, techniques described for the production of single chain antibodies (U.S. Patent 4,946,778; Bird, 1988, Science 242:423-426; Huston et al., 1988, Proc. Natl. Acad. Sci. USA 85:5879-5883; and Ward et *al.,* 1989, Nature 334:544-546) can be adapted to produce single chain antibodies against NGPCR gene products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include but are not limited to: the F(ab*'*) ₂ fragments which can be produced by pepsin digestion of the antibody molecule and the Fab fragments which can be generated by reducing the disulfide bridges of the F (ab*'*) ₂ fragments. Alternatively, Fab expression libraries may be constructed (Huse *et al.,* 1989, Science, 246:1275-1281) to allow rapid and easy identification of monoclonal Fab fragments with the desired specificity.

Antibodies to a NGPCR can, in turn, be uti generate anti-idiotype antibodies that "mimic" a given NGPCR, using techniques well known to those skilled in the art. (See, *e.g*., Greenspan & Bona, 1993, FASEB J 7(5):437-444; and Nissinoff, 1991, J. Immunol. 147(8):2429-2438). For example antibodies which bind to a NGPCR ECD and competitively inhibit the binding of a ligand of NGPCR can be used to generate anti-idiotypes that "mimic" a NGPCR ECD and, therefore, bind and neutralize a ligand. Such neutralizing anti-idiotypes or Fab fragments of such anti-idiotypes can be used in therapeutic regimens involving the NGPCR signaling pathway.

### 5.4 DIAGNOSIS OF ABNORMALITIES RELATED TO A NGPCR

A variety of methods can be employed for the diagnostic and prognostic evaluation of disorders related to NGPCR function, and for the identification of subjects having a predisposition to such disorders.

Such methods may, for example, utilize reagents such as the NGPCR nucleotide sequences described in Section 5.1, and NGPCR antibodies, as described, in Section 5.3. Specifically, such reagents may be used, for example, for: (1) the detection of the presence of NGPCR gene mutations, or the detection of either over- or under-expression of NGPCR mRNA relative to a given phenotype; (2) the detection of either an over- or an under-abundance of NGPCR gene product relative to a given phenotype; and (3) the detection of perturbations or abnormalities in the signal transduction pathway mediated by NGPCR.

The methods described herein may be performed, for example, by utilizing pre-packaged diagnostic kits comprising at least one specific NGPCR nucleotide sequence or NGPCR antibody reagent described herein, which may be conveniently used, *e.g*., in clinical settings, to diagnose patients exhibiting body weight disorder abnormalities.

For the detection of NGPCR mutations, any nucleated cell can be used as a starting source for genomic nucleic acid. For the detection of NGPCR gene expression or NGPCR gene products, any cell type or tissue in which the NGPCR gene is expressed, such as, for example, brain or adipose cells, may be utilized.

Nucleic acid-based detection techniques are described, below, in Section 5.4.1. Peptide detection techniques are described, below, in Section 5.4.2.

### 5.4.1 DETECTION OF NGPCR NUCLEOTIDES AND TRANSCRIPTS

Mutations within a NGPCR gene can be detected by utilizing a number of techniques. Nucleic acid from any nucleated cell can be used as the starting point for such assay techniques, and may be isolated according to standard nucleic acid preparation procedures which are well known to those of skill in the art.

DNA may be used in hybridization or amplification assays of biological samples to detect abnormalities involving NGPCR gene structure, including point mutations, insertions, deletions and chromosomal rearrangements. Such assays may include, but are not limited to, Southern analyses, single stranded conformational polymorphism analyses (SSCP), and PCR analyses.

Such diagnostic methods for the detection of NGPCR genespecific mutations can involve for example, contacting and incubating nucleic acids including recombinant DNA molecules, cloned genes or degenerate variants thereof, obtained from a sample, *e.g*., derived from a patient sample or other appropriate cellular source, with one or more labeled nucleic acid reagents including recombinant DNA molecules, cloned genes or degenerate variants thereof, as described in Section 5.1, under conditions favorable for the specific annealing of these reagents to their complementary sequences within a given NGPCR gene. Preferably, the lengths of these nucleic acid reagents are at least 15 to 30 nucleotides. After incubation, all non-annealed nucleic acids are removed from the nucleic acid:NGPCR molecule hybrid. The presence of nucleic acids which have hybridized, if any such molecules exist, is then detected. Using such a detection scheme, the nucleic acid from the cell type or tissue of interest can be immobilized, for example, to a solid support such as a membrane, or a plastic surface such as that on a microtiter plate or polystyrene beads. In this case, after incubation, non-annealed, labeled nucleic acid reagents of the type described in Section 5.1 are easily removed. Detection of the remaining, annealed, labeled NGPCR nucleic acid reagents is accomplished using standard techniques well-known to those in the art. The NGPCR gene sequences to which the nucleic acid reagents have annealed can be compared to the annealing pattern expected from a normal NGPCR gene sequence in order to determine whether a NGPCR gene mutation is present.

Alternative diagnostic methods for the detection of NGPCR gene specific nucleic acid molecules, in patient samples or other appropriate cell sources, may involve their amplification, *e.g*., by PCR (the experimental embodiment set forth in Mullis, K.B., 1987, U.S. Patent No. 4,683,202), followed by the detection of the amplified molecules using techniques well known to those of skill in the art. The resulting amplified sequences can be compared to those which would be expected if the nucleic acid being amplified contained only normal copies of a NGPCR gene in order to determine whether a NGPCR gene mutation exists.

Additionally, well-known genotyping techniques can be performed to identify individuals carrying NGPCR gene mutations. Such techniques include, for example, the use of restriction fragment length polymorphisms (RFLPs), which involve sequence variations in one of the recognition sites for the specific restriction enzyme used.

Additionally, improved methods for analyzing DNA polymorphisms which can be utilized for the identification of NGPCR gene mutations have been described which capitalize on the presence of variable numbers of short, tandemly repeated DNA sequences between the restriction enzyme sites. For example, Weber (U.S. Pat. No. 5,075,217, which is incorporated herein by reference in its entirety) describes a DNA marker based on length polymorphisms in blocks of (dC-dA)n-(dG-dT)n short tandem repeats. The average separation of (dC-dA)n-(dG-dT)n blocks is estimated to be 30,000-60,000 bp. Markers which are so closely spaced exhibit a high frequency co-inheritance, and are extremely useful in the identification of genetic mutations, such as, for example, mutations within a given NGPCR gene, and the diagnosis of diseases and disorders related to NGPCR mutations.

Also, Caskey et *al.* (U.S. Pat. No. 5,364,759, which is incorporated herein by reference in its entirety) describe a DNA profiling assay for detecting short tri and tetra nucleotide repeat sequences. The process includes extracting the DNA of interest, such as the NGPCR gene, amplifying the extracted DNA, and labeling the repeat sequences to form a genotypic map of the individual's DNA.

The level of NGPCR gene expression can also be assayed by detecting and measuring NGPCR transcription. For example, RNA from a cell type or tissue known, or suspected to express the NGPCR gene, such as brain, may be isolated and tested utilizing hybridization or PCR techniques such as are described, above. The isolated cells can be derived from cell culture or from a patient. The analysis of cells taken from culture may be a necessary step in the assessment of cells to be used as part of a cell-based gene therapy technique or, alternatively, to test the effect of compounds on the expression of a NGPCR gene. Such analyses may reveal both quantitative and qualitative aspects of the expression pattern of the NGPCR gene, including activation or inactivation of NGPCR gene expression.

In one embodiment of such a detection scheme, cDNAs are synthesized from the RNAs of interest (*e.g*., by reverse transcription of the RNA molecule into cDNA). A sequence within the cDNA is then used as the template for a nucleic acid amplification reaction, such as a PCR amplification reaction, or the like. The nucleic acid reagents used as synthesis initiation reagents (e.g., primers) in the reverse transcription and nucleic acid amplification steps of this method are chosen from among the NGPCR nucleic acid reagents described in Section 5.1. The preferred lengths of such nucleic acid reagents are at least 9-30 nucleotides. For detection of the amplified product, the nucleic acid amplification may be performed using radioactively or non-radioactively labeled nucleotides. Alternatively, enough amplified product may be made such that the product may be visualized by standard ethidium bromide staining, by utilizing any other suitable nucleic acid staining method, or by sequencing.

Additionally, it is possible to perform such NGPCR crene expression assays *"in situ",* i.e., directly upon tissue sections (fixed and/or frozen) of patient tissue obtained from biopsies or resections, such that no nucleic acid purification is necessary. Nucleic acid reagents such as those described in Section 5.1 may be used as probes and/or primers for such in situ procedures (See, for example, Nuovo, G.J., 1992, "PCR In Situ Hybridization: Protocols And Applications", Raven Press, NY).

Alternatively,·if a sufficient quantity of the appropriate cells can be obtained, standard Northern analysis can be performed to determine the level of mRNA expression of the NGPCR gene.

### 5.4.2 DETECTION OF NGPCR GENE PRODUCTS

Antibodies directed against wild type or mutant NGPCR gene products or conserved variants or peptide fragments thereof, which are discussed, above, in Section 5.3, may also be used as diagnostics and prognostics, as described herein. Such diagnostic methods, may be used to detect abnormalities in the level of NGPCR gene expression, or abnormalities in the structure and/or temporal, tissue, cellular, or subcellular location of the NGPCR, and may be performed in vivo or in vitro, such as, for example, on biopsy tissue.

For example, antibodies directed to epitopes of the NGPCR ECD can be used *in vivo* to detect the pattern and level of expression of the NGPCR in the body. Such antibodies can be labeled, *e.g*., with a radio-opaque or other appropriate compound and injected into a subject in order to visualize binding to the NGPCR expressed in the body using methods such as X-rays, CAT-scans, or MRI. Labeled antibody fragments, e.g., the Fab or single chain antibody comprising the smallest portion of the antigen binding region, are preferred for this purpose to promote crossing the blood-brain barrier and permit labeling NGPCRs expressed in the brain.

Additionally, any NGPCR fusion protein or NGPCR conjugated protein whose presence can be detected, can be administered. For example, NGPCR fusion or conjugated proteins labeled with a radio-opaque or other appropriate compound can be administered and visualized in vivo, as discussed, above for labeled antibodies. Further such NGPCR fusion proteins as alkaline phosphatase-NGPCR (AP-NGPCR) or NGPCR-AP fusion proteins can be utilized for in *vitro* diagnostic procedures.

Alternatively, immunoassays or fusion protein detection assays, as described above, can be utilized on biopsy and autopsy samples *in vitro* to permit assessment of the expression pattern of the NGPCR. Such assays are not confined to the use of antibodies that define a NGPCR ECD, but can include the use of antibodies directed to epitopes of any of the domains of a NGPCR, e.g., the ECD, the TM and/or CD. The use of each or all of these labeled antibodies will yield useful information regarding translation and intracellular transport of the NGPCR to the cell surface, and can identify defects in processing.

The tissue or cell type to be analyzed will generally include those which are known, or suspected, to express a NGPCR gene, such as, for example, brain cells. The protein isolation methods employed herein may, for example, be such as those described in Harlow and Lane (Harlow, E. and Lane, D., 1988, "Antibodies: A Laboratory Manual", Cold Spring Harbor Laboratory Press, Cold Spring Harbor, New York), which is incorporated herein by reference in its entirety. The isolated cells can be derived from cell culture or from a patient. The analysis of cells taken from culture may be a necessary step in the assessment of cells that could be used as part of a cell-based gene therapy technique or, alternatively, to test the effect of compounds on the expression of a NGPCR gene.

For example, antibodies, or fragments of antibodies, such as those described, above, in Section 5.3, useful in the present invention may be used to quantitatively or qualitatively detect the presence of NGPCR gene products or conserved variants or peptide fragments thereof. This can be accomplished, for example, by immunofluorescence techniques employing a fluorescently labeled antibody (see below, this Section) coupled with light microscopic, flow cytometric, or fluorimetric detection. Such techniques are especially preferred if such NGPCR gene products are expressed on the cell surface.

The antibodies (or fragments thereof) or NGPCR fusion or conjugated proteins useful in the present invention may, additionally, be employed histologically, as in immunofluorescence, immunoelectron microscopy or non-immuno assays, for in situ detection of NGPCR gene products or conserved variants or peptide fragments thereof, or for NGPCR binding (in the case of labeled NGPCR ligand fusion protein).

In situ detection may be accomplished by removing a histological specimen from a patient, and applying thereto a labeled antibody or fusion protein of the present invention. The antibody (or fragment) or fusion protein is preferably applied by overlaying the labeled antibody (or fragment) onto a biological sample. Through the use of such a procedure, it is possible to determine not only the presence of the NGPCR gene product, or conserved variants or peptide fragments, or NGPCR binding, but also its distribution in the examined tissue. Using the present invention, those of ordinary skill will readily perceive that any of a wide variety of histological methods (such as staining procedures) can be modified in order to achieve such in situ detection.

Immunoassays and non-immunoassays for NGPCR gene products or conserved variants or peptide fragments thereof will typically comprise incubating a sample, such as a biological fluid, a tissue extract, freshly harvested cells, or lysates of cells which have been incubated in cell culture, in the presence of a detectably labeled antibody capable of identifying NGPCR gene products or conserved variants or peptide fragments thereof, and detecting the bound antibody by any of a number of techniques well-known in the art.

The biological sample may be brought in contact with and immobilized onto a solid phase support or carrier such as nitrocellulose, or other solid support which is capable of immobilizing cells, cell particles or soluble proteins. The support may then be washed with suitable buffers followed by treatment with the detectably labeled NGPCR antibody or NGPCR ligand fusion protein. The solid phase support may then be washed with the buffer a second time to remove unbound antibody or fusion protein. The amount of bound label on solid support may then be detected by conventional means.

By "solid phase support or carrier" is intended any support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

The binding activity of a given lot of NGPCR antibody or NGPCR ligand fusion protein may be determined according to well known methods. Those skilled in the art will be able to determine operative and optimal assay conditions for each determination by employing routine experimentation.

With respect to antibodies, one of the ways in which the NGPCR antibody can be detectably labeled is by linking the same to an enzyme and use in an enzyme immunoassay (EIA) (Voller, A., "The Enzyme Linked Immunosorbent Assay (ELISA)", 1978, Diagnostic Horizons 2:1-7, Microbiological Associates Quarterly Publication, Walkersville, MD); Voller, A. et *al.,* 1978, J. Clin. Pathol. *31*:507-520; Butler, J.E., 1981, Meth. Enzymol. 73:482-523; Maggio, E. (ed.), 1980, Enzyme Immunoassay, CRC Press, Boca Raton, FL,; Ishikawa, E. et *al.,* (eds.), 1981, Enzyme Immunoassay, Kgaku Shoin, Tokyo). The enzyme that is bound to the antibody will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. The detection can be accomplished by colorimetric methods which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible to detect NGPCR through the use of a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in, which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

### 5.5 SCREENING ASSAYS FOR COMPOUNDS THAT MODULATE NGPCR EXPRESSION OR ACTIVITY

The following assays are designed to identify compounds that interact with *(e.g*., bind to) NGPCRs (including, but not limited to an ECD or CD of a NGPCR), compounds that interact with (*e.g*., bind to) intracellular proteins that interact with NGPCR (including but not limited to the TM and CD of NGPCR), compounds that interfere with the interaction of NGPCR with transmembrane or intracellular proteins involved in NGPCR-mediated signal transduction, and to compounds which modulate the activity of NGPCR gene (*i.e*., modulate the level of NGPCR gene expression) or modulate the level of NGPCR. Assays may additionally be utilized which identify compounds which bind to NGPCR gene regulatory sequences (*e.g*., promoter sequences) and which may modulate NGPCR gene expression. See e.g., Platt, K.A., 1994, J. Biol. Chem. 269:28558-28562, which is incorporated herein by reference in its entirety.

The compounds which may be screened in accordance with the invention include but are not limited to peptides, antibodies and fragments thereof, and other organic compounds (*e.g.*, peptidomimetics) that bind to an ECD of a NGPCR and either mimic the activity triggered by the natural ligand (*i.e*., agonists) or inhibit the activity triggered by the natural ligand (*i.e*., antagonists); as well as peptides, antibodies or fragments thereof, and other organic compounds that mimic the ECD of the NGPCR (or a portion thereof) and bind to and "neutralize" natural ligand.

Such compounds can include, but are not limited to, peptides such as, for example, soluble peptides, including but not limited to members of random peptide libraries; (see, e.g., Lam, K.S. et *al.,* 1991, Nature 354:82-84; Houghten, R. *et al.,* 1991, Nature 354:84-86), and combinatorial chemistry-derived molecular library made of D- and/or L- configuration amino acids, phosphopeptides (including, but not limited to members of random or partially degenerate, directed phosphopeptide libraries; see, e.g., Songyang, Z. et *al.,* 1993, Cell 72:767-778), antibodies (including, but not limited to, polyclonal, monoclonal, humanized, anti-idiotypic, chimeric or single chain antibodies, and FAb, F(ab*'*)₂ and FAb expression library fragments, and epitope-binding fragments thereof), and small organic or inorganic molecules.

Other compounds that can be screened in accordance with the invention include but are not limited to small organic molecules that are able to cross the blood-brain barrier, gain entry into an appropriate cell (*e.g*., in the choroid plexus, the hypothalamus, etc.) and affect the expression of a NGPCR gene or some other gene involved in the NGPCR signal transduction pathway (*e.g*., by interacting with the regulatory region or transcription factors involved in gene expression); or such compounds that affect the activity of the NGPCR (*e.g*., by inhibiting or enhancing the enzymatic activity of a CD) or the activity of some other intracellular factor involved in the NGPCR signal transduction pathway.

Computer modeling and searching technologies permit identification of compounds, or the improvement of already identified compounds, that can modulate NGPCR expression or activity. Having identified such a compound or composition, the active sites or regions are identified. Such active sites might typically be ligand binding sites. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

Next, the three dimensional geometric structure of the active site is determined. This can be done by known methods, including X-ray crystallography, which can determine a complete molecular structure. On the other hand, solid or liquid phase NMR can be used to determine certain intramolecular distances. Any other experimental method of structure determination can be used to obtain partial or complete geometric structures. The geometric structures may be measured with a complexed ligand, natural or artificial, which may increase the accuracy of the active site structure determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the active site, either experimentally, by modeling, or by a combination, candidate modulating compounds can be identified by searching databases containing compounds along with information on their molecular structure. Such a search seeks compounds having structures that match the determined active site structure and that interact with the groups defining the active site. Such a search can be manual, but is preferably computer assisted. These compounds found from this search are potential NGPCR modulating compounds.

Alternatively, these methods can be used to identify improved modulating compounds from an already known modulating compound or ligand. The composition of the known compound can be modified and the structural effects of modification can be determined using the experimental and computer modeling methods described above applied to the new composition. The altered structure is then compared to the active site structure of the compound to determine if an improved fit or interaction results. In this manner systematic variations in composition, such as by varying side groups, can be quickly evaluated to obtain modified modulating compounds or ligands of improved specificity or activity.

Further experimental and computer modeling methods useful to identify modulating compounds based upon identification of the active sites of a NGPCR, and related transduction and transcription factors will be apparent to those of skill in the art.

Examples of molecular modeling systems are the CHARMm and QUANTA programs (Polygen Corporation, Waltham, MA). CHARMm performs the energy minimization and molecular dynamics functions. QUANTA performs the construction, graphic modeling and analysis of molecular structure. QUANTA allows interactive construction, modification, visualization, and analysis of the behavior of molecules with each other.

A number of articles review computer modeling of drugs interactive with specific proteins, such as Rotivinen, et *al*., 1988, Acta Pharmaceutical Fennica 97:159-166; Ripka, New Scientist 54-57 (June 16, 1988); McKinaly and Rossmann, 1989, Annu. Rev. Pharmacol. Toxiciol. 29:111-122; Perry and Davies, OSAR: Quantitative Structure-Activity Relationships in Drug Design pp. 189-193 (Alan R. Liss, Inc. 1989); Lewis and Dean, 1989 Proc. R. Soc. Lond. 236:125-140 and 141-162; and, with respect to a model receptor for nucleic acid components, Askew, et al., 1989, J. Am. Chem. Soc. *111*:1082-1090. Other computer programs that screen and graphically depict chemicals are available from companies such as BioDesign, Inc. (Pasadena, CA.), Allelix, Inc. (Mississauga, Ontario, Canada), and Hypercube, Inc. (Cambridge, Ontario). Although these are primarily designed for application to drugs specific to particular proteins, they can be adapted to design of drugs specific to regions of DNA or RNA, once that region is identified.

Although described above with reference to design and generation of compounds which could alter binding, one could also screen libraries of known compounds, including natural products or synthetic chemicals, and biologically active materials, including proteins, for compounds which are inhibitors or activators.

Cell-based systems can also be used to identify compounds that bind NGPCRs as well as assess the altered activity associated with such binding in living cells. One tool of particular interest for such assays is green fluorescent protein which is described, *inter alia,* in U.S. Patent No. 5,625,048, herein incorporated by reference. Cells that may be used in such cellular assays include, but are not limited to, leukocytes, or cell lines derived from leukocytes, lymphocytes, stem cells, including embryonic stem cells, and the like. In addition, expression host cells *(e.g.,* B95 cells, COS cells, CHO cells, OMK cells, fibroblasts, Sf9 cells) genetically engineered to express a functional NGPCR of interest and to respond to activation by the test, or natural, ligand, as measured by a chemical or phenotypic change, or induction of another host cell gene, can be used as an end point in the assay.

Compounds identified via assays such as those described herein may be useful, for example, in elaborating the biological function of a NGPCR gene product. Such compounds can be administered to a patient at therapeutically effective doses to treat any of a variety of physiological or mental disorders. A therapeutically effective dose refers to that amount of the compound sufficient to result in any amelioration, impediment, prevention, or alteration of any biological or overt symptom.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

Pharmaceutical compositions for use in accordance with the present invention may be formulated in conventional manner using one or more physiologically acceptable carriers or excipients. Thus, the compounds and their physiologically acceptable salts and solvates may be formulated for administration by inhalation or insufflation (either through the mouth or the nose) or oral, buccal, parenteral, intracranial, intrathecal, or rectal administration.

For oral administration, the pharmaceutical compositions may take the form of, for example, tablets or capsules prepared by conventional means with pharmaceutically acceptable excipients such as binding agents (*e.g*., pregelatinised maize starch, polyvinylpyrrolidone or hydroxypropyl methylcellulose); fillers (*e.g*., lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents *(e.g.,* sorbitol syrup, cellulose derivatives or hydrogenated edible fats); emulsifying agents (*e.g*., lecithin or acacia); non-aqueous vehicles (*e.g*., almond oil, oily esters, ethyl alcohol or fractionated vegetable oils); and preservatives (*e.g*., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g*. gelatin for use in an inhaler or insufflator may be formulated containing a powder mix of the compound and a suitable powder base such as lactose or starch.

The compounds may be formulated for parenteral administration by injection, *e.g*., by bolus injection or continuous infusion. Formulations for injection may be presented in unit dosage form, *e.g*., in ampoules or in multidose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g*., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### 5.5.1 IN VITRO SCREENING ASSAYS FOR COMPOUNDS THAT BIND TO NGPCRs

*In vitro* systems may be designed to identify compounds capable of interacting with (*e.g*., binding to) NGPCR (including, but not limited to, a ECD or CD of NGPCR). Compounds identified may be useful, for example, in modulating the activity of wild type and/or mutant NGPCR gene products; may be useful in elaborating the biological function of the NGPCR; may be utilized in screens for identifying compounds that disrupt normal NGPCR interactions; or may in themselves disrupt such interactions.

The principle of the assays used to identify compounds that bind to the NGPCR involves preparing a reaction mixture of the NGPCR and the test compound under conditions and for a time sufficient to allow the two components to interact and bind, thus forming a complex which can be removed and/or detected in the reaction mixture. The NGPCR species used can vary depending upon the goal of the screening assay. For example, where agonists of the natural ligand are sought, the full length NGPCR, or a soluble truncated NGPCR, *e.g.,* in which the TM and/or CD is deleted from the molecule, a peptide corresponding to a ECD or a fusion protein containing one or more NGPCR ECD fused to a protein or polypeptide that affords advantages in the assay system (*e.g*., labeling, isolation of the resulting complex, etc.) can be utilized. Where compounds that interact with the cytoplasmic domain are sought to be identified, peptides corresponding to the NGPCR CD and fusion proteins containing the NGPCR CD can be used.

The screening assays can be conducted in a variety of ways. For example, one method to conduct such an assay would involve anchoring the NGPCR protein, polypeptide, peptide or fusion protein or the test substance onto a solid phase and detecting NGPCR/test compound complexes anchored on the solid phase at the end of the reaction. In one embodiment of such a method, the NGPCR reactant may be anchored onto a solid surface, and the test compound, which is not anchored, may be labeled, either directly or indirectly.

In practice, microtiter plates may conveniently be utilized as the solid phase. The anchored component may be immobilized by non-covalent or covalent attachments. Non-covalent attachment may be accomplished by simply coating the solid surface with a solution of the protein and drying. Alternatively, an immobilized antibody, preferably a monoclonal antibody, specific for the protein to be immobilized may be used to anchor the protein to the solid surface. The surfaces may be prepared in advance and stored.

In order to conduct the assay, the nonimmobilized component is added to the coated surface containing the anchored component. After the reaction is complete, unreacted components are removed (*e.g*., by washing) under conditions such that any complexes formed will remain immobilized on the solid surface. The detection of complexes anchored on the solid surface can be accomplished in a number of ways. Where the previously nonimmobilized component is pre-labeled, the detection of label immobilized on the surface indicates that complexes were formed. Where the previously nonimmobilized component is not pre-labeled, an indirect label can be used to detect complexes anchored on the surface; *e.g*., using a labeled antibody specific for the previously nonimmobilized component (the antibody, in turn, may be directly labeled or indirectly labeled with a labeled anti-Ig antibody).

Alternatively, a reaction can be conducted in a liquid phase, the reaction products separated from unreacted components, and complexes detected; *e.g*., using an immobilized antibody specific for a NGPCR protein, polypeptide, peptide or fusion protein or the test compound to anchor any complexes formed in solution, and a labeled antibody specific for the other component of the possible complex to detect anchored complexes.

Alternatively, cell-based assays can be used to identify compounds that interact with NGPCR. To this end, cell lines that express a NGPCR, or cell lines (*e.g*., COS cells, CHO cells, fibroblasts, etc.) that have been genetically engineered to express a NGPCR (e.g., by transfection or transduction of NGPCR DNA) can be used. Interaction of the test compound with, for example, a ECD of a NGPCR expressed by the host cell can be determined by comparison or competition with native ligand.

### 5.5.2 ASSAYS FOR INTRACELLULAR PROTEINS THAT INTERACT WITH NGPCRs

Any method suitable for detecting protein-protein interactions can be employed for identifying transmembrane proteins or intracellular proteins that interact with a NGPCR. Among the traditional methods which may be employed are co-immunoprecipitation, crosslinking and co-purification through gradients or chromatographic columns of cell lysates or proteins obtained from cell lysates and a NGPCR to identify proteins in the lysate that interact with the NGPCR. For these assays, the NGPCR component used can be a full length NGPCR, a soluble derivative lacking the membrane-anchoring region (*e.g*., a truncated NGPCR in which a TM is deleted resulting in a truncated molecule containing a ECD fused to a CD), a peptide corresponding to a CD or a fusion protein containing a CD of a NGPCR. Once isolated, such an intracellular protein can be identified and can, in turn, be used, in conjunction with standard techniques, to identify proteins with which it interacts. For example, at least a portion of the amino acid sequence of an intracellular protein which interacts with a NGPCR can be ascertained using techniques well known to those of skill in the art, such as via the Edman degradation technique. (See, *e.g*., Creighton, 1983, "Proteins: Structures and Molecular Principles", W.H. Freeman & Co., N.Y., pp.34-49). The amino acid sequence obtained may be used as a guide for the generation of oligonucleotide mixtures that can be used to screen for gene sequences encoding such intracellular proteins. Screening may be accomplished, for example, by standard hybridization or PCR techniques. Techniques for the generation of oligonucleotide mixtures and the screening are well-known. (See, *e.g.,* Ausubel, supra, and PCR Protocols: A Guide to Methods and Applications, 1990, Innis, M. et *al.,* eds. Academic Press, Inc., New York).

Additionally, methods may be employed which result in the simultaneous identification of genes which encode the transmembrane or intracellular proteins interacting with NGPCR. These methods include, for example, probing expression, libraries, in a manner similar to the well known technique of antibody probing of λgt11 libraries, using labeled NGPCR protein, or an NGPCR polypeptide, peptide or fusion protein, *e.g*., an NGPCR polypeptide or NGPCR domain fused to a marker (*e.g*., an enzyme, fluor, luminescent protein, or dye), or an Ig-Fc domain.

One method which detects protein interactions *in vivo,* the two-hybrid system, is described in detail for illustration only and not by way of limitation. One version of this system has been described (Chien et *al.,* 1991, Proc. Natl. Acad. Sci. USA, 88:9578-9582) and is commercially available from Clontech (Palo Alto, CA).

Briefly, utilizing such a system, plasmids are constructed that encode two hybrid proteins: one plasmid consists of nucleotides encoding the DNA-binding domain of a transcription activator protein fused to a NGPCR nucleotide sequence encoding NGPCR, an NGPCR polypeptide, peptide or fusion protein, and the other plasmid consists of nucleotides encoding the transcription activator protein's activation domain fused to a cDNA encoding an unknown protein which has been recombined into this plasmid as part of a cDNA library. The DNA-binding domain fusion plasmid and the cDNA library are transformed into a strain of the yeast *Saccharomyces cerevisiae* that contains a reporter gene *(e.g.,* HBS or *lacZ)* whose regulatory region contains the transcription activator's binding site. Either hybrid protein alone cannot activate transcription of the reporter gene: the DNA-binding domain hybrid cannot because it does not provide activation function and the activation domain hybrid cannot because it cannot localize to the activator's binding sites. Interaction of the two hybrid proteins reconstitutes the functional activator protein and results in expression of the reporter gene, which is detected by an assay for the reporter gene product.

The two-hybrid system or related methodology may be used to screen activation domain libraries for proteins that interact with the "bait" gene product. By way of example, and not by way of limitation, a NGPCR may be used as the bait gene product. Total genomic or cDNA sequences are fused to the DNA encoding an activation domain. This library and a plasmid encoding a hybrid of a bait NGPCR gene product fused to'the DNA-binding domain are cotransformed into a yeast reporter strain, and the resulting transformants are screened for those that express the reporter gene. For example, and not by way of limitation, a bait NGPCR gene sequence, such as the open reading frame of a NGPCR (or a domain of a NGPCR) can be cloned into a vector such that it is translationally fused to the DNA encoding the DNA-binding domain of the GAL4 protein. These colonies are purified and the library plasmids responsible for reporter gene expression are isolated. DNA sequencing is then used to identify the proteins encoded by the library plasmids.

A cDNA library of the cell line from which proteins that interact with bait NGPCR gene product are to be detected can be made using methods routinely practiced in the art. According to the particular system described herein, for example, the cDNA fragments can be inserted into a vector such that they are translationally fused to the transcriptional activation domain of GAL4. This library can be co-transformed along with the bait NGPCR gene-GAL4 fusion plasmid into a yeast strain which contains a lacZ gene driven by a promoter which contains GAL4 activation sequence. A cDNA encoded protein, fused to GAL4 transcriptional activation domain, that interacts with bait NGPCR gene product will reconstitute an active GAL4 protein and thereby drive expression of the HIS3 gene. Colonies which express HIS3 can be detected by their growth on petri dishes containing semi-solid agar based media lacking histidine. The cDNA can then be purified from these strains, and used to produce and isolate the bait NGPCR gene-interacting protein using techniques routinely practiced in the art.

### 5.5.3 ASSAYS FOR COMPOUNDS THAT INTERFERE WITH NGPCR/INTRACELLULAR OR NGPCR/TRANSMEMBRANE MACROMOLECULE INTERACTION

The macromolecules that interact with the NGPCR are referred to, for purposes of this discussion, as "binding partners". These binding partners are likely to be involved in the NGPCR signal transduction pathway. Therefore, it is desirable to identify compounds that interfere with or disrupt the interaction of such binding partners which may be useful in regulating the activity of a NGPCR and controlling disorders associated with NGPCR activity.

The basic principle of the assay systems used to identify compounds that interfere with the interaction between a NGPCR and its binding partner or partners involves preparing a reaction mixture containing NGPCR protein, polypeptide, peptide or fusion protein as described in Sections 5.5.1 and 5.5.2 above, and the binding partner under conditions and for a time sufficient to allow the two to interact and bind, thus forming a complex. In order to test a compound for inhibitory activity, the reaction mixture is prepared in the presence and absence of the test compound. The test compound may be initially included in the reaction mixture, or may be added at a time subsequent to the addition of the NGPCR moiety and its binding partner. Control reaction mixtures are incubated without the test compound or with a placebo. The formation of any complexes between the NGPCR moiety and the binding partner is then detected. The formation of a complex in the control reaction, but not in the reaction mixture containing the test compound, indicates that the compound interferes with the interaction of the NGPCR and the interactive binding partner. Additionally, complex formation within reaction mixtures containing the test compound and normal NGPCR protein may also be compared to complex formation within reaction mixtures containing the test compound and a mutant NGPCR. This comparison may be important in those cases wherein it is desirable to identify compounds that specifically disrupt interactions of mutant, or mutated, NGPCRs but not normal NGPCRs.

The assay for compounds that interfere with the interaction of the NGPCR and binding partners can be conducted in a heterogeneous or homogeneous format. Heterogeneous assays involve anchoring either the NGPCR moiety product or the binding partner onto a solid phase and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction by competition can be identified by conducting the reaction in the presence of the test substance; *i.e*., by adding the test substance to the reaction mixture prior to, or simultaneously with, a NGPCR moiety and interactive binding partner. Alternatively, test compounds that disrupt preformed complexes, *e.g*. compounds with higher binding constants that displace one of the components from the complex, can be tested by adding the test compound to the reaction mixture after complexes have been formed. The various formats are described briefly below.

In a heterogeneous assay system, either a NGPCR moiety or an interactive binding partner, is anchored onto a solid surface, while the non-anchored species is labeled, either directly or indirectly. In practice, microtiter plates are conveniently utilized. The anchored species may be immobilized by non-covalent or covalent attachments. Non-covalent attachment may be accomplished simply by coating the solid surface with a solution of the NGPCR gene product or binding partner and drying. Alternatively, an immobilized antibody specific for the species to be anchored may be used to anchor the species to the solid surface. The surfaces may be prepared in advance and stored.

In order to conduct the assay, the partner of the immobilized species is exposed to the coated surface with or without the test compound. After the reaction is complete, unreacted components are removed (*e.g*., by washing) and any complexes formed will remain immobilized on the solid surface. The detection of complexes anchored on the solid surface can be accomplished in a number of ways. Where the non-immobilized species is pre-labeled, the detection of label immobilized on the surface indicates that complexes were formed. Where the non-immobilized species is not pre-labeled, an indirect label can be used to detect complexes anchored on the surface; *e.g.,* using a labeled antibody specific for the initially non-immobilized species (the antibody, in turn, may be directly labeled or indirectly labeled with a labeled anti-Ig antibody). Depending upon the order of addition of reaction components, test compounds which inhibit complex formation or which disrupt preformed complexes can be detected.

Alternatively, the reaction can be conducted in a liquid phase in the presence or absence of the test compound, the reaction products separated from unreacted components, and complexes detected; *e.g*., using an immobilized antibody specific for one of the binding components to anchor any complexes formed in solution, and a labeled antibody specific for the other partner to detect anchored complexes. Again, depending upon the order of addition of reactants to the liquid phase, test compounds which inhibit complex or which disrupt preformed complexes can be identified.

In an alternate embodiment of the invention, a homogeneous assay can be used. In this approach, a preformed complex of a MGPCR moiety and an interactive binding partner is prepared in which either the NGPCR or its binding partners is labeled, but the signal generated by the label is quenched due to formation of the complex (see, e.g., U.S. Patent No. 4,109,496 by Rubenstein which utilizes this approach for immunoassays). The addition of a test substance that competes with and displaces one of the species from the preformed complex will result in the generation of a signal above background. In this way, test substances which disrupt NGPCR/intracellular binding partner interaction can be identified.

In a particular embodiment, a NGPCR fusion can be prepared for immobilization. For example, a NGPCR or a peptide fragment, *e.g*., corresponding to a CD, can be fused to a glutathione-S-transferase (GST) gene using a fusion vector, such as pGEX-5X-1, in such a manner that its binding activity is maintained in the resulting fusion protein. The interactive binding partner can be purified and used to raise a monoclonal antibody, using methods routinely practiced in the art and described above, in Section 5.3. This antibody can be labeled with the radioactive isotope ¹²⁵I, for example, by methods routinely practiced in the art. In a heterogeneous assay, *e.g*., the GST-NGPCR fusion protein can be anchored to glutathione-agarose beads. The interactive binding partner can then be added in the presence or absence of the test compound in a manner that allows interaction and binding to occur. At the end of the reaction period, unbound material can be washed away, and the labeled monoclonal antibody can be added to the system and allowed to bind to the complexed components. The interaction between a NGPCR gene product and the interactive binding partner can be detected by measuring the amount of radioactivity that remains associated with the glutathione-agarose beads. A successful inhibition of the interaction by the test compound will result in a decrease in measured radioactivity.

Alternatively, the GST-NGPCR fusion protein and the interactive binding partner can be mixed together in liquid in the absence of the solid glutathione-agarose beads. The test compound can be added either during or after the species are allowed to interact. This mixture can then be added to the glutathione-agarose beads and unbound material is washed away. Again the extent of inhibition of the NGPCR/binding partner interaction can be detected by adding the labeled antibody and measuring the radioactivity associated with the beads.

In another embodiment of the invention, these same techniques can be employed using peptide fragments that correspond to the binding domains of a NGPCR and/or the interactive or binding partner (in cases where the binding partner is a protein), in place of one or both of the full length proteins. Any number of methods routinely practiced in the art can be used to identify and isolate the binding sites. These methods include, but are not limited to, mutagenesis of the gene encoding one of the proteins and screening for disruption of binding in a co-immunoprecipitation assay. Compensatory mutations in the gene encoding the second species in the complex can then be selected. Sequence analysis of the genes encoding the respective proteins will reveal the mutations that correspond to the region of the protein involved in interactive binding. Alternatively, one protein can be anchored to a solid surface using methods described above, and allowed to interact with and bind to its labeled binding partner, which has been treated with a proteolytic enzyme, such as trypsin. After washing, a relatively short, labeled peptide comprising the binding domain may remain associated with the solid material, which can be isolated and identified by amino acid sequencing. Also, once the gene coding for the intracellular binding partner is obtained, short gene segments can be engineered to express peptide fragments of the protein, which can then be tested for binding activity and purified or synthesized.

For example, and not by way of limitation, a NGPCR gene product can be anchored to a solid material as described, above, by making a GST-NGPCR fusion protein and allowing it to bind to glutathione agarose beads. The interactive binding partner can be labeled with a radioactive isotope, such as ³⁵S, and cleaved with a proteolytic enzyme such as trypsin. Cleavage products can then be added to the anchored GST-NGPCR fusion protein and allowed to bind. After washing away unbound peptides, labeled bound material, representing the intracellular binding partner binding domain, can be eluted, purified, and analyzed for amino acid sequence by well-known methods. Peptides so identified can be produced synthetically or fused to appropriate facilitative proteins using recombinant DNA technology.

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims. All of the cited references, publications, patents and patent applications are herein incorporated by reference in their entirety.

## Claims

1. An isolated nucleic acid molecule containing at least 24 contiguous bases of nucleotide sequence first disclosed in the NGPCR nucleotide sequence described in SEQ ID NO: 1.

2. An isolated nucleic acid molecule having a nucleotide sequence that:
(a) encodes the amino acid sequence shown in SEQ ID NO: 2; and
(b) hybridizes under stringent conditions to the nucleotide sequence of SEQ ID NO: 1 or the complement thereof.

3. An isolated nucleic acid molecule having a nucleotide sequence that:
(a) encodes the amino acid sequence shown in SEQ ID NO: 4; and
(b) hybridizes under stringent conditions to the nucleotide sequence of SEQ ID NO: 3 or the complete thereof.

4. An isolated nucleic acid molecule having a nucleotide sequence that:
(a) encodes the amino acid sequence shown in SEQ ID NO: 40; and
(b) hybridizes under stringent conditions to the nucleotide sequence of SEQ ID NO: 39 or the complement thereof.

5. An isolated nucleic acid molecule having a nucleotide sequence that:
(a) encodes the amino acid sequence shown in SEQ ID NO: 58; and
(b) hybridizes under stringent conditions to the nucleotide sequence of the SEQ ID NO: 57 or the complement thereof.

6. An expression vector comprising an isolated polynucleotide encoding the amino acid sequence presented in SEQ ID NO: 2.

7. A polypeptide encoded by a nucleic acid as claimed in any one of claims 1 to 5 or an expression vector as claimed in claim 6.

8. An antibody that binds to a polypeptide as claimed in claim 7, or a fragment or a derivative of said antibody, for example, a polyclonal or monoclonal antibody, a single chain antibody, or a Fab fragment, a F(ab¹)2, a fragment produced by a Fab expression library, or an anti-idiotypic (anti-Id) antibody, or an expitodpe binding fragment of any of said antibodies or fragments.

9. A host cell that comprises a nucleic acid as claimed in any one of the claims 1 to 5 or an expression vector as claimed in claim 6 and is capable of expressing the polypeptide encoded by the said nucleic acid, optionally on the surface of the cell.

10. An animal that is capable of expressing a nucleic acid as claimed in any one of claim 1 to 5, or that is an optionally conditional "knock-out" animal that does not express a functional polypeptide as claimed in claim 7.


Sequence Listing

<110> Turner, C. Alexander Jr.
      Nehls, Michael
      Friedrich, Glenn
      Scoville, John
      Zambrowicz, Brian
      Sands, Arthur T.

<120> Novel Human 7TM Proteins and Receptors and
  Polynucleotides Encoding the Same

<130> 8170(1) EP JSvn/TJH

<150> US 60/153,366
<151> 1999-09-10

<150> US 60/165,510
<151> 1999-11-15

<160> 59

<170> FastSEQ for Windows Version 4.0

<210> 1
<211> 3753
<212> DNA
<213> homo sapiens

<400> 1
atgtttcgct cagatcgaat gtggagctgc cattggaaat ggaagcccag tcctctcctg 60
ttcttatttg ctttatatat catgtgtgtt cctcactcag cagtgtgggg atgtgccaac 120
tgccgagtgg ttttgtccaa cccttctggg acctttactt ctccatgcta ccctaacgac 180
tacccaaaca gccaggcttg catgtggacg ctccgagccc ccaccggtta tatcattcag 240
ataacattta acgactttga cattgaagaa gctcccaatt gcatttatga ctcattatcc 300
cttgataatg gagagagcca gactaaattt tgtggagcaa ctgccaaagg cctatcattt 360
aactcaagtg cgaatgagat gcatgtgtcc ttttcaagtg actttagcat ccagaagaaa 420
ggtttcaatg ccagctacat cagagttgcc gtgtccttaa ggaatcaaaa ggtcatttta 480
ccccagacat cagatgctta ccaggtatct gttgcaaaaa gcatctctat tccagagctc 540
agtgctttca cactctgctt tgaagcaacc aaagttggcc atgaagacag tgattggaca 600
gctttctcct actcaaatgc atccttcaca caattgctca gttttggaaa ggccaagagt 660
ggctactttc tatccatttc tgattcaaaa tgtttgttga ataatgcatt acctgtcaaa 720
gaaaaagaag acatttttgc agaaagcttt gaacagctct gccttgtttg gaataattct 780
ttgggctcta ttggtgtaaa tttcaaaaga aactatgaaa cagttccatg tgattctacc 840
attagtaaag ttattcctgg gaatgggaaa ttgttgttgg gctccaatca aaatgaaatt 900
gtctctctaa aaggggacat ttataacttt cgactttgga attttaccat gaatgccaaa 960
atcctctcca acctcagctg taatgtgaaa gggaatgtag tcgactggca aaatgacttc 1020
tggaatatcc caaacctagc tctgaaagct gaaagcaacc taagctgtgg ttcctacctg 1080
atcccgctcc cagcagcaga actggccagc tgtgcagacc tggggaccct ctgtcaagct 1140
actgtaaact ctcctagtac tacaccaccc actgtcacca ctaacatgcc tgttactaac 1200
agaatcgata aacaaaggaa tgatggaatt atctatagaa tatccgtagt gattcagaac 1260
atccttcgtc accctgaggt aaaagtacag agcaaggtgg cagaatggct caattcaacc 1320
ttccaaaatt ggaactacac ggtttatgtc gttaatatca gttttcacct gagtgctgga 1380
gaggacaaga ttaaagtcaa gagaagcctt gaggatgagc caaggttggt gctttgggcc 1440
cttctagttt acaatgctac caacaatact aatttagaag gaaaaatcat tcagcagaag 1500
ctcctaaaaa ataatgagtc cttggatgaa ggcttgaggc tacatacagt gaatgtgaga 1560
caactgggtc attgtcttgc catggaggaa cccaaaggct actactggcc atctatccaa 1620
ccttctgaat acgttcttcc ttgtccagac aagcctggct tttctgcttc tcggatatgt 1680
ttttacaatg ctaccaaccc attggtaacc tactggggac ctgttgatat ctccaactgt 1740
ttaaaagaag caaatgaagt tgctaaccag attttaaatt taactgctga tgggcagaac 1800
ttaacctcag ccaatattac caacattgtg gaacaggtca aaagaattgt gaataaagaa 1860
gaaaacattg atataacact tggctcaact ctaatgaata tattttctaa tatcttaagc 1920
agttcagaca gtgacttgct tgagtcatct tctgaagctt taaaaacaat tgatgaattg 1980
gccttcaaga tagacctaaa tagcacatca catgtgaata ttacaactcg gaacttggct 2040
ctcagcgtat catccctgtt accagggaca aatgcaattt caaattttag cattggtctt 2100
ccaagcaata atgaatcgta tttccagatg gattttgaga gtggacaagt ggatccactg 2160
gcatctgtaa ttttgcctcc aaacttactt gagaatttaa gtccagaaga ttctgtatta 2220
gttagaagag cacagtttac tttcttcaac aaaactggac ttttccagga tgtaggaccc 2280
caaagaaaaa ctttagtgag ttatgtgatg gcgtgcagta ttggaaacat tactatccag 2340

aatctgaagg atcctgttca aataaaaatc aaacatacaa gaactcagga agtgcatcat 2400
cccatctgtg ccttctggga tctgaacaaa aacaaaagtt ttggaggatg gaacacgtca 2460
ggatgtgttg cacacagaga ttcagatgca agtgagacag tctgcctgtg taaccacttc 2520
acacactttg gagttctgat ggaccttcca agaagtgcct cacagttaga tgcaagaaac 2580
actaaagtcc tcactttcat cagctatatt gggtgtggaa tatctgctat tttttcagca 2640
gcaactctcc tgacatatgt tgcttttgag aaattgcgaa gggattatcc ctccaaaatc 2700
ttgatgaacc tgagcacagc cctgctgttc ctgaatctcc tcttcctcct agatggctgg 2760
atcacctcct tcaatgtgga tggactttgc attgctgttg cagtcctgtt gcatttcttc 2820
cttctggcaa cctttacctg gatggggcta gaagcaattc acatgtacat tgctctagtt 2880
aaagtattta acacttacat tcgccgatac attctaaaat tctgcatcat tggctggggt 2940
ttgcctgcct tagtggtgtc agttgttcta gcgagcagaa acaacaatga agtctatgga 3000
aaagaaagtt atgggaaaga aaaaggtgat gaattctgtt ggattcaaga tccagtcata 3060
ttttatgtga cctgtgctgg gtattttgga gtcatgtttt ttctgaacat tgccatgttc 3120
attgtggtaa tggtgcagat ctgtgggagg aatggcaaga gaagcaaccg gaccctgaga 3180
gaagaagtgt taaggaacct gcgcagtgtg gttagcttga cctttctgtt gggcatgaca 3240
tggggttttg cattctttgc ctggggaccc ttaaatatcc ccttcatgta cctcttctcc 3300
atcttcaatt cattacaagg cttatttata ttcatcttcc actgtgctat gaaggagaat 3360
gttcagaaac agtggcggcg gcatctctgc tgtggtagat ttcggttagc agataactca 3420
gattggagta agacagctac caatatcatc aagaaaagtt ctgataatct aggaaaatct 3480
ttgtcttcaa gctccattgg ttccaactca acctatctta catccaaatc taaatccagc 3540
tctaccacct atttcaaaag gaatagccac acagacagtg cttccatgga caagtccttg 3600
tcaaaactgg cccatgctga tggagatcaa acatcaatca tccctgtcca tcaggtcatt 3660
gataaggtca agggttattg caatgctcat tcagacaact tctataaaaa tattatcatg 3720
tcagacacct tcagccacag cacaaagttt taa 3753

<210> 2
<211> 1250
<212> PRT
<213> homo sapiens

<400> 2
   Met Phe Arg Ser Asp Arg Met Trp Ser Cys His Trp Lys Trp Lys Pro
   1 5 10 15
   Ser Pro Leu Leu Phe Leu Phe Ala Leu Tyr Ile Met Cys Val Pro His
   20 25 30
   Ser Ala Val Trp Gly Cys Ala Asn Cys Arg Val Val Leu Ser Asn Pro
   35 40 45
   Ser Gly Thr Phe Thr Ser Pro Cys Tyr Pro Asn Asp Tyr Pro Asn Ser
   50 55 60
   Gln Ala Cys Met Trp Thr Leu Arg Ala Pro Thr Gly Tyr Ile Ile Gln
   65 70 75 80
   Ile Thr Phe Asn Asp Phe Asp Ile Glu Glu Ala Pro Asn Cys Ile Tyr
   85 90 95
   Asp Ser Leu Ser Leu Asp Asn Gly Glu Ser Gln Thr Lys Phe Cys Gly
   100 105 110
   Ala Thr Ala Lys Gly Leu Ser Phe Asn Ser Ser Ala Asn Glu Met His
   115 120 125
   Val Ser Phe Ser Ser Asp Phe Ser Ile Gln Lys Lys Gly Phe Asn Ala
   130 135 140
   Ser Tyr Ile Arg Val Ala Val Ser Leu Arg Asn Gln Lys Val Ile Leu
   145 150 155 160
   Pro Gln Thr Ser Asp Ala Tyr Gln Val Ser Val Ala Lys Ser Ile Ser
   165 170 175
   Ile Pro Glu Leu Ser Ala Phe Thr Leu Cys Phe Glu Ala Thr Lys Val
   180 185 190
   Gly His Glu Asp Ser Asp Trp Thr Ala Phe Ser Tyr Ser Asn Ala Ser
   195 200 205
   Phe Thr Gln Leu Leu Ser Phe Gly Lys Ala Lys Ser Gly Tyr Phe Leu
   210 215 220
   Ser Ile Ser Asp Ser Lys Cys Leu Leu Asn Asn Ala Leu Pro Val Lys
   225 230 235 240
   Glu Lys Glu Asp Ile Phe Ala Glu Ser Phe Glu Gln Leu Cys Leu Val
   245 250 255
   Trp Asn Asn Ser Leu Gly Ser Ile Gly Val Asn Phe Lys Arg Asn Tyr
   260 265 270
   Glu Thr Val Pro Cys Asp Ser Thr Ile Ser Lys Val Ile Pro Gly Asn
   275 280 285
   Gly Lys Leu Leu Leu Gly Ser Asn Gln Asn Glu Ile Val Ser Leu Lys
   290                 295                 300
   Gly Asp Ile Tyr Asn Phe Arg Leu Trp Asn Phe Thr Met Asn Ala Lys
   305                 310                 315                 320
   Ile Leu Ser Asn Leu Ser Cys Asn Val Lys Gly Asn Val Val Asp Trp
                   325                 330                 335
   Gln Asn Asp Phe Trp Asn Ile Pro Asn Leu Ala Leu Lys Ala Glu Ser
               340                 345                 350
   Asn Leu Ser Cys Gly Ser Tyr Leu Ile Pro Leu Pro Ala Ala Glu Leu
           355                 360                 365
   Ala Ser Cys Ala Asp Leu Gly Thr Leu Cys Gln Ala Thr Val Asn Ser
       370                 375                 380
   Pro Ser Thr Thr Pro Pro Thr Val Thr Thr Asn Met Pro Val Thr Asn
   385                 390                 395                 400
   Arg Ile Asp Lys Gln Arg Asn Asp Gly Ile Ile Tyr Arg Ile Ser Val
                   405                 410                 415
   Val Ile Gln Asn Ile Leu Arg His Pro Glu Val Lys Val Gln Ser Lys
               420                 425                 430
   Val Ala Glu Trp Leu Asn Ser Thr Phe Gln Asn Trp Asn Tyr Thr Val
           435                 440                 445
   Tyr Val Val Asn Ile Ser Phe His Leu Ser Ala Gly Glu Asp Lys Ile
       450                 455                 460
   Lys Val Lys Arg Ser Leu Glu Asp Glu Pro Arg Leu Val Leu Trp Ala
   465                 470                 475                 480
   Leu Leu Val Tyr Asn Ala Thr Asn Asn Thr Asn Leu Glu Gly Lys Ile
                   485                 490                 495
   Ile Gln Gln Lys Leu Leu Lys Asn Asn Glu Ser Leu Asp Glu Gly Leu
               500                 505                 510
   Arg Leu His Thr Val Asn Val Arg Gln Leu Gly His Cys Leu Ala Met
           515                 520                 525
   Glu Glu Pro Lys Gly Tyr Tyr Trp Pro Ser Ile Gln Pro Ser Glu Tyr
       530                 535                 540
   Val Leu Pro Cys Pro Asp Lys Pro Gly Phe Ser Ala Ser Arg Ile Cys
   545                 550                 555                 560
   Phe Tyr Asn Ala Thr Asn Pro Leu Val Thr Tyr Trp Gly Pro Val Asp
                   565                 570                 575
   Ile Ser Asn Cys Leu Lys Glu Ala Asn Glu Val Ala Asn Gln Ile Leu
               580                 585                 590
   Asn Leu Thr Ala Asp Gly Gln Asn Leu Thr Ser Ala Asn Ile Thr Asn
           595                 600                 605
   Ile Val Glu Gln Val Lys Arg Ile Val Asn Lys Glu Glu Asn Ile Asp
       610                 615                 620
   Ile Thr Leu Gly Ser Thr Leu Met Asn Ile Phe Ser Asn Ile Leu Ser
   625                 630                 635                 640
   Ser Ser Asp Ser Asp Leu Leu Glu Ser Ser Ser Glu Ala Leu Lys Thr
                   645                 650                 655
   Ile Asp Glu Leu Ala Phe Lys Ile Asp Leu Asn Ser Thr Ser His Val
               660                 665                 670
   Asn Ile Thr Thr Arg Asn Leu Ala Leu Ser Val Ser Ser Leu Leu Pro
           675                 680                 685
   Gly Thr Asn Ala Ile Ser Asn Phe Ser Ile Gly Leu Pro Ser Asn Asn
       690                 695                 700
   Glu Ser Tyr Phe Gln Met Asp Phe Glu Ser Gly Gln Val Asp Pro Leu
   705                 710                 715                 720
   Ala Ser Val Ile Leu Pro Pro Asn Leu Leu Glu Asn Leu Ser Pro Glu
                   725                 730                 735
   Asp Ser Val Leu Val Arg Arg Ala Gln Phe Thr Phe Phe Asn Lys Thr
               740                 745                 750
   Gly Leu Phe Gln Asp Val Gly Pro Gln Arg Lys Thr Leu Val Ser Tyr
           755                 760                 765
   Val Met Ala Cys Ser Ile Gly Asn Ile Thr Ile Gln Asn Leu Lys Asp
       770                 775                 780
   Pro Val Gln Ile Lys Ile Lys His Thr Arg Thr Gln Glu Val His His
   785                 790                 795                 800
   Pro Ile Cys Ala Phe Trp Asp Leu Asn Lys Asn Lys Ser Phe Gly Gly
                   805                 810                 815
   Trp Asn Thr Ser Gly Cys Val Ala His Arg Asp Ser Asp Ala Ser Glu
               820                 825                 830
   Thr Val Cys Leu Cys Asn His Phe Thr His Phe Gly Val Leu Met Asp
   835 840 845
   Leu Pro Arg Ser Ala Ser Gln Leu Asp Ala Arg Asn Thr Lys Val Leu
   850 855 860
   Thr Phe Ile Ser Tyr Ile Gly Cys Gly Ile Ser Ala Ile Phe Ser Ala
   865 870 875 880
   Ala Thr Leu Leu Thr Tyr Val Ala Phe Glu Lys Leu Arg Arg Asp Tyr
   885 890 895
   Pro Ser Lys Ile Leu Met Asn Leu Ser Thr Ala Leu Leu Phe Leu Asn
   900 905 910
   Leu Leu Phe Leu Leu Asp Gly Trp Ile Thr Ser Phe Asn Val Asp Gly
   915 920 925
   Leu Cys Ile Ala Val Ala Val Leu Leu His Phe Phe Leu Leu Ala Thr
   930 935 940
   Phe Thr Trp Met Gly Leu Glu Ala Ile His Met Tyr Ile Ala Leu Val
   945 950 955 960
   Lys Val Phe Asn Thr Tyr Ile Arg Arg Tyr Ile Leu Lys Phe Cys Ile
   965 970 975
   Ile Gly Trp Gly Leu Pro Ala Leu Val Val Ser Val Val Leu Ala Ser
   980 985 990
   Arg Asn Asn Asn Glu Val Tyr Gly Lys Glu Ser Tyr Gly Lys Glu Lys
   995 1000 1005
   Gly Asp Glu Phe Cys Trp Ile Gln Asp Pro Val Ile Phe Tyr Val Thr
   1010 1015 1020
   Cys Ala Gly Tyr Phe Gly Val Met Phe Phe Leu Asn Ile Ala Met Phe
   1025 1030 1035 1040
   Ile Val Val Met Val Gln Ile Cys Gly Arg Asn Gly Lys Arg Ser Asn
   1045 1050 1055
   Arg Thr Leu Arg Glu Glu Val Leu Arg Asn Leu Arg Ser Val Val Ser
   1060 1065 1070
   Leu Thr Phe Leu Leu Gly Met Thr Trp Gly Phe Ala Phe Phe Ala Trp
   1075 1080 1085
   Gly Pro Leu Asn Ile Pro Phe Met Tyr Leu Phe Ser Ile Phe Asn Ser
   1090 1095 1100
   Leu Gln Gly Leu Phe Ile Phe Ile Phe His Cys Ala Met Lys Glu Asn
   1105 1110 1115 1120
   Val Gln Lys Gln Trp Arg Arg His Leu Cys Cys Gly Arg Phe Arg Leu
   1125 1130 1135
   Ala Asp Asn Ser Asp Trp Ser Lys Thr Ala Thr Asn Ile Ile Lys Lys
   1140 1145 1150
   Ser Ser Asp Asn Leu Gly Lys Ser Leu Ser Ser Ser Ser Ile Gly Ser
   1155 1160 1165
   Asn Ser Thr Tyr Leu Thr Ser Lys Ser Lys Ser Ser Ser Thr Thr Tyr
   1170 1175 1180
   Phe Lys Arg Asn Ser His Thr Asp Ser Ala Ser Met Asp Lys Ser Leu
   1185 1190 1195 1200
   Ser Lys Leu Ala His Ala Asp Gly Asp Gln Thr Ser Ile Ile Pro Val
   1205 1210 1215
   His Gln Val Ile Asp Lys Val Lys Gly Tyr Cys Asn Ala His Ser Asp
   1220 1225 1230
   Asn Phe Tyr Lys Asn Ile Ile Met Ser Asp Thr Phe Ser His Ser Thr
   1235 1240 1245
   Lys Phe
   1250

<210> 3
<211> 3666
<212> DNA
<213> homo sapiens

<400> 3
atgtttcgct cagatcgaat gtggagctgc cattggaaat ggaagcccag tcctctcctg      60
ttcttatttg ctttatatat catgtgtgtt cctcactcag cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480

ccccagacat cagatgctta ccaggtatct gttgcaaaaa gcatctctat tccagagctc 540
agtgctttca cactctgctt tgaagcaacc aaagttggcc atgaagacag tgattggaca 600
gctttctcct actcaaatgc atccttcaca caattgctca gttttggaaa ggccaagagt 660
ggctactttc tatccatttc tgattcaaaa tgtttgttga ataatgcatt acctgtcaaa 720
gaaaaagaag acatttttgc agaaagcttt gaacagctct gccttgtttg gaataattct 780
ttgggctcta ttggtgtaaa tttcaaaaga aactatgaaa cagttccatg tgattctacc 840
attagtaaag ttattcctgg gaatgggaaa ttgttgttgg gctccaatca aaatgaaatt 900
gtctctctaa aaggggacat ttataacttt cgactttgga attttaccat gaatgccaaa 960
atcctctcca acctcagctg taatgtgaaa gggaatgtag tcgactggca aaatgacttc 1020
tggaatatcc caaacctagc tctgaaagct gaaagcaacc taagctgtgg ttcctacctg 1080
atcccgctcc cagcagcaga actggccagc tgtgcagacc tggggaccct ctgtcaagct 1140
actgtaaact ctcctagtac tacaccaccc actgtcacca ctaacatgcc tgttactaac 1200
agaatcgata aacaaaggaa tgatggaatt atctatagaa tatccgtagt gattcagaac 1260
atccttcgtc accctgaggt aaaagtacag agcaaggtgg cagaatggct caattcaacc 1320
ttccaaaatt ggaactacac ggtttatgtc gttaatatca gttttcacct gagtgctgga 1380
gaggacaaga ttaaagtcaa gagaagcctt gaggatgagc caaggttggt gctttgggcc 1440
cttctagttt acaatgctac caacaatact aatttagaag gaaaaatcat tcagcagaag 1500
ctcctaaaaa ataatgagtc cttggatgaa ggcttgaggc tacatacagt gaatgtgaga 1560
caactgggtc attgtcttgc catggaggaa cccaaaggct actactggcc atctatccaa 1620
ccttctgaat acgttcttcc ttgtccagac aagcctggct tttctgcttc tcggatatgt 1680
ttttacaatg ctaccaaccc attggtaacc tactggggac ctgttgatat ctccaactgt 1740
ttaaaagaag caaatgaagt tgctaaccag attttaaatt taactgctga tgggcagaac 1800
ttaacctcag ccaatattac caacattgtg gaacaggtca aaagaattgt gaataaagaa 1860
gaaaacattg atataacact tggctcaact ctaatgaata tattttctaa tatcttaagc 1920
agttcagaca gtgacttgct tgagtcatct tctgaagctt taaaaacaat tgatgaattg 1980
gccttcaaga tagacctaaa tagcacatca catgtgaata ttacaactcg gaacttggct 2040
ctcagcgtat catccctgtt accagggaca aatgcaattt caaattttag cattggtctt 2100
ccaagcaata atgaatcgta tttccagatg gattttgaga gtggacaagt ggatccactg 2160
gcatctgtaa ttttgcctcc aaacttactt gagaatttaa gtccagaaga ttctgtatta 2220
gttagaagag cacagtttac tttcttcaac aaaactggac ttttccagga tgtaggaccc 2280
caaagaaaaa ctttagtgag ttatgtgatg gcgtgcagta ttggaaacat tactatccag 2340
aatctgaagg atcctgttca aataaaaatc aaacatacaa gaactcagga agtgcatcat 2400
cccatctgtg ccttctggga tctgaacaaa aacaaaagtt ttggaggatg gaacacgtca 2460
ggatgtgttg cacacagaga ttcagatgca agtgagacag tctgcctgtg taaccacttc 2520
acacactttg gagttctgat ggaccttcca agaagtgcct cacagttaga tgcaagaaac 2580
actaaagtcc tcactttcat cagctatatt gggtgtggaa tatctgctat tttttcagca 2640
gcaactctcc tgacatatgt tgcttttgag aaattgcgaa gggattatcc ctccaaaatc 2700
ttgatgaacc tgagcacagc cctgctgttc ctgaatctcc tcttcctcct agatggctgg 2760
atcacctcct tcaatgtgga tggactttgc attgctgttg cagtcctgtt gcatttcttc 2820
cttctggcaa cctttacctg gatggggcta gaagcaattc acatgtacat tgctctagtt 2880
aaagtattta acacttacat tcgccgatac attctaaaat tctgcatcat tggctggggt 2940
ttgcctgcct tagtggtgtc agttgttcta gcgagcagaa acaacaatga agtctatgga 3000
aaagaaagtt atgggaaaga aaaaggtgat gaattctgtt ggattcaaga tccagtcata 3060
ttttatgtga cctgtgctgg gtattttgga gtcatgtttt ttctgaacat tgccatgttc 3120
attgtggtaa tggtgcagat ctgtgggagg aatggcaaga gaagcaaccg gaccctgaga 3180
gaagaagtgt taaggaacct gcgcagtgtg gttagcttga cctttctgtt gggcatgaca 3240
tggggttttg cattctttgc ctggggaccc ttaaatatcc ccttcatgta cctcttctcc 3300
atcttcaatt cattacaagg cttatttata ttcatcttcc actgtgctat gaaggagaat 3360
gttcagaaac agtggcggcg gcatctctgc tgtggtagat ttcggttagc agataactca 3420
gattggagta agacagctac caatatcatc aagaaaagtt ctgataatct aggaaaatct 3480
ttgtcttcaa gctccattgg ttccaactca acctatctta catccaaatc taaatccagc 3540
tctaccacct atttcaaaag gaatagccac acagataatg tctcctatga gcattccttc 3600
aacaaaagtg gatcactcag acagtgcttc catggacaag tccttgtcaa aactggccca 3660
tgctga 3666

<210> 4
<211> 1221
<212> PRT
<213> homo sapiens

<400> 4
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Ala Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser

50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Ala Thr Val Asn Ser
    370                 375                 380
Pro Ser Thr Thr Pro Pro Thr Val Thr Thr Asn Met Pro Val Thr Asn
385                 390                 395                 400
Arg Ile Asp Lys Gln Arg Asn Asp Gly Ile Ile Tyr Arg Ile Ser Val
                405                 410                 415
Val Ile Gln Asn Ile Leu Arg His Pro Glu Val Lys Val Gln Ser Lys
            420                 425                 430
Val Ala Glu Trp Leu Asn Ser Thr Phe Gln Asn Trp Asn Tyr Thr Val
        435                 440                 445
Tyr Val Val Asn Ile Ser Phe His Leu Ser Ala Gly Glu Asp Lys Ile
    450                 455                 460
Lys Val Lys Arg Ser Leu Glu Asp Glu Pro Arg Leu Val Leu Trp Ala
465                 470                 475                 480
Leu Leu Val Tyr Asn Ala Thr Asn Asn Thr Asn Leu Glu Gly Lys Ile
                485                 490                 495
Ile Gln Gln Lys Leu Leu Lys Asn Asn Glu Ser Leu Asp Glu Gly Leu
            500                 505                 510
Arg Leu His Thr Val Asn Val Arg Gln Leu Gly His Cys Leu Ala Met
        515                 520                 525
Glu Glu Pro Lys Gly Tyr Tyr Trp Pro Ser Ile Gln Pro Ser Glu Tyr
    530                 535                 540
Val Leu Pro Cys Pro Asp Lys Pro Gly Phe Ser Ala Ser Arg Ile Cys
545                 550                 555                 560
Phe Tyr Asn Ala Thr Asn Pro Leu Val Thr Tyr Trp Gly Pro Val Asp
                565                 570                 575
Ile Ser Asn Cys Leu Lys Glu Ala Asn Glu Val Ala Asn Gln Ile Leu
            580                 585                 590
Asn Leu Thr Ala Asp Gly Gln Asn Leu Thr Ser Ala Asn Ile Thr Asn

595                 600                 605
Ile Val Glu Gln Val Lys Arg Ile Val Asn Lys Glu Glu Asn Ile Asp
    610                 615                 620
Ile Thr Leu Gly Ser Thr Leu Met Asn Ile Phe Ser Asn Ile Leu Ser
625                 630                 635                 640
Ser Ser Asp Ser Asp Leu Leu Glu Ser Ser Ser Glu Ala Leu Lys Thr
                645                 650                 655
Ile Asp Glu Leu Ala Phe Lys Ile Asp Leu Asn Ser Thr Ser His Val
            660                 665                 670
Asn Ile Thr Thr Arg Asn Leu Ala Leu Ser Val Ser Ser Leu Leu Pro
        675                 680                 685
Gly Thr Asn Ala Ile Ser Asn Phe Ser Ile Gly Leu Pro Ser Asn Asn
    690                 695                 700
Glu Ser Tyr Phe Gln Met Asp Phe Glu Ser Gly Gln Val Asp Pro Leu
705                 710                 715                 720
Ala Ser Val Ile Leu Pro Pro Asn Leu Leu Glu Asn Leu Ser Pro Glu
                725                 730                 735
Asp Ser Val Leu Val Arg Arg Ala Gln Phe Thr Phe Phe Asn Lys Thr
            740                 745                 750
Gly Leu Phe Gln Asp Val Gly Pro Gln Arg Lys Thr Leu Val Ser Tyr
        755                 760                 765
Val Met Ala Cys Ser Ile Gly Asn Ile Thr Ile Gln Asn Leu Lys Asp
    770                 775                 780
Pro Val Gln Ile Lys Ile Lys His Thr Arg Thr Gln Glu Val His His
785                 790                 795                 800
Pro Ile Cys Ala Phe Trp Asp Leu Asn Lys Asn Lys Ser Phe Gly Gly
                805                 810                 815
Trp Asn Thr Ser Gly Cys Val Ala His Arg Asp Ser Asp Ala Ser Glu
            820                 825                 830
Thr Val Cys Leu Cys Asn His Phe Thr His Phe Gly Val Leu Met Asp
        835                 840                 845
Leu Pro Arg Ser Ala Ser Gln Leu Asp Ala Arg Asn Thr Lys Val Leu
    850                 855                 860
Thr Phe Ile Ser Tyr Ile Gly Cys Gly Ile Ser Ala Ile Phe Ser Ala
865                 870                 875                 880
Ala Thr Leu Leu Thr Tyr Val Ala Phe Glu Lys Leu Arg Arg Asp Tyr
                885                 890                 895
Pro Ser Lys Ile Leu Met Asn Leu Ser Thr Ala Leu Leu Phe Leu Asn
            900                 905                 910
Leu Leu Phe Leu Leu Asp Gly Trp Ile Thr Ser Phe Asn Val Asp Gly
        915                 920                 925
Leu Cys Ile Ala Val Ala Val Leu Leu His Phe Phe Leu Leu Ala Thr
    930                 935                 940
Phe Thr Trp Met Gly Leu Glu Ala Ile His Met Tyr Ile Ala Leu Val
945                 950                 955                 960
Lys Val Phe Asn Thr Tyr Ile Arg Arg Tyr Ile Leu Lys Phe Cys Ile
                965                 970                 975
Ile Gly Trp Gly Leu Pro Ala Leu Val Val Ser Val Val Leu Ala Ser
            980                 985                 990
Arg Asn Asn Asn Glu Val Tyr Gly Lys Glu Ser Tyr Gly Lys Glu Lys
        995                 1000                1005
Gly Asp Glu Phe Cys Trp Ile Gln Asp Pro Val Ile Phe Tyr Val Thr
    1010                1015                1020
Cys Ala Gly Tyr Phe Gly Val Met Phe Phe Leu Asn Ile Ala Met Phe
1025                1030                1035                1040
Ile Val Val Met Val Gln Ile Cys Gly Arg Asn Gly Lys Arg Ser Asn
                1045                1050                1055
Arg Thr Leu Arg Glu Glu Val Leu Arg Asn Leu Arg Ser Val Val Ser
            1060                1065                1070
Leu Thr Phe Leu Leu Gly Met Thr Trp Gly Phe Ala Phe Phe Ala Trp
        1075                1080                1085
Gly Pro Leu Asn Ile Pro Phe Met Tyr Leu Phe Ser Ile Phe Asn Ser
    1090                1095                1100
Leu Gln Gly Leu Phe Ile Phe Ile Phe His Cys Ala Met Lys Glu Asn
1105                1110                1115                1120
Val Gln Lys Gln Trp Arg Arg His Leu Cys Cys Gly Arg Phe Arg Leu
                1125                1130                1135
Ala Asp Asn Ser Asp Trp Ser Lys Thr Ala Thr Asn Ile Ile Lys Lys

1140                    1145                    1150
Ser Ser Asp Asn Leu Gly Lys Ser Leu Ser Ser Ser Ser Ile Gly Ser
        1155                    1160                    1165
Asn Ser Thr Tyr Leu Thr Ser Lys Ser Lys Ser Ser Ser Thr Thr Tyr
    1170                    1175                    1180
Phe Lys Arg Asn Ser His Thr Asp Asn Val Ser Tyr Glu His Ser Phe
1185                    1190                    1195                    1200
Asn Lys Ser Gly Ser Leu Arg Gln Cys Phe His Gly Gln Val Leu Val
                1205                    1210                    1215
Lys Thr Gly Pro Cys
            1220

<210> 5
<211> 2157
<212> DNA
<213> homo sapiens

<400> 5
atgtttcgct cagatcgaat gtggagctgc cattggaaat ggaagcccag tcctctcctg      60
ttcttatttg ctttatatat catgtgtgtt cctcactcag cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080
atcccgctcc cagcagcaga actggccagc tgtgcagacc tggggaccct ctgtcaagct    1140
actgtaaact ctcctagtac tacaccaccc actgtcacca ctaacatgcc tgttactaac    1200
agaatcgata aacaaaggaa tgatggaatt atctatagaa tatccgtagt gattcagaac    1260
atccttcgtc accctgaggt aaaagtacag agcaaggtgg cagaatggct caattcaacc    1320
ttccaaaatt ggaactacac ggtttatgtc gttaatatca gttttcacct gagtgctgga    1380
gaggacaaga ttaaagtcaa gagaagcctt gaggatgagc caaggttggt gctttgggcc    1440
cttctagttt acaatgctac caacaatact aatttagaag gaaaaatcat tcagcagaag    1500
ctcctaaaaa ataatgagtc cttggatgaa ggcttgaggc tacatacagt gaatgtgaga    1560
caactgggtc attgtcttgc catggaggaa cccaaaggct actactggcc atctatccaa    1620
ccttctgaat acgttcttcc ttgtccagac aagcctggct tttctgcttc tcggatatgt    1680
ttttacaatg ctaccaaccc attggtaacc tactggggac ctgttgatat ctccaactgt    1740
ttaaaagaag caaatgaagt tgctaaccag attttaaatt taactgctga tgggcagaac    1800
ttaacctcag ccaatattac caacattgtg gaacaggtca aaagaattgt gaataaagaa    1860
gaaaacattg atataacact tggctcaact ctaatgaata tattttctaa tatcttaagc    1920
agttcagaca gtgacttgct tgagtcatct tctgaagctt taaaaacaat tgatgaattg    1980
gccttcaaga tagacctaaa tagcacatca catgtgaata ttacaactcg gaacttggct    2040
ctcagcgtat catccctgtt accagggaca aatgcaattt caaattttag cattggtctt    2100
ccaagcaata atgaatcgta tttccaggta atgagccagt ggtttctttc attttaa       2157

<210> 6
<211> 718
<212> PRT
<213> homo sapiens

<400> 6
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Ala Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser

50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Ala Thr Val Asn Ser
    370                 375                 380
Pro Ser Thr Thr Pro Pro Thr Val Thr Thr Asn Met Pro Val Thr Asn
385                 390                 395                 400
Arg Ile Asp Lys Gln Arg Asn Asp Gly Ile Ile Tyr Arg Ile Ser Val
                405                 410                 415
Val Ile Gln Asn Ile Leu Arg His Pro Glu Val Lys Val Gln Ser Lys
            420                 425                 430
Val Ala Glu Trp Leu Asn Ser Thr Phe Gln Asn Trp Asn Tyr Thr Val
        435                 440                 445
Tyr Val Val Asn Ile Ser Phe His Leu Ser Ala Gly Glu Asp Lys Ile
    450                 455                 460
Lys Val Lys Arg Ser Leu Glu Asp Glu Pro Arg Leu Val Leu Trp Ala
465                 470                 475                 480
Leu Leu Val Tyr Asn Ala Thr Asn Asn Thr Asn Leu Glu Gly Lys Ile
                485                 490                 495
Ile Gln Gln Lys Leu Leu Lys Asn Asn Glu Ser Leu Asp Glu Gly Leu
            500                 505                 510
Arg Leu His Thr Val Asn Val Arg Gln Leu Gly His Cys Leu Ala Met
        515                 520                 525
Glu Glu Pro Lys Gly Tyr Tyr Trp Pro Ser Ile Gln Pro Ser Glu Tyr
    530                 535                 540
Val Leu Pro Cys Pro Asp Lys Pro Gly Phe Ser Ala Ser Arg Ile Cys
545                 550                 555                 560
Phe Tyr Asn Ala Thr Asn Pro Leu Val Thr Tyr Trp Gly Pro Val Asp
                565                 570                 575
Ile Ser Asn Cys Leu Lys Glu Ala Asn Glu Val Ala Asn Gln Ile Leu
            580                 585                 590
Asn Leu Thr Ala Asp Gly Gln Asn Leu Thr Ser Ala Asn Ile Thr Asn

595                 600                 605
Ile Val Glu Gln Val Lys Arg Ile Val Asn Lys Glu Glu Asn Ile Asp
    610                 615                 620
Ile Thr Leu Gly Ser Thr Leu Met Asn Ile Phe Ser Asn Ile Leu Ser
625                 630                 635                 640
Ser Ser Asp Ser Asp Leu Leu Glu Ser Ser Ser Glu Ala Leu Lys Thr
                645                 650                 655
Ile Asp Glu Leu Ala Phe Lys Ile Asp Leu Asn Ser Thr Ser His Val
            660                 665                 670
Asn Ile Thr Thr Arg Asn Leu Ala Leu Ser Val Ser Ser Leu Leu Pro
        675                 680                 685
Gly Thr Asn Ala Ile Ser Asn Phe Ser Ile Gly Leu Pro Ser Asn Asn
    690                 695                 700
Glu Ser Tyr Phe Gln Val Met Ser Gln Trp Phe Leu Ser Phe
705                 710                 715

<210> 7
<211> 3339
<212> DNA
<213> homo sapiens

<400> 7
atgtttcgct cagatcgaat gtggagctgc cattggaaat ggaagcccag tcctctcctg      60
ttcttatttg ctttatatat catgtgtgtt cctcactcag cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080
atcccgctcc cagcagcaga actggccagc tgtgcagacc tggggaccct ctgtcaagct    1140
actgtaaact ctcctagtac tacaccaccc actgtcacca ctaacatgcc tgttactaac    1200
agaatcgata aacaaaggaa tgatggaatt atctatagaa tatccgtagt gattcagaac    1260
atccttcgtc accctgaggt aaaagtacag agcaaggtgg cagaatggct caattcaacc    1320
ttccaaaatt ggaactacac ggtttatgtc gttaatatca gttttcacct gagtgctgga    1380
gaggacaaga ttaaagtcaa gagaagcctt gaggatgagc caaggttggt gctttgggcc    1440
cttctagttt acaatgctac caacaatact aatttagaag gaaaaatcat tcagcagaag    1500
ctcctaaaaa ataatgagtc cttggatgaa ggcttgaggc tacatacagt gaatgtgaga    1560
caactgggtc attgtcttgc catggaggaa cccaaaggct actactggcc atctatccaa    1620
ccttctgaat acgttcttcc ttgtccagac aagcctggct tttctgcttc tcggatatgt    1680
ttttacaatg ctaccaaccc attggtaacc tactggggac ctgttgatat ctccaactgt    1740
ttaaaagaag caaatgaagt tgctaaccag attttaaatt taactgctga tgggcagaac    1800
ttaacctcag ccaatattac caacattgtg gaacaggtca aaagaattgt gaataaagaa    1860
gaaaacattg atataacact tggctcaact ctaatgaata tattttctaa tatcttaagc    1920
agttcagaca gtgacttgct tgagtcatct tctgaagctt taaaaacaat tgatgaattg    1980
gccttcaaga tagacctaaa tagcacatca catgtgaata ttacaactcg gaacttggct    2040
ctcagcgtat catccctgtt accagggaca aatgcaattt caaattttag cattggtctt    2100
ccaagcaata atgaatcgta tttccagatg gattttgaga gtggacaagt ggatccactg    2160
gcatctgtaa ttttgcctcc aaacttactt gagaatttaa gtccagaaga ttctgtatta    2220
gttagaagag cacagtttac tttcttcaac aaaactggac ttttccagga tgtaggaccc    2280
caaagaaaaa ctttagtgag ttatgtgatg gcgtgcagta ttggaaacat tactatccag    2340
aatctgaagg atcctgttca aataaaaatc aaacatacaa gaactcagga agtgcatcat    2400
cccatctgtg ccttctggga tctgaacaaa aacaaaagtt ttggaggatg gaacacgtca    2460
ggatgtgttg cacacagaga ttcagatgca agtgagacag tctgcctgtg taaccacttc    2520
acacactttg gagttctgat ggaccttcca agaagtgcct cacagttaga tgcaagaaac    2580
actaaagtcc tcactttcat cagctatatt gggtgtggaa tatctgctat tttttcagca    2640
gcaactctcc tgacatatgt tgcttttgag aaattgcgaa gggattatcc ctccaaaatc    2700
ttgatgaacc tgagcacagc cctgctgttc ctgaatctcc tcttcctcct agatggctgg    2760

atcacctcct tcaatgtgga tggactttgc attgctgttg cagtcctgtt gcatttcttc 2820
cttctggcaa cctttacctg gatggggcta gaagcaattc acatgtacat tgctctagtt 2880
aaagtattta acacttacat tcgccgatac attctaaaat tctgcatcat tggctggggt 2940
ttgcctgcct tagtggtgtc agttgttcta gcgagcagaa acaacaatga agtctatgga 3000
aaagaaagtt atgggaaaga aaaaggtgat gaattctgtt ggattcaaga tccagtcata 3060
ttttatgtga cctgtgctgg gtattttgga gtcatgtttt ttctgaacat tgccatgttc 3120
attgtggtaa tggtgcagat ctgtgggagg aatggcaaga gaagcaaccg gaccctgaga 3180
gaagaagtgt taaggaacct gcgcagtgtg gttagcttga cctttctgtt gggcatgaca 3240
tggggttttg cattctttgc ctggggaccc ttaaatatcc ccttcatgta cctcttctcc 3300
atcttcaatt cattacaagg taagataaat tgtacatga 3339

<210> 8
<211> 1112
<212> PRT
<213> homo sapiens

<400> 8
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Ala Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Ala Thr Val Asn Ser
    370                 375                 380
Pro Ser Thr Thr Pro Pro Thr Val Thr Thr Asn Met Pro Val Thr Asn
385                 390                 395                 400
Arg Ile Asp Lys Gln Arg Asn Asp Gly Ile Ile Tyr Arg Ile Ser Val

                405                 410                 415
Val Ile Gln Asn Ile Leu Arg His Pro Glu Val Lys Val Gln Ser Lys
            420                 425                 430
Val Ala Glu Trp Leu Asn Ser Thr Phe Gln Asn Trp Asn Tyr Thr Val
        435                 440                 445
Tyr Val Val Asn Ile Ser Phe His Leu Ser Ala Gly Glu Asp Lys Ile
    450                 455                 460
Lys Val Lys Arg Ser Leu Glu Asp Glu Pro Arg Leu Val Leu Trp Ala
465                 470                 475                 480
Leu Leu Val Tyr Asn Ala Thr Asn Asn Thr Asn Leu Glu Gly Lys Ile
                485                 490                 495
Ile Gln Gln Lys Leu Leu Lys Asn Asn Glu Ser Leu Asp Glu Gly Leu
            500                 505                 510
Arg Leu His Thr Val Asn Val Arg Gln Leu Gly His Cys Leu Ala Met
        515                 520                 525
Glu Glu Pro Lys Gly Tyr Tyr Trp Pro Ser Ile Gln Pro Ser Glu Tyr
    530                 535                 540
Val Leu Pro Cys Pro Asp Lys Pro Gly Phe Ser Ala Ser Arg Ile Cys
545                 550                 555                 560
Phe Tyr Asn Ala Thr Asn Pro Leu Val Thr Tyr Trp Gly Pro Val Asp
                565                 570                 575
Ile Ser Asn Cys Leu Lys Glu Ala Asn Glu Val Ala Asn Gln Ile Leu
            580                 585                 590
Asn Leu Thr Ala Asp Gly Gln Asn Leu Thr Ser Ala Asn Ile Thr Asn
        595                 600                 605
Ile Val Glu Gln Val Lys Arg Ile Val Asn Lys Glu Glu Asn Ile Asp
    610                 615                 620
Ile Thr Leu Gly Ser Thr Leu Met Asn Ile Phe Ser Asn Ile Leu Ser
625                 630                 635                 640
Ser Ser Asp Ser Asp Leu Leu Glu Ser Ser Ser Glu Ala Leu Lys Thr
                645                 650                 655
Ile Asp Glu Leu Ala Phe Lys Ile Asp Leu Asn Ser Thr Ser His Val
            660                 665                 670
Asn Ile Thr Thr Arg Asn Leu Ala Leu Ser Val Ser Ser Leu Leu Pro
        675                 680                 685
Gly Thr Asn Ala Ile Ser Asn Phe Ser Ile Gly Leu Pro Ser Asn Asn
    690                 695                 700
Glu Ser Tyr Phe Gln Met Asp Phe Glu Ser Gly Gln Val Asp Pro Leu
705                 710                 715                 720
Ala Ser Val Ile Leu Pro Pro Asn Leu Leu Glu Asn Leu Ser Pro Glu
                725                 730                 735
Asp Ser Val Leu Val Arg Arg Ala Gln Phe Thr Phe Phe Asn Lys Thr
            740                 745                 750
Gly Leu Phe Gln Asp Val Gly Pro Gln Arg Lys Thr Leu Val Ser Tyr
        755                 760                 765
Val Met Ala Cys Ser Ile Gly Asn Ile Thr Ile Gln Asn Leu Lys Asp
    770                 775                 780
Pro Val Gln Ile Lys Ile Lys His Thr Arg Thr Gln Glu Val His His
785                 790                 795                 800
Pro Ile Cys Ala Phe Trp Asp Leu Asn Lys Asn Lys Ser Phe Gly Gly
                805                 810                 815
Trp Asn Thr Ser Gly Cys Val Ala His Arg Asp Ser Asp Ala Ser Glu
            820                 825                 830
Thr Val Cys Leu Cys Asn His Phe Thr His Phe Gly Val Leu Met Asp
        835                 840                 845
Leu Pro Arg Ser Ala Ser Gln Leu Asp Ala Arg Asn Thr Lys Val Leu
    850                 855                 860
Thr Phe Ile Ser Tyr Ile Gly Cys Gly Ile Ser Ala Ile Phe Ser Ala
865                 870                 875                 880
Ala Thr Leu Leu Thr Tyr Val Ala Phe Glu Lys Leu Arg Arg Asp Tyr
                885                 890                 895
Pro Ser Lys Ile Leu Met Asn Leu Ser Thr Ala Leu Leu Phe Leu Asn
            900                 905                 910
Leu Leu Phe Leu Leu Asp Gly Trp Ile Thr Ser Phe Asn Val Asp Gly
        915                 920                 925
Leu Cys Ile Ala Val Ala Val Leu Leu His Phe Phe Leu Leu Ala Thr
    930                 935                 940
Phe Thr Trp Met Gly Leu Glu Ala Ile His Met Tyr Ile Ala Leu Val

945                 950                 955                 960
Lys Val Phe Asn Thr Tyr Ile Arg Arg Tyr Ile Leu Lys Phe Cys Ile
                965                 970                 975
Ile Gly Trp Gly Leu Pro Ala Leu Val Val Ser Val Val Leu Ala Ser
            980                 985                 990
Arg Asn Asn Asn Glu Val Tyr Gly Lys Glu Ser Tyr Gly Lys Glu Lys
        995                 1000                1005
Gly Asp Glu Phe Cys Trp Ile Gln Asp Pro Val Ile Phe Tyr Val Thr
    1010                1015                1020
Cys Ala Gly Tyr Phe Gly Val Met Phe Phe Leu Asn Ile Ala Met Phe
1025                1030                1035                1040
Ile Val Val Met Val Gln Ile Cys Gly Arg Asn Gly Lys Arg Ser Asn
                1045                1050                1055
Arg Thr Leu Arg Glu Glu Val Leu Arg Asn Leu Arg Ser Val Val Ser
            1060                1065                1070
Leu Thr Phe Leu Leu Gly Met Thr Trp Gly Phe Ala Phe Phe Ala Trp
        1075                1080                1085
Gly Pro Leu Asn Ile Pro Phe Met Tyr Leu Phe Ser Ile Phe Asn Ser
    1090                1095                1100
Leu Gln Gly Lys Ile Asn Cys Thr
1105                1110

<210> 9
<211> 3750
<212> DNA
<213> homo sapiens

<400> 9
atgtttcgct cagatcgaat gtggagctgc cattggaaat ggaagcccag tcctctcctg      60
ttcttatttg ctttatatat catgtgtgtt cctcactcag tgtggggatg tgccaactgc     120
cgagtggttt tgtccaaccc ttctgggacc tttacttctc catgctaccc taacgactac     180
ccaaacagcc aggcttgcat gtggacgctc cgagccccca ccggttatat cattcagata     240
acatttaacg actttgacat tgaagaagct cccaattgca tttatgactc attatccctt     300
gataatggag agagccagac taaattttgt ggagcaactg ccaaaggcct atcatttaac     360
tcaagtgcga atgagatgca tgtgtccttt tcaagtgact ttagcatcca gaagaaaggt     420
ttcaatgcca gctacatcag agttgccgtg tccttaagga atcaaaaggt cattttaccc      480
cagacatcag atgcttacca ggtatctgtt gcaaaaagca tctctattcc agagctcagt     540
gctttcacac tctgctttga agcaaccaaa gttggccatg aagacagtga ttggacagct     600
ttctcctact caaatgcatc cttcacacaa ttgctcagtt ttggaaaggc caagagtggc     660
tactttctat ccatttctga ttcaaaatgt ttgttgaata atgcattacc tgtcaaagaa     720
aaagaagaca tttttgcaga aagctttgaa cagctctgcc ttgtttggaa taattctttg     780
ggctctattg gtgtaaattt caaaagaaac tatgaaacag ttccatgtga ttctaccatt     840
agtaaagtta ttcctgggaa tgggaaattg ttgttgggct ccaatcaaaa tgaaattgtc     900
tctctaaaag ggacatttta taactttcga ctttggaatt ttaccatgaa tgccaaaatc     960
ctctccaacc tcagctgtaa tgtgaaaggg aatgtagtcg actggcaaaa tgacttctgg    1020
aatatcccaa acctagctct gaaagctgaa agcaacctaa gctgtggttc ctacctgatc    1080
ccgctcccag cagcagaact ggccagctgt gcagacctgg ggaccctctg tcaagctact    1140
gtaaactctc ctagtactac accacccact gtcaccacta acatgcctgt tactaacaga    1200
atcgataaac aaaggaatga tggaattatc tatagaatat ccgtagtgat tcagaacatc    1260
cttcgtcacc ctgaggtaaa agtacagagc aaggtggcag aatggctcaa ttcaaccttc    1320
caaaattgga actacacggt ttatgtcgtt aatatcagtt ttcacctgag tgctggagag    1380
gacaagatta aagtcaagag aagccttgag gatgagccaa ggttggtgct ttgggccctt    1440
ctagtttaca atgctaccaa caatactaat ttagaaggaa aaatcattca gcagaagctc    1500
ctaaaaaata atgagtcctt ggatgaaggc ttgaggctac atacagtgaa tgtgagacaa    1560
ctgggtcatt gtcttgccat ggaggaaccc aaaggctact actggccatc tatccaacct    1620
tctgaatacg ttcttccttg tccagacaag cctggctttt ctgcttctcg gatatgtttt    1680
tacaatgcta ccaacccatt ggtaacctac tggggacctg ttgatatctc caactgttta    1740
aaagaagcaa atgaagttgc taaccagatt ttaaatttaa ctgctgatgg gcagaactta    1800
acctcagcca atattaccaa cattgtggaa caggtcaaaa gaattgtgaa taaagaagaa    1860
aacattgata taacacttgg ctcaactcta atgaatatat tttctaatat cttaagcagt    1920
tcagacagtg acttgcttga gtcatcttct gaagctttaa aaacaattga tgaattggcc    1980
ttcaagatag acctaaatag cacatcacat gtgaatatta caactcggaa cttggctctc    2040
agcgtatcat ccctgttacc agggacaaat gcaatttcaa attttagcat tggtcttcca    2100
agcaataatg aatcgtattt ccagatggat tttgagagtg gacaagtgga tccactggca    2160
tctgtaattt tgcctccaaa cttacttgag aatttaagtc cagaagattc tgtattagtt    2220
agaagagcac agtttacttt cttcaacaaa actggacttt tccaggatgt aggaccccaa    2280
agaaaaactt tagtgagtta tgtgatggcg tgcagtattg gaaacattac tatccagaat    2340
ctgaaggatc ctgttcaaat aaaaatcaaa catacaagaa ctcaggaagt gcatcatccc    2400

atctgtgcct tctgggatct gaacaaaaac aaaagttttg gaggatggaa cacgtcagga 2460
tgtgttgcac acagagattc agatgcaagt gagacagtct gcctgtgtaa ccacttcaca 2520
cactttggag ttctgatgga ccttccaaga agtgcctcac agttagatgc aagaaacact 2580
aaagtcctca ctttcatcag ctatattggg tgtggaatat ctgctatttt ttcagcagca 2640
actctcctga catatgttgc ttttgagaaa ttgcgaaggg attatccctc caaaatcttg 2700
atgaacctga gcacagccct gctgttcctg aatctcctct tcctcctaga tggctggatc 2760
acctccttca atgtggatgg actttgcatt gctgttgcag tcctgttgca tttcttcctt 2820
ctggcaacct ttacctggat ggggctagaa gcaattcaca tgtacattgc tctagttaaa 2880
gtatttaaca cttacattcg ccgatacatt ctaaaattct gcatcattgg ctggggtttg 2940
cctgccttag tggtgtcagt tgttctagcg agcagaaaca acaatgaagt ctatggaaaa 3000
gaaagttatg ggaaagaaaa aggtgatgaa ttctgttgga ttcaagatcc agtcatattt 3060
tatgtgacct gtgctgggta ttttggagtc atgttttttc tgaacattgc catgttcatt 3120
gtggtaatgg tgcagatctg tgggaggaat ggcaagagaa gcaaccggac cctgagagaa 3180
gaagtgttaa ggaacctgcg cagtgtggtt agcttgacct ttctgttggg catgacatgg 3240
ggttttgcat tctttgcctg gggaccctta aatatcccct tcatgtacct cttctccatc 3300
ttcaattcat tacaaggctt atttatattc atcttccact gtgctatgaa ggagaatgtt 3360
cagaaacagt ggcggcggca tctctgctgt ggtagatttc ggttagcaga taactcagat 3420
tggagtaaga cagctaccaa tatcatcaag aaaagttctg ataatctagg aaaatctttg 3480
tcttcaagct ccattggttc caactcaacc tatcttacat ccaaatctaa atccagctct 3540
accacctatt tcaaaaggaa tagccacaca gacagtgctt ccatggacaa gtccttgtca 3600
aaactggccc atgctgatgg agatcaaaca tcaatcatcc ctgtccatca ggtcattgat 3660
aaggtcaagg gttattgcaa tgctcattca gacaacttct ataaaaatat tatcatgtca 3720
gacaccttca gccacagcac aaagttttaa 3750

<210> 10
<211> 1249
<212> PRT
<213> homo sapiens

<400> 10
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Val Trp Gly Cys Ala Asn Cys Arg Val Val Leu Ser Asn Pro Ser
        35                  40                  45
Gly Thr Phe Thr Ser Pro Cys Tyr Pro Asn Asp Tyr Pro Asn Ser Gln
    50                  55                  60
Ala Cys Met Trp Thr Leu Arg Ala Pro Thr Gly Tyr Ile Ile Gln Ile
65                  70                  75                  80
Thr Phe Asn Asp Phe Asp Ile Glu Glu Ala Pro Asn Cys Ile Tyr Asp
                85                  90                  95
Ser Leu Ser Leu Asp Asn Gly Glu Ser Gln Thr Lys Phe Cys Gly Ala
            100                 105                 110
Thr Ala Lys Gly Leu Ser Phe Asn Ser Ser Ala Asn Glu Met His Val
        115                 120                 125
Ser Phe Ser Ser Asp Phe Ser Ile Gln Lys Lys Gly Phe Asn Ala Ser
    130                 135                 140
Tyr Ile Arg Val Ala Val Ser Leu Arg Asn Gln Lys Val Ile Leu Pro
145                 150                 155                 160
Gln Thr Ser Asp Ala Tyr Gln Val Ser Val Ala Lys Ser Ile Ser Ile
                165                 170                 175
Pro Glu Leu Ser Ala Phe Thr Leu Cys Phe Glu Ala Thr Lys Val Gly
            180                 185                 190
His Glu Asp Ser Asp Trp Thr Ala Phe Ser Tyr Ser Asn Ala Ser Phe
        195                 200                 205
Thr Gln Leu Leu Ser Phe Gly Lys Ala Lys Ser Gly Tyr Phe Leu Ser
    210                 215                 220
Ile Ser Asp Ser Lys Cys Leu Leu Asn Asn Ala Leu Pro Val Lys Glu
225                 230                 235                 240
Lys Glu Asp Ile Phe Ala Glu Ser Phe Glu Gln Leu Cys Leu Val Trp
                245                 250                 255
Asn Asn Ser Leu Gly Ser Ile Gly Val Asn Phe Lys Arg Asn Tyr Glu
            260                 265                 270
Thr Val Pro Cys Asp Ser Thr Ile Ser Lys Val Ile Pro Gly Asn Gly
        275                 280                 285
Lys Leu Leu Leu Gly Ser Asn Gln Asn Glu Ile Val Ser Leu Lys Gly
    290                 295                 300

Asp Ile Tyr Asn Phe Arg Leu Trp Asn Phe Thr Met Asn Ala Lys Ile
305                 310                 315                 320
Leu Ser Asn Leu Ser Cys Asn Val Lys Gly Asn Val Val Asp Trp Gln
                325                 330                 335
Asn Asp Phe Trp Asn Ile Pro Asn Leu Ala Leu Lys Ala Glu Ser Asn
            340                 345                 350
Leu Ser Cys Gly Ser Tyr Leu Ile Pro Leu Pro Ala Ala Glu Leu Ala
        355                 360                 365
Ser Cys Ala Asp Leu Gly Thr Leu Cys Gln Ala Thr Val Asn Ser Pro
    370                 375                 380
Ser Thr Thr Pro Pro Thr Val Thr Thr Asn Met Pro Val Thr Asn Arg
385                 390                 395                 400
Ile Asp Lys Gln Arg Asn Asp Gly Ile Ile Tyr Arg Ile Ser Val Val
                405                 410                 415
Ile Gln Asn Ile Leu Arg His Pro Glu Val Lys Val Gln Ser Lys Val
            420                 425                 430
Ala Glu Trp Leu Asn Ser Thr Phe Gln Asn Trp Asn Tyr Thr Val Tyr
        435                 440                 445
Val Val Asn Ile Ser Phe His Leu Ser Ala Gly Glu Asp Lys Ile Lys
    450                 455                 460
Val Lys Arg Ser Leu Glu Asp Glu Pro Arg Leu Val Leu Trp Ala Leu
465                 470                 475                 480
Leu Val Tyr Asn Ala Thr Asn Asn Thr Asn Leu Glu Gly Lys Ile Ile
                485                 490                 495
Gln Gln Lys Leu Leu Lys Asn Asn Glu Ser Leu Asp Glu Gly Leu Arg
            500                 505                 510
Leu His Thr Val Asn Val Arg Gln Leu Gly His Cys Leu Ala Met Glu
        515                 520                 525
Glu Pro Lys Gly Tyr Tyr Trp Pro Ser Ile Gln Pro Ser Glu Tyr Val
    530                 535                 540
Leu Pro Cys Pro Asp Lys Pro Gly Phe Ser Ala Ser Arg Ile Cys Phe
545                 550                 555                 560
Tyr Asn Ala Thr Asn Pro Leu Val Thr Tyr Trp Gly Pro Val Asp Ile
                565                 570                 575
Ser Asn Cys Leu Lys Glu Ala Asn Glu Val Ala Asn Gln Ile Leu Asn
            580                 585                 590
Leu Thr Ala Asp Gly Gln Asn Leu Thr Ser Ala Asn Ile Thr Asn Ile
        595                 600                 605
Val Glu Gln Val Lys Arg Ile Val Asn Lys Glu Glu Asn Ile Asp Ile
    610                 615                 620
Thr Leu Gly Ser Thr Leu Met Asn Ile Phe Ser Asn Ile Leu Ser Ser
625                 630                 635                 640
Ser Asp Ser Asp Leu Leu Glu Ser Ser Ser Glu Ala Leu Lys Thr Ile
                645                 650                 655
Asp Glu Leu Ala Phe Lys Ile Asp Leu Asn Ser Thr Ser His Val Asn
            660                 665                 670
Ile Thr Thr Arg Asn Leu Ala Leu Ser Val Ser Ser Leu Leu Pro Gly
        675                 680                 685
Thr Asn Ala Ile Ser Asn Phe Ser Ile Gly Leu Pro Ser Asn Asn Glu
    690                 695                 700
Ser Tyr Phe Gln Met Asp Phe Glu Ser Gly Gln Val Asp Pro Leu Ala
705                 710                 715                 720
Ser Val Ile Leu Pro Pro Asn Leu Leu Glu Asn Leu Ser Pro Glu Asp
                725                 730                 735
Ser Val Leu Val Arg Arg Ala Gln Phe Thr Phe Phe Asn Lys Thr Gly
            740                 745                 750
Leu Phe Gln Asp Val Gly Pro Gln Arg Lys Thr Leu Val Ser Tyr Val
        755                 760                 765
Met Ala Cys Ser Ile Gly Asn Ile Thr Ile Gln Asn Leu Lys Asp Pro
    770                 775                 780
Val Gln Ile Lys Ile Lys His Thr Arg Thr Gln Glu Val His His Pro
785                 790                 795                 800
Ile Cys Ala Phe Trp Asp Leu Asn Lys Asn Lys Ser Phe Gly Gly Trp
                805                 810                 815
Asn Thr Ser Gly Cys Val Ala His Arg Asp Ser Asp Ala Ser Glu Thr
            820                 825                 830
Val Cys Leu Cys Asn His Phe Thr His Phe Gly Val Leu Met Asp Leu
        835                 840                 845

Pro Arg Ser Ala Ser Gln Leu Asp Ala Arg Asn Thr Lys Val Leu Thr
    850                 855                 860
Phe Ile Ser Tyr Ile Gly Cys Gly Ile Ser Ala Ile Phe Ser Ala Ala
865                 870                 875                 880
Thr Leu Leu Thr Tyr Val Ala Phe Glu Lys Leu Arg Arg Asp Tyr Pro
                885                 890                 895
Ser Lys Ile Leu Met Asn Leu Ser Thr Ala Leu Leu Phe Leu Asn Leu
            900                 905                 910
Leu Phe Leu Leu Asp Gly Trp Ile Thr Ser Phe Asn Val Asp Gly Leu
        915                 920                 925
Cys Ile Ala Val Ala Val Leu Leu His Phe Phe Leu Leu Ala Thr Phe
    930                 935                 940
Thr Trp Met Gly Leu Glu Ala Ile His Met Tyr Ile Ala Leu Val Lys
945                 950                 955                 960
Val Phe Asn Thr Tyr Ile Arg Arg Tyr Ile Leu Lys Phe Cys Ile Ile
                965                 970                 975
Gly Trp Gly Leu Pro Ala Leu Val Val Ser Val Val Leu Ala Ser Arg
            980                 985                 990
Asn Asn Asn Glu Val Tyr Gly Lys Glu Ser Tyr Gly Lys Glu Lys Gly
        995                 1000                1005
Asp Glu Phe Cys Trp Ile Gln Asp Pro Val Ile Phe Tyr Val Thr Cys
    1010                1015                1020
Ala Gly Tyr Phe Gly Val Met Phe Phe Leu Asn Ile Ala Met Phe Ile
1025                1030                1035                1040
Val Val Met Val Gln Ile Cys Gly Arg Asn Gly Lys Arg Ser Asn Arg
                1045                1050                1055
Thr Leu Arg Glu Glu Val Leu Arg Asn Leu Arg Ser Val Val Ser Leu
            1060                1065                1070
Thr Phe Leu Leu Gly Met Thr Trp Gly Phe Ala Phe Phe Ala Trp Gly
        1075                1080                1085
Pro Leu Asn Ile Pro Phe Met Tyr Leu Phe Ser Ile Phe Asn Ser Leu
    1090                1095                1100
Gln Gly Leu Phe Ile Phe Ile Phe His Cys Ala Met Lys Glu Asn Val
1105                1110                1115                1120
Gln Lys Gln Trp Arg Arg His Leu Cys Cys Gly Arg Phe Arg Leu Ala
                1125                1130                1135
Asp Asn Ser Asp Trp Ser Lys Thr Ala Thr Asn Ile Ile Lys Lys Ser
            1140                1145                1150
Ser Asp Asn Leu Gly Lys Ser Leu Ser Ser Ser Ser Ile Gly Ser Asn
        1155                1160                1165
Ser Thr Tyr Leu Thr Ser Lys Ser Lys Ser Ser Ser Thr Thr Tyr Phe
    1170                1175                1180
Lys Arg Asn Ser His Thr Asp Ser Ala Ser Met Asp Lys Ser Leu Ser
1185                1190                1195                1200
Lys Leu Ala His Ala Asp Gly Asp Gln Thr Ser Ile Ile Pro Val His
                1205                1210                1215
Gln Val Ile Asp Lys Val Lys Gly Tyr Cys Asn Ala His Ser Asp Asn
            1220                1225                1230
Phe Tyr Lys Asn Ile Ile Met Ser Asp Thr Phe Ser His Ser Thr Lys
        1235                1240                1245
Phe

<210> 11
<211> 3663
<212> DNA
<213> homo sapiens

<400> 11
atgtttcgct cagatcgaat gtggagctgc cattggaaat ggaagcccag tcctctcctg      60
ttcttatttg ctttatatat catgtgtgtt cctcactcag tgtggggatg tgccaactgc     120
cgagtggttt tgtccaaccc ttctgggacc tttacttctc catgctaccc taacgactac     180
ccaaacagcc aggcttgcat gtggacgctc cgagccccca ccggttatat cattcagata     240
acatttaacg actttgacat tgaagaagct cccaattgca tttatgactc attatccctt     300
gataatggag agagccagac taaattttgt ggagcaactg ccaaaggcct atcatttaac     360
tcaagtgcga atgagatgca tgtgtccttt tcaagtgact ttagcatcca gaagaaaggt     420
ttcaatgcca gctacatcag agttgccgtg tccttaagga atcaaaaggt cattttaccc     480
cagacatcag atgcttacca ggtatctgtt gcaaaaagca tctctattcc agagctcagt     540

gctttcacac tctgctttga agcaaccaaa gttggccatg aagacagtga ttggacagct 600
ttctcctact caaatgcatc cttcacacaa ttgctcagtt ttggaaaggc caagagtggc 660
tactttctat ccatttctga ttcaaaatgt ttgttgaata atgcattacc tgtcaaagaa 720
aaagaagaca tttttgcaga aagctttgaa cagctctgcc ttgtttggaa taattctttg 780
ggctctattg gtgtaaattt caaaagaaac tatgaaacag ttccatgtga ttctaccatt 840
agtaaagtta ttcctgggaa tgggaaattg ttgttgggct ccaatcaaaa tgaaattgtc 900
tctctaaaag ggacatttta taactttcga ctttggaatt ttaccatgaa tgccaaaatc 960
ctctccaacc tcagctgtaa tgtgaaaggg aatgtagtcg actggcaaaa tgacttctgg 1020
aatatcccaa acctagctct gaaagctgaa agcaacctaa gctgtggttc ctacctgatc 1080
ccgctcccag cagcagaact ggccagctgt gcagacctgg ggaccctctg tcaagctact 1140
gtaaactctc ctagtactac accacccact gtcaccacta acatgcctgt tactaacaga 1200
atcgataaac aaaggaatga tggaattatc tatagaatat ccgtagtgat tcagaacatc 1260
cttcgtcacc ctgaggtaaa agtacagagc aaggtggcag aatggctcaa ttcaaccttc 1320
caaaattgga actacacggt ttatgtcgtt aatatcagtt ttcacctgag tgctggagag 1380
gacaagatta aagtcaagag aagccttgag gatgagccaa ggttggtgct ttgggccctt 1440
ctagtttaca atgctaccaa caatactaat ttagaaggaa aaatcattca gcagaagctc 1500
ctaaaaaata atgagtcctt ggatgaaggc ttgaggctac atacagtgaa tgtgagacaa 1560
ctgggtcatt gtcttgccat ggaggaaccc aaaggctact actggccatc tatccaacct 1620
tctgaatacg ttcttccttg tccagacaag cctggctttt ctgcttctcg gatatgtttt 1680
tacaatgcta ccaacccatt ggtaacctac tggggacctg ttgatatctc caactgttta 1740
aaagaagcaa atgaagttgc taaccagatt ttaaatttaa ctgctgatgg gcagaactta 1800
acctcagcca atattaccaa cattgtggaa caggtcaaaa gaattgtgaa taaagaagaa 1860
aacattgata taacacttgg ctcaactcta atgaatatat tttctaatat cttaagcagt 1920
tcagacagtg acttgcttga gtcatcttct gaagctttaa aaacaattga tgaattggcc 1980
ttcaagatag acctaaatag cacatcacat gtgaatatta caactcggaa cttggctctc 2040
agcgtatcat ccctgttacc agggacaaat gcaatttcaa attttagcat tggtcttcca 2100
agcaataatg aatcgtattt ccagatggat tttgagagtg gacaagtgga tccactggca 2160
tctgtaattt tgcctccaaa cttacttgag aatttaagtc cagaagattc tgtattagtt 2220
agaagagcac agtttacttt cttcaacaaa actggacttt tccaggatgt aggaccccaa 2280
agaaaaactt tagtgagtta tgtgatggcg tgcagtattg gaaacattac tatccagaat 2340
ctgaaggatc ctgttcaaat aaaaatcaaa catacaagaa ctcaggaagt gcatcatccc 2400
atctgtgcct tctgggatct gaacaaaaac aaaagttttg gaggatggaa cacgtcagga 2460
tgtgttgcac acagagattc agatgcaagt gagacagtct gcctgtgtaa ccacttcaca 2520
cactttggag ttctgatgga ccttccaaga agtgcctcac agttagatgc aagaaacact 2580
aaagtcctca ctttcatcag ctatattggg tgtggaatat ctgctatttt ttcagcagca 2640
actctcctga catatgttgc ttttgagaaa ttgcgaaggg attatccctc caaaatcttg 2700
atgaacctga gcacagccct gctgttcctg aatctcctct tcctcctaga tggctggatc 2760
acctccttca atgtggatgg actttgcatt gctgttgcag tcctgttgca tttcttcctt 2820
ctggcaacct ttacctggat ggggctagaa gcaattcaca tgtacattgc tctagttaaa 2880
gtatttaaca cttacattcg ccgatacatt ctaaaattct gcatcattgg ctggggtttg 2940
cctgccttag tggtgtcagt tgttctagcg agcagaaaca acaatgaagt ctatggaaaa 3000
gaaagttatg ggaaagaaaa aggtgatgaa ttctgttgga ttcaagatcc agtcatattt 3060
tatgtgacct gtgctgggta ttttggagtc atgttttttc tgaacattgc catgttcatt 3120
gtggtaatgg tgcagatctg tgggaggaat ggcaagagaa gcaaccggac cctgagagaa 3180
gaagtgttaa ggaacctgcg cagtgtggtt agcttgacct ttctgttggg catgacatgg 3240
ggttttgcat tctttgcctg gggaccctta aatatcccct tcatgtacct cttctccatc 3300
ttcaattcat tacaaggctt atttatattc atcttccact gtgctatgaa ggagaatgtt 3360
cagaaacagt ggcggcggca tctctgctgt ggtagatttc ggttagcaga taactcagat 3420
tggagtaaga cagctaccaa tatcatcaag aaaagttctg ataatctagg aaaatctttg 3480
tcttcaagct ccattggttc caactcaacc tatcttacat ccaaatctaa atccagctct 3540
accacctatt tcaaaaggaa tagccacaca gataatgtct cctatgagca ttccttcaac 3600
aaaagtggat cactcagaca gtgcttccat ggacaagtcc ttgtcaaaac tggcccatgc 3660
tga 3663

<210> 12
<211> 1220
<212> PRT
<213> homo sapiens

<400> 12
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Val Trp Gly Cys Ala Asn Cys Arg Val Val Leu Ser Asn Pro Ser
        35                  40                  45
Gly Thr Phe Thr Ser Pro Cys Tyr Pro Asn Asp Tyr Pro Asn Ser Gln
    50                  55                  60

Ala Cys Met Trp Thr Leu Arg Ala Pro Thr Gly Tyr Ile Ile Gln Ile
65                  70                  75                  80
Thr Phe Asn Asp Phe Asp Ile Glu Glu Ala Pro Asn Cys Ile Tyr Asp
                85                  90                  95
Ser Leu Ser Leu Asp Asn Gly Glu Ser Gln Thr Lys Phe Cys Gly Ala
            100                 105                 110
Thr Ala Lys Gly Leu Ser Phe Asn Ser Ser Ala Asn Glu Met His Val
        115                 120                 125
Ser Phe Ser Ser Asp Phe Ser Ile Gln Lys Lys Gly Phe Asn Ala Ser
    130                 135                 140
Tyr Ile Arg Val Ala Val Ser Leu Arg Asn Gln Lys Val Ile Leu Pro
145                 150                 155                 160
Gln Thr Ser Asp Ala Tyr Gln Val Ser Val Ala Lys Ser Ile Ser Ile
                165                 170                 175
Pro Glu Leu Ser Ala Phe Thr Leu Cys Phe Glu Ala Thr Lys Val Gly
            180                 185                 190
His Glu Asp Ser Asp Trp Thr Ala Phe Ser Tyr Ser Asn Ala Ser Phe
        195                 200                 205
Thr Gln Leu Leu Ser Phe Gly Lys Ala Lys Ser Gly Tyr Phe Leu Ser
    210                 215                 220
Ile Ser Asp Ser Lys Cys Leu Leu Asn Asn Ala Leu Pro Val Lys Glu
225                 230                 235                 240
Lys Glu Asp Ile Phe Ala Glu Ser Phe Glu Gln Leu Cys Leu Val Trp
                245                 250                 255
Asn Asn Ser Leu Gly Ser Ile Gly Val Asn Phe Lys Arg Asn Tyr Glu
            260                 265                 270
Thr Val Pro Cys Asp Ser Thr Ile Ser Lys Val Ile Pro Gly Asn Gly
        275                 280                 285
Lys Leu Leu Leu Gly Ser Asn Gln Asn Glu Ile Val Ser Leu Lys Gly
    290                 295                 300
Asp Ile Tyr Asn Phe Arg Leu Trp Asn Phe Thr Met Asn Ala Lys Ile
305                 310                 315                 320
Leu Ser Asn Leu Ser Cys Asn Val Lys Gly Asn Val Val Asp Trp Gln
                325                 330                 335
Asn Asp Phe Trp Asn Ile Pro Asn Leu Ala Leu Lys Ala Glu Ser Asn
            340                 345                 350
Leu Ser Cys Gly Ser Tyr Leu Ile Pro Leu Pro Ala Ala Glu Leu Ala
        355                 360                 365
Ser Cys Ala Asp Leu Gly Thr Leu Cys Gln Ala Thr Val Asn Ser Pro
    370                 375                 380
Ser Thr Thr Pro Pro Thr Val Thr Thr Asn Met Pro Val Thr Asn Arg
385                 390                 395                 400
Ile Asp Lys Gln Arg Asn Asp Gly Ile Ile Tyr Arg Ile Ser Val Val
                405                 410                 415
Ile Gln Asn Ile Leu Arg His Pro Glu Val Lys Val Gln Ser Lys Val
            420                 425                 430
Ala Glu Trp Leu Asn Ser Thr Phe Gln Asn Trp Asn Tyr Thr Val Tyr
        435                 440                 445
Val Val Asn Ile Ser Phe His Leu Ser Ala Gly Glu Asp Lys Ile Lys
    450                 455                 460
Val Lys Arg Ser Leu Glu Asp Glu Pro Arg Leu Val Leu Trp Ala Leu
465                 470                 475                 480
Leu Val Tyr Asn Ala Thr Asn Asn Thr Asn Leu Glu Gly Lys Ile Ile
                485                 490                 495
Gln Gln Lys Leu Leu Lys Asn Asn Glu Ser Leu Asp Glu Gly Leu Arg
            500                 505                 510
Leu His Thr Val Asn Val Arg Gln Leu Gly His Cys Leu Ala Met Glu
        515                 520                 525
Glu Pro Lys Gly Tyr Tyr Trp Pro Ser Ile Gln Pro Ser Glu Tyr Val
    530                 535                 540
Leu Pro Cys Pro Asp Lys Pro Gly Phe Ser Ala Ser Arg Ile Cys Phe
545                 550                 555                 560
Tyr Asn Ala Thr Asn Pro Leu Val Thr Tyr Trp Gly Pro Val Asp Ile
                565                 570                 575
Ser Asn Cys Leu Lys Glu Ala Asn Glu Val Ala Asn Gln Ile Leu Asn
            580                 585                 590
Leu Thr Ala Asp Gly Gln Asn Leu Thr Ser Ala Asn Ile Thr Asn Ile
        595                 600                 605

Val Glu Gln Val Lys Arg Ile Val Asn Lys Glu Glu Asn Ile Asp Ile
    610                 615                 620
Thr Leu Gly Ser Thr Leu Met Asn Ile Phe Ser Asn Ile Leu Ser Ser
625                 630                 635                 640
Ser Asp Ser Asp Leu Leu Glu Ser Ser Ser Glu Ala Leu Lys Thr Ile
                645                 650                 655
Asp Glu Leu Ala Phe Lys Ile Asp Leu Asn Ser Thr Ser His Val Asn
            660                 665                 670
Ile Thr Thr Arg Asn Leu Ala Leu Ser Val Ser Ser Leu Leu Pro Gly
        675                 680                 685
Thr Asn Ala Ile Ser Asn Phe Ser Ile Gly Leu Pro Ser Asn Asn Glu
    690                 695                 700
Ser Tyr Phe Gln Met Asp Phe Glu Ser Gly Gln Val Asp Pro Leu Ala
705                 710                 715                 720
Ser Val Ile Leu Pro Pro Asn Leu Leu Glu Asn Leu Ser Pro Glu Asp
                725                 730                 735
Ser Val Leu Val Arg Arg Ala Gln Phe Thr Phe Phe Asn Lys Thr Gly
            740                 745                 750
Leu Phe Gln Asp Val Gly Pro Gln Arg Lys Thr Leu Val Ser Tyr Val
        755                 760                 765
Met Ala Cys Ser Ile Gly Asn Ile Thr Ile Gln Asn Leu Lys Asp Pro
    770                 775                 780
Val Gln Ile Lys Ile Lys His Thr Arg Thr Gln Glu Val His His Pro
785                 790                 795                 800
Ile Cys Ala Phe Trp Asp Leu Asn Lys Asn Lys Ser Phe Gly Gly Trp
                805                 810                 815
Asn Thr Ser Gly Cys Val Ala His Arg Asp Ser Asp Ala Ser Glu Thr
            820                 825                 830
Val Cys Leu Cys Asn His Phe Thr His Phe Gly Val Leu Met Asp Leu
        835                 840                 845
Pro Arg Ser Ala Ser Gln Leu Asp Ala Arg Asn Thr Lys Val Leu Thr
    850                 855                 860
Phe Ile Ser Tyr Ile Gly Cys Gly Ile Ser Ala Ile Phe Ser Ala Ala
865                 870                 875                 880
Thr Leu Leu Thr Tyr Val Ala Phe Glu Lys Leu Arg Arg Asp Tyr Pro
                885                 890                 895
Ser Lys Ile Leu Met Asn Leu Ser Thr Ala Leu Leu Phe Leu Asn Leu
            900                 905                 910
Leu Phe Leu Leu Asp Gly Trp Ile Thr Ser Phe Asn Val Asp Gly Leu
        915                 920                 925
Cys Ile Ala Val Ala Val Leu Leu His Phe Phe Leu Leu Ala Thr Phe
    930                 935                 940
Thr Trp Met Gly Leu Glu Ala Ile His Met Tyr Ile Ala Leu Val Lys
945                 950                 955                 960
Val Phe Asn Thr Tyr Ile Arg Arg Tyr Ile Leu Lys Phe Cys Ile Ile
                965                 970                 975
Gly Trp Gly Leu Pro Ala Leu Val Val Ser Val Val Leu Ala Ser Arg
            980                 985                 990
Asn Asn Asn Glu Val Tyr Gly Lys Glu Ser Tyr Gly Lys Glu Lys Gly
        995                 1000                1005
Asp Glu Phe Cys Trp Ile Gln Asp Pro Val Ile Phe Tyr Val Thr Cys
    1010                1015                1020
Ala Gly Tyr Phe Gly Val Met Phe Phe Leu Asn Ile Ala Met Phe Ile
1025                1030                1035                1040
Val Val Met Val Gln Ile Cys Gly Arg Asn Gly Lys Arg Ser Asn Arg
                1045                1050                1055
Thr Leu Arg Glu Glu Val Leu Arg Asn Leu Arg Ser Val Val Ser Leu
            1060                1065                1070
Thr Phe Leu Leu Gly Met Thr Trp Gly Phe Ala Phe Phe Ala Trp Gly
        1075                1080                1085
Pro Leu Asn Ile Pro Phe Met Tyr Leu Phe Ser Ile Phe Asn Ser Leu
    1090                1095                1100
Gln Gly Leu Phe Ile Phe Ile Phe His Cys Ala Met Lys Glu Asn Val
1105                1110                1115                1120
Gln Lys Gln Trp Arg Arg His Leu Cys Cys Gly Arg Phe Arg Leu Ala
                1125                1130                1135
Asp Asn Ser Asp Trp Ser Lys Thr Ala Thr Asn Ile Ile Lys Lys Ser
            1140                1145                1150

Ser Asp Asn Leu Gly Lys Ser Leu Ser Ser Ser Ser Ile Gly Ser Asn
        1155                1160                1165
Ser Thr Tyr Leu Thr Ser Lys Ser Lys Ser Ser Ser Thr Thr Tyr Phe
    1170                1175                1180
Lys Arg Asn Ser His Thr Asp Asn Val Ser Tyr Glu His Ser Phe Asn
1185                1190                1195                1200
Lys Ser Gly Ser Leu Arg Gln Cys Phe His Gly Gln Val Leu Val Lys
                1205                1210                1215
Thr Gly Pro Cys
            1220

<210> 13
<211> 2154
<212> DNA
<213> homo sapiens

<400> 13
atgtttcgct cagatcgaat gtggagctgc cattggaaat ggaagcccag tcctctcctg      60
ttcttatttg ctttatatat catgtgtgtt cctcactcag tgtggggatg tgccaactgc     120
cgagtggttt tgtccaaccc ttctgggacc tttacttctc catgctaccc taacgactac     180
ccaaacagcc aggcttgcat gtggacgctc cgagccccca ccggttatat cattcagata     240
acatttaacg actttgacat tgaagaagct cccaattgca tttatgactc attatccctt     300
gataatggag agagccagac taaattttgt ggagcaactg ccaaaggcct atcatttaac     360
tcaagtgcga atgagatgca tgtgtccttt tcaagtgact ttagcatcca gaagaaaggt     420
ttcaatgcca gctacatcag agttgccgtg tccttaagga atcaaaaggt cattttaccc      480
cagacatcag atgcttacca ggtatctgtt gcaaaaagca tctctattcc agagctcagt     540
gctttcacac tctgctttga agcaaccaaa gttggccatg aagacagtga ttggacagct     600
ttctcctact caaatgcatc cttcacacaa ttgctcagtt ttggaaaggc caagagtggc     660
tactttctat ccatttctga ttcaaaatgt ttgttgaata atgcattacc tgtcaaagaa     720
aaagaagaca tttttgcaga aagctttgaa cagctctgcc ttgtttggaa taattctttg     780
ggctctattg gtgtaaattt caaaagaaac tatgaaacag ttccatgtga ttctaccatt     840
agtaaagtta ttcctgggaa tgggaaattg ttgttgggct ccaatcaaaa tgaaattgtc     900
tctctaaaag gggacattta taactttcga ctttggaatt ttaccatgaa tgccaaaatc     960
ctctccaacc tcagctgtaa tgtgaaaggg aatgtagtcg actggcaaaa tgacttctgg    1020
aatatcccaa acctagctct gaaagctgaa agcaacctaa gctgtggttc ctacctgatc    1080
ccgctcccag cagcagaact ggccagctgt gcagacctgg ggaccctctg tcaagctact    1140
gtaaactctc ctagtactac accacccact gtcaccacta acatgcctgt tactaacaga    1200
atcgataaac aaaggaatga tggaattatc tatagaatat ccgtagtgat tcagaacatc    1260
cttcgtcacc ctgaggtaaa agtacagagc aaggtggcag aatggctcaa ttcaaccttc    1320
caaaattgga actacacggt ttatgtcgtt aatatcagtt ttcacctgag tgctggagag    1380
gacaagatta aagtcaagag aagccttgag gatgagccaa ggttggtgct ttgggccctt    1440
ctagtttaca atgctaccaa caatactaat ttagaaggaa aaatcattca gcagaagctc    1500
ctaaaaaata atgagtcctt ggatgaaggc ttgaggctac atacagtgaa tgtgagacaa    1560
ctgggtcatt gtcttgccat ggaggaaccc aaaggctact actggccatc tatccaacct    1620
tctgaatacg ttcttccttg tccagacaag cctggctttt ctgcttctcg gatatgtttt    1680
tacaatgcta ccaacccatt ggtaacctac tggggacctg ttgatatctc caactgttta    1740
aaagaagcaa atgaagttgc taaccagatt ttaaatttaa ctgctgatgg gcagaactta    1800
acctcagcca atattaccaa cattgtggaa caggtcaaaa gaattgtgaa taaagaagaa    1860
aacattgata taacacttgg ctcaactcta atgaatatat tttctaatat cttaagcagt    1920
tcagacagtg acttgcttga gtcatcttct gaagctttaa aaacaattga tgaattggcc    1980
ttcaagatag acctaaatag cacatcacat gtgaatatta caactcggaa cttggctctc    2040
agcgtatcat ccctgttacc agggacaaat gcaatttcaa attttagcat tggtcttcca    2100
agcaataatg aatcgtattt ccaggtaatg agccagtggt ttctttcatt ttaa          2154

<210> 14
<211> 717
<212> PRT
<213> homo sapiens

<400> 14
Met Phe Arg Ser Asp Arg Met Trp Ser Cys His Trp Lys Trp Lys Pro
 1               5                  10                  15
Ser Pro Leu Leu Phe Leu Phe Ala Leu Tyr Ile Met Cys Val Pro His
            20                  25                  30
Ser Val Trp Gly Cys Ala Asn Cys Arg Val Val Leu Ser Asn Pro Ser
        35                  40                  45
Gly Thr Phe Thr Ser Pro Cys Tyr Pro Asn Asp Tyr Pro Asn Ser Gln
    50                  55                  60

Ala Cys Met Trp Thr Leu Arg Ala Pro Thr Gly Tyr Ile Ile Gln Ile
65                  70                  75                  80
Thr Phe Asn Asp Phe Asp Ile Glu Glu Ala Pro Asn Cys Ile Tyr Asp
                85                  90                  95
Ser Leu Ser Leu Asp Asn Gly Glu Ser Gln Thr Lys Phe Cys Gly Ala
            100                 105                 110
Thr Ala Lys Gly Leu Ser Phe Asn Ser Ser Ala Asn Glu Met His Val
        115                 120                 125
Ser Phe Ser Ser Asp Phe Ser Ile Gln Lys Lys Gly Phe Asn Ala Ser
    130                 135                 140
Tyr Ile Arg Val Ala Val Ser Leu Arg Asn Gln Lys Val Ile Leu Pro
145                 150                 155                 160
Gln Thr Ser Asp Ala Tyr Gln Val Ser Val Ala Lys Ser Ile Ser Ile
                165                 170                 175
Pro Glu Leu Ser Ala Phe Thr Leu Cys Phe Glu Ala Thr Lys Val Gly
            180                 185                 190
His Glu Asp Ser Asp Trp Thr Ala Phe Ser Tyr Ser Asn Ala Ser Phe
        195                 200                 205
Thr Gln Leu Leu Ser Phe Gly Lys Ala Lys Ser Gly Tyr Phe Leu Ser
    210                 215                 220
Ile Ser Asp Ser Lys Cys Leu Leu Asn Asn Ala Leu Pro Val Lys Glu
225                 230                 235                 240
Lys Glu Asp Ile Phe Ala Glu Ser Phe Glu Gln Leu Cys Leu Val Trp
                245                 250                 255
Asn Asn Ser Leu Gly Ser Ile Gly Val Asn Phe Lys Arg Asn Tyr Glu
            260                 265                 270
Thr Val Pro Cys Asp Ser Thr Ile Ser Lys Val Ile Pro Gly Asn Gly
        275                 280                 285
Lys Leu Leu Leu Gly Ser Asn Gln Asn Glu Ile Val Ser Leu Lys Gly
    290                 295                 300
Asp Ile Tyr Asn Phe Arg Leu Trp Asn Phe Thr Met Asn Ala Lys Ile
305                 310                 315                 320
Leu Ser Asn Leu Ser Cys Asn Val Lys Gly Asn Val Val Asp Trp Gln
                325                 330                 335
Asn Asp Phe Trp Asn Ile Pro Asn Leu Ala Leu Lys Ala Glu Ser Asn
            340                 345                 350
Leu Ser Cys Gly Ser Tyr Leu Ile Pro Leu Pro Ala Ala Glu Leu Ala
        355                 360                 365
Ser Cys Ala Asp Leu Gly Thr Leu Cys Gln Ala Thr Val Asn Ser Pro
    370                 375                 380
Ser Thr Thr Pro Pro Thr Val Thr Thr Asn Met Pro Val Thr Asn Arg
385                 390                 395                 400
Ile Asp Lys Gln Arg Asn Asp Gly Ile Ile Tyr Arg Ile Ser Val Val
                405                 410                 415
Ile Gln Asn Ile Leu Arg His Pro Glu Val Lys Val Gln Ser Lys Val
            420                 425                 430
Ala Glu Trp Leu Asn Ser Thr Phe Gln Asn Trp Asn Tyr Thr Val Tyr
        435                 440                 445
Val Val Asn Ile Ser Phe His Leu Ser Ala Gly Glu Asp Lys Ile Lys
    450                 455                 460
Val Lys Arg Ser Leu Glu Asp Glu Pro Arg Leu Val Leu Trp Ala Leu
465                 470                 475                 480
Leu Val Tyr Asn Ala Thr Asn Asn Thr Asn Leu Glu Gly Lys Ile Ile
                485                 490                 495
Gln Gln Lys Leu Leu Lys Asn Asn Glu Ser Leu Asp Glu Gly Leu Arg
            500                 505                 510
Leu His Thr Val Asn Val Arg Gln Leu Gly His Cys Leu Ala Met Glu
        515                 520                 525
Glu Pro Lys Gly Tyr Tyr Trp Pro Ser Ile Gln Pro Ser Glu Tyr Val
    530                 535                 540
Leu Pro Cys Pro Asp Lys Pro Gly Phe Ser Ala Ser Arg Ile Cys Phe
545                 550                 555                 560
Tyr Asn Ala Thr Asn Pro Leu Val Thr Tyr Trp Gly Pro Val Asp Ile
                565                 570                 575
Ser Asn Cys Leu Lys Glu Ala Asn Glu Val Ala Asn Gln Ile Leu Asn
            580                 585                 590
Leu Thr Ala Asp Gly Gln Asn Leu Thr Ser Ala Asn Ile Thr Asn Ile
        595                 600                 605

Val Glu Gln Val Lys Arg Ile Val Asn Lys Glu Glu Asn Ile Asp Ile
    610                 615                 620
Thr Leu Gly Ser Thr Leu Met Asn Ile Phe Ser Asn Ile Leu Ser Ser
625                 630                 635                 640
Ser Asp Ser Asp Leu Leu Glu Ser Ser Ser Glu Ala Leu Lys Thr Ile
                645                 650                 655
Asp Glu Leu Ala Phe Lys Ile Asp Leu Asn Ser Thr Ser His Val Asn
            660                 665                 670
Ile Thr Thr Arg Asn Leu Ala Leu Ser Val Ser Ser Leu Leu Pro Gly
        675                 680                 685
Thr Asn Ala Ile Ser Asn Phe Ser Ile Gly Leu Pro Ser Asn Asn Glu
    690                 695                 700
Ser Tyr Phe Gln Val Met Ser Gln Trp Phe Leu Ser Phe
705                 710                 715

<210> 15
<211> 3336
<212> DNA
<213> homo sapiens

<400> 15
atgtttcgct cagatcgaat gtggagctgc cattggaaat ggaagcccag tcctctcctg      60
ttcttatttg ctttatatat catgtgtgtt cctcactcag tgtggggatg tgccaactgc     120
cgagtggttt tgtccaaccc ttctgggacc tttacttctc catgctaccc taacgactac     180
ccaaacagcc aggcttgcat gtggacgctc cgagccccca ccggttatat cattcagata     240
acatttaacg actttgacat tgaagaagct cccaattgca tttatgactc attatccctt     300
gataatggag agagccagac taaattttgt ggagcaactg ccaaaggcct atcatttaac     360
tcaagtgcga atgagatgca tgtgtccttt tcaagtgact ttagcatcca gaagaaaggt     420
ttcaatgcca gctacatcag agttgccgtg tccttaagga atcaaaaggt cattttaccc     480
cagacatcag atgcttacca ggtatctgtt gcaaaaagca tctctattcc agagctcagt     540
gctttcacac tctgctttga agcaaccaaa gttggccatg aagacagtga ttggacagct     600
ttctcctact caaatgcatc cttcacacaa ttgctcagtt ttggaaaggc caagagtggc     660
tactttctat ccatttctga ttcaaaatgt ttgttgaata atgcattacc tgtcaaagaa     720
aaagaagaca tttttgcaga aagctttgaa cagctctgcc ttgtttggaa taattctttg     780
ggctctattg gtgtaaattt caaaagaaac tatgaaacag ttccatgtga ttctaccatt     840
agtaaagtta ttcctgggaa tgggaaattg ttgttgggct ccaatcaaaa tgaaattgtc     900
tctctaaaag ggacatttta taactttcga ctttggaatt ttaccatgaa tgccaaaatc     960
ctctccaacc tcagctgtaa tgtgaaaggg aatgtagtcg actggcaaaa tgacttctgg    1020
aatatcccaa acctagctct gaaagctgaa agcaacctaa gctgtggttc ctacctgatc    1080
ccgctcccag cagcagaact ggccagctgt gcagacctgg ggaccctctg tcaagctact    1140
gtaaactctc ctagtactac accacccact gtcaccacta acatgcctgt tactaacaga    1200
atcgataaac aaaggaatga tggaattatc tatagaatat ccgtagtgat tcagaacatc    1260
cttcgtcacc ctgaggtaaa agtacagagc aaggtggcag aatggctcaa ttcaaccttc    1320
caaaattgga actacacggt ttatgtcgtt aatatcagtt ttcacctgag tgctggagag    1380
gacaagatta aagtcaagag aagccttgag gatgagccaa ggttggtgct ttgggccctt    1440
ctagtttaca atgctaccaa caatactaat ttagaaggaa aaatcattca gcagaagctc    1500
ctaaaaaata atgagtcctt ggatgaaggc ttgaggctac atacagtgaa tgtgagacaa    1560
ctgggtcatt gtcttgccat ggaggaaccc aaaggctact actggccatc tatccaacct    1620
tctgaatacg ttcttccttg tccagacaag cctggctttt ctgcttctcg gatatgtttt    1680
tacaatgcta ccaacccatt ggtaacctac tggggacctg ttgatatctc caactgttta    1740
aaagaagcaa atgaagttgc taaccagatt ttaaatttaa ctgctgatgg gcagaactta    1800
acctcagcca atattaccaa cattgtggaa caggtcaaaa gaattgtgaa taaagaagaa    1860
aacattgata taacacttgg ctcaactcta atgaatatat tttctaatat cttaagcagt    1920
tcagacagtg acttgcttga gtcatcttct gaagctttaa aaacaattga tgaattggcc    1980
ttcaagatag acctaaatag cacatcacat gtgaatatta caactcggaa cttggctctc    2040
agcgtatcat ccctgttacc agggacaaat gcaatttcaa attttagcat tggtcttcca    2100
agcaataatg aatcgtattt ccagatggat tttgagagtg gacaagtgga tccactggca    2160
tctgtaattt tgcctccaaa cttacttgag aatttaagtc cagaagattc tgtattagtt    2220
agaagagcac agtttacttt cttcaacaaa actggacttt tccaggatgt aggaccccaa    2280
agaaaaactt tagtgagtta tgtgatggcg tgcagtattg gaaacattac tatccagaat    2340
ctgaaggatc ctgttcaaat aaaaatcaaa catacaagaa ctcaggaagt gcatcatccc    2400
atctgtgcct tctgggatct gaacaaaaac aaaagttttg gaggatggaa cacgtcagga    2460
tgtgttgcac acagagattc agatgcaagt gagacagtct gcctgtgtaa ccacttcaca    2520
cactttggag ttctgatgga ccttccaaga agtgcctcac agttagatgc aagaaacact    2580
aaagtcctca ctttcatcag ctatattggg tgtggaatat ctgctatttt ttcagcagca    2640
actctcctga catatgttgc ttttgagaaa ttgcgaaggg attatccctc caaaatcttg    2700
atgaacctga gcacagccct gctgttcctg aatctcctct tcctcctaga tggctggatc    2760
acctccttca atgtggatgg actttgcatt gctgttgcag tcctgttgca tttcttcctt    2820

ctggcaacct ttacctggat ggggctagaa gcaattcaca tgtacattgc tctagttaaa 2880
gtatttaaca cttacattcg ccgatacatt ctaaaattct gcatcattgg ctggggtttg 2940
cctgccttag tggtgtcagt tgttctagcg agcagaaaca acaatgaagt ctatggaaaa 3000
gaaagttatg ggaaagaaaa aggtgatgaa ttctgttgga ttcaagatcc agtcatattt 3060
tatgtgacct gtgctgggta ttttggagtc atgttttttc tgaacattgc catgttcatt 3120
gtggtaatgg tgcagatctg tgggaggaat ggcaagagaa gcaaccggac cctgagagaa 3180
gaagtgttaa ggaacctgcg cagtgtggtt agcttgacct ttctgttggg catgacatgg 3240
ggttttgcat tctttgcctg gggaccctta aatatcccct tcatgtacct cttctccatc 3300
ttcaattcat tacaaggtaa gataaattgt acatga 3336

<210> 16
<211> 1111
<212> PRT
<213> homo sapiens

<400> 16
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Val Trp Gly Cys Ala Asn Cys Arg Val Val Leu Ser Asn Pro Ser
        35                  40                  45
Gly Thr Phe Thr Ser Pro Cys Tyr Pro Asn Asp Tyr Pro Asn Ser Gln
    50                  55                  60
Ala Cys Met Trp Thr Leu Arg Ala Pro Thr Gly Tyr Ile Ile Gln Ile
65                  70                  75                  80
Thr Phe Asn Asp Phe Asp Ile Glu Glu Ala Pro Asn Cys Ile Tyr Asp
                85                  90                  95
Ser Leu Ser Leu Asp Asn Gly Glu Ser Gln Thr Lys Phe Cys Gly Ala
            100                 105                 110
Thr Ala Lys Gly Leu Ser Phe Asn Ser Ser Ala Asn Glu Met His Val
        115                 120                 125
Ser Phe Ser Ser Asp Phe Ser Ile Gln Lys Lys Gly Phe Asn Ala Ser
    130                 135                 140
Tyr Ile Arg Val Ala Val Ser Leu Arg Asn Gln Lys Val Ile Leu Pro
145                 150                 155                 160
Gln Thr Ser Asp Ala Tyr Gln Val Ser Val Ala Lys Ser Ile Ser Ile
                165                 170                 175
Pro Glu Leu Ser Ala Phe Thr Leu Cys Phe Glu Ala Thr Lys Val Gly
            180                 185                 190
His Glu Asp Ser Asp Trp Thr Ala Phe Ser Tyr Ser Asn Ala Ser Phe
        195                 200                 205
Thr Gln Leu Leu Ser Phe Gly Lys Ala Lys Ser Gly Tyr Phe Leu Ser
    210                 215                 220
Ile Ser Asp Ser Lys Cys Leu Leu Asn Asn Ala Leu Pro Val Lys Glu
225                 230                 235                 240
Lys Glu Asp Ile Phe Ala Glu Ser Phe Glu Gln Leu Cys Leu Val Trp
                245                 250                 255
Asn Asn Ser Leu Gly Ser Ile Gly Val Asn Phe Lys Arg Asn Tyr Glu
            260                 265                 270
Thr Val Pro Cys Asp Ser Thr Ile Ser Lys Val Ile Pro Gly Asn Gly
        275                 280                 285
Lys Leu Leu Leu Gly Ser Asn Gln Asn Glu Ile Val Ser Leu Lys Gly
    290                 295                 300
Asp Ile Tyr Asn Phe Arg Leu Trp Asn Phe Thr Met Asn Ala Lys Ile
305                 310                 315                 320
Leu Ser Asn Leu Ser Cys Asn Val Lys Gly Asn Val Val Asp Trp Gln
                325                 330                 335
Asn Asp Phe Trp Asn Ile Pro Asn Leu Ala Leu Lys Ala Glu Ser Asn
            340                 345                 350
Leu Ser Cys Gly Ser Tyr Leu Ile Pro Leu Pro Ala Ala Glu Leu Ala
        355                 360                 365
Ser Cys Ala Asp Leu Gly Thr Leu Cys Gln Ala Thr Val Asn Ser Pro
    370                 375                 380
Ser Thr Thr Pro Pro Thr Val Thr Thr Asn Met Pro Val Thr Asn Arg
385                 390                 395                 400
Ile Asp Lys Gln Arg Asn Asp Gly Ile Ile Tyr Arg Ile Ser Val Val
                405                 410                 415

Ile Gln Asn Ile Leu Arg His Pro Glu Val Lys Val Gln Ser Lys Val
            420                 425                 430
Ala Glu Trp Leu Asn Ser Thr Phe Gln Asn Trp Asn Tyr Thr Val Tyr
        435                 440                 445
Val Val Asn Ile Ser Phe His Leu Ser Ala Gly Glu Asp Lys Ile Lys
    450                 455                 460
Val Lys Arg Ser Leu Glu Asp Glu Pro Arg Leu Val Leu Trp Ala Leu
465                 470                 475                 480
Leu Val Tyr Asn Ala Thr Asn Asn Thr Asn Leu Glu Gly Lys Ile Ile
                485                 490                 495
Gln Gln Lys Leu Leu Lys Asn Asn Glu Ser Leu Asp Glu Gly Leu Arg
            500                 505                 510
Leu His Thr Val Asn Val Arg Gln Leu Gly His Cys Leu Ala Met Glu
        515                 520                 525
Glu Pro Lys Gly Tyr Tyr Trp Pro Ser Ile Gln Pro Ser Glu Tyr Val
    530                 535                 540
Leu Pro Cys Pro Asp Lys Pro Gly Phe Ser Ala Ser Arg Ile Cys Phe
545                 550                 555                 560
Tyr Asn Ala Thr Asn Pro Leu Val Thr Tyr Trp Gly Pro Val Asp Ile
                565                 570                 575
Ser Asn Cys Leu Lys Glu Ala Asn Glu Val Ala Asn Gln Ile Leu Asn
            580                 585                 590
Leu Thr Ala Asp Gly Gln Asn Leu Thr Ser Ala Asn Ile Thr Asn Ile
        595                 600                 605
Val Glu Gln Val Lys Arg Ile Val Asn Lys Glu Glu Asn Ile Asp Ile
    610                 615                 620
Thr Leu Gly Ser Thr Leu Met Asn Ile Phe Ser Asn Ile Leu Ser Ser
625                 630                 635                 640
Ser Asp Ser Asp Leu Leu Glu Ser Ser Ser Glu Ala Leu Lys Thr Ile
                645                 650                 655
Asp Glu Leu Ala Phe Lys Ile Asp Leu Asn Ser Thr Ser His Val Asn
            660                 665                 670
Ile Thr Thr Arg Asn Leu Ala Leu Ser Val Ser Ser Leu Leu Pro Gly
        675                 680                 685
Thr Asn Ala Ile Ser Asn Phe Ser Ile Gly Leu Pro Ser Asn Asn Glu
    690                 695                 700
Ser Tyr Phe Gln Met Asp Phe Glu Ser Gly Gln Val Asp Pro Leu Ala
705                 710                 715                 720
Ser Val Ile Leu Pro Pro Asn Leu Leu Glu Asn Leu Ser Pro Glu Asp
                725                 730                 735
Ser Val Leu Val Arg Arg Ala Gln Phe Thr Phe Phe Asn Lys Thr Gly
            740                 745                 750
Leu Phe Gln Asp Val Gly Pro Gln Arg Lys Thr Leu Val Ser Tyr Val
        755                 760                 765
Met Ala Cys Ser Ile Gly Asn Ile Thr Ile Gln Asn Leu Lys Asp Pro
    770                 775                 780
Val Gln Ile Lys Ile Lys His Thr Arg Thr Gln Glu Val His His Pro
785                 790                 795                 800
Ile Cys Ala Phe Trp Asp Leu Asn Lys Asn Lys Ser Phe Gly Gly Trp
                805                 810                 815
Asn Thr Ser Gly Cys Val Ala His Arg Asp Ser Asp Ala Ser Glu Thr
            820                 825                 830
Val Cys Leu Cys Asn His Phe Thr His Phe Gly Val Leu Met Asp Leu
        835                 840                 845
Pro Arg Ser Ala Ser Gln Leu Asp Ala Arg Asn Thr Lys Val Leu Thr
    850                 855                 860
Phe Ile Ser Tyr Ile Gly Cys Gly Ile Ser Ala Ile Phe Ser Ala Ala
865                 870                 875                 880
Thr Leu Leu Thr Tyr Val Ala Phe Glu Lys Leu Arg Arg Asp Tyr Pro
                885                 890                 895
Ser Lys Ile Leu Met Asn Leu Ser Thr Ala Leu Leu Phe Leu Asn Leu
            900                 905                 910
Leu Phe Leu Leu Asp Gly Trp Ile Thr Ser Phe Asn Val Asp Gly Leu
        915                 920                 925
Cys Ile Ala Val Ala Val Leu Leu His Phe Phe Leu Leu Ala Thr Phe
    930                 935                 940
Thr Trp Met Gly Leu Glu Ala Ile His Met Tyr Ile Ala Leu Val Lys
945                 950                 955                 960

Val Phe Asn Thr Tyr Ile Arg Arg Tyr Ile Leu Lys Phe Cys Ile Ile
                965                 970                 975
Gly Trp Gly Leu Pro Ala Leu Val Val Ser Val Val Leu Ala Ser Arg
            980                 985                 990
Asn Asn Asn Glu Val Tyr Gly Lys Glu Ser Tyr Gly Lys Glu Lys Gly
        995                 1000                1005
Asp Glu Phe Cys Trp Ile Gln Asp Pro Val Ile Phe Tyr Val Thr Cys
    1010                1015                1020
Ala Gly Tyr Phe Gly Val Met Phe Phe Leu Asn Ile Ala Met Phe Ile
1025                1030                1035                1040
Val Val Met Val Gln Ile Cys Gly Arg Asn Gly Lys Arg Ser Asn Arg
                1045                1050                1055
Thr Leu Arg Glu Glu Val Leu Arg Asn Leu Arg Ser Val Val Ser Leu
            1060                1065                1070
Thr Phe Leu Leu Gly Met Thr Trp Gly Phe Ala Phe Phe Ala Trp Gly
        1075                1080                1085
Pro Leu Asn Ile Pro Phe Met Tyr Leu Phe Ser Ile Phe Asn Ser Leu
    1090                1095                1100
Gln Gly Lys Ile Asn Cys Thr
1105                1110

<210> 17
<211> 3753
<212> DNA
<213> homo sapiens

<400> 17
atgatgtttc gctcagatcg aatgtggagc tgccattgga aatggaagcc cagtcctctc      60
ctgttcttat ttgctttata tatcatgtgt gttcctcact cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080
atcccgctcc cagcagcaga actggccagc tgtgcagacc tggggaccct ctgtcaagct    1140
actgtaaact ctcctagtac tacaccaccc actgtcacca ctaacatgcc tgttactaac    1200
agaatcgata aacaaaggaa tgatggaatt atctatagaa tatccgtagt gattcagaac    1260
atccttcgtc accctgaggt aaaagtacag agcaaggtgg cagaatggct caattcaacc    1320
ttccaaaatt ggaactacac ggtttatgtc gttaatatca gtttcacctt gagtgctgga    1380
gaggacaaga ttaaagtcaa gagaagcctt gaggatgagc caaggttggt gctttgggcc    1440
cttctagttt acaatgctac caacaatact aatttagaag gaaaaatcat tcagcagaag    1500
ctcctaaaaa ataatgagtc cttggatgaa ggcttgaggc tacatacagt gaatgtgaga    1560
caactgggtc attgtcttgc catggaggaa cccaaaggct actactggcc atctatccaa    1620
ccttctgaat acgttcttcc ttgtccagac aagcctggct tttctgcttc tcggatatgt    1680
ttttacaatg ctaccaaccc attggtaacc tactggggac ctgttgatat ctccaactgt    1740
ttaaaagaag caaatgaagt tgctaaccag attttaaatt taactgctga tgggcagaac    1800
ttaacctcag ccaatattac caacattgtg gaacaggtca aaagaattgt gaataaagaa    1860
gaaaacattg atataacact tggctcaact ctaatgaata tattttctaa tatcttaagc    1920
agttcagaca gtgacttgct tgagtcatct tctgaagctt taaaaacaat tgatgaattg    1980
gccttcaaga tagacctaaa tagcacatca catgtgaata ttacaactcg gaacttggct    2040
ctcagcgtat catccctgtt accagggaca aatgcaattt caaattttag cattggtctt    2100
ccaagcaata atgaatcgta tttccagatg gattttgaga gtggacaagt ggatccactg    2160
gcatctgtaa ttttgcctcc aaacttactt gagaatttaa gtccagaaga ttctgtatta    2220
gttagaagag cacagtttac tttcttcaac aaaactggac ttttccagga tgtaggaccc    2280
caaagaaaaa ctttagtgag ttatgtgatg gcgtgcagta ttggaaacat tactatccag    2340
aatctgaagg atcctgttca aataaaaatc aaacatacaa gaactcagga agtgcatcat    2400
cccatctgtg ccttctggga tctgaacaaa aacaaaagtt ttggaggatg gaacacgtca    2460

ggatgtgttg cacacagaga ttcagatgca agtgagacag tctgcctgtg taaccacttc 2520
acacactttg gagttctgat ggaccttcca agaagtgcct cacagttaga tgcaagaaac 2580
actaaagtcc tcactttcat cagctatatt gggtgtggaa tatctgctat tttttcagca 2640
gcaactctcc tgacatatgt tgcttttgag aaattgcgaa gggattatcc ctccaaaatc 2700
ttgatgaacc tgagcacagc cctgctgttc ctgaatctcc tcttcctcct agatggctgg 2760
atcacctcct tcaatgtgga tggactttgc attgctgttg cagtcctgtt gcatttcttc 2820
cttctggcaa cctttacctg gatggggcta gaagcaattc acatgtacat tgctctagtt 2880
aaagtattta acacttacat tcgccgatac attctaaaat tctgcatcat tggctggggt 2940
ttgcctgcct tagtggtgtc agttgttcta gcgagcagaa acaacaatga agtctatgga 3000
aaagaaagtt atgggaaaga aaaaggtgat gaattctgtt ggattcaaga tccagtcata 3060
ttttatgtga cctgtgctgg gtattttgga gtcatgtttt ttctgaacat tgccatgttc 3120
attgtggtaa tggtgcagat ctgtgggagg aatggcaaga gaagcaaccg gaccctgaga 3180
gaagaagtgt taaggaacct gcgcagtgtg gttagcttga cctttctgtt gggcatgaca 3240
tggggttttg cattctttgc ctggggaccc ttaaatatcc ccttcatgta cctcttctcc 3300
atcttcaatt cattacaagg cttatttata ttcatcttcc actgtgctat gaaggagaat 3360
gttcagaaac agtggcggcg gcatctctgc tgtggtagat ttcggttagc agataactca 3420
gattggagta agacagctac caatatcatc aagaaaagtt ctgataatct aggaaaatct 3480
ttgtcttcaa gctccattgg ttccaactca acctatctta catccaaatc taaatccagc 3540
tctaccacct atttcaaaag gaatagccac acagacagtg cttccatgga caagtccttg 3600
tcaaaactgg cccatgctga tggagatcaa acatcaatca tccctgtcca tcaggtcatt 3660
gataaggtca agggttattg caatgctcat tcagacaact tctataaaaa tattatcatg 3720
tcagacacct tcagccacag cacaaagttt taa 3753

<210> 18
<211> 1250
<212> PRT
<213> homo sapiens

<400> 18
Met Met Phe Arg Ser Asp Arg Met Trp Ser Cys His Trp Lys Trp Lys
1               5                   10                  15
Pro Ser Pro Leu Leu Phe Leu Phe Ala Leu Tyr Ile Met Cys Val Pro
            20                  25                  30
His Ser Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys

305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Ala Thr Val Asn Ser
    370                 375                 380
Pro Ser Thr Thr Pro Pro Thr Val Thr Thr Asn Met Pro Val Thr Asn
385                 390                 395                 400
Arg Ile Asp Lys Gln Arg Asn Asp Gly Ile Ile Tyr Arg Ile Ser Val
                405                 410                 415
Val Ile Gln Asn Ile Leu Arg His Pro Glu Val Lys Val Gln Ser Lys
            420                 425                 430
Val Ala Glu Trp Leu Asn Ser Thr Phe Gln Asn Trp Asn Tyr Thr Val
        435                 440                 445
Tyr Val Val Asn Ile Ser Phe His Leu Ser Ala Gly Glu Asp Lys Ile
    450                 455                 460
Lys Val Lys Arg Ser Leu Glu Asp Glu Pro Arg Leu Val Leu Trp Ala
465                 470                 475                 480
Leu Leu Val Tyr Asn Ala Thr Asn Asn Thr Asn Leu Glu Gly Lys Ile
                485                 490                 495
Ile Gln Gln Lys Leu Leu Lys Asn Asn Glu Ser Leu Asp Glu Gly Leu
            500                 505                 510
Arg Leu His Thr Val Asn Val Arg Gln Leu Gly His Cys Leu Ala Met
        515                 520                 525
Glu Glu Pro Lys Gly Tyr Tyr Trp Pro Ser Ile Gln Pro Ser Glu Tyr
    530                 535                 540
Val Leu Pro Cys Pro Asp Lys Pro Gly Phe Ser Ala Ser Arg Ile Cys
545                 550                 555                 560
Phe Tyr Asn Ala Thr Asn Pro Leu Val Thr Tyr Trp Gly Pro Val Asp
                565                 570                 575
Ile Ser Asn Cys Leu Lys Glu Ala Asn Glu Val Ala Asn Gln Ile Leu
            580                 585                 590
Asn Leu Thr Ala Asp Gly Gln Asn Leu Thr Ser Ala Asn Ile Thr Asn
        595                 600                 605
Ile Val Glu Gln Val Lys Arg Ile Val Asn Lys Glu Glu Asn Ile Asp
    610                 615                 620
Ile Thr Leu Gly Ser Thr Leu Met Asn Ile Phe Ser Asn Ile Leu Ser
625                 630                 635                 640
Ser Ser Asp Ser Asp Leu Leu Glu Ser Ser Ser Glu Ala Leu Lys Thr
                645                 650                 655
Ile Asp Glu Leu Ala Phe Lys Ile Asp Leu Asn Ser Thr Ser His Val
            660                 665                 670
Asn Ile Thr Thr Arg Asn Leu Ala Leu Ser Val Ser Ser Leu Leu Pro
        675                 680                 685
Gly Thr Asn Ala Ile Ser Asn Phe Ser Ile Gly Leu Pro Ser Asn Asn
    690                 695                 700
Glu Ser Tyr Phe Gln Met Asp Phe Glu Ser Gly Gln Val Asp Pro Leu
705                 710                 715                 720
Ala Ser Val Ile Leu Pro Pro Asn Leu Leu Glu Asn Leu Ser Pro Glu
                725                 730                 735
Asp Ser Val Leu Val Arg Arg Ala Gln Phe Thr Phe Phe Asn Lys Thr
            740                 745                 750
Gly Leu Phe Gln Asp Val Gly Pro Gln Arg Lys Thr Leu Val Ser Tyr
        755                 760                 765
Val Met Ala Cys Ser Ile Gly Asn Ile Thr Ile Gln Asn Leu Lys Asp
    770                 775                 780
Pro Val Gln Ile Lys Ile Lys His Thr Arg Thr Gln Glu Val His His
785                 790                 795                 800
Pro Ile Cys Ala Phe Trp Asp Leu Asn Lys Asn Lys Ser Phe Gly Gly
                805                 810                 815
Trp Asn Thr Ser Gly Cys Val Ala His Arg Asp Ser Asp Ala Ser Glu
            820                 825                 830
Thr Val Cys Leu Cys Asn His Phe Thr His Phe Gly Val Leu Met Asp
        835                 840                 845
Leu Pro Arg Ser Ala Ser Gln Leu Asp Ala Arg Asn Thr Lys Val Leu

850                 855                 860
Thr Phe Ile Ser Tyr Ile Gly Cys Gly Ile Ser Ala Ile Phe Ser Ala
865                 870                 875                 880
Ala Thr Leu Leu Thr Tyr Val Ala Phe Glu Lys Leu Arg Arg Asp Tyr
                885                 890                 895
Pro Ser Lys Ile Leu Met Asn Leu Ser Thr Ala Leu Leu Phe Leu Asn
            900                 905                 910
Leu Leu Phe Leu Leu Asp Gly Trp Ile Thr Ser Phe Asn Val Asp Gly
        915                 920                 925
Leu Cys Ile Ala Val Ala Val Leu Leu His Phe Phe Leu Leu Ala Thr
    930                 935                 940
Phe Thr Trp Met Gly Leu Glu Ala Ile His Met Tyr Ile Ala Leu Val
945                 950                 955                 960
Lys Val Phe Asn Thr Tyr Ile Arg Arg Tyr Ile Leu Lys Phe Cys Ile
                965                 970                 975
Ile Gly Trp Gly Leu Pro Ala Leu Val Val Ser Val Val Leu Ala Ser
            980                 985                 990
Arg Asn Asn Asn Glu Val Tyr Gly Lys Glu Ser Tyr Gly Lys Glu Lys
        995                 1000                1005
Gly Asp Glu Phe Cys Trp Ile Gln Asp Pro Val Ile Phe Tyr Val Thr
    1010                1015                1020
Cys Ala Gly Tyr Phe Gly Val Met Phe Phe Leu Asn Ile Ala Met Phe
1025                1030                1035                1040
Ile Val Val Met Val Gln Ile Cys Gly Arg Asn Gly Lys Arg Ser Asn
                1045                1050                1055
Arg Thr Leu Arg Glu Glu Val Leu Arg Asn Leu Arg Ser Val Val Ser
            1060                1065                1070
Leu Thr Phe Leu Leu Gly Met Thr Trp Gly Phe Ala Phe Phe Ala Trp
        1075                1080                1085
Gly Pro Leu Asn Ile Pro Phe Met Tyr Leu Phe Ser Ile Phe Asn Ser
    1090                1095                1100
Leu Gln Gly Leu Phe Ile Phe Ile Phe His Cys Ala Met Lys Glu Asn
1105                1110                1115                1120
Val Gln Lys Gln Trp Arg Arg His Leu Cys Cys Gly Arg Phe Arg Leu
                1125                1130                1135
Ala Asp Asn Ser Asp Trp Ser Lys Thr Ala Thr Asn Ile Ile Lys Lys
            1140                1145                1150
Ser Ser Asp Asn Leu Gly Lys Ser Leu Ser Ser Ser Ser Ile Gly Ser
        1155                1160                1165
Asn Ser Thr Tyr Leu Thr Ser Lys Ser Lys Ser Ser Ser Thr Thr Tyr
    1170                1175                1180
Phe Lys Arg Asn Ser His Thr Asp Ser Ala Ser Met Asp Lys Ser Leu
1185                1190                1195                1200
Ser Lys Leu Ala His Ala Asp Gly Asp Gln Thr Ser Ile Ile Pro Val
                1205                1210                1215
His Gln Val Ile Asp Lys Val Lys Gly Tyr Cys Asn Ala His Ser Asp
            1220                1225                1230
Asn Phe Tyr Lys Asn Ile Ile Met Ser Asp Thr Phe Ser His Ser Thr
        1235                1240                1245
Lys Phe
    1250

<210> 19
<211> 3666
<212> DNA
<213> homo sapiens

<400> 19
atgatgtttc gctcagatcg aatgtggagc tgccattgga aatggaagcc cagtcctctc      60
ctgttcttat ttgctttata tatcatgtgt gttcctcact cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600

gctttctcct actcaaatgc atccttcaca caattgctca gttttggaaa ggccaagagt 660
ggctactttc tatccatttc tgattcaaaa tgtttgttga ataatgcatt acctgtcaaa 720
gaaaaagaag acatttttgc agaaagcttt gaacagctct gccttgtttg gaataattct 780
ttgggctcta ttggtgtaaa tttcaaaaga aactatgaaa cagttccatg tgattctacc 840
attagtaaag ttattcctgg gaatgggaaa ttgttgttgg gctccaatca aaatgaaatt 900
gtctctctaa aaggggacat ttataacttt cgactttgga attttaccat gaatgccaaa 960
atcctctcca acctcagctg taatgtgaaa gggaatgtag tcgactggca aaatgacttc 1020
tggaatatcc caaacctagc tctgaaagct gaaagcaacc taagctgtgg ttcctacctg 1080
atcccgctcc cagcagcaga actggccagc tgtgcagacc tggggaccct ctgtcaagct 1140
actgtaaact ctcctagtac tacaccaccc actgtcacca ctaacatgcc tgttactaac 1200
agaatcgata aacaaaggaa tgatggaatt atctatagaa tatccgtagt gattcagaac 1260
atccttcgtc accctgaggt aaaagtacag agcaaggtgg cagaatggct caattcaacc 1320
ttccaaaatt ggaactacac ggtttatgtc gttaatatca gttttcacct gagtgctgga 1380
gaggacaaga ttaaagtcaa gagaagcctt gaggatgagc caaggttggt gctttgggcc 1440
cttctagttt acaatgctac caacaatact aatttagaag gaaaaatcat tcagcagaag 1500
ctcctaaaaa ataatgagtc cttggatgaa ggcttgaggc tacatacagt gaatgtgaga 1560
caactgggtc attgtcttgc catggaggaa cccaaaggct actactggcc atctatccaa 1620
ccttctgaat acgttcttcc ttgtccagac aagcctggct tttctgcttc tcggatatgt 1680
ttttacaatg ctaccaaccc attggtaacc tactggggac ctgttgatat ctccaactgt 1740
ttaaaagaag caaatgaagt tgctaaccag attttaaatt taactgctga tgggcagaac 1800
ttaacctcag ccaatattac caacattgtg gaacaggtca aaagaattgt gaataaagaa 1860
gaaaacattg atataacact tggctcaact ctaatgaata tattttctaa tatcttaagc 1920
agttcagaca gtgacttgct tgagtcatct tctgaagctt taaaaacaat tgatgaattg 1980
gccttcaaga tagacctaaa tagcacatca catgtgaata ttacaactcg gaacttggct 2040
ctcagcgtat catccctgtt accagggaca aatgcaattt caaattttag cattggtctt 2100
ccaagcaata atgaatcgta tttccagatg gattttgaga gtggacaagt ggatccactg 2160
gcatctgtaa ttttgcctcc aaacttactt gagaatttaa gtccagaaga ttctgtatta 2220
gttagaagag cacagtttac tttcttcaac aaaactggac ttttccagga tgtaggaccc 2280
caaagaaaaa ctttagtgag ttatgtgatg gcgtgcagta ttggaaacat tactatccag 2340
aatctgaagg atcctgttca aataaaaatc aaacatacaa gaactcagga agtgcatcat 2400
cccatctgtg ccttctggga tctgaacaaa aacaaaagtt ttggaggatg gaacacgtca 2460
ggatgtgttg cacacagaga ttcagatgca agtgagacag tctgcctgtg taaccacttc 2520
acacactttg gagttctgat ggaccttcca agaagtgcct cacagttaga tgcaagaaac 2580
actaaagtcc tcactttcat cagctatatt gggtgtggaa tatctgctat tttttcagca 2640
gcaactctcc tgacatatgt tgcttttgag aaattgcgaa gggattatcc ctccaaaatc 2700
ttgatgaacc tgagcacagc cctgctgttc ctgaatctcc tcttcctcct agatggctgg 2760
atcacctcct tcaatgtgga tggactttgc attgctgttg cagtcctgtt gcatttcttc 2820
cttctggcaa cctttacctg gatggggcta gaagcaattc acatgtacat tgctctagtt 2880
aaagtattta acacttacat tcgccgatac attctaaaat tctgcatcat tggctggggt 2940
ttgcctgcct tagtggtgtc agttgttcta gcgagcagaa acaacaatga agtctatgga 3000
aaagaaagtt atgggaaaga aaaaggtgat gaattctgtt ggattcaaga tccagtcata 3060
ttttatgtga cctgtgctgg gtattttgga gtcatgtttt ttctgaacat tgccatgttc 3120
attgtggtaa tggtgcagat ctgtgggagg aatggcaaga gaagcaaccg gaccctgaga 3180
gaagaagtgt taaggaacct gcgcagtgtg gttagcttga cctttctgtt gggcatgaca 3240
tggggttttg cattctttgc ctggggaccc ttaaatatcc ccttcatgta cctcttctcc 3300
atcttcaatt cattacaagg cttatttata ttcatcttcc actgtgctat gaaggagaat 3360
gttcagaaac agtggcggcg gcatctctgc tgtggtagat ttcggttagc agataactca 3420
gattggagta agacagctac caatatcatc aagaaaagtt ctgataatct aggaaaatct 3480
ttgtcttcaa gctccattgg ttccaactca acctatctta catccaaatc taaatccagc 3540
tctaccacct atttcaaaag gaatagccac acagataatg tctcctatga gcattccttc 3600
aacaaaagtg gatcactcag acagtgcttc catggacaag tccttgtcaa aactggccca 3660
tgctga 3666

<210> 20
<211> 1221
<212> PRT
<213> homo sapiens

<400> 20
Met Met Phe Arg Ser Asp Arg Met Trp Ser Cys His Trp Lys Trp Lys
1               5                   10                  15
Pro Ser Pro Leu Leu Phe Leu Phe Ala Leu Tyr Ile Met Cys Val Pro
            20                  25                  30
His Ser Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln

65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Ala Thr Val Asn Ser
    370                 375                 380
Pro Ser Thr Thr Pro Pro Thr Val Thr Thr Asn Met Pro Val Thr Asn
385                 390                 395                 400
Arg Ile Asp Lys Gln Arg Asn Asp Gly Ile Ile Tyr Arg Ile Ser Val
                405                 410                 415
Val Ile Gln Asn Ile Leu Arg His Pro Glu Val Lys Val Gln Ser Lys
            420                 425                 430
Val Ala Glu Trp Leu Asn Ser Thr Phe Gln Asn Trp Asn Tyr Thr Val
        435                 440                 445
Tyr Val Val Asn Ile Ser Phe His Leu Ser Ala Gly Glu Asp Lys Ile
    450                 455                 460
Lys Val Lys Arg Ser Leu Glu Asp Glu Pro Arg Leu Val Leu Trp Ala
465                 470                 475                 480
Leu Leu Val Tyr Asn Ala Thr Asn Asn Thr Asn Leu Glu Gly Lys Ile
                485                 490                 495
Ile Gln Gln Lys Leu Leu Lys Asn Asn Glu Ser Leu Asp Glu Gly Leu
            500                 505                 510
Arg Leu His Thr Val Asn Val Arg Gln Leu Gly His Cys Leu Ala Met
        515                 520                 525
Glu Glu Pro Lys Gly Tyr Tyr Trp Pro Ser Ile Gln Pro Ser Glu Tyr
    530                 535                 540
Val Leu Pro Cys Pro Asp Lys Pro Gly Phe Ser Ala Ser Arg Ile Cys
545                 550                 555                 560
Phe Tyr Asn Ala Thr Asn Pro Leu Val Thr Tyr Trp Gly Pro Val Asp
                565                 570                 575
Ile Ser Asn Cys Leu Lys Glu Ala Asn Glu Val Ala Asn Gln Ile Leu
            580                 585                 590
Asn Leu Thr Ala Asp Gly Gln Asn Leu Thr Ser Ala Asn Ile Thr Asn
        595                 600                 605
Ile Val Glu Gln Val Lys Arg Ile Val Asn Lys Glu Glu Asn Ile Asp

    610                 615                 620
Ile Thr Leu Gly Ser Thr Leu Met Asn Ile Phe Ser Asn Ile Leu Ser
625                 630                 635                 640
Ser Ser Asp Ser Asp Leu Leu Glu Ser Ser Ser Glu Ala Leu Lys Thr
                645                 650                 655
Ile Asp Glu Leu Ala Phe Lys Ile Asp Leu Asn Ser Thr Ser His Val
            660                 665                 670
Asn Ile Thr Thr Arg Asn Leu Ala Leu Ser Val Ser Ser Leu Leu Pro
        675                 680                 685
Gly Thr Asn Ala Ile Ser Asn Phe Ser Ile Gly Leu Pro Ser Asn Asn
    690                 695                 700
Glu Ser Tyr Phe Gln Met Asp Phe Glu Ser Gly Gln Val Asp Pro Leu
705                 710                 715                 720
Ala Ser Val Ile Leu Pro Pro Asn Leu Leu Glu Asn Leu Ser Pro Glu
                725                 730                 735
Asp Ser Val Leu Val Arg Arg Ala Gln Phe Thr Phe Phe Asn Lys Thr
            740                 745                 750
Gly Leu Phe Gln Asp Val Gly Pro Gln Arg Lys Thr Leu Val Ser Tyr
        755                 760                 765
Val Met Ala Cys Ser Ile Gly Asn Ile Thr Ile Gln Asn Leu Lys Asp
    770                 775                 780
Pro Val Gln Ile Lys Ile Lys His Thr Arg Thr Gln Glu Val His His
785                 790                 795                 800
Pro Ile Cys Ala Phe Trp Asp Leu Asn Lys Asn Lys Ser Phe Gly Gly
                805                 810                 815
Trp Asn Thr Ser Gly Cys Val Ala His Arg Asp Ser Asp Ala Ser Glu
            820                 825                 830
Thr Val Cys Leu Cys Asn His Phe Thr His Phe Gly Val Leu Met Asp
        835                 840                 845
Leu Pro Arg Ser Ala Ser Gln Leu Asp Ala Arg Asn Thr Lys Val Leu
    850                 855                 860
Thr Phe Ile Ser Tyr Ile Gly Cys Gly Ile Ser Ala Ile Phe Ser Ala
865                 870                 875                 880
Ala Thr Leu Leu Thr Tyr Val Ala Phe Glu Lys Leu Arg Arg Asp Tyr
                885                 890                 895
Pro Ser Lys Ile Leu Met Asn Leu Ser Thr Ala Leu Leu Phe Leu Asn
            900                 905                 910
Leu Leu Phe Leu Leu Asp Gly Trp Ile Thr Ser Phe Asn Val Asp Gly
        915                 920                 925
Leu Cys Ile Ala Val Ala Val Leu Leu His Phe Phe Leu Leu Ala Thr
    930                 935                 940
Phe Thr Trp Met Gly Leu Glu Ala Ile His Met Tyr Ile Ala Leu Val
945                 950                 955                 960
Lys Val Phe Asn Thr Tyr Ile Arg Arg Tyr Ile Leu Lys Phe Cys Ile
                965                 970                 975
Ile Gly Trp Gly Leu Pro Ala Leu Val Val Ser Val Val Leu Ala Ser
            980                 985                 990
Arg Asn Asn Asn Glu Val Tyr Gly Lys Glu Ser Tyr Gly Lys Glu Lys
        995                 1000                1005
Gly Asp Glu Phe Cys Trp Ile Gln Asp Pro Val Ile Phe Tyr Val Thr
    1010                1015                1020
Cys Ala Gly Tyr Phe Gly Val Met Phe Phe Leu Asn Ile Ala Met Phe
1025                1030                1035                1040
Ile Val Val Met Val Gln Ile Cys Gly Arg Asn Gly Lys Arg Ser Asn
                1045                1050                1055
Arg Thr Leu Arg Glu Glu Val Leu Arg Asn Leu Arg Ser Val Val Ser
            1060                1065                1070
Leu Thr Phe Leu Leu Gly Met Thr Trp Gly Phe Ala Phe Phe Ala Trp
        1075                1080                1085
Gly Pro Leu Asn Ile Pro Phe Met Tyr Leu Phe Ser Ile Phe Asn Ser
    1090                1095                1100
Leu Gln Gly Leu Phe Ile Phe Ile Phe His Cys Ala Met Lys Glu Asn
1105                1110                1115                1120
Val Gln Lys Gln Trp Arg Arg His Leu Cys Cys Gly Arg Phe Arg Leu
                1125                1130                1135
Ala Asp Asn Ser Asp Trp Ser Lys Thr Ala Thr Asn Ile Ile Lys Lys
            1140                1145                1150
Ser Ser Asp Asn Leu Gly Lys Ser Leu Ser Ser Ser Ser Ile Gly Ser

1155                 1160                1165
Asn Ser Thr Tyr Leu Thr Ser Lys Ser Lys Ser Ser Ser Thr Thr Tyr
    1170                1175                1180
Phe Lys Arg Asn Ser His Thr Asp Asn Val Ser Tyr Glu His Ser Phe
1185                1190                1195                1200
Asn Lys Ser Gly Ser Leu Arg Gln Cys Phe His Gly Gln Val Leu Val
                1205                1210                1215
Lys Thr Gly Pro Cys
            1220

<210> 21
<211> 2157
<212> DNA
<213> homo sapiens

<400> 21
atgatgtttc gctcagatcg aatgtggagc tgccattgga aatggaagcc cagtcctctc      60
ctgttcttat ttgctttata tatcatgtgt gttcctcact cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080
atcccgctcc cagcagcaga actggccagc tgtgcagacc tggggaccct ctgtcaagct    1140
actgtaaact ctcctagtac tacaccaccc actgtcacca ctaacatgcc tgttactaac    1200
agaatcgata aacaaaggaa tgatggaatt atctatagaa tatccgtagt gattcagaac    1260
atccttcgtc accctgaggt aaaagtacag agcaaggtgg cagaatggct caattcaacc    1320
ttccaaaatt ggaactacac ggtttatgtc gttaatatca gttttcacct gagtgctgga    1380
gaggacaaga ttaaagtcaa gagaagcctt gaggatgagc caaggttggt gctttgggcc    1440
cttctagttt acaatgctac caacaatact aatttagaag gaaaaatcat tcagcagaag    1500
ctcctaaaaa ataatgagtc cttggatgaa ggcttgaggc tacatacagt gaatgtgaga    1560
caactgggtc attgtcttgc catggaggaa cccaaaggct actactggcc atctatccaa    1620
ccttctgaat acgttcttcc ttgtccagac aagcctggct tttctgcttc tcggatatgt    1680
ttttacaatg ctaccaaccc attggtaacc tactggggac ctgttgatat ctccaactgt    1740
ttaaaagaag caaatgaagt tgctaaccag attttaaatt taactgctga tgggcagaac    1800
ttaacctcag ccaatattac caacattgtg gaacaggtca aaagaattgt gaataaagaa    1860
gaaaacattg atataacact tggctcaact ctaatgaata tattttctaa tatcttaagc    1920
agttcagaca gtgacttgct tgagtcatct tctgaagctt taaaaacaat tgatgaattg    1980
gccttcaaga tagacctaaa tagcacatca catgtgaata ttacaactcg gaacttggct    2040
ctcagcgtat catccctgtt accagggaca aatgcaattt caaattttag cattggtctt    2100
ccaagcaata atgaatcgta tttccaggta atgagccagt ggtttctttc attttaa       2157

<210> 22
<211> 718
<212> PRT
<213> homo sapiens

<400> 22
Met Met Phe Arg Ser Asp Arg Met Trp Ser Cys His Trp Lys Trp Lys
1               5                   10                  15
Pro Ser Pro Leu Leu Phe Leu Phe Ala Leu Tyr Ile Met Cys Val Pro
            20                  25                  30
His Ser Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln

65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Ala Thr Val Asn Ser
    370                 375                 380
Pro Ser Thr Thr Pro Pro Thr Val Thr Thr Asn Met Pro Val Thr Asn
385                 390                 395                 400
Arg Ile Asp Lys Gln Arg Asn Asp Gly Ile Ile Tyr Arg Ile Ser Val
                405                 410                 415
Val Ile Gln Asn Ile Leu Arg His Pro Glu Val Lys Val Gln Ser Lys
            420                 425                 430
Val Ala Glu Trp Leu Asn Ser Thr Phe Gln Asn Trp Asn Tyr Thr Val
        435                 440                 445
Tyr Val Val Asn Ile Ser Phe His Leu Ser Ala Gly Glu Asp Lys Ile
    450                 455                 460
Lys Val Lys Arg Ser Leu Glu Asp Glu Pro Arg Leu Val Leu Trp Ala
465                 470                 475                 480
Leu Leu Val Tyr Asn Ala Thr Asn Asn Thr Asn Leu Glu Gly Lys Ile
                485                 490                 495
Ile Gln Gln Lys Leu Leu Lys Asn Asn Glu Ser Leu Asp Glu Gly Leu
            500                 505                 510
Arg Leu His Thr Val Asn Val Arg Gln Leu Gly His Cys Leu Ala Met
        515                 520                 525
Glu Glu Pro Lys Gly Tyr Tyr Trp Pro Ser Ile Gln Pro Ser Glu Tyr
    530                 535                 540
Val Leu Pro Cys Pro Asp Lys Pro Gly Phe Ser Ala Ser Arg Ile Cys
545                 550                 555                 560
Phe Tyr Asn Ala Thr Asn Pro Leu Val Thr Tyr Trp Gly Pro Val Asp
                565                 570                 575
Ile Ser Asn Cys Leu Lys Glu Ala Asn Glu Val Ala Asn Gln Ile Leu
            580                 585                 590
Asn Leu Thr Ala Asp Gly Gln Asn Leu Thr Ser Ala Asn Ile Thr Asn
        595                 600                 605
Ile Val Glu Gln Val Lys Arg Ile Val Asn Lys Glu Glu Asn Ile Asp

610                 615                 620
Ile Thr Leu Gly Ser Thr Leu Met Asn Ile Phe Ser Asn Ile Leu Ser
625                 630                 635                 640
Ser Ser Asp Ser Asp Leu Leu Glu Ser Ser Ser Glu Ala Leu Lys Thr
                645                 650                 655
Ile Asp Glu Leu Ala Phe Lys Ile Asp Leu Asn Ser Thr Ser His Val
            660                 665                 670
Asn Ile Thr Thr Arg Asn Leu Ala Leu Ser Val Ser Ser Leu Leu Pro
        675                 680                 685
Gly Thr Asn Ala Ile Ser Asn Phe Ser Ile Gly Leu Pro Ser Asn Asn
    690                 695                 700
Glu Ser Tyr Phe Gln Val Met Ser Gln Trp Phe Leu Ser Phe
705                 710                 715

<210> 23
<211> 3339
<212> DNA
<213> homo sapiens

<400> 23
atgatgtttc gctcagatcg aatgtggagc tgccattgga aatggaagcc cagtcctctc      60
ctgttcttat ttgctttata tatcatgtgt gttcctcact cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080
atcccgctcc cagcagcaga actggccagc tgtgcagacc tggggaccct ctgtcaagct    1140
actgtaaact ctcctagtac tacaccaccc actgtcacca ctaacatgcc tgttactaac    1200
agaatcgata aacaaaggaa tgatggaatt atctatagaa tatccgtagt gattcagaac    1260
atccttcgtc accctgaggt aaaagtacag agcaaggtgg cagaatggct caattcaacc    1320
ttccaaaatt ggaactacac ggtttatgtc gttaatatca gttttcacct gagtgctgga    1380
gaggacaaga ttaaagtcaa gagaagcctt gaggatgagc caaggttggt gctttgggcc    1440
cttctagttt acaatgctac caacaatact aatttagaag gaaaaatcat tcagcagaag    1500
ctcctaaaaa ataatgagtc cttggatgaa ggcttgaggc tacatacagt gaatgtgaga    1560
caactgggtc attgtcttgc catggaggaa cccaaaggct actactggcc atctatccaa    1620
ccttctgaat acgttcttcc ttgtccagac aagcctggct tttctgcttc tcggatatgt    1680
ttttacaatg ctaccaaccc attggtaacc tactggggac ctgttgatat ctccaactgt    1740
ttaaaagaag caaatgaagt tgctaaccag attttaaatt taactgctga tgggcagaac    1800
ttaacctcag ccaatattac caacattgtg gaacaggtca aaagaattgt gaataaagaa    1860
gaaaacattg atataacact tggctcaact ctaatgaata tattttctaa tatcttaagc    1920
agttcagaca gtgacttgct tgagtcatct tctgaagctt taaaaacaat tgatgaattg    1980
gccttcaaga tagacctaaa tagcacatca catgtgaata ttacaactcg gaacttggct    2040
ctcagcgtat catccctgtt accagggaca aatgcaattt caaattttag cattggtctt    2100
ccaagcaata atgaatcgta tttccagatg gattttgaga gtggacaagt ggatccactg    2160
gcatctgtaa ttttgcctcc aaacttactt gagaatttaa gtccagaaga ttctgtatta    2220
gttagaagag cacagtttac tttcttcaac aaaactggac ttttccagga tgtaggaccc    2280
caaagaaaaa ctttagtgag ttatgtgatg gcgtgcagta ttggaaacat tactatccag    2340
aatctgaagg atcctgttca aataaaaatc aaacatacaa gaactcagga agtgcatcat    2400
cccatctgtg ccttctggga tctgaacaaa aacaaaagtt ttggaggatg gaacacgtca    2460
ggatgtgttg cacacagaga ttcagatgca agtgagacag tctgcctgtg taaccacttc    2520
acacactttg gagttctgat ggaccttcca agaagtgcct cacagttaga tgcaagaaac    2580
actaaagtcc tcactttcat cagctatatt gggtgtggaa tatctgctat tttttcagca    2640
gcaactctcc tgacatatgt tgcttttgag aaattgcgaa gggattatcc ctccaaaatc    2700
ttgatgaacc tgagcacagc cctgctgttc ctgaatctcc tcttcctcct agatggctgg    2760
atcacctcct tcaatgtgga tggactttgc attgctgttg cagtcctgtt gcatttcttc    2820
cttctggcaa cctttacctg gatggggcta gaagcaattc acatgtacat tgctctagtt    2880

aaagtattta acacttacat tcgccgatac attctaaaat tctgcatcat tggctggggt 2940
ttgcctgcct tagtggtgtc agttgttcta gcgagcagaa acaacaatga agtctatgga 3000
aaagaaagtt atgggaaaga aaaaggtgat gaattctgtt ggattcaaga tccagtcata 3060
ttttatgtga cctgtgctgg gtattttgga gtcatgtttt ttctgaacat tgccatgttc 3120
attgtggtaa tggtgcagat ctgtgggagg aatggcaaga gaagcaaccg gaccctgaga 3180
gaagaagtgt taaggaacct gcgcagtgtg gttagcttga cctttctgtt gggcatgaca 3240
tggggttttg cattctttgc ctggggaccc ttaaatatcc ccttcatgta cctcttctcc 3300
atcttcaatt cattacaagg taagataaat tgtacatga 3339

<210> 24
<211> 1112
<212> PRT
<213> homo sapiens

<400> 24
Met Met Phe Arg Ser Asp Arg Met Trp Ser Cys His Trp Lys Trp Lys
1               5                   10                  15
Pro Ser Pro Leu Leu Phe Leu Phe Ala Leu Tyr Ile Met Cys Val Pro
            20                  25                  30
His Ser Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Ala Thr Val Asn Ser
    370                 375                 380
Pro Ser Thr Thr Pro Pro Thr Val Thr Thr Asn Met Pro Val Thr Asn
385                 390                 395                 400
Arg Ile Asp Lys Gln Arg Asn Asp Gly Ile Ile Tyr Arg Ile Ser Val
                405                 410                 415
Val Ile Gln Asn Ile Leu Arg His Pro Glu Val Lys Val Gln Ser Lys

            420                 425                 430
Val Ala Glu Trp Leu Asn Ser Thr Phe Gln Asn Trp Asn Tyr Thr Val
        435                 440                 445
Tyr Val Val Asn Ile Ser Phe His Leu Ser Ala Gly Glu Asp Lys Ile
    450                 455                 460
Lys Val Lys Arg Ser Leu Glu Asp Glu Pro Arg Leu Val Leu Trp Ala
465                 470                 475                 480
Leu Leu Val Tyr Asn Ala Thr Asn Asn Thr Asn Leu Glu Gly Lys Ile
                485                 490                 495
Ile Gln Gln Lys Leu Leu Lys Asn Asn Glu Ser Leu Asp Glu Gly Leu
            500                 505                 510
Arg Leu His Thr Val Asn Val Arg Gln Leu Gly His Cys Leu Ala Met
        515                 520                 525
Glu Glu Pro Lys Gly Tyr Tyr Trp Pro Ser Ile Gln Pro Ser Glu Tyr
    530                 535                 540
Val Leu Pro Cys Pro Asp Lys Pro Gly Phe Ser Ala Ser Arg Ile Cys
545                 550                 555                 560
Phe Tyr Asn Ala Thr Asn Pro Leu Val Thr Tyr Trp Gly Pro Val Asp
                565                 570                 575
Ile Ser Asn Cys Leu Lys Glu Ala Asn Glu Val Ala Asn Gln Ile Leu
            580                 585                 590
Asn Leu Thr Ala Asp Gly Gln Asn Leu Thr Ser Ala Asn Ile Thr Asn
        595                 600                 605
Ile Val Glu Gln Val Lys Arg Ile Val Asn Lys Glu Glu Asn Ile Asp
    610                 615                 620
Ile Thr Leu Gly Ser Thr Leu Met Asn Ile Phe Ser Asn Ile Leu Ser
625                 630                 635                 640
Ser Ser Asp Ser Asp Leu Leu Glu Ser Ser Ser Glu Ala Leu Lys Thr
                645                 650                 655
Ile Asp Glu Leu Ala Phe Lys Ile Asp Leu Asn Ser Thr Ser His Val
            660                 665                 670
Asn Ile Thr Thr Arg Asn Leu Ala Leu Ser Val Ser Ser Leu Leu Pro
        675                 680                 685
Gly Thr Asn Ala Ile Ser Asn Phe Ser Ile Gly Leu Pro Ser Asn Asn
    690                 695                 700
Glu Ser Tyr Phe Gln Met Asp Phe Glu Ser Gly Gln Val Asp Pro Leu
705                 710                 715                 720
Ala Ser Val Ile Leu Pro Pro Asn Leu Leu Glu Asn Leu Ser Pro Glu
                725                 730                 735
Asp Ser Val Leu Val Arg Arg Ala Gln Phe Thr Phe Phe Asn Lys Thr
            740                 745                 750
Gly Leu Phe Gln Asp Val Gly Pro Gln Arg Lys Thr Leu Val Ser Tyr
        755                 760                 765
Val Met Ala Cys Ser Ile Gly Asn Ile Thr Ile Gln Asn Leu Lys Asp
    770                 775                 780
Pro Val Gln Ile Lys Ile Lys His Thr Arg Thr Gln Glu Val His His
785                 790                 795                 800
Pro Ile Cys Ala Phe Trp Asp Leu Asn Lys Asn Lys Ser Phe Gly Gly
                805                 810                 815
Trp Asn Thr Ser Gly Cys Val Ala His Arg Asp Ser Asp Ala Ser Glu
            820                 825                 830
Thr Val Cys Leu Cys Asn His Phe Thr His Phe Gly Val Leu Met Asp
        835                 840                 845
Leu Pro Arg Ser Ala Ser Gln Leu Asp Ala Arg Asn Thr Lys Val Leu
    850                 855                 860
Thr Phe Ile Ser Tyr Ile Gly Cys Gly Ile Ser Ala Ile Phe Ser Ala
865                 870                 875                 880
Ala Thr Leu Leu Thr Tyr Val Ala Phe Glu Lys Leu Arg Arg Asp Tyr
                885                 890                 895
Pro Ser Lys Ile Leu Met Asn Leu Ser Thr Ala Leu Leu Phe Leu Asn
            900                 905                 910
Leu Leu Phe Leu Leu Asp Gly Trp Ile Thr Ser Phe Asn Val Asp Gly
        915                 920                 925
Leu Cys Ile Ala Val Ala Val Leu Leu His Phe Phe Leu Leu Ala Thr
    930                 935                 940
Phe Thr Trp Met Gly Leu Glu Ala Ile His Met Tyr Ile Ala Leu Val
945                 950                 955                 960
Lys Val Phe Asn Thr Tyr Ile Arg Arg Tyr Ile Leu Lys Phe Cys Ile

                965                 970                 975
Ile Gly Trp Gly Leu Pro Ala Leu Val Val Ser Val Val Leu Ala Ser
            980                 985                 990
Arg Asn Asn Asn Glu Val Tyr Gly Lys Glu Ser Tyr Gly Lys Glu Lys
        995                 1000                1005
Gly Asp Glu Phe Cys Trp Ile Gln Asp Pro Val Ile Phe Tyr Val Thr
    1010                1015                1020
Cys Ala Gly Tyr Phe Gly Val Met Phe Phe Leu Asn Ile Ala Met Phe
1025                1030                1035                1040
Ile Val Val Met Val Gln Ile Cys Gly Arg Asn Gly Lys Arg Ser Asn
                1045                1050                1055
Arg Thr Leu Arg Glu Glu Val Leu Arg Asn Leu Arg Ser Val Val Ser
            1060                1065                1070
Leu Thr Phe Leu Leu Gly Met Thr Trp Gly Phe Ala Phe Phe Ala Trp
        1075                1080                1085
Gly Pro Leu Asn Ile Pro Phe Met Tyr Leu Phe Ser Ile Phe Asn Ser
    1090                1095                1100
Leu Gln Gly Lys Ile Asn Cys Thr
1105                1110

<210> 25
<211> 1626
<212> DNA
<213> homo sapiens

<400> 25
atggattttg agagtggaca agtggatcca ctggcatctg taattttgcc tccaaactta 60
cttgagaatt taagtccaga agattctgta ttagttagaa gagcacagtt tactttcttc 120
aacaaaactg gacttttcca ggatgtagga ccccaaagaa aaactttagt gagttatgtg 180
atggcgtgca gtattggaaa cattactatc cagaatctga aggatcctgt tcaaataaaa 240
atcaaacata caagaactca ggaagtgcat catcccatct gtgccttctg ggatctgaac 300
aaaaacaaaa gttttggagg atggaacacg tcaggatgtg ttgcacacag agattcagat 360
gcaagtgaga cagtctgcct gtgtaaccac ttcacacact ttggagttct gatggacctt 420
ccaagaagtg cctcacagtt agatgcaaga aacactaaag tcctcacttt catcagctat 480
attgggtgtg gaatatctgc tattttttca gcagcaactc tcctgacata tgttgctttt 540
gagaaattgc gaagggatta tccctccaaa atcttgatga acctgagcac agccctgctg 600
ttcctgaatc tcctcttcct cctagatggc tggatcacct ccttcaatgt ggatggactt 660
tgcattgctg ttgcagtcct gttgcatttc ttccttctgg caacctttac ctggatgggg 720
ctagaagcaa ttcacatgta cattgctcta gttaaagtat ttaacactta cattcgccga 780
tacattctaa aattctgcat cattggctgg ggtttgcctg ccttagtggt gtcagttgtt 840
ctagcgagca gaaacaacaa tgaagtctat ggaaaagaaa gttatgggaa agaaaaaggt 900
gatgaattct gttggattca agatccagtc atattttatg tgacctgtgc tgggtatttt 960
ggagtcatgt tttttctgaa cattgccatg ttcattgtgg taatggtgca gatctgtggg 1020
aggaatggca agagaagcaa ccggaccctg agagaagaag tgttaaggaa cctgcgcagt 1080
gtggttagct tgacctttct gttgggcatg acatggggtt ttgcattctt tgcctgggga 1140
cccttaaata tccccttcat gtacctcttc tccatcttca attcattaca aggcttattt 1200
atattcatct tccactgtgc tatgaaggag aatgttcaga aacagtggcg gcggcatctc 1260
tgctgtggta gatttcggtt agcagataac tcagattgga gtaagacagc taccaatatc 1320
atcaagaaaa gttctgataa tctaggaaaa tctttgtctt caagctccat tggttccaac 1380
tcaacctatc ttacatccaa atctaaatcc agctctacca cctatttcaa aaggaatagc 1440
cacacagaca gtgcttccat ggacaagtcc ttgtcaaaac tggcccatgc tgatggagat 1500
caaacatcaa tcatccctgt ccatcaggtc attgataagg tcaagggtta ttgcaatgct 1560
cattcagaca acttctataa aaatattatc atgtcagaca ccttcagcca cagcacaaag 1620
ttttaa 1626

<210> 26
<211> 541
<212> PRT
<213> homo sapiens

<400> 26
Met Asp Phe Glu Ser Gly Gln Val Asp Pro Leu Ala Ser Val Ile Leu
1               5                   10                  15
Pro Pro Asn Leu Leu Glu Asn Leu Ser Pro Glu Asp Ser Val Leu Val
            20                  25                  30
Arg Arg Ala Gln Phe Thr Phe Phe Asn Lys Thr Gly Leu Phe Gln Asp
        35                  40                  45
Val Gly Pro Gln Arg Lys Thr Leu Val Ser Tyr Val Met Ala Cys Ser

50                  55                  60
Ile Gly Asn Ile Thr Ile Gln Asn Leu Lys Asp Pro Val Gln Ile Lys
65                  70                  75                  80
Ile Lys His Thr Arg Thr Gln Glu Val His His Pro Ile Cys Ala Phe
                85                  90                  95
Trp Asp Leu Asn Lys Asn Lys Ser Phe Gly Gly Trp Asn Thr Ser Gly
            100                 105                 110
Cys Val Ala His Arg Asp Ser Asp Ala Ser Glu Thr Val Cys Leu Cys
        115                 120                 125
Asn His Phe Thr His Phe Gly Val Leu Met Asp Leu Pro Arg Ser Ala
    130                 135                 140
Ser Gln Leu Asp Ala Arg Asn Thr Lys Val Leu Thr Phe Ile Ser Tyr
145                 150                 155                 160
Ile Gly Cys Gly Ile Ser Ala Ile Phe Ser Ala Ala Thr Leu Leu Thr
                165                 170                 175
Tyr Val Ala Phe Glu Lys Leu Arg Arg Asp Tyr Pro Ser Lys Ile Leu
            180                 185                 190
Met Asn Leu Ser Thr Ala Leu Leu Phe Leu Asn Leu Leu Phe Leu Leu
        195                 200                 205
Asp Gly Trp Ile Thr Ser Phe Asn Val Asp Gly Leu Cys Ile Ala Val
    210                 215                 220
Ala Val Leu Leu His Phe Phe Leu Leu Ala Thr Phe Thr Trp Met Gly
225                 230                 235                 240
Leu Glu Ala Ile His Met Tyr Ile Ala Leu Val Lys Val Phe Asn Thr
                245                 250                 255
Tyr Ile Arg Arg Tyr Ile Leu Lys Phe Cys Ile Ile Gly Trp Gly Leu
            260                 265                 270
Pro Ala Leu Val Val Ser Val Val Leu Ala Ser Arg Asn Asn Asn Glu
        275                 280                 285
Val Tyr Gly Lys Glu Ser Tyr Gly Lys Glu Lys Gly Asp Glu Phe Cys
    290                 295                 300
Trp Ile Gln Asp Pro Val Ile Phe Tyr Val Thr Cys Ala Gly Tyr Phe
305                 310                 315                 320
Gly Val Met Phe Phe Leu Asn Ile Ala Met Phe Ile Val Val Met Val
                325                 330                 335
Gln Ile Cys Gly Arg Asn Gly Lys Arg Ser Asn Arg Thr Leu Arg Glu
            340                 345                 350
Glu Val Leu Arg Asn Leu Arg Ser Val Val Ser Leu Thr Phe Leu Leu
        355                 360                 365
Gly Met Thr Trp Gly Phe Ala Phe Phe Ala Trp Gly Pro Leu Asn Ile
    370                 375                 380
Pro Phe Met Tyr Leu Phe Ser Ile Phe Asn Ser Leu Gln Gly Leu Phe
385                 390                 395                 400
Ile Phe Ile Phe His Cys Ala Met Lys Glu Asn Val Gln Lys Gln Trp
                405                 410                 415
Arg Arg His Leu Cys Cys Gly Arg Phe Arg Leu Ala Asp Asn Ser Asp
            420                 425                 430
Trp Ser Lys Thr Ala Thr Asn Ile Ile Lys Lys Ser Ser Asp Asn Leu
        435                 440                 445
Gly Lys Ser Leu Ser Ser Ser Ser Ile Gly Ser Asn Ser Thr Tyr Leu
    450                 455                 460
Thr Ser Lys Ser Lys Ser Ser Ser Thr Thr Tyr Phe Lys Arg Asn Ser
465                 470                 475                 480
His Thr Asp Ser Ala Ser Met Asp Lys Ser Leu Ser Lys Leu Ala His
                485                 490                 495
Ala Asp Gly Asp Gln Thr Ser Ile Ile Pro Val His Gln Val Ile Asp
            500                 505                 510
Lys Val Lys Gly Tyr Cys Asn Ala His Ser Asp Asn Phe Tyr Lys Asn
        515                 520                 525
Ile Ile Met Ser Asp Thr Phe Ser His Ser Thr Lys Phe
    530                 535                 540

<210> 27
<211> 1539
<212> DNA
<213> homo sapiens

<400> 27

atggattttg agagtggaca agtggatcca ctggcatctg taattttgcc tccaaactta 60
cttgagaatt taagtccaga agattctgta ttagttagaa gagcacagtt tactttcttc 120
aacaaaactg gacttttcca ggatgtagga ccccaaagaa aaactttagt gagttatgtg 180
atggcgtgca gtattggaaa cattactatc cagaatctga aggatcctgt tcaaataaaa 240
atcaaacata caagaactca ggaagtgcat catcccatct gtgccttctg ggatctgaac 300
aaaaacaaaa gttttggagg atggaacacg tcaggatgtg ttgcacacag agattcagat 360
gcaagtgaga cagtctgcct gtgtaaccac ttcacacact ttggagttct gatggacctt 420
ccaagaagtg cctcacagtt agatgcaaga aacactaaag tcctcacttt catcagctat 480
attgggtgtg gaatatctgc tattttttca gcagcaactc tcctgacata tgttgctttt 540
gagaaattgc gaagggatta tccctccaaa atcttgatga acctgagcac agccctgctg 600
ttcctgaatc tcctcttcct cctagatggc tggatcacct ccttcaatgt ggatggactt 660
tgcattgctg ttgcagtcct gttgcatttc ttccttctgg caacctttac ctggatgggg 720
ctagaagcaa ttcacatgta cattgctcta gttaaagtat ttaacactta cattcgccga 780
tacattctaa aattctgcat cattggctgg ggtttgcctg ccttagtggt gtcagttgtt 840
ctagcgagca gaaacaacaa tgaagtctat ggaaaagaaa gttatgggaa agaaaaaggt 900
gatgaattct gttggattca agatccagtc atattttatg tgacctgtgc tgggtatttt 960
ggagtcatgt tttttctgaa cattgccatg ttcattgtgg taatggtgca gatctgtggg 1020
aggaatggca agagaagcaa ccggaccctg agagaagaag tgttaaggaa cctgcgcagt 1080
gtggttagct tgacctttct gttgggcatg acatggggtt ttgcattctt tgcctgggga 1140
cccttaaata tccccttcat gtacctcttc tccatcttca attcattaca aggcttattt 1200
atattcatct tccactgtgc tatgaaggag aatgttcaga aacagtggcg gcggcatctc 1260
tgctgtggta gatttcggtt agcagataac tcagattgga gtaagacagc taccaatatc 1320
atcaagaaaa gttctgataa tctaggaaaa tctttgtctt caagctccat tggttccaac 1380
tcaacctatc ttacatccaa atctaaatcc agctctacca cctatttcaa aaggaatagc 1440
cacacagata atgtctccta tgagcattcc ttcaacaaaa gtggatcact cagacagtgc 1500
ttccatggac aagtccttgt caaaactggc ccatgctga 1539

<210> 28
<211> 512
<212> PRT
<213> homo sapiens

<400> 28
Met Asp Phe Glu Ser Gly Gln Val Asp Pro Leu Ala Ser Val Ile Leu
1               5                   10                  15
Pro Pro Asn Leu Leu Glu Asn Leu Ser Pro Glu Asp Ser Val Leu Val
            20                  25                  30
Arg Arg Ala Gln Phe Thr Phe Phe Asn Lys Thr Gly Leu Phe Gln Asp
        35                  40                  45
Val Gly Pro Gln Arg Lys Thr Leu Val Ser Tyr Val Met Ala Cys Ser
    50                  55                  60
Ile Gly Asn Ile Thr Ile Gln Asn Leu Lys Asp Pro Val Gln Ile Lys
65                  70                  75                  80
Ile Lys His Thr Arg Thr Gln Glu Val His His Pro Ile Cys Ala Phe
                85                  90                  95
Trp Asp Leu Asn Lys Asn Lys Ser Phe Gly Gly Trp Asn Thr Ser Gly
            100                 105                 110
Cys Val Ala His Arg Asp Ser Asp Ala Ser Glu Thr Val Cys Leu Cys
        115                 120                 125
Asn His Phe Thr His Phe Gly Val Leu Met Asp Leu Pro Arg Ser Ala
    130                 135                 140
Ser Gln Leu Asp Ala Arg Asn Thr Lys Val Leu Thr Phe Ile Ser Tyr
145                 150                 155                 160
Ile Gly Cys Gly Ile Ser Ala Ile Phe Ser Ala Ala Thr Leu Leu Thr
                165                 170                 175
Tyr Val Ala Phe Glu Lys Leu Arg Arg Asp Tyr Pro Ser Lys Ile Leu
            180                 185                 190
Met Asn Leu Ser Thr Ala Leu Leu Phe Leu Asn Leu Leu Phe Leu Leu
        195                 200                 205
Asp Gly Trp Ile Thr Ser Phe Asn Val Asp Gly Leu Cys Ile Ala Val
    210                 215                 220
Ala Val Leu Leu His Phe Phe Leu Leu Ala Thr Phe Thr Trp Met Gly
225                 230                 235                 240
Leu Glu Ala Ile His Met Tyr Ile Ala Leu Val Lys Val Phe Asn Thr
                245                 250                 255
Tyr Ile Arg Arg Tyr Ile Leu Lys Phe Cys Ile Ile Gly Trp Gly Leu
            260                 265                 270
Pro Ala Leu Val Val Ser Val Val Leu Ala Ser Arg Asn Asn Asn Glu

        275                 280                 285
Val Tyr Gly Lys Glu Ser Tyr Gly Lys Glu Lys Gly Asp Glu Phe Cys
    290                 295                 300
Trp Ile Gln Asp Pro Val Ile Phe Tyr Val Thr Cys Ala Gly Tyr Phe
305                 310                 315                 320
Gly Val Met Phe Phe Leu Asn Ile Ala Met Phe Ile Val Val Met Val
                325                 330                 335
Gln Ile Cys Gly Arg Asn Gly Lys Arg Ser Asn Arg Thr Leu Arg Glu
            340                 345                 350
Glu Val Leu Arg Asn Leu Arg Ser Val Val Ser Leu Thr Phe Leu Leu
        355                 360                 365
Gly Met Thr Trp Gly Phe Ala Phe Phe Ala Trp Gly Pro Leu Asn Ile
    370                 375                 380
Pro Phe Met Tyr Leu Phe Ser Ile Phe Asn Ser Leu Gln Gly Leu Phe
385                 390                 395                 400
Ile Phe Ile Phe His Cys Ala Met Lys Glu Asn Val Gln Lys Gln Trp
                405                 410                 415
Arg Arg His Leu Cys Cys Gly Arg Phe Arg Leu Ala Asp Asn Ser Asp
            420                 425                 430
Trp Ser Lys Thr Ala Thr Asn Ile Ile Lys Lys Ser Ser Asp Asn Leu
        435                 440                 445
Gly Lys Ser Leu Ser Ser Ser Ser Ile Gly Ser Asn Ser Thr Tyr Leu
    450                 455                 460
Thr Ser Lys Ser Lys Ser Ser Ser Thr Thr Tyr Phe Lys Arg Asn Ser
465                 470                 475                 480
His Thr Asp Asn Val Ser Tyr Glu His Ser Phe Asn Lys Ser Gly Ser
                485                 490                 495
Leu Arg Gln Cys Phe His Gly Gln Val Leu Val Lys Thr Gly Pro Cys
            500                 505                 510

<210> 29
<211> 27
<212> DNA
<213> homo sapiens

<400> 29
atgagccagt ggtttctttc attttaa 27

<210> 30
<211> 8
<212> PRT
<213> homo sapiens

<400> 30
Met Ser Gln Trp Phe Leu Ser Phe
1               5

<210> 31
<211> 1212
<212> DNA
<213> homo sapiens

<400> 31
atggattttg agagtggaca agtggatcca ctggcatctg taattttgcc tccaaactta 60
cttgagaatt taagtccaga agattctgta ttagttagaa gagcacagtt tactttcttc 120
aacaaaactg gacttttcca ggatgtagga ccccaaagaa aaactttagt gagttatgtg 180
atggcgtgca gtattggaaa cattactatc cagaatctga aggatcctgt tcaaataaaa 240
atcaaacata caagaactca ggaagtgcat catcccatct gtgccttctg ggatctgaac 300
aaaaacaaaa gttttggagg atggaacacg tcaggatgtg ttgcacacag agattcagat 360
gcaagtgaga cagtctgcct gtgtaaccac ttcacacact ttggagttct gatggacctt 420
ccaagaagtg cctcacagtt agatgcaaga aacactaaag tcctcacttt catcagctat 480
attgggtgtg gaatatctgc tattttttca gcagcaactc tcctgacata tgttgctttt 540
gagaaattgc gaagggatta tccctccaaa atcttgatga acctgagcac agccctgctg 600
ttcctgaatc tcctcttcct cctagatggc tggatcacct ccttcaatgt ggatggactt 660
tgcattgctg ttgcagtcct gttgcatttc ttccttctgg caacctttac ctggatgggg 720
ctagaagcaa ttcacatgta cattgctcta gttaaagtat ttaacactta cattcgccga 780
tacattctaa aattctgcat cattggctgg ggtttgcctg ccttagtggt gtcagttgtt 840
ctagcgagca gaaacaacaa tgaagtctat ggaaaagaaa gttatgggaa agaaaaaggt 900

gatgaattct gttggattca agatccagtc atattttatg tgacctgtgc tgggtatttt 960
ggagtcatgt tttttctgaa cattgccatg ttcattgtgg taatggtgca gatctgtggg 1020
aggaatggca agagaagcaa ccggaccctg agagaagaag tgttaaggaa cctgcgcagt 1080
gtggttagct tgacctttct gttgggcatg acatggggtt ttgcattctt tgcctgggga 1140
cccttaaata tccccttcat gtacctcttc tccatcttca attcattaca aggtaagata 1200
aattgtacat ga 1212

<210> 32
<211> 403
<212> PRT
<213> homo sapiens

<400> 32
Met Asp Phe Glu Ser Gly Gln Val Asp Pro Leu Ala Ser Val Ile Leu
1               5                   10                  15
Pro Pro Asn Leu Leu Glu Asn Leu Ser Pro Glu Asp Ser Val Leu Val
            20                  25                  30
Arg Arg Ala Gln Phe Thr Phe Phe Asn Lys Thr Gly Leu Phe Gln Asp
        35                  40                  45
Val Gly Pro Gln Arg Lys Thr Leu Val Ser Tyr Val Met Ala Cys Ser
    50                  55                  60
Ile Gly Asn Ile Thr Ile Gln Asn Leu Lys Asp Pro Val Gln Ile Lys
65                  70                  75                  80
Ile Lys His Thr Arg Thr Gln Glu Val His His Pro Ile Cys Ala Phe
                85                  90                  95
Trp Asp Leu Asn Lys Asn Lys Ser Phe Gly Gly Trp Asn Thr Ser Gly
            100                 105                 110
Cys Val Ala His Arg Asp Ser Asp Ala Ser Glu Thr Val Cys Leu Cys
        115                 120                 125
Asn His Phe Thr His Phe Gly Val Leu Met Asp Leu Pro Arg Ser Ala
    130                 135                 140
Ser Gln Leu Asp Ala Arg Asn Thr Lys Val Leu Thr Phe Ile Ser Tyr
145                 150                 155                 160
Ile Gly Cys Gly Ile Ser Ala Ile Phe Ser Ala Ala Thr Leu Leu Thr
                165                 170                 175
Tyr Val Ala Phe Glu Lys Leu Arg Arg Asp Tyr Pro Ser Lys Ile Leu
            180                 185                 190
Met Asn Leu Ser Thr Ala Leu Leu Phe Leu Asn Leu Leu Phe Leu Leu
        195                 200                 205
Asp Gly Trp Ile Thr Ser Phe Asn Val Asp Gly Leu Cys Ile Ala Val
    210                 215                 220
Ala Val Leu Leu His Phe Phe Leu Leu Ala Thr Phe Thr Trp Met Gly
225                 230                 235                 240
Leu Glu Ala Ile His Met Tyr Ile Ala Leu Val Lys Val Phe Asn Thr
                245                 250                 255
Tyr Ile Arg Arg Tyr Ile Leu Lys Phe Cys Ile Ile Gly Trp Gly Leu
            260                 265                 270
Pro Ala Leu Val Val Ser Val Val Leu Ala Ser Arg Asn Asn Asn Glu
        275                 280                 285
Val Tyr Gly Lys Glu Ser Tyr Gly Lys Glu Lys Gly Asp Glu Phe Cys
    290                 295                 300
Trp Ile Gln Asp Pro Val Ile Phe Tyr Val Thr Cys Ala Gly Tyr Phe
305                 310                 315                 320
Gly Val Met Phe Phe Leu Asn Ile Ala Met Phe Ile Val Val Met Val
                325                 330                 335
Gln Ile Cys Gly Arg Asn Gly Lys Arg Ser Asn Arg Thr Leu Arg Glu
            340                 345                 350
Glu Val Leu Arg Asn Leu Arg Ser Val Val Ser Leu Thr Phe Leu Leu
        355                 360                 365
Gly Met Thr Trp Gly Phe Ala Phe Phe Ala Trp Gly Pro Leu Asn Ile
    370                 375                 380
Pro Phe Met Tyr Leu Phe Ser Ile Phe Asn Ser Leu Gln Gly Lys Ile
385                 390                 395                 400
Asn Cys Thr

<210> 33
<211> 3669

<212> DNA
<213> homo sapiens

<400> 33
atgtttcgct cagatcgaat gtggagctgc cattggaaat ggaagcccag tcctctcctg 60
ttcttatttg ctttatatat catgtgtgtt cctcactcag cagtgtgggg atgtgccaac 120
tgccgagtgg ttttgtccaa cccttctggg acctttactt ctccatgcta ccctaacgac 180
tacccaaaca gccaggcttg catgtggacg ctccgagccc ccaccggtta tatcattcag 240
ataacattta acgactttga cattgaagaa gctcccaatt gcatttatga ctcattatcc 300
cttgataatg gagagagcca gactaaattt tgtggagcaa ctgccaaagg cctatcattt 360
aactcaagtg cgaatgagat gcatgtgtcc ttttcaagtg actttagcat ccagaagaaa 420
ggtttcaatg ccagctacat cagagttgcc gtgtccttaa ggaatcaaaa ggtcatttta 480
ccccagacat cagatgctta ccaggtatct gttgcaaaaa gcatctctat tccagagctc 540
agtgctttca cactctgctt tgaagcaacc aaagttggcc atgaagacag tgattggaca 600
gctttctcct actcaaatgc atccttcaca caattgctca gttttggaaa ggccaagagt 660
ggctactttc tatccatttc tgattcaaaa tgtttgttga ataatgcatt acctgtcaaa 720
gaaaaagaag acatttttgc agaaagcttt gaacagctct gccttgtttg gaataattct 780
ttgggctcta ttggtgtaaa tttcaaaaga aactatgaaa cagttccatg tgattctacc 840
attagtaaag ttattcctgg gaatgggaaa ttgttgttgg gctccaatca aaatgaaatt 900
gtctctctaa aaggggacat ttataacttt cgactttgga attttaccat gaatgccaaa 960
atcctctcca acctcagctg taatgtgaaa gggaatgtag tcgactggca aaatgacttc 1020
tggaatatcc caaacctagc tctgaaagct gaaagcaacc taagctgtgg ttcctacctg 1080
atcccgctcc cagcagcaga actggccagc tgtgcagacc tggggaccct ctgtcaagat 1140
ggaattatct atagaatatc cgtagtgatt cagaacatcc ttcgtcaccc tgaggtaaaa 1200
gtacagagca aggtggcaga atggctcaat tcaaccttcc aaaattggaa ctacacggtt 1260
tatgtcgtta atatcagttt tcacctgagt gctggagagg acaagattaa agtcaagaga 1320
agccttgagg atgagccaag gttggtgctt tgggcccttc tagtttacaa tgctaccaac 1380
aatactaatt tagaaggaaa aatcattcag cagaagctcc taaaaaataa tgagtccttg 1440
gatgaaggct tgaggctaca tacagtgaat gtgagacaac tgggtcattg tcttgccatg 1500
gaggaaccca aaggctacta ctggccatct atccaacctt ctgaatacgt tcttccttgt 1560
ccagacaagc ctggcttttc tgcttctcgg atatgttttt acaatgctac caacccattg 1620
gtaacctact ggggacctgt tgatatctcc aactgtttaa aagaagcaaa tgaagttgct 1680
aaccagattt taaatttaac tgctgatggg cagaacttaa cctcagccaa tattaccaac 1740
attgtggaac aggtcaaaag aattgtgaat aaagaagaaa acattgatat aacacttggc 1800
tcaactctaa tgaatatatt ttctaatatc ttaagcagtt cagacagtga cttgcttgag 1860
tcatcttctg aagctttaaa aacaattgat gaattggcct tcaagataga cctaaatagc 1920
acatcacatg tgaatattac aactcggaac ttggctctca gcgtatcatc cctgttacca 1980
gggacaaatg caatttcaaa ttttagcatt ggtcttccaa gcaataatga atcgtatttc 2040
cagatggatt ttgagagtgg acaagtggat ccactggcat ctgtaatttt gcctccaaac 2100
ttacttgaga atttaagtcc agaagattct gtattagtta gaagagcaca gtttactttc 2160
ttcaacaaaa ctggactttt ccaggatgta ggaccccaaa gaaaaacttt agtgagttat 2220
gtgatggcgt gcagtattgg aaacattact atccagaatc tgaaggatcc tgttcaaata 2280
aaaatcaaac atacaagaac tcaggaagtg catcatccca tctgtgcctt ctgggatctg 2340
aacaaaaaca aaagttttgg aggatggaac acgtcaggat gtgttgcaca cagagattca 2400
gatgcaagtg agacagtctg cctgtgtaac cacttcacac actttggagt tctgatggac 2460
cttccaagaa gtgcctcaca gttagatgca agaaacacta aagtcctcac tttcatcagc 2520
tatattgggt gtggaatatc tgctattttt tcagcagcaa ctctcctgac atatgttgct 2580
tttgagaaat tgcgaaggga ttatccctcc aaaatcttga tgaacctgag cacagccctg 2640
ctgttcctga atctcctctt cctcctagat ggctggatca cctccttcaa tgtggatgga 2700
ctttgcattg ctgttgcagt cctgttgcat ttcttccttc tggcaacctt tacctggatg 2760
gggctagaag caattcacat gtacattgct ctagttaaag tatttaacac ttacattcgc 2820
cgatacattc taaaattctg catcattggc tggggtttgc ctgccttagt ggtgtcagtt 2880
gttctagcga gcagaaacaa caatgaagtc tatggaaaag aaagttatgg gaaagaaaaa 2940
ggtgatgaat tctgttggat tcaagatcca gtcatatttt atgtgacctg tgctgggtat 3000
tttggagtca tgttttttct gaacattgcc atgttcattg tggtaatggt gcagatctgt 3060
gggaggaatg gcaagagaag caaccggacc ctgagagaag aagtgttaag gaacctgcgc 3120
agtgtggtta gcttgacctt tctgttgggc atgacatggg gttttgcatt ctttgcctgg 3180
ggacccttaa atatcccctt catgtacctc ttctccatct tcaattcatt acaaggctta 3240
tttatattca tcttccactg tgctatgaag gagaatgttc agaaacagtg gcggcggcat 3300
ctctgctgtg gtagatttcg gttagcagat aactcagatt ggagtaagac agctaccaat 3360
atcatcaaga aaagttctga taatctagga aaatctttgt cttcaagctc cattggttcc 3420
aactcaacct atcttacatc caaatctaaa tccagctcta ccacctattt caaaaggaat 3480
agccacacag acagtgcttc catggacaag tccttgtcaa aactggccca tgctgatgga 3540
gatcaaacat caatcatccc tgtccatcag gtcattgata aggtcaaggg ttattgcaat 3600
gctcattcag acaacttcta taaaaatatt atcatgtcag acaccttcag ccacagcaca 3660
aagttttaa 3669

<210> 34

<211> 1222
<212> PRT
<213> homo sapiens

<400> 34
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Ala Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Asp Gly Ile Ile Tyr
    370                 375                 380
Arg Ile Ser Val Val Ile Gln Asn Ile Leu Arg His Pro Glu Val Lys
385                 390                 395                 400
Val Gln Ser Lys Val Ala Glu Trp Leu Asn Ser Thr Phe Gln Asn Trp
                405                 410                 415
Asn Tyr Thr Val Tyr Val Val Asn Ile Ser Phe His Leu Ser Ala Gly
            420                 425                 430
Glu Asp Lys Ile Lys Val Lys Arg Ser Leu Glu Asp Glu Pro Arg Leu
        435                 440                 445
Val Leu Trp Ala Leu Leu Val Tyr Asn Ala Thr Asn Asn Thr Asn Leu
    450                 455                 460
Glu Gly Lys Ile Ile Gln Gln Lys Leu Leu Lys Asn Asn Glu Ser Leu
465                 470                 475                 480
Asp Glu Gly Leu Arg Leu His Thr Val Asn Val Arg Gln Leu Gly His
                485                 490                 495
Cys Leu Ala Met Glu Glu Pro Lys Gly Tyr Tyr Trp Pro Ser Ile Gln

            500                 505                 510
Pro Ser Glu Tyr Val Leu Pro Cys Pro Asp Lys Pro Gly Phe Ser Ala
        515                 520                 525
Ser Arg Ile Cys Phe Tyr Asn Ala Thr Asn Pro Leu Val Thr Tyr Trp
    530                 535                 540
Gly Pro Val Asp Ile Ser Asn Cys Leu Lys Glu Ala Asn Glu Val Ala
545                 550                 555                 560
Asn Gln Ile Leu Asn Leu Thr Ala Asp Gly Gln Asn Leu Thr Ser Ala
                565                 570                 575
Asn Ile Thr Asn Ile Val Glu Gln Val Lys Arg Ile Val Asn Lys Glu
            580                 585                 590
Glu Asn Ile Asp Ile Thr Leu Gly Ser Thr Leu Met Asn Ile Phe Ser
        595                 600                 605
Asn Ile Leu Ser Ser Ser Asp Ser Asp Leu Leu Glu Ser Ser Ser Glu
    610                 615                 620
Ala Leu Lys Thr Ile Asp Glu Leu Ala Phe Lys Ile Asp Leu Asn Ser
625                 630                 635                 640
Thr Ser His Val Asn Ile Thr Thr Arg Asn Leu Ala Leu Ser Val Ser
                645                 650                 655
Ser Leu Leu Pro Gly Thr Asn Ala Ile Ser Asn Phe Ser Ile Gly Leu
            660                 665                 670
Pro Ser Asn Asn Glu Ser Tyr Phe Gln Met Asp Phe Glu Ser Gly Gln
        675                 680                 685
Val Asp Pro Leu Ala Ser Val Ile Leu Pro Pro Asn Leu Leu Glu Asn
    690                 695                 700
Leu Ser Pro Glu Asp Ser Val Leu Val Arg Arg Ala Gln Phe Thr Phe
705                 710                 715                 720
Phe Asn Lys Thr Gly Leu Phe Gln Asp Val Gly Pro Gln Arg Lys Thr
                725                 730                 735
Leu Val Ser Tyr Val Met Ala Cys Ser Ile Gly Asn Ile Thr Ile Gln
            740                 745                 750
Asn Leu Lys Asp Pro Val Gln Ile Lys Ile Lys His Thr Arg Thr Gln
        755                 760                 765
Glu Val His His Pro Ile Cys Ala Phe Trp Asp Leu Asn Lys Asn Lys
    770                 775                 780
Ser Phe Gly Gly Trp Asn Thr Ser Gly Cys Val Ala His Arg Asp Ser
785                 790                 795                 800
Asp Ala Ser Glu Thr Val Cys Leu Cys Asn His Phe Thr His Phe Gly
                805                 810                 815
Val Leu Met Asp Leu Pro Arg Ser Ala Ser Gln Leu Asp Ala Arg Asn
            820                 825                 830
Thr Lys Val Leu Thr Phe Ile Ser Tyr Ile Gly Cys Gly Ile Ser Ala
        835                 840                 845
Ile Phe Ser Ala Ala Thr Leu Leu Thr Tyr Val Ala Phe Glu Lys Leu
    850                 855                 860
Arg Arg Asp Tyr Pro Ser Lys Ile Leu Met Asn Leu Ser Thr Ala Leu
865                 870                 875                 880
Leu Phe Leu Asn Leu Leu Phe Leu Leu Asp Gly Trp Ile Thr Ser Phe
                885                 890                 895
Asn Val Asp Gly Leu Cys Ile Ala Val Ala Val Leu Leu His Phe Phe
            900                 905                 910
Leu Leu Ala Thr Phe Thr Trp Met Gly Leu Glu Ala Ile His Met Tyr
        915                 920                 925
Ile Ala Leu Val Lys Val Phe Asn Thr Tyr Ile Arg Arg Tyr Ile Leu
    930                 935                 940
Lys Phe Cys Ile Ile Gly Trp Gly Leu Pro Ala Leu Val Val Ser Val
945                 950                 955                 960
Val Leu Ala Ser Arg Asn Asn Asn Glu Val Tyr Gly Lys Glu Ser Tyr
                965                 970                 975
Gly Lys Glu Lys Gly Asp Glu Phe Cys Trp Ile Gln Asp Pro Val Ile
            980                 985                 990
Phe Tyr Val Thr Cys Ala Gly Tyr Phe Gly Val Met Phe Phe Leu Asn
        995                 1000                1005
Ile Ala Met Phe Ile Val Val Met Val Gln Ile Cys Gly Arg Asn Gly
    1010                1015                1020
Lys Arg Ser Asn Arg Thr Leu Arg Glu Glu Val Leu Arg Asn Leu Arg
1025                1030                1035                1040
Ser Val Val Ser Leu Thr Phe Leu Leu Gly Met Thr Trp Gly Phe Ala

                1045                    1050                    1055
Phe Phe Ala Trp Gly Pro Leu Asn Ile Pro Phe Met Tyr Leu Phe Ser
            1060                    1065                    1070
Ile Phe Asn Ser Leu Gln Gly Leu Phe Ile Phe Ile Phe His Cys Ala
        1075                    1080                    1085
Met Lys Glu Asn Val Gln Lys Gln Trp Arg Arg His Leu Cys Cys Gly
    1090                    1095                    1100
Arg Phe Arg Leu Ala Asp Asn Ser Asp Trp Ser Lys Thr Ala Thr Asn
1105                    1110                    1115                    1120
Ile Ile Lys Lys Ser Ser Asp Asn Leu Gly Lys Ser Leu Ser Ser Ser
                1125                    1130                    1135
Ser Ile Gly Ser Asn Ser Thr Tyr Leu Thr Ser Lys Ser Lys Ser Ser
            1140                    1145                    1150
Ser Thr Thr Tyr Phe Lys Arg Asn Ser His Thr Asp Ser Ala Ser Met
        1155                    1160                    1165
Asp Lys Ser Leu Ser Lys Leu Ala His Ala Asp Gly Asp Gln Thr Ser
    1170                    1175                    1180
Ile Ile Pro Val His Gln Val Ile Asp Lys Val Lys Gly Tyr Cys Asn
1185                    1190                    1195                    1200
Ala His Ser Asp Asn Phe Tyr Lys Asn Ile Ile Met Ser Asp Thr Phe
                1205                    1210                    1215
Ser His Ser Thr Lys Phe
            1220

<210> 35
<211> 3582
<212> DNA
<213> homo sapiens

<400> 35
atgtttcgct cagatcgaat gtggagctgc cattggaaat ggaagcccag tcctctcctg      60
ttcttatttg ctttatatat catgtgtgtt cctcactcag cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080
atcccgctcc cagcagcaga actggccagc tgtgcagacc tggggaccct ctgtcaagat    1140
ggaattatct atagaatatc cgtagtgatt cagaacatcc ttcgtcaccc tgaggtaaaa    1200
gtacagagca aggtggcaga atggctcaat tcaaccttcc aaaattggaa ctacacggtt    1260
tatgtcgtta atatcagttt tcacctgagt gctggagagg acaagattaa agtcaagaga    1320
agccttgagg atgagccaag gttggtgctt tgggcccttc tagtttacaa tgctaccaac    1380
aatactaatt tagaaggaaa aatcattcag cagaagctcc taaaaaataa tgagtccttg    1440
gatgaaggct tgaggctaca tacagtgaat gtgagacaac tgggtcattg tcttgccatg    1500
gaggaaccca aaggctacta ctggccatct atccaacctt ctgaatacgt tcttccttgt    1560
ccagacaagc ctggcttttc tgcttctcgg atatgttttt acaatgctac caacccattg    1620
gtaacctact ggggacctgt tgatatctcc aactgtttaa aagaagcaaa tgaagttgct    1680
aaccagattt taaatttaac tgctgatggg cagaacttaa cctcagccaa tattaccaac    1740
attgtggaac aggtcaaaag aattgtgaat aaagaagaaa acattgatat aacacttggc    1800
tcaactctaa tgaatatatt ttctaatatc ttaagcagtt cagacagtga cttgcttgag    1860
tcatcttctg aagctttaaa aacaattgat gaattggcct tcaagataga cctaaatagc    1920
acatcacatg tgaatattac aactcggaac ttggctctca gcgtatcatc cctgttacca    1980
gggacaaatg caatttcaaa ttttagcatt ggtcttccaa gcaataatga atcgtatttc    2040
cagatggatt ttgagagtgg acaagtggat ccactggcat ctgtaatttt gcctccaaac    2100
ttacttgaga atttaagtcc agaagattct gtattagtta gaagagcaca gtttactttc    2160
ttcaacaaaa ctggactttt ccaggatgta ggaccccaaa gaaaaacttt agtgagttat    2220
gtgatggcgt gcagtattgg aaacattact atccagaatc tgaaggatcc tgttcaaata    2280

aaaatcaaac atacaagaac tcaggaagtg catcatccca tctgtgcctt ctgggatctg 2340
aacaaaaaca aaagttttgg aggatggaac acgtcaggat gtgttgcaca cagagattca 2400
gatgcaagtg agacagtctg cctgtgtaac cacttcacac actttggagt tctgatggac 2460
cttccaagaa gtgcctcaca gttagatgca agaaacacta aagtcctcac tttcatcagc 2520
tatattgggt gtggaatatc tgctattttt tcagcagcaa ctctcctgac atatgttgct 2580
tttgagaaat tgcgaaggga ttatccctcc aaaatcttga tgaacctgag cacagccctg 2640
ctgttcctga atctcctctt cctcctagat ggctggatca cctccttcaa tgtggatgga 2700
ctttgcattg ctgttgcagt cctgttgcat ttcttccttc tggcaacctt tacctggatg 2760
gggctagaag caattcacat gtacattgct ctagttaaag tatttaacac ttacattcgc 2820
cgatacattc taaaattctg catcattggc tggggtttgc ctgccttagt ggtgtcagtt 2880
gttctagcga gcagaaacaa caatgaagtc tatggaaaag aaagttatgg gaaagaaaaa 2940
ggtgatgaat tctgttggat tcaagatcca gtcatatttt atgtgacctg tgctgggtat 3000
tttggagtca tgttttttct gaacattgcc atgttcattg tggtaatggt gcagatctgt 3060
gggaggaatg gcaagagaag caaccggacc ctgagagaag aagtgttaag gaacctgcgc 3120
agtgtggtta gcttgacctt tctgttgggc atgacatggg gttttgcatt ctttgcctgg 3180
ggacccttaa atatcccctt catgtacctc ttctccatct tcaattcatt acaaggctta 3240
tttatattca tcttccactg tgctatgaag gagaatgttc agaaacagtg gcggcggcat 3300
ctctgctgtg gtagatttcg gttagcagat aactcagatt ggagtaagac agctaccaat 3360
atcatcaaga aaagttctga taatctagga aaatctttgt cttcaagctc cattggttcc 3420
aactcaacct atcttacatc caaatctaaa tccagctcta ccacctattt caaaaggaat 3480
agccacacag ataatgtctc ctatgagcat tccttcaaca aaagtggatc actcagacag 3540
tgcttccatg gacaagtcct tgtcaaaact ggcccatgct ga 3582

<210> 36
<211> 1193
<212> PRT
<213> homo sapiens

<400> 36
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Ala Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys

305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Asp Gly Ile Ile Tyr
    370                 375                 380
Arg Ile Ser Val Val Ile Gln Asn Ile Leu Arg His Pro Glu Val Lys
385                 390                 395                 400
Val Gln Ser Lys Val Ala Glu Trp Leu Asn Ser Thr Phe Gln Asn Trp
                405                 410                 415
Asn Tyr Thr Val Tyr Val Val Asn Ile Ser Phe His Leu Ser Ala Gly
            420                 425                 430
Glu Asp Lys Ile Lys Val Lys Arg Ser Leu Glu Asp Glu Pro Arg Leu
        435                 440                 445
Val Leu Trp Ala Leu Leu Val Tyr Asn Ala Thr Asn Asn Thr Asn Leu
    450                 455                 460
Glu Gly Lys Ile Ile Gln Gln Lys Leu Leu Lys Asn Asn Glu Ser Leu
465                 470                 475                 480
Asp Glu Gly Leu Arg Leu His Thr Val Asn Val Arg Gln Leu Gly His
                485                 490                 495
Cys Leu Ala Met Glu Glu Pro Lys Gly Tyr Tyr Trp Pro Ser Ile Gln
            500                 505                 510
Pro Ser Glu Tyr Val Leu Pro Cys Pro Asp Lys Pro Gly Phe Ser Ala
        515                 520                 525
Ser Arg Ile Cys Phe Tyr Asn Ala Thr Asn Pro Leu Val Thr Tyr Trp
    530                 535                 540
Gly Pro Val Asp Ile Ser Asn Cys Leu Lys Glu Ala Asn Glu Val Ala
545                 550                 555                 560
Asn Gln Ile Leu Asn Leu Thr Ala Asp Gly Gln Asn Leu Thr Ser Ala
                565                 570                 575
Asn Ile Thr Asn Ile Val Glu Gln Val Lys Arg Ile Val Asn Lys Glu
            580                 585                 590
Glu Asn Ile Asp Ile Thr Leu Gly Ser Thr Leu Met Asn Ile Phe Ser
        595                 600                 605
Asn Ile Leu Ser Ser Ser Asp Ser Asp Leu Leu Glu Ser Ser Ser Glu
    610                 615                 620
Ala Leu Lys Thr Ile Asp Glu Leu Ala Phe Lys Ile Asp Leu Asn Ser
625                 630                 635                 640
Thr Ser His Val Asn Ile Thr Thr Arg Asn Leu Ala Leu Ser Val Ser
                645                 650                 655
Ser Leu Leu Pro Gly Thr Asn Ala Ile Ser Asn Phe Ser Ile Gly Leu
            660                 665                 670
Pro Ser Asn Asn Glu Ser Tyr Phe Gln Met Asp Phe Glu Ser Gly Gln
        675                 680                 685
Val Asp Pro Leu Ala Ser Val Ile Leu Pro Pro Asn Leu Leu Glu Asn
    690                 695                 700
Leu Ser Pro Glu Asp Ser Val Leu Val Arg Arg Ala Gln Phe Thr Phe
705                 710                 715                 720
Phe Asn Lys Thr Gly Leu Phe Gln Asp Val Gly Pro Gln Arg Lys Thr
                725                 730                 735
Leu Val Ser Tyr Val Met Ala Cys Ser Ile Gly Asn Ile Thr Ile Gln
            740                 745                 750
Asn Leu Lys Asp Pro Val Gln Ile Lys Ile Lys His Thr Arg Thr Gln
        755                 760                 765
Glu Val His His Pro Ile Cys Ala Phe Trp Asp Leu Asn Lys Asn Lys
    770                 775                 780
Ser Phe Gly Gly Trp Asn Thr Ser Gly Cys Val Ala His Arg Asp Ser
785                 790                 795                 800
Asp Ala Ser Glu Thr Val Cys Leu Cys Asn His Phe Thr His Phe Gly
                805                 810                 815
Val Leu Met Asp Leu Pro Arg Ser Ala Ser Gln Leu Asp Ala Arg Asn
            820                 825                 830
Thr Lys Val Leu Thr Phe Ile Ser Tyr Ile Gly Cys Gly Ile Ser Ala
        835                 840                 845
Ile Phe Ser Ala Ala Thr Leu Leu Thr Tyr Val Ala Phe Glu Lys Leu

850                 855                 860
Arg Arg Asp Tyr Pro Ser Lys Ile Leu Met Asn Leu Ser Thr Ala Leu
865                 870                 875                 880
Leu Phe Leu Asn Leu Leu Phe Leu Leu Asp Gly Trp Ile Thr Ser Phe
                885                 890                 895
Asn Val Asp Gly Leu Cys Ile Ala Val Ala Val Leu Leu His Phe Phe
            900                 905                 910
Leu Leu Ala Thr Phe Thr Trp Met Gly Leu Glu Ala Ile His Met Tyr
        915                 920                 925
Ile Ala Leu Val Lys Val Phe Asn Thr Tyr Ile Arg Arg Tyr Ile Leu
    930                 935                 940
Lys Phe Cys Ile Ile Gly Trp Gly Leu Pro Ala Leu Val Val Ser Val
945                 950                 955                 960
Val Leu Ala Ser Arg Asn Asn Asn Glu Val Tyr Gly Lys Glu Ser Tyr
                965                 970                 975
Gly Lys Glu Lys Gly Asp Glu Phe Cys Trp Ile Gln Asp Pro Val Ile
            980                 985                 990
Phe Tyr Val Thr Cys Ala Gly Tyr Phe Gly Val Met Phe Phe Leu Asn
        995                 1000                1005
Ile Ala Met Phe Ile Val Val Met Val Gln Ile Cys Gly Arg Asn Gly
    1010                1015                1020
Lys Arg Ser Asn Arg Thr Leu Arg Glu Glu Val Leu Arg Asn Leu Arg
1025                1030                1035                1040
Ser Val Val Ser Leu Thr Phe Leu Leu Gly Met Thr Trp Gly Phe Ala
                1045                1050                1055
Phe Phe Ala Trp Gly Pro Leu Asn Ile Pro Phe Met Tyr Leu Phe Ser
            1060                1065                1070
Ile Phe Asn Ser Leu Gln Gly Leu Phe Ile Phe Ile Phe His Cys Ala
        1075                1080                1085
Met Lys Glu Asn Val Gln Lys Gln Trp Arg Arg His Leu Cys Cys Gly
    1090                1095                1100
Arg Phe Arg Leu Ala Asp Asn Ser Asp Trp Ser Lys Thr Ala Thr Asn
1105                1110                1115                1120
Ile Ile Lys Lys Ser Ser Asp Asn Leu Gly Lys Ser Leu Ser Ser Ser
                1125                1130                1135
Ser Ile Gly Ser Asn Ser Thr Tyr Leu Thr Ser Lys Ser Lys Ser Ser
            1140                1145                1150
Ser Thr Thr Tyr Phe Lys Arg Asn Ser His Thr Asp Asn Val Ser Tyr
        1155                1160                1165
Glu His Ser Phe Asn Lys Ser Gly Ser Leu Arg Gln Cys Phe His Gly
    1170                1175                1180
Gln Val Leu Val Lys Thr Gly Pro Cys
1185                1190

<210> 37
<211> 2073
<212> DNA
<213> homo sapiens

<400> 37
atgtttcgct cagatcgaat gtggagctgc cattggaaat ggaagcccag tcctctcctg      60
ttcttatttg ctttatatat catgtgtgtt cctcactcag cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080

atcccgctcc cagcagcaga actggccagc tgtgcagacc tggggaccct ctgtcaagat 1140
ggaattatct atagaatatc cgtagtgatt cagaacatcc ttcgtcaccc tgaggtaaaa 1200
gtacagagca aggtggcaga atggctcaat tcaaccttcc aaaattggaa ctacacggtt 1260
tatgtcgtta atatcagttt tcacctgagt gctggagagg acaagattaa agtcaagaga 1320
agccttgagg atgagccaag gttggtgctt tgggcccttc tagtttacaa tgctaccaac 1380
aatactaatt tagaaggaaa aatcattcag cagaagctcc taaaaaataa tgagtccttg 1440
gatgaaggct tgaggctaca tacagtgaat gtgagacaac tgggtcattg tcttgccatg 1500
gaggaaccca aaggctacta ctggccatct atccaacctt ctgaatacgt tcttccttgt 1560
ccagacaagc ctggcttttc tgcttctcgg atatgttttt acaatgctac caacccattg 1620
gtaacctact ggggacctgt tgatatctcc aactgtttaa aagaagcaaa tgaagttgct 1680
aaccagattt taaatttaac tgctgatggg cagaacttaa cctcagccaa tattaccaac 1740
attgtggaac aggtcaaaag aattgtgaat aaagaagaaa acattgatat aacacttggc 1800
tcaactctaa tgaatatatt ttctaatatc ttaagcagtt cagacagtga cttgcttgag 1860
tcatcttctg aagctttaaa aacaattgat gaattggcct tcaagataga cctaaatagc 1920
acatcacatg tgaatattac aactcggaac ttggctctca gcgtatcatc cctgttacca 1980
gggacaaatg caatttcaaa ttttagcatt ggtcttccaa gcaataatga atcgtatttc 2040
caggtaatga gccagtggtt tctttcattt taa 2073

<210> 38
<211> 690
<212> PRT
<213> homo sapiens

<400> 38
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Ala Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350

Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Asp Gly Ile Ile Tyr
    370                 375                 380
Arg Ile Ser Val Val Ile Gln Asn Ile Leu Arg His Pro Glu Val Lys
385                 390                 395                 400
Val Gln Ser Lys Val Ala Glu Trp Leu Asn Ser Thr Phe Gln Asn Trp
                405                 410                 415
Asn Tyr Thr Val Tyr Val Val Asn Ile Ser Phe His Leu Ser Ala Gly
            420                 425                 430
Glu Asp Lys Ile Lys Val Lys Arg Ser Leu Glu Asp Glu Pro Arg Leu
        435                 440                 445
Val Leu Trp Ala Leu Leu Val Tyr Asn Ala Thr Asn Asn Thr Asn Leu
    450                 455                 460
Glu Gly Lys Ile Ile Gln Gln Lys Leu Leu Lys Asn Asn Glu Ser Leu
465                 470                 475                 480
Asp Glu Gly Leu Arg Leu His Thr Val Asn Val Arg Gln Leu Gly His
                485                 490                 495
Cys Leu Ala Met Glu Glu Pro Lys Gly Tyr Tyr Trp Pro Ser Ile Gln
            500                 505                 510
Pro Ser Glu Tyr Val Leu Pro Cys Pro Asp Lys Pro Gly Phe Ser Ala
        515                 520                 525
Ser Arg Ile Cys Phe Tyr Asn Ala Thr Asn Pro Leu Val Thr Tyr Trp
    530                 535                 540
Gly Pro Val Asp Ile Ser Asn Cys Leu Lys Glu Ala Asn Glu Val Ala
545                 550                 555                 560
Asn Gln Ile Leu Asn Leu Thr Ala Asp Gly Gln Asn Leu Thr Ser Ala
                565                 570                 575
Asn Ile Thr Asn Ile Val Glu Gln Val Lys Arg Ile Val Asn Lys Glu
            580                 585                 590
Glu Asn Ile Asp Ile Thr Leu Gly Ser Thr Leu Met Asn Ile Phe Ser
        595                 600                 605
Asn Ile Leu Ser Ser Ser Asp Ser Asp Leu Leu Glu Ser Ser Ser Glu
    610                 615                 620
Ala Leu Lys Thr Ile Asp Glu Leu Ala Phe Lys Ile Asp Leu Asn Ser
625                 630                 635                 640
Thr Ser His Val Asn Ile Thr Thr Arg Asn Leu Ala Leu Ser Val Ser
                645                 650                 655
Ser Leu Leu Pro Gly Thr Asn Ala Ile Ser Asn Phe Ser Ile Gly Leu
            660                 665                 670
Pro Ser Asn Asn Glu Ser Tyr Phe Gln Val Met Ser Gln Trp Phe Leu
        675                 680                 685
Ser Phe
    690

<210> 39
<211> 3255
<212> DNA
<213> homo sapiens

<400> 39
   atgtttcgct cagatcgaat gtggagctgc cattggaaat ggaagcccag tcctctcctg      60
   ttcttatttg ctttatatat catgtgtgtt cctcactcag cagtgtgggg atgtgccaac     120
   tgccgagtgg ttttgtccaa cccttctggg acctttactt ctccatgcta ccctaacgac     180
   tacccaaaca gccaggcttg catgtggacg ctccgagccc ccaccggtta tatcattcag     240
   ataacattta acgactttga cattgaagaa gctcccaatt gcatttatga ctcattatcc     300
   cttgataatg gagagagcca gactaaattt tgtggagcaa ctgccaaagg cctatcattt     360
   aactcaagtg cgaatgagat gcatgtgtcc ttttcaagtg actttagcat ccagaagaaa     420
   ggtttcaatg ccagctacat cagagttgcc gtgtccttaa ggaatcaaaa ggtcatttta     480
   ccccagacat cagatgctta ccaggtatct gttgcaaaaa gcatctctat tccagagctc     540
   agtgctttca cactctgctt tgaagcaacc aaagttggcc atgaagacag tgattggaca     600
   gctttctcct actcaaatgc atccttcaca caattgctca gttttggaaa ggccaagagt     660
   ggctactttc tatccatttc tgattcaaaa tgtttgttga ataatgcatt acctgtcaaa     720
   gaaaaagaag acatttttgc agaaagcttt gaacagctct gccttgtttg gaataattct     780
   ttgggctcta ttggtgtaaa tttcaaaaga aactatgaaa cagttccatg tgattctacc     840
   attagtaaag ttattcctgg gaatggaaaa ttgttgttgg gctccaatca aaatgaaatt     900
   gtctctctaa aaggggacat ttataacttt cgactttgga attttaccat gaatgccaaa     960
   atcctctcca acctcagctg taatgtgaaa gggaatgtag tcgactggca aaatgacttc    1020
   tggaatatcc caaacctagc tctgaaagct gaaagcaacc taagctgtgg ttcctacctg 1080
   atcccgctcc cagcagcaga actggccagc tgtgcagacc tggggaccct ctgtcaagat 1140
   ggaattatct atagaatatc cgtagtgatt cagaacatcc ttcgtcaccc tgaggtaaaa 1200
   gtacagagca aggtggcaga atggctcaat tcaaccttcc aaaattggaa ctacacggtt 1260
   tatgtcgtta atatcagttt tcacctgagt gctggagagg acaagattaa agtcaagaga 1320
   agccttgagg atgagccaag gttggtgctt tgggcccttc tagtttacaa tgctaccaac 1380
   aatactaatt tagaaggaaa aatcattcag cagaagctcc taaaaaataa tgagtccttg 1440
   gatgaaggct tgaggctaca tacagtgaat gtgagacaac tgggtcattg tcttgccatg 1500
   gaggaaccca aaggctacta ctggccatct atccaacctt ctgaatacgt tcttccttgt 1560
   ccagacaagc ctggcttttc tgcttctcgg atatgttttt acaatgctac caacccattg 1620
   gtaacctact ggggacctgt tgatatctcc aactgtttaa aagaagcaaa tgaagttgct 1680
   aaccagattt taaatttaac tgctgatggg cagaacttaa cctcagccaa tattaccaac 1740
   attgtggaac aggtcaaaag aattgtgaat aaagaagaaa acattgatat aacacttggc 1800
   tcaactctaa tgaatatatt ttctaatatc ttaagcagtt cagacagtga cttgcttgag 1860
   tcatcttctg aagctttaaa aacaattgat gaattggcct tcaagataga cctaaatagc 1920
   acatcacatg tgaatattac aactcggaac ttggctctca gcgtatcatc cctgttacca 1980
   gggacaaatg caatttcaaa ttttagcatt ggtcttccaa gcaataatga atcgtatttc 2040
   cagatggatt ttgagagtgg acaagtggat ccactggcat ctgtaatttt gcctccaaac 2100
   ttacttgaga atttaagtcc agaagattct gtattagtta gaagagcaca gtttactttc 2160
   ttcaacaaaa ctggactttt ccaggatgta ggaccccaaa gaaaaacttt agtgagttat 2220
   gtgatggcgt gcagtattgg aaacattact atccagaatc tgaaggatcc tgttcaaata 2280
   aaaatcaaac atacaagaac tcaggaagtg catcatccca tctgtgcctt ctgggatctg 2340
   aacaaaaaca aaagttttgg aggatggaac acgtcaggat gtgttgcaca cagagattca 2400
   gatgcaagtg agacagtctg cctgtgtaac cacttcacac actttggagt tctgatggac 2460
   cttccaagaa gtgcctcaca gttagatgca agaaacacta aagtcctcac tttcatcagc 2520
   tatattgggt gtggaatatc tgctattttt tcagcagcaa ctctcctgac atatgttgct 2580
   tttgagaaat tgcgaaggga ttatccctcc aaaatcttga tgaacctgag cacagccctg 2640
   ctgttcctga atctcctctt cctcctagat ggctggatca cctccttcaa tgtggatgga 2700
   ctttgcattg ctgttgcagt cctgttgcat ttcttccttc tggcaacctt tacctggatg 2760
   gggctagaag caattcacat gtacattgct ctagttaaag tatttaacac ttacattcgc 2820
   cgatacattc taaaattctg catcattggc tggggtttgc ctgccttagt ggtgtcagtt 2880
   gttctagcga gcagaaacaa caatgaagtc tatggaaaag aaagttatgg gaaagaaaaa 2940
   ggtgatgaat tctgttggat tcaagatcca gtcatatttt atgtgacctg tgctgggtat 3000
   tttggagtca tgttttttct gaacattgcc atgttcattg tggtaatggt gcagatctgt 3060
   gggaggaatg gcaagagaag caaccggacc ctgagagaag aagtgttaag gaacctgcgc 3120
   agtgtggtta gcttgacctt tctgttgggc atgacatggg gttttgcatt ctttgcctgg 3180
   ggacccttaa atatcccctt catgtacctc ttctccatct tcaattcatt acaaggtaag 3240
   ataaattgta catga 3255

<210> 40
<211> 1084
<212> PRT
<213> homo sapiens

<400> 40
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Ala Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val

            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Asp Gly Ile Ile Tyr
    370                 375                 380
Arg Ile Ser Val Val Ile Gln Asn Ile Leu Arg His Pro Glu Val Lys
385                 390                 395                 400
Val Gln Ser Lys Val Ala Glu Trp Leu Asn Ser Thr Phe Gln Asn Trp
                405                 410                 415
Asn Tyr Thr Val Tyr Val Val Asn Ile Ser Phe His Leu Ser Ala Gly
            420                 425                 430
Glu Asp Lys Ile Lys Val Lys Arg Ser Leu Glu Asp Glu Pro Arg Leu
        435                 440                 445
Val Leu Trp Ala Leu Leu Val Tyr Asn Ala Thr Asn Asn Thr Asn Leu
    450                 455                 460
Glu Gly Lys Ile Ile Gln Gln Lys Leu Leu Lys Asn Asn Glu Ser Leu
465                 470                 475                 480
Asp Glu Gly Leu Arg Leu His Thr Val Asn Val Arg Gln Leu Gly His
                485                 490                 495
Cys Leu Ala Met Glu Glu Pro Lys Gly Tyr Tyr Trp Pro Ser Ile Gln
            500                 505                 510
Pro Ser Glu Tyr Val Leu Pro Cys Pro Asp Lys Pro Gly Phe Ser Ala
        515                 520                 525
Ser Arg Ile Cys Phe Tyr Asn Ala Thr Asn Pro Leu Val Thr Tyr Trp
    530                 535                 540
Gly Pro Val Asp Ile Ser Asn Cys Leu Lys Glu Ala Asn Glu Val Ala
545                 550                 555                 560
Asn Gln Ile Leu Asn Leu Thr Ala Asp Gly Gln Asn Leu Thr Ser Ala
                565                 570                 575
Asn Ile Thr Asn Ile Val Glu Gln Val Lys Arg Ile Val Asn Lys Glu
            580                 585                 590
Glu Asn Ile Asp Ile Thr Leu Gly Ser Thr Leu Met Asn Ile Phe Ser
        595                 600                 605
Asn Ile Leu Ser Ser Ser Asp Ser Asp Leu Leu Glu Ser Ser Ser Glu
    610                 615                 620
Ala Leu Lys Thr Ile Asp Glu Leu Ala Phe Lys Ile Asp Leu Asn Ser
625                 630                 635                 640
Thr Ser His Val Asn Ile Thr Thr Arg Asn Leu Ala Leu Ser Val Ser
                645                 650                 655
Ser Leu Leu Pro Gly Thr Asn Ala Ile Ser Asn Phe Ser Ile Gly Leu
            660                 665                 670
Pro Ser Asn Asn Glu Ser Tyr Phe Gln Met Asp Phe Glu Ser Gly Gln
        675                 680                 685
Val Asp Pro Leu Ala Ser Val Ile Leu Pro Pro Asn Leu Leu Glu Asn
    690                 695                 700
Leu Ser Pro Glu Asp Ser Val Leu Val Arg Arg Ala Gln Phe Thr Phe
705                 710                 715                 720
Phe Asn Lys Thr Gly Leu Phe Gln Asp Val Gly Pro Gln Arg Lys Thr

                725                 730                 735
Leu Val Ser Tyr Val Met Ala Cys Ser Ile Gly Asn Ile Thr Ile Gln
            740                 745                 750
Asn Leu Lys Asp Pro Val Gln Ile Lys Ile Lys His Thr Arg Thr Gln
        755                 760                 765
Glu Val His His Pro Ile Cys Ala Phe Trp Asp Leu Asn Lys Asn Lys
    770                 775                 780
Ser Phe Gly Gly Trp Asn Thr Ser Gly Cys Val Ala His Arg Asp Ser
785                 790                 795                 800
Asp Ala Ser Glu Thr Val Cys Leu Cys Asn His Phe Thr His Phe Gly
                805                 810                 815
Val Leu Met Asp Leu Pro Arg Ser Ala Ser Gln Leu Asp Ala Arg Asn
            820                 825                 830
Thr Lys Val Leu Thr Phe Ile Ser Tyr Ile Gly Cys Gly Ile Ser Ala
        835                 840                 845
Ile Phe Ser Ala Ala Thr Leu Leu Thr Tyr Val Ala Phe Glu Lys Leu
    850                 855                 860
Arg Arg Asp Tyr Pro Ser Lys Ile Leu Met Asn Leu Ser Thr Ala Leu
865                 870                 875                 880
Leu Phe Leu Asn Leu Leu Phe Leu Leu Asp Gly Trp Ile Thr Ser Phe
                885                 890                 895
Asn Val Asp Gly Leu Cys Ile Ala Val Ala Val Leu Leu His Phe Phe
            900                 905                 910
Leu Leu Ala Thr Phe Thr Trp Met Gly Leu Glu Ala Ile His Met Tyr
        915                 920                 925
Ile Ala Leu Val Lys Val Phe Asn Thr Tyr Ile Arg Arg Tyr Ile Leu
    930                 935                 940
Lys Phe Cys Ile Ile Gly Trp Gly Leu Pro Ala Leu Val Val Ser Val
945                 950                 955                 960
Val Leu Ala Ser Arg Asn Asn Asn Glu Val Tyr Gly Lys Glu Ser Tyr
                965                 970                 975
Gly Lys Glu Lys Gly Asp Glu Phe Cys Trp Ile Gln Asp Pro Val Ile
            980                 985                 990
Phe Tyr Val Thr Cys Ala Gly Tyr Phe Gly Val Met Phe Phe Leu Asn
        995                 1000                1005
Ile Ala Met Phe Ile Val Val Met Val Gln Ile Cys Gly Arg Asn Gly
    1010                1015                1020
Lys Arg Ser Asn Arg Thr Leu Arg Glu Glu Val Leu Arg Asn Leu Arg
1025                1030                1035                1040
Ser Val Val Ser Leu Thr Phe Leu Leu Gly Met Thr Trp Gly Phe Ala
                1045                1050                1055
Phe Phe Ala Trp Gly Pro Leu Asn Ile Pro Phe Met Tyr Leu Phe Ser
            1060                1065                1070
Ile Phe Asn Ser Leu Gln Gly Lys Ile Asn Cys Thr
        1075                1080

<210> 41
<211> 3666
<212> DNA
<213> homo sapiens

<400> 41
atgtttcgct cagatcgaat gtggagctgc cattggaaat ggaagcccag tcctctcctg 60
ttcttatttg ctttatatat catgtgtgtt cctcactcag tgtggggatg tgccaactgc 120
cgagtggttt tgtccaaccc ttctgggacc tttacttctc catgctaccc taacgactac 180
ccaaacagcc aggcttgcat gtggacgctc cgagccccca ccggttatat cattcagata 240
acatttaacg actttgacat tgaagaagct cccaattgca tttatgactc attatccctt 300
gataatggag agagccagac taaattttgt ggagcaactg ccaaaggcct atcatttaac 360
tcaagtgcga atgagatgca tgtgtccttt tcaagtgact ttagcatcca gaagaaaggt 420
ttcaatgcca gctacatcag agttgccgtg tccttaagga atcaaaaggt cattttaccc 480
cagacatcag atgcttacca ggtatctgtt gcaaaaagca tctctattcc agagctcagt 540
gctttcacac tctgctttga agcaaccaaa gttggccatg aagacagtga ttggacagct 600
ttctcctact caaatgcatc cttcacacaa ttgctcagtt ttggaaaggc caagagtggc 660
tactttctat ccatttctga ttcaaaatgt ttgttgaata atgcattacc tgtcaaagaa 720
aaagaagaca tttttgcaga aagctttgaa cagctctgcc ttgtttggaa taattctttg 780
ggctctattg gtgtaaattt caaaagaaac tatgaaacag ttccatgtga ttctaccatt 840
agtaaagtta ttcctggaaa tgggaaattg ttgttgggct ccaatcaaaa tgaaattgtc 900
tctctaaaag ggacatttta taactttcga ctttggaatt ttaccatgaa tgccaaaatc 960

ctctccaacc tcagctgtaa tgtgaaaggg aatgtagtcg actggcaaaa tgacttctgg 1020
aatatcccaa acctagctct gaaagctgaa agcaacctaa gctgtggttc ctacctgatc 1080
ccgctcccag cagcagaact ggccagctgt gcagacctgg ggaccctctg tcaagatgga 1140
attatctata gaatatccgt agtgattcag aacatccttc gtcaccctga ggtaaaagta 1200
cagagcaagg tggcagaatg gctcaattca accttccaaa attggaacta cacggtttat 1260
gtcgttaata tcagttttca cctgagtgct ggagaggaca agattaaagt caagagaagc 1320
cttgaggatg agccaaggtt ggtgctttgg gcccttctag tttacaatgc taccaacaat 1380
actaatttag aaggaaaaat cattcagcag aagctcctaa aaaataatga gtccttggat 1440
gaaggcttga ggctacatac agtgaatgtg agacaactgg gtcattgtct tgccatggag 1500
gaacccaaag gctactactg gccatctatc caaccttctg aatacgttct tccttgtcca 1560
gacaagcctg gcttttctgc ttctcggata tgtttttaca atgctaccaa cccattggta 1620
acctactggg gacctgttga tatctccaac tgtttaaaag aagcaaatga agttgctaac 1680
cagattttaa atttaactgc tgatgggcag aacttaacct cagccaatat taccaacatt 1740
gtggaacagg tcaaaagaat tgtgaataaa gaagaaaaca ttgatataac acttggctca 1800
actctaatga atatattttc taatatctta agcagttcag acagtgactt gcttgagtca 1860
tcttctgaag ctttaaaaac aattgatgaa ttggccttca agatagacct aaatagcaca 1920
tcacatgtga atattacaac tcggaacttg gctctcagcg tatcatccct gttaccaggg 1980
acaaatgcaa tttcaaattt tagcattggt cttccaagca ataatgaatc gtatttccag 2040
atggattttg agagtggaca agtggatcca ctggcatctg taattttgcc tccaaactta 2100
cttgagaatt taagtccaga agattctgta ttagttagaa gagcacagtt tactttcttc 2160
aacaaaactg gacttttcca ggatgtagga ccccaaagaa aaactttagt gagttatgtg 2220
atggcgtgca gtattggaaa cattactatc cagaatctga aggatcctgt tcaaataaaa 2280
atcaaacata caagaactca ggaagtgcat catcccatct gtgccttctg ggatctgaac 2340
aaaaacaaaa gttttggagg atggaacacg tcaggatgtg ttgcacacag agattcagat 2400
gcaagtgaga cagtctgcct gtgtaaccac ttcacacact ttggagttct gatggacctt 2460
ccaagaagtg cctcacagtt agatgcaaga aacactaaag tcctcacttt catcagctat 2520
attgggtgtg gaatatctgc tattttttca gcagcaactc tcctgacata tgttgctttt 2580
gagaaattgc gaagggatta tccctccaaa atcttgatga acctgagcac agccctgctg 2640
ttcctgaatc tcctcttcct cctagatggc tggatcacct ccttcaatgt ggatggactt 2700
tgcattgctg ttgcagtcct gttgcatttc ttccttctgg caacctttac ctggatgggg 2760
ctagaagcaa ttcacatgta cattgctcta gttaaagtat ttaacactta cattcgccga 2820
tacattctaa aattctgcat cattggctgg ggtttgcctg ccttagtggt gtcagttgtt 2880
ctagcgagca gaaacaacaa tgaagtctat ggaaaagaaa gttatgggaa agaaaaaggt 2940
gatgaattct gttggattca agatccagtc atattttatg tgacctgtgc tgggtatttt 3000
ggagtcatgt tttttctgaa cattgccatg ttcattgtgg taatggtgca gatctgtggg 3060
aggaatggca agagaagcaa ccggaccctg agagaagaag tgttaaggaa cctgcgcagt 3120
gtggttagct tgacctttct gttgggcatg acatggggtt ttgcattctt tgcctgggga 3180
cccttaaata tccccttcat gtacctcttc tccatcttca attcattaca aggcttattt 3240
atattcatct tccactgtgc tatgaaggag aatgttcaga aacagtggcg gcggcatctc 3300
tgctgtggta gatttcggtt agcagataac tcagattgga gtaagacagc taccaatatc 3360
atcaagaaaa gttctgataa tctaggaaaa tctttgtctt caagctccat tggttccaac 3420
tcaacctatc ttacatccaa atctaaatcc agctctacca cctatttcaa aaggaatagc 3480
cacacagaca gtgcttccat ggacaagtcc ttgtcaaaac tggcccatgc tgatggagat 3540
caaacatcaa tcatccctgt ccatcaggtc attgataagg tcaagggtta ttgcaatgct 3600
cattcagaca acttctataa aaatattatc atgtcagaca ccttcagcca cagcacaaag 3660
ttttaa 3666

<210> 42
<211> 1221
<212> PRT
<213> homo sapiens

<400> 42
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Val Trp Gly Cys Ala Asn Cys Arg Val Val Leu Ser Asn Pro Ser
        35                  40                  45
Gly Thr Phe Thr Ser Pro Cys Tyr Pro Asn Asp Tyr Pro Asn Ser Gln
    50                  55                  60
Ala Cys Met Trp Thr Leu Arg Ala Pro Thr Gly Tyr Ile Ile Gln Ile
65                  70                  75                  80
Thr Phe Asn Asp Phe Asp Ile Glu Glu Ala Pro Asn Cys Ile Tyr Asp
                85                  90                  95
Ser Leu Ser Leu Asp Asn Gly Glu Ser Gln Thr Lys Phe Cys Gly Ala
            100                 105                 110
Thr Ala Lys Gly Leu Ser Phe Asn Ser Ser Ala Asn Glu Met His Val

        115                 120                 125
Ser Phe Ser Ser Asp Phe Ser Ile Gln Lys Lys Gly Phe Asn Ala Ser
    130                 135                 140
Tyr Ile Arg Val Ala Val Ser Leu Arg Asn Gln Lys Val Ile Leu Pro
145                 150                 155                 160
Gln Thr Ser Asp Ala Tyr Gln Val Ser Val Ala Lys Ser Ile Ser Ile
                165                 170                 175
Pro Glu Leu Ser Ala Phe Thr Leu Cys Phe Glu Ala Thr Lys Val Gly
            180                 185                 190
His Glu Asp Ser Asp Trp Thr Ala Phe Ser Tyr Ser Asn Ala Ser Phe
        195                 200                 205
Thr Gln Leu Leu Ser Phe Gly Lys Ala Lys Ser Gly Tyr Phe Leu Ser
    210                 215                 220
Ile Ser Asp Ser Lys Cys Leu Leu Asn Asn Ala Leu Pro Val Lys Glu
225                 230                 235                 240
Lys Glu Asp Ile Phe Ala Glu Ser Phe Glu Gln Leu Cys Leu Val Trp
                245                 250                 255
Asn Asn Ser Leu Gly Ser Ile Gly Val Asn Phe Lys Arg Asn Tyr Glu
            260                 265                 270
Thr Val Pro Cys Asp Ser Thr Ile Ser Lys Val Ile Pro Gly Asn Gly
        275                 280                 285
Lys Leu Leu Leu Gly Ser Asn Gln Asn Glu Ile Val Ser Leu Lys Gly
    290                 295                 300
Asp Ile Tyr Asn Phe Arg Leu Trp Asn Phe Thr Met Asn Ala Lys Ile
305                 310                 315                 320
Leu Ser Asn Leu Ser Cys Asn Val Lys Gly Asn Val Val Asp Trp Gln
                325                 330                 335
Asn Asp Phe Trp Asn Ile Pro Asn Leu Ala Leu Lys Ala Glu Ser Asn
            340                 345                 350
Leu Ser Cys Gly Ser Tyr Leu Ile Pro Leu Pro Ala Ala Glu Leu Ala
        355                 360                 365
Ser Cys Ala Asp Leu Gly Thr Leu Cys Gln Asp Gly Ile Ile Tyr Arg
    370                 375                 380
Ile Ser Val Val Ile Gln Asn Ile Leu Arg His Pro Glu Val Lys Val
385                 390                 395                 400
Gln Ser Lys Val Ala Glu Trp Leu Asn Ser Thr Phe Gln Asn Trp Asn
                405                 410                 415
Tyr Thr Val Tyr Val Val Asn Ile Ser Phe His Leu Ser Ala Gly Glu
            420                 425                 430
Asp Lys Ile Lys Val Lys Arg Ser Leu Glu Asp Glu Pro Arg Leu Val
        435                 440                 445
Leu Trp Ala Leu Leu Val Tyr Asn Ala Thr Asn Asn Thr Asn Leu Glu
    450                 455                 460
Gly Lys Ile Ile Gln Gln Lys Leu Leu Lys Asn Asn Glu Ser Leu Asp
465                 470                 475                 480
Glu Gly Leu Arg Leu His Thr Val Asn Val Arg Gln Leu Gly His Cys
                485                 490                 495
Leu Ala Met Glu Glu Pro Lys Gly Tyr Tyr Trp Pro Ser Ile Gln Pro
            500                 505                 510
Ser Glu Tyr Val Leu Pro Cys Pro Asp Lys Pro Gly Phe Ser Ala Ser
        515                 520                 525
Arg Ile Cys Phe Tyr Asn Ala Thr Asn Pro Leu Val Thr Tyr Trp Gly
    530                 535                 540
Pro Val Asp Ile Ser Asn Cys Leu Lys Glu Ala Asn Glu Val Ala Asn
545                 550                 555                 560
Gln Ile Leu Asn Leu Thr Ala Asp Gly Gln Asn Leu Thr Ser Ala Asn
                565                 570                 575
Ile Thr Asn Ile Val Glu Gln Val Lys Arg Ile Val Asn Lys Glu Glu
            580                 585                 590
Asn Ile Asp Ile Thr Leu Gly Ser Thr Leu Met Asn Ile Phe Ser Asn
        595                 600                 605
Ile Leu Ser Ser Ser Asp Ser Asp Leu Leu Glu Ser Ser Ser Glu Ala
    610                 615                 620
Leu Lys Thr Ile Asp Glu Leu Ala Phe Lys Ile Asp Leu Asn Ser Thr
625                 630                 635                 640
Ser His Val Asn Ile Thr Thr Arg Asn Leu Ala Leu Ser Val Ser Ser
                645                 650                 655
Leu Leu Pro Gly Thr Asn Ala Ile Ser Asn Phe Ser Ile Gly Leu Pro

            660                 665                 670
Ser Asn Asn Glu Ser Tyr Phe Gln Met Asp Phe Glu Ser Gly Gln Val
        675                 680                 685
Asp Pro Leu Ala Ser Val Ile Leu Pro Pro Asn Leu Leu Glu Asn Leu
    690                 695                 700
Ser Pro Glu Asp Ser Val Leu Val Arg Arg Ala Gln Phe Thr Phe Phe
705                 710                 715                 720
Asn Lys Thr Gly Leu Phe Gln Asp Val Gly Pro Gln Arg Lys Thr Leu
                725                 730                 735
Val Ser Tyr Val Met Ala Cys Ser Ile Gly Asn Ile Thr Ile Gln Asn
            740                 745                 750
Leu Lys Asp Pro Val Gln Ile Lys Ile Lys His Thr Arg Thr Gln Glu
        755                 760                 765
Val His His Pro Ile Cys Ala Phe Trp Asp Leu Asn Lys Asn Lys Ser
    770                 775                 780
Phe Gly Gly Trp Asn Thr Ser Gly Cys Val Ala His Arg Asp Ser Asp
785                 790                 795                 800
Ala Ser Glu Thr Val Cys Leu Cys Asn His Phe Thr His Phe Gly Val
                805                 810                 815
Leu Met Asp Leu Pro Arg Ser Ala Ser Gln Leu Asp Ala Arg Asn Thr
            820                 825                 830
Lys Val Leu Thr Phe Ile Ser Tyr Ile Gly Cys Gly Ile Ser Ala Ile
        835                 840                 845
Phe Ser Ala Ala Thr Leu Leu Thr Tyr Val Ala Phe Glu Lys Leu Arg
    850                 855                 860
Arg Asp Tyr Pro Ser Lys Ile Leu Met Asn Leu Ser Thr Ala Leu Leu
865                 870                 875                 880
Phe Leu Asn Leu Leu Phe Leu Leu Asp Gly Trp Ile Thr Ser Phe Asn
                885                 890                 895
Val Asp Gly Leu Cys Ile Ala Val Ala Val Leu Leu His Phe Phe Leu
            900                 905                 910
Leu Ala Thr Phe Thr Trp Met Gly Leu Glu Ala Ile His Met Tyr Ile
        915                 920                 925
Ala Leu Val Lys Val Phe Asn Thr Tyr Ile Arg Arg Tyr Ile Leu Lys
    930                 935                 940
Phe Cys Ile Ile Gly Trp Gly Leu Pro Ala Leu Val Val Ser Val Val
945                 950                 955                 960
Leu Ala Ser Arg Asn Asn Asn Glu Val Tyr Gly Lys Glu Ser Tyr Gly
                965                 970                 975
Lys Glu Lys Gly Asp Glu Phe Cys Trp Ile Gln Asp Pro Val Ile Phe
            980                 985                 990
Tyr Val Thr Cys Ala Gly Tyr Phe Gly Val Met Phe Phe Leu Asn Ile
        995                 1000                1005
Ala Met Phe Ile Val Val Met Val Gln Ile Cys Gly Arg Asn Gly Lys
    1010                1015                1020
Arg Ser Asn Arg Thr Leu Arg Glu Glu Val Leu Arg Asn Leu Arg Ser
1025                1030                1035                1040
Val Val Ser Leu Thr Phe Leu Leu Gly Met Thr Trp Gly Phe Ala Phe
                1045                1050                1055
Phe Ala Trp Gly Pro Leu Asn Ile Pro Phe Met Tyr Leu Phe Ser Ile
            1060                1065                1070
Phe Asn Ser Leu Gln Gly Leu Phe Ile Phe Ile Phe His Cys Ala Met
        1075                1080                1085
Lys Glu Asn Val Gln Lys Gln Trp Arg Arg His Leu Cys Cys Gly Arg
    1090                1095                1100
Phe Arg Leu Ala Asp Asn Ser Asp Trp Ser Lys Thr Ala Thr Asn Ile
1105                1110                1115                1120
Ile Lys Lys Ser Ser Asp Asn Leu Gly Lys Ser Leu Ser Ser Ser Ser
                1125                1130                1135
Ile Gly Ser Asn Ser Thr Tyr Leu Thr Ser Lys Ser Lys Ser Ser Ser
            1140                1145                1150
Thr Thr Tyr Phe Lys Arg Asn Ser His Thr Asp Ser Ala Ser Met Asp
        1155                1160                1165
Lys Ser Leu Ser Lys Leu Ala His Ala Asp Gly Asp Gln Thr Ser Ile
    1170                1175                1180
Ile Pro Val His Gln Val Ile Asp Lys Val Lys Gly Tyr Cys Asn Ala
1185                1190                1195                1200
His Ser Asp Asn Phe Tyr Lys Asn Ile Ile Met Ser Asp Thr Phe Ser

1205                    1210                    1215
His Ser Thr Lys Phe
            1220

<210> 43
<211> 3579
<212> DNA
<213> homo sapiens

<400> 43
atgtttcgct cagatcgaat gtggagctgc cattggaaat ggaagcccag tcctctcctg      60
ttcttatttg ctttatatat catgtgtgtt cctcactcag tgtggggatg tgccaactgc     120
cgagtggttt tgtccaaccc ttctgggacc tttacttctc catgctaccc taacgactac     180
ccaaacagcc aggcttgcat gtggacgctc cgagccccca ccggttatat cattcagata     240
acatttaacg actttgacat tgaagaagct cccaattgca tttatgactc attatccctt     300
gataatggag agagccagac taaattttgt ggagcaactg ccaaaggcct atcatttaac     360
tcaagtgcga atgagatgca tgtgtccttt tcaagtgact ttagcatcca gaagaaaggt     420
ttcaatgcca gctacatcag agttgccgtg tccttaagga atcaaaaggt cattttaccc     480
cagacatcag atgcttacca ggtatctgtt gcaaaaagca tctctattcc agagctcagt     540
gctttcacac tctgctttga agcaaccaaa gttggccatg aagacagtga ttggacagct     600
ttctcctact caaatgcatc cttcacacaa ttgctcagtt ttggaaaggc caagagtggc     660
tactttctat ccatttctga ttcaaaatgt ttgttgaata atgcattacc tgtcaaagaa     720
aaagaagaca tttttgcaga aagctttgaa cagctctgcc ttgtttggaa taattctttg      780
ggctctattg gtgtaaattt caaaagaaac tatgaaacag ttccatgtga ttctaccatt     840
agtaaagtta ttcctgggaa tgggaaattg ttgttgggct ccaatcaaaa tgaaattgtc     900
tctctaaaag ggacatttta taactttcga ctttggaatt ttaccatgaa tgccaaaatc     960
ctctccaacc tcagctgtaa tgtgaaaggg aatgtagtcg actggcaaaa tgacttctgg    1020
aatatcccaa acctagctct gaaagctgaa agcaacctaa gctgtggttc ctacctgatc    1080
ccgctcccag cagcagaact ggccagctgt gcagacctgg ggaccctctg tcaagatgga    1140
attatctata gaatatccgt agtgattcag aacatccttc gtcaccctga ggtaaaagta    1200
cagagcaagg tggcagaatg gctcaattca accttccaaa attggaacta cacggtttat    1260
gtcgttaata tcagttttca cctgagtgct ggagaggaca agattaaagt caagagaagc    1320
cttgaggatg agccaaggtt ggtgctttgg gcccttctag tttacaatgc taccaacaat    1380
actaatttag aaggaaaaat cattcagcag aagctcctaa aaaataatga gtccttggat    1440
gaaggcttga ggctacatac agtgaatgtg agacaactgg gtcattgtct tgccatggag    1500
gaacccaaag gctactactg gccatctatc caaccttctg aatacgttct tccttgtcca    1560
gacaagcctg gcttttctgc ttctcggata tgtttttaca atgctaccaa cccattggta    1620
acctactggg gacctgttga tatctccaac tgtttaaaag aagcaaatga agttgctaac    1680
cagattttaa atttaactgc tgatgggcag aacttaacct cagccaatat taccaacatt    1740
gtggaacagg tcaaaagaat tgtgaataaa gaagaaaaca ttgatataac acttggctca    1800
actctaatga atatattttc taatatctta agcagttcag acagtgactt gcttgagtca    1860
tcttctgaag ctttaaaaac aattgatgaa ttggccttca agatagacct aaatagcaca    1920
tcacatgtga atattacaac tcggaacttg gctctcagcg tatcatccct gttaccaggg    1980
acaaatgcaa tttcaaattt tagcattggt cttccaagca ataatgaatc gtatttccag    2040
atggattttg agagtggaca agtggatcca ctggcatctg taattttgcc tccaaactta    2100
cttgagaatt taagtccaga agattctgta ttagttagaa gagcacagtt tactttcttc    2160
aacaaaactg gacttttcca ggatgtagga ccccaaagaa aaactttagt gagttatgtg    2220
atggcgtgca gtattggaaa cattactatc cagaatctga aggatcctgt tcaaataaaa    2280
atcaaacata caagaactca ggaagtgcat catcccatct gtgccttctg ggatctgaac    2340
aaaaacaaaa gttttggagg atggaacacg tcaggatgtg ttgcacacag agattcagat    2400
gcaagtgaga cagtctgcct gtgtaaccac ttcacacact ttggagttct gatggacctt    2460
ccaagaagtg cctcacagtt agatgcaaga aacactaaag tcctcacttt catcagctat    2520
attgggtgtg gaatatctgc tattttttca gcagcaactc tcctgacata tgttgctttt    2580
gagaaattgc gaagggatta tccctccaaa atcttgatga acctgagcac agccctgctg    2640
ttcctgaatc tcctcttcct cctagatggc tggatcacct ccttcaatgt ggatggactt    2700
tgcattgctg ttgcagtcct gttgcatttc ttccttctgg caacctttac ctggatgggg    2760
ctagaagcaa ttcacatgta cattgctcta gttaaagtat ttaacactta cattcgccga    2820
tacattctaa aattctgcat cattggctgg ggtttgcctg ccttagtggt gtcagttgtt    2880
ctagcgagca gaaacaacaa tgaagtctat ggaaaagaaa gttatgggaa agaaaaaggt    2940
gatgaattct gttggattca agatccagtc atattttatg tgacctgtgc tgggtatttt    3000
ggagtcatgt tttttctgaa cattgccatg ttcattgtgg taatggtgca gatctgtggg    3060
aggaatggca agagaagcaa ccggaccctg agagaagaag tgttaaggaa cctgcgcagt    3120
gtggttagct tgacctttct gttgggcatg acatggggtt ttgcattctt tgcctgggga    3180
cccttaaata tccccttcat gtacctcttc tccatcttca attcattaca aggcttattt    3240
atattcatct tccactgtgc tatgaaggag aatgttcaga aacagtggcg gcggcatctc    3300
tgctgtggta gatttcggtt agcagataac tcagattgga gtaagacagc taccaatatc    3360
atcaagaaaa gttctgataa tctaggaaaa tctttgtctt caagctccat tggttccaac    3420
tcaacctatc ttacatccaa atctaaatcc agctctacca cctatttcaa aaggaatagc    3480

cacacagata atgtctccta tgagcattcc ttcaacaaaa gtggatcact cagacagtgc 3540
ttccatggac aagtccttgt caaaactggc ccatgctga 3579

<210> 44
<211> 1192
<212> PRT
<213> homo sapiens

<400> 44
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Val Trp Gly Cys Ala Asn Cys Arg Val Val Leu Ser Asn Pro Ser
        35                  40                  45
Gly Thr Phe Thr Ser Pro Cys Tyr Pro Asn Asp Tyr Pro Asn Ser Gln
    50                  55                  60
Ala Cys Met Trp Thr Leu Arg Ala Pro Thr Gly Tyr Ile Ile Gln Ile
65                  70                  75                  80
Thr Phe Asn Asp Phe Asp Ile Glu Glu Ala Pro Asn Cys Ile Tyr Asp
                85                  90                  95
Ser Leu Ser Leu Asp Asn Gly Glu Ser Gln Thr Lys Phe Cys Gly Ala
            100                 105                 110
Thr Ala Lys Gly Leu Ser Phe Asn Ser Ser Ala Asn Glu Met His Val
        115                 120                 125
Ser Phe Ser Ser Asp Phe Ser Ile Gln Lys Lys Gly Phe Asn Ala Ser
    130                 135                 140
Tyr Ile Arg Val Ala Val Ser Leu Arg Asn Gln Lys Val Ile Leu Pro
145                 150                 155                 160
Gln Thr Ser Asp Ala Tyr Gln Val Ser Val Ala Lys Ser Ile Ser Ile
                165                 170                 175
Pro Glu Leu Ser Ala Phe Thr Leu Cys Phe Glu Ala Thr Lys Val Gly
            180                 185                 190
His Glu Asp Ser Asp Trp Thr Ala Phe Ser Tyr Ser Asn Ala Ser Phe
        195                 200                 205
Thr Gln Leu Leu Ser Phe Gly Lys Ala Lys Ser Gly Tyr Phe Leu Ser
    210                 215                 220
Ile Ser Asp Ser Lys Cys Leu Leu Asn Asn Ala Leu Pro Val Lys Glu
225                 230                 235                 240
Lys Glu Asp Ile Phe Ala Glu Ser Phe Glu Gln Leu Cys Leu Val Trp
                245                 250                 255
Asn Asn Ser Leu Gly Ser Ile Gly Val Asn Phe Lys Arg Asn Tyr Glu
            260                 265                 270
Thr Val Pro Cys Asp Ser Thr Ile Ser Lys Val Ile Pro Gly Asn Gly
        275                 280                 285
Lys Leu Leu Leu Gly Ser Asn Gln Asn Glu Ile Val Ser Leu Lys Gly
    290                 295                 300
Asp Ile Tyr Asn Phe Arg Leu Trp Asn Phe Thr Met Asn Ala Lys Ile
305                 310                 315                 320
Leu Ser Asn Leu Ser Cys Asn Val Lys Gly Asn Val Val Asp Trp Gln
                325                 330                 335
Asn Asp Phe Trp Asn Ile Pro Asn Leu Ala Leu Lys Ala Glu Ser Asn
            340                 345                 350
Leu Ser Cys Gly Ser Tyr Leu Ile Pro Leu Pro Ala Ala Glu Leu Ala
        355                 360                 365
Ser Cys Ala Asp Leu Gly Thr Leu Cys Gln Asp Gly Ile Ile Tyr Arg
    370                 375                 380
Ile Ser Val Val Ile Gln Asn Ile Leu Arg His Pro Glu Val Lys Val
385                 390                 395                 400
Gln Ser Lys Val Ala Glu Trp Leu Asn Ser Thr Phe Gln Asn Trp Asn
                405                 410                 415
Tyr Thr Val Tyr Val Val Asn Ile Ser Phe His Leu Ser Ala Gly Glu
            420                 425                 430
Asp Lys Ile Lys Val Lys Arg Ser Leu Glu Asp Glu Pro Arg Leu Val
        435                 440                 445
Leu Trp Ala Leu Leu Val Tyr Asn Ala Thr Asn Asn Thr Asn Leu Glu
    450                 455                 460
Gly Lys Ile Ile Gln Gln Lys Leu Leu Lys Asn Asn Glu Ser Leu Asp

465                 470                 475                 480
Glu Gly Leu Arg Leu His Thr Val Asn Val Arg Gln Leu Gly His Cys
                485                 490                 495
Leu Ala Met Glu Glu Pro Lys Gly Tyr Tyr Trp Pro Ser Ile Gln Pro
            500                 505                 510
Ser Glu Tyr Val Leu Pro Cys Pro Asp Lys Pro Gly Phe Ser Ala Ser
        515                 520                 525
Arg Ile Cys Phe Tyr Asn Ala Thr Asn Pro Leu Val Thr Tyr Trp Gly
    530                 535                 540
Pro Val Asp Ile Ser Asn Cys Leu Lys Glu Ala Asn Glu Val Ala Asn
545                 550                 555                 560
Gln Ile Leu Asn Leu Thr Ala Asp Gly Gln Asn Leu Thr Ser Ala Asn
                565                 570                 575
Ile Thr Asn Ile Val Glu Gln Val Lys Arg Ile Val Asn Lys Glu Glu
            580                 585                 590
Asn Ile Asp Ile Thr Leu Gly Ser Thr Leu Met Asn Ile Phe Ser Asn
        595                 600                 605
Ile Leu Ser Ser Ser Asp Ser Asp Leu Leu Glu Ser Ser Ser Glu Ala
    610                 615                 620
Leu Lys Thr Ile Asp Glu Leu Ala Phe Lys Ile Asp Leu Asn Ser Thr
625                 630                 635                 640
Ser His Val Asn Ile Thr Thr Arg Asn Leu Ala Leu Ser Val Ser Ser
                645                 650                 655
Leu Leu Pro Gly Thr Asn Ala Ile Ser Asn Phe Ser Ile Gly Leu Pro
            660                 665                 670
Ser Asn Asn Glu Ser Tyr Phe Gln Met Asp Phe Glu Ser Gly Gln Val
        675                 680                 685
Asp Pro Leu Ala Ser Val Ile Leu Pro Pro Asn Leu Leu Glu Asn Leu
    690                 695                 700
Ser Pro Glu Asp Ser Val Leu Val Arg Arg Ala Gln Phe Thr Phe Phe
705                 710                 715                 720
Asn Lys Thr Gly Leu Phe Gln Asp Val Gly Pro Gln Arg Lys Thr Leu
                725                 730                 735
Val Ser Tyr Val Met Ala Cys Ser Ile Gly Asn Ile Thr Ile Gln Asn
            740                 745                 750
Leu Lys Asp Pro Val Gln Ile Lys Ile Lys His Thr Arg Thr Gln Glu
        755                 760                 765
Val His His Pro Ile Cys Ala Phe Trp Asp Leu Asn Lys Asn Lys Ser
    770                 775                 780
Phe Gly Gly Trp Asn Thr Ser Gly Cys Val Ala His Arg Asp Ser Asp
785                 790                 795                 800
Ala Ser Glu Thr Val Cys Leu Cys Asn His Phe Thr His Phe Gly Val
                805                 810                 815
Leu Met Asp Leu Pro Arg Ser Ala Ser Gln Leu Asp Ala Arg Asn Thr
            820                 825                 830
Lys Val Leu Thr Phe Ile Ser Tyr Ile Gly Cys Gly Ile Ser Ala Ile
        835                 840                 845
Phe Ser Ala Ala Thr Leu Leu Thr Tyr Val Ala Phe Glu Lys Leu Arg
    850                 855                 860
Arg Asp Tyr Pro Ser Lys Ile Leu Met Asn Leu Ser Thr Ala Leu Leu
865                 870                 875                 880
Phe Leu Asn Leu Leu Phe Leu Leu Asp Gly Trp Ile Thr Ser Phe Asn
                885                 890                 895
Val Asp Gly Leu Cys Ile Ala Val Ala Val Leu Leu His Phe Phe Leu
            900                 905                 910
Leu Ala Thr Phe Thr Trp Met Gly Leu Glu Ala Ile His Met Tyr Ile
        915                 920                 925
Ala Leu Val Lys Val Phe Asn Thr Tyr Ile Arg Arg Tyr Ile Leu Lys
    930                 935                 940
Phe Cys Ile Ile Gly Trp Gly Leu Pro Ala Leu Val Val Ser Val Val
945                 950                 955                 960
Leu Ala Ser Arg Asn Asn Asn Glu Val Tyr Gly Lys Glu Ser Tyr Gly
                965                 970                 975
Lys Glu Lys Gly Asp Glu Phe Cys Trp Ile Gln Asp Pro Val Ile Phe
            980                 985                 990
Tyr Val Thr Cys Ala Gly Tyr Phe Gly Val Met Phe Phe Leu Asn Ile
        995                 1000                1005
Ala Met Phe Ile Val Val Met Val Gln Ile Cys Gly Arg Asn Gly Lys

1010                1015                1020
Arg Ser Asn Arg Thr Leu Arg Glu Glu Val Leu Arg Asn Leu Arg Ser
1025                1030                1035                1040
Val Val Ser Leu Thr Phe Leu Leu Gly Met Thr Trp Gly Phe Ala Phe
                1045                1050                1055
Phe Ala Trp Gly Pro Leu Asn Ile Pro Phe Met Tyr Leu Phe Ser Ile
            1060                1065                1070
Phe Asn Ser Leu Gln Gly Leu Phe Ile Phe Ile Phe His Cys Ala Met
        1075                1080                1085
Lys Glu Asn Val Gln Lys Gln Trp Arg Arg His Leu Cys Cys Gly Arg
    1090                1095                1100
Phe Arg Leu Ala Asp Asn Ser Asp Trp Ser Lys Thr Ala Thr Asn Ile
1105                1110                1115                1120
Ile Lys Lys Ser Ser Asp Asn Leu Gly Lys Ser Leu Ser Ser Ser Ser
                1125                1130                1135
Ile Gly Ser Asn Ser Thr Tyr Leu Thr Ser Lys Ser Lys Ser Ser Ser
            1140                1145                1150
Thr Thr Tyr Phe Lys Arg Asn Ser His Thr Asp Asn Val Ser Tyr Glu
        1155                1160                1165
His Ser Phe Asn Lys Ser Gly Ser Leu Arg Gln Cys Phe His Gly Gln
    1170                1175                1180
Val Leu Val Lys Thr Gly Pro Cys
1185                1190

<210> 45
<211> 2070
<212> DNA
<213> homo sapiens

<400> 45
atgtttcgct cagatcgaat gtggagctgc cattggaaat ggaagcccag tcctctcctg      60
ttcttatttg ctttatatat catgtgtgtt cctcactcag tgtggggatg tgccaactgc     120
cgagtggttt tgtccaaccc ttctgggacc tttacttctc catgctaccc taacgactac     180
ccaaacagcc aggcttgcat gtggacgctc cgagccccca ccggttatat cattcagata     240
acatttaacg actttgacat tgaagaagct cccaattgca tttatgactc attatccctt     300
gataatggag agagccagac taaattttgt ggagcaactg ccaaaggcct atcatttaac     360
tcaagtgcga atgagatgca tgtgtccttt tcaagtgact ttagcatcca gaagaaaggt     420
ttcaatgcca gctacatcag agttgccgtg tccttaagga atcaaaaggt cattttaccc     480
cagacatcag atgcttacca ggtatctgtt gcaaaaagca tctctattcc agagctcagt     540
gctttcacac tctgctttga agcaaccaaa gttggccatg aagacagtga ttggacagct     600
ttctcctact caaatgcatc cttcacacaa ttgctcagtt ttggaaaggc caagagtggc     660
tactttctat ccatttctga ttcaaaatgt ttgttgaata atgcattacc tgtcaaagaa     720
aaagaagaca tttttgcaga aagctttgaa cagctctgcc ttgtttggaa taattctttg     780
ggctctattg gtgtaaattt caaaagaaac tatgaaacag ttccatgtga ttctaccatt     840
agtaaagtta ttcctgggaa tgggaaattg ttgttgggct ccaatcaaaa tgaaattgtc     900
tctctaaaag ggacatttta taactttcga ctttggaatt ttaccatgaa tgccaaaatc     960
ctctccaacc tcagctgtaa tgtgaaaggg aatgtagtcg actggcaaaa tgacttctgg    1020
aatatcccaa acctagctct gaaagctgaa agcaacctaa gctgtggttc ctacctgatc    1080
ccgctcccag cagcagaact ggccagctgt gcagacctgg ggaccctctg tcaagatgga    1140
attatctata gaatatccgt agtgattcag aacatccttc gtcaccctga ggtaaaagta    1200
cagagcaagg tggcagaatg gctcaattca accttccaaa attggaacta cacggtttat    1260
gtcgttaata tcagttttca cctgagtgct ggagaggaca agattaaagt caagagaagc    1320
cttgaggatg agccaaggtt ggtgctttgg gcccttctag tttacaatgc taccaacaat    1380
actaatttag aaggaaaaat cattcagcag aagctcctaa aaaataatga gtccttggat    1440
gaaggcttga ggctacatac agtgaatgtg agacaactgg gtcattgtct tgccatggag    1500
gaacccaaag gctactactg gccatctatc caaccttctg aatacgttct tccttgtcca    1560
gacaagcctg gcttttctgc ttctcggata tgtttttaca atgctaccaa cccattggta    1620
acctactggg gacctgttga tatctccaac tgtttaaaag aagcaaatga agttgctaac    1680
cagattttaa atttaactgc tgatgggcag aacttaacct cagccaatat taccaacatt    1740
gtggaacagg tcaaaagaat tgtgaataaa gaagaaaaca ttgatataac acttggctca    1800
actctaatga atatattttc taatatctta agcagttcag acagtgactt gcttgagtca    1860
tcttctgaag ctttaaaaac aattgatgaa ttggccttca agatagacct aaatagcaca    1920
tcacatgtga atattacaac tcggaacttg gctctcagcg tatcatccct gttaccaggg    1980
acaaatgcaa tttcaaattt tagcattggt cttccaagca ataatgaatc gtatttccag    2040
gtaatgagcc agtggtttct ttcattttaa                                     2070

<210> 46
<211> 689

<212> PRT
<213> homo sapiens

<400> 46
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Val Trp Gly Cys Ala Asn Cys Arg Val Val Leu Ser Asn Pro Ser
        35                  40                  45
Gly Thr Phe Thr Ser Pro Cys Tyr Pro Asn Asp Tyr Pro Asn Ser Gln
    50                  55                  60
Ala Cys Met Trp Thr Leu Arg Ala Pro Thr Gly Tyr Ile Ile Gln Ile
65                  70                  75                  80
Thr Phe Asn Asp Phe Asp Ile Glu Glu Ala Pro Asn Cys Ile Tyr Asp
                85                  90                  95
Ser Leu Ser Leu Asp Asn Gly Glu Ser Gln Thr Lys Phe Cys Gly Ala
            100                 105                 110
Thr Ala Lys Gly Leu Ser Phe Asn Ser Ser Ala Asn Glu Met His Val
        115                 120                 125
Ser Phe Ser Ser Asp Phe Ser Ile Gln Lys Lys Gly Phe Asn Ala Ser
    130                 135                 140
Tyr Ile Arg Val Ala Val Ser Leu Arg Asn Gln Lys Val Ile Leu Pro
145                 150                 155                 160
Gln Thr Ser Asp Ala Tyr Gln Val Ser Val Ala Lys Ser Ile Ser Ile
                165                 170                 175
Pro Glu Leu Ser Ala Phe Thr Leu Cys Phe Glu Ala Thr Lys Val Gly
            180                 185                 190
His Glu Asp Ser Asp Trp Thr Ala Phe Ser Tyr Ser Asn Ala Ser Phe
        195                 200                 205
Thr Gln Leu Leu Ser Phe Gly Lys Ala Lys Ser Gly Tyr Phe Leu Ser
    210                 215                 220
Ile Ser Asp Ser Lys Cys Leu Leu Asn Asn Ala Leu Pro Val Lys Glu
225                 230                 235                 240
Lys Glu Asp Ile Phe Ala Glu Ser Phe Glu Gln Leu Cys Leu Val Trp
                245                 250                 255
Asn Asn Ser Leu Gly Ser Ile Gly Val Asn Phe Lys Arg Asn Tyr Glu
            260                 265                 270
Thr Val Pro Cys Asp Ser Thr Ile Ser Lys Val Ile Pro Gly Asn Gly
        275                 280                 285
Lys Leu Leu Leu Gly Ser Asn Gln Asn Glu Ile Val Ser Leu Lys Gly
    290                 295                 300
Asp Ile Tyr Asn Phe Arg Leu Trp Asn Phe Thr Met Asn Ala Lys Ile
305                 310                 315                 320
Leu Ser Asn Leu Ser Cys Asn Val Lys Gly Asn Val Val Asp Trp Gln
                325                 330                 335
Asn Asp Phe Trp Asn Ile Pro Asn Leu Ala Leu Lys Ala Glu Ser Asn
            340                 345                 350
Leu Ser Cys Gly Ser Tyr Leu Ile Pro Leu Pro Ala Ala Glu Leu Ala
        355                 360                 365
Ser Cys Ala Asp Leu Gly Thr Leu Cys Gln Asp Gly Ile Ile Tyr Arg
    370                 375                 380
Ile Ser Val Val Ile Gln Asn Ile Leu Arg His Pro Glu Val Lys Val
385                 390                 395                 400
Gln Ser Lys Val Ala Glu Trp Leu Asn Ser Thr Phe Gln Asn Trp Asn
                405                 410                 415
Tyr Thr Val Tyr Val Val Asn Ile Ser Phe His Leu Ser Ala Gly Glu
            420                 425                 430
Asp Lys Ile Lys Val Lys Arg Ser Leu Glu Asp Glu Pro Arg Leu Val
        435                 440                 445
Leu Trp Ala Leu Leu Val Tyr Asn Ala Thr Asn Asn Thr Asn Leu Glu
    450                 455                 460
Gly Lys Ile Ile Gln Gln Lys Leu Leu Lys Asn Asn Glu Ser Leu Asp
465                 470                 475                 480
Glu Gly Leu Arg Leu His Thr Val Asn Val Arg Gln Leu Gly His Cys
                485                 490                 495
Leu Ala Met Glu Glu Pro Lys Gly Tyr Tyr Trp Pro Ser Ile Gln Pro
            500                 505                 510

Ser Glu Tyr Val Leu Pro Cys Pro Asp Lys Pro Gly Phe Ser Ala Ser
        515                 520                 525
Arg Ile Cys Phe Tyr Asn Ala Thr Asn Pro Leu Val Thr Tyr Trp Gly
    530                 535                 540
Pro Val Asp Ile Ser Asn Cys Leu Lys Glu Ala Asn Glu Val Ala Asn
545                 550                 555                 560
Gln Ile Leu Asn Leu Thr Ala Asp Gly Gln Asn Leu Thr Ser Ala Asn
                565                 570                 575
Ile Thr Asn Ile Val Glu Gln Val Lys Arg Ile Val Asn Lys Glu Glu
            580                 585                 590
Asn Ile Asp Ile Thr Leu Gly Ser Thr Leu Met Asn Ile Phe Ser Asn
        595                 600                 605
Ile Leu Ser Ser Ser Asp Ser Asp Leu Leu Glu Ser Ser Ser Glu Ala
    610                 615                 620
Leu Lys Thr Ile Asp Glu Leu Ala Phe Lys Ile Asp Leu Asn Ser Thr
625                 630                 635                 640
Ser His Val Asn Ile Thr Thr Arg Asn Leu Ala Leu Ser Val Ser Ser
                645                 650                 655
Leu Leu Pro Gly Thr Asn Ala Ile Ser Asn Phe Ser Ile Gly Leu Pro
            660                 665                 670
Ser Asn Asn Glu Ser Tyr Phe Gln Val Met Ser Gln Trp Phe Leu Ser
        675                 680                 685
Phe

<210> 47
<211> 3252
<212> DNA
<213> homo sapiens

<400> 47
atgtttcgct cagatcgaat gtggagctgc cattggaaat ggaagcccag tcctctcctg      60
ttcttatttg ctttatatat catgtgtgtt cctcactcag tgtggggatg tgccaactgc     120
cgagtggttt tgtccaaccc ttctgggacc tttacttctc catgctaccc taacgactac     180
ccaaacagcc aggcttgcat gtggacgctc cgagccccca ccggttatat cattcagata     240
acatttaacg actttgacat tgaagaagct cccaattgca tttatgactc attatccctt     300
gataatggag agagccagac taaattttgt ggagcaactg ccaaaggcct atcatttaac     360
tcaagtgcga atgagatgca tgtgtccttt tcaagtgact ttagcatcca gaagaaaggt     420
ttcaatgcca gctacatcag agttgccgtg tccttaagga atcaaaaggt cattttaccc     480
cagacatcag atgcttacca ggtatctgtt gcaaaaagca tctctattcc agagctcagt     540
gctttcacac tctgctttga agcaaccaaa gttggccatg aagacagtga ttggacagct     600
ttctcctact caaatgcatc cttcacacaa ttgctcagtt ttggaaaggc caagagtggc     660
tactttctat ccatttctga ttcaaaatgt ttgttgaata atgcattacc tgtcaaagaa     720
aaagaagaca tttttgcaga aagctttgaa cagctctgcc ttgtttggaa taattctttg     780
ggctctattg gtgtaaattt caaaagaaac tatgaaacag ttccatgtga ttctaccatt     840
agtaaagtta ttcctgggaa tgggaaattg ttgttgggct ccaatcaaaa tgaaattgtc     900
tctctaaaag gggacattta taactttcga ctttggaatt ttaccatgaa tgccaaaatc     960
ctctccaacc tcagctgtaa tgtgaaaggg aatgtagtcg actggcaaaa tgacttctgg    1020
aatatcccaa acctagctct gaaagctgaa agcaacctaa gctgtggttc ctacctgatc    1080
ccgctcccag cagcagaact ggccagctgt gcagacctgg ggaccctctg tcaagatgga    1140
attatctata gaatatccgt agtgattcag aacatccttc gtcaccctga ggtaaaagta    1200
cagagcaagg tggcagaatg gctcaattca accttccaaa attggaacta cacggtttat    1260
gtcgttaata tcagttttca cctgagtgct ggagaggaca agattaaagt caagagaagc    1320
cttgaggatg agccaaggtt ggtgctttgg gcccttctag tttacaatgc taccaacaat    1380
actaatttag aaggaaaaat cattcagcag aagctcctaa aaaataatga gtccttggat    1440
gaaggcttga ggctacatac agtgaatgtg agacaactgg gtcattgtct tgccatggag    1500
gaacccaaag gctactactg gccatctatc caaccttctg aatacgttct tccttgtcca    1560
gacaagcctg gcttttctgc ttctcggata tgtttttaca atgctaccaa cccattggta    1620
acctactggg gacctgttga tatctccaac tgtttaaaag aagcaaatga agttgctaac    1680
cagattttaa atttaactgc tgatgggcag aacttaacct cagccaatat taccaacatt    1740
gtggaacagg tcaaaagaat tgtgaataaa gaagaaaaca ttgatataac acttggctca    1800
actctaatga atatattttc taatatctta agcagttcag acagtgactt gcttgagtca    1860
tcttctgaag ctttaaaaac aattgatgaa ttggccttca agatagacct aaatagcaca    1920
tcacatgtga atattacaac tcggaacttg gctctcagcg tatcatccct gttaccaggg    1980
acaaatgcaa tttcaaattt tagcattggt cttccaagca ataatgaatc gtatttccag    2040
atggattttg agagtggaca agtggatcca ctggcatctg taattttgcc tccaaactta    2100
cttgagaatt taagtccaga agattctgta ttagttagaa gagcacagtt tactttcttc    2160
aacaaaactg gacttttcca ggatgtagga ccccaaagaa aaactttagt gagttatgtg    2220

atggcgtgca gtattggaaa cattactatc cagaatctga aggatcctgt tcaaataaaa 2280
atcaaacata caagaactca ggaagtgcat catcccatct gtgccttctg ggatctgaac 2340
aaaaacaaaa gttttggagg atggaacacg tcaggatgtg ttgcacacag agattcagat 2400
gcaagtgaga cagtctgcct gtgtaaccac ttcacacact ttggagttct gatggacctt 2460
ccaagaagtg cctcacagtt agatgcaaga aacactaaag tcctcacttt catcagctat 2520
attgggtgtg gaatatctgc tattttttca gcagcaactc tcctgacata tgttgctttt 2580
gagaaattgc gaagggatta tccctccaaa atcttgatga acctgagcac agccctgctg 2640
ttcctgaatc tcctcttcct cctagatggc tggatcacct ccttcaatgt ggatggactt 2700
tgcattgctg ttgcagtcct gttgcatttc ttccttctgg caacctttac ctggatgggg 2760
ctagaagcaa ttcacatgta cattgctcta gttaaagtat ttaacactta cattcgccga 2820
tacattctaa aattctgcat cattggctgg ggtttgcctg ccttagtggt gtcagttgtt 2880
ctagcgagca gaaacaacaa tgaagtctat ggaaaagaaa gttatgggaa agaaaaaggt 2940
gatgaattct gttggattca agatccagtc atattttatg tgacctgtgc tgggtatttt 3000
ggagtcatgt tttttctgaa cattgccatg ttcattgtgg taatggtgca gatctgtggg 3060
aggaatggca agagaagcaa ccggaccctg agagaagaag tgttaaggaa cctgcgcagt 3120
gtggttagct tgacctttct gttgggcatg acatggggtt ttgcattctt tgcctgggga 3180
cccttaaata tccccttcat gtacctcttc tccatcttca attcattaca aggtaagata 3240
aattgtacat ga 3252

<210> 48
<211> 1083
<212> PRT
<213> homo sapiens

<400> 48
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Val Trp Gly Cys Ala Asn Cys Arg Val Val Leu Ser Asn Pro Ser
        35                  40                  45
Gly Thr Phe Thr Ser Pro Cys Tyr Pro Asn Asp Tyr Pro Asn Ser Gln
    50                  55                  60
Ala Cys Met Trp Thr Leu Arg Ala Pro Thr Gly Tyr Ile Ile Gln Ile
65                  70                  75                  80
Thr Phe Asn Asp Phe Asp Ile Glu Glu Ala Pro Asn Cys Ile Tyr Asp
                85                  90                  95
Ser Leu Ser Leu Asp Asn Gly Glu Ser Gln Thr Lys Phe Cys Gly Ala
            100                 105                 110
Thr Ala Lys Gly Leu Ser Phe Asn Ser Ser Ala Asn Glu Met His Val
        115                 120                 125
Ser Phe Ser Ser Asp Phe Ser Ile Gln Lys Lys Gly Phe Asn Ala Ser
    130                 135                 140
Tyr Ile Arg Val Ala Val Ser Leu Arg Asn Gln Lys Val Ile Leu Pro
145                 150                 155                 160
Gln Thr Ser Asp Ala Tyr Gln Val Ser Val Ala Lys Ser Ile Ser Ile
                165                 170                 175
Pro Glu Leu Ser Ala Phe Thr Leu Cys Phe Glu Ala Thr Lys Val Gly
            180                 185                 190
His Glu Asp Ser Asp Trp Thr Ala Phe Ser Tyr Ser Asn Ala Ser Phe
        195                 200                 205
Thr Gln Leu Leu Ser Phe Gly Lys Ala Lys Ser Gly Tyr Phe Leu Ser
    210                 215                 220
Ile Ser Asp Ser Lys Cys Leu Leu Asn Asn Ala Leu Pro Val Lys Glu
225                 230                 235                 240
Lys Glu Asp Ile Phe Ala Glu Ser Phe Glu Gln Leu Cys Leu Val Trp
                245                 250                 255
Asn Asn Ser Leu Gly Ser Ile Gly Val Asn Phe Lys Arg Asn Tyr Glu
            260                 265                 270
Thr Val Pro Cys Asp Ser Thr Ile Ser Lys Val Ile Pro Gly Asn Gly
        275                 280                 285
Lys Leu Leu Leu Gly Ser Asn Gln Asn Glu Ile Val Ser Leu Lys Gly
    290                 295                 300
Asp Ile Tyr Asn Phe Arg Leu Trp Asn Phe Thr Met Asn Ala Lys Ile
305                 310                 315                 320
Leu Ser Asn Leu Ser Cys Asn Val Lys Gly Asn Val Val Asp Trp Gln
                325                 330                 335
Asn Asp Phe Trp Asn Ile Pro Asn Leu Ala Leu Lys Ala Glu Ser Asn

            340                 345                 350
Leu Ser Cys Gly Ser Tyr Leu Ile Pro Leu Pro Ala Ala Glu Leu Ala
        355                 360                 365
Ser Cys Ala Asp Leu Gly Thr Leu Cys Gln Asp Gly Ile Ile Tyr Arg
    370                 375                 380
Ile Ser Val Val Ile Gln Asn Ile Leu Arg His Pro Glu Val Lys Val
385                 390                 395                 400
Gln Ser Lys Val Ala Glu Trp Leu Asn Ser Thr Phe Gln Asn Trp Asn
                405                 410                 415
Tyr Thr Val Tyr Val Val Asn Ile Ser Phe His Leu Ser Ala Gly Glu
            420                 425                 430
Asp Lys Ile Lys Val Lys Arg Ser Leu Glu Asp Glu Pro Arg Leu Val
        435                 440                 445
Leu Trp Ala Leu Leu Val Tyr Asn Ala Thr Asn Asn Thr Asn Leu Glu
    450                 455                 460
Gly Lys Ile Ile Gln Gln Lys Leu Leu Lys Asn Asn Glu Ser Leu Asp
465                 470                 475                 480
Glu Gly Leu Arg Leu His Thr Val Asn Val Arg Gln Leu Gly His Cys
                485                 490                 495
Leu Ala Met Glu Glu Pro Lys Gly Tyr Tyr Trp Pro Ser Ile Gln Pro
            500                 505                 510
Ser Glu Tyr Val Leu Pro Cys Pro Asp Lys Pro Gly Phe Ser Ala Ser
        515                 520                 525
Arg Ile Cys Phe Tyr Asn Ala Thr Asn Pro Leu Val Thr Tyr Trp Gly
    530                 535                 540
Pro Val Asp Ile Ser Asn Cys Leu Lys Glu Ala Asn Glu Val Ala Asn
545                 550                 555                 560
Gln Ile Leu Asn Leu Thr Ala Asp Gly Gln Asn Leu Thr Ser Ala Asn
                565                 570                 575
Ile Thr Asn Ile Val Glu Gln Val Lys Arg Ile Val Asn Lys Glu Glu
            580                 585                 590
Asn Ile Asp Ile Thr Leu Gly Ser Thr Leu Met Asn Ile Phe Ser Asn
        595                 600                 605
Ile Leu Ser Ser Ser Asp Ser Asp Leu Leu Glu Ser Ser Ser Glu Ala
    610                 615                 620
Leu Lys Thr Ile Asp Glu Leu Ala Phe Lys Ile Asp Leu Asn Ser Thr
625                 630                 635                 640
Ser His Val Asn Ile Thr Thr Arg Asn Leu Ala Leu Ser Val Ser Ser
                645                 650                 655
Leu Leu Pro Gly Thr Asn Ala Ile Ser Asn Phe Ser Ile Gly Leu Pro
            660                 665                 670
Ser Asn Asn Glu Ser Tyr Phe Gln Met Asp Phe Glu Ser Gly Gln Val
        675                 680                 685
Asp Pro Leu Ala Ser Val Ile Leu Pro Pro Asn Leu Leu Glu Asn Leu
    690                 695                 700
Ser Pro Glu Asp Ser Val Leu Val Arg Arg Ala Gln Phe Thr Phe Phe
705                 710                 715                 720
Asn Lys Thr Gly Leu Phe Gln Asp Val Gly Pro Gln Arg Lys Thr Leu
                725                 730                 735
Val Ser Tyr Val Met Ala Cys Ser Ile Gly Asn Ile Thr Ile Gln Asn
            740                 745                 750
Leu Lys Asp Pro Val Gln Ile Lys Ile Lys His Thr Arg Thr Gln Glu
        755                 760                 765
Val His His Pro Ile Cys Ala Phe Trp Asp Leu Asn Lys Asn Lys Ser
    770                 775                 780
Phe Gly Gly Trp Asn Thr Ser Gly Cys Val Ala His Arg Asp Ser Asp
785                 790                 795                 800
Ala Ser Glu Thr Val Cys Leu Cys Asn His Phe Thr His Phe Gly Val
                805                 810                 815
Leu Met Asp Leu Pro Arg Ser Ala Ser Gln Leu Asp Ala Arg Asn Thr
            820                 825                 830
Lys Val Leu Thr Phe Ile Ser Tyr Ile Gly Cys Gly Ile Ser Ala Ile
        835                 840                 845
Phe Ser Ala Ala Thr Leu Leu Thr Tyr Val Ala Phe Glu Lys Leu Arg
    850                 855                 860
Arg Asp Tyr Pro Ser Lys Ile Leu Met Asn Leu Ser Thr Ala Leu Leu
865                 870                 875                 880
Phe Leu Asn Leu Leu Phe Leu Leu Asp Gly Trp Ile Thr Ser Phe Asn

885                 890                 895
Val Asp Gly Leu Cys Ile Ala Val Ala Val Leu Leu His Phe Phe Leu
            900                 905                 910
Leu Ala Thr Phe Thr Trp Met Gly Leu Glu Ala Ile His Met Tyr Ile
        915                 920                 925
Ala Leu Val Lys Val Phe Asn Thr Tyr Ile Arg Arg Tyr Ile Leu Lys
    930                 935                 940
Phe Cys Ile Ile Gly Trp Gly Leu Pro Ala Leu Val Val Ser Val Val
945                 950                 955                 960
Leu Ala Ser Arg Asn Asn Asn Glu Val Tyr Gly Lys Glu Ser Tyr Gly
                965                 970                 975
Lys Glu Lys Gly Asp Glu Phe Cys Trp Ile Gln Asp Pro Val Ile Phe
            980                 985                 990
Tyr Val Thr Cys Ala Gly Tyr Phe Gly Val Met Phe Phe Leu Asn Ile
        995                 1000                1005
Ala Met Phe Ile Val Val Met Val Gln Ile Cys Gly Arg Asn Gly Lys
    1010                1015                1020
Arg Ser Asn Arg Thr Leu Arg Glu Glu Val Leu Arg Asn Leu Arg Ser
1025                1030                1035                1040
Val Val Ser Leu Thr Phe Leu Leu Gly Met Thr Trp Gly Phe Ala Phe
                1045                1050                1055
Phe Ala Trp Gly Pro Leu Asn Ile Pro Phe Met Tyr Leu Phe Ser Ile
            1060                1065                1070
Phe Asn Ser Leu Gln Gly Lys Ile Asn Cys Thr
        1075                1080

<210> 49
<211> 3669
<212> DNA
<213> homo sapiens

<400> 49
atgatgtttc gctcagatcg aatgtggagc tgccattgga aatggaagcc cagtcctctc      60
ctgttcttat ttgctttata tatcatgtgt gttcctcact cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080
atcccgctcc cagcagcaga actggccagc tgtgcagacc tggggaccct ctgtcaagat    1140
ggaattatct atagaatatc cgtagtgatt cagaacatcc ttcgtcaccc tgaggtaaaa    1200
gtacagagca aggtggcaga atggctcaat tcaaccttcc aaaattggaa ctacacggtt    1260
tatgtcgtta atatcagttt tcacctgagt gctggagagg acaagattaa agtcaagaga    1320
agccttgagg atgagccaag gttggtgctt tgggcccttc tagtttacaa tgctaccaac    1380
aatactaatt tagaaggaaa aatcattcag cagaagctcc taaaaaataa tgagtccttg    1440
gatgaaggct tgaggctaca tacagtgaat gtgagacaac tgggtcattg tcttgccatg    1500
gaggaaccca aaggctacta ctggccatct atccaacctt ctgaatacgt tcttccttgt    1560
ccagacaagc ctggcttttc tgcttctcgg atatgttttt acaatgctac caacccattg    1620
gtaacctact ggggacctgt tgatatctcc aactgtttaa aagaagcaaa tgaagttgct    1680
aaccagattt taaatttaac tgctgatggg cagaacttaa cctcagccaa tattaccaac    1740
attgtggaac aggtcaaaag aattgtgaat aaagaagaaa acattgatat aacacttggc    1800
tcaactctaa tgaatatatt ttctaatatc ttaagcagtt cagacagtga cttgcttgag    1860
tcatcttctg aagctttaaa aacaattgat gaattggcct tcaagataga cctaaatagc    1920
acatcacatg tgaatattac aactcggaac ttggctctca gcgtatcatc cctgttacca    1980
gggacaaatg caatttcaaa ttttagcatt ggtcttccaa gcaataatga atcgtatttc    2040
cagatggatt ttgagagtgg acaagtggat ccactggcat ctgtaatttt gcctccaaac    2100
ttacttgaga atttaagtcc agaagattct gtattagtta gaagagcaca gtttactttc    2160

ttcaacaaaa ctggactttt ccaggatgta ggaccccaaa gaaaaacttt agtgagttat 2220
gtgatggcgt gcagtattgg aaacattact atccagaatc tgaaggatcc tgttcaaata 2280
aaaatcaaac atacaagaac tcaggaagtg catcatccca tctgtgcctt ctgggatctg 2340
aacaaaaaca aaagttttgg aggatggaac acgtcaggat gtgttgcaca cagagattca 2400
gatgcaagtg agacagtctg cctgtgtaac cacttcacac actttggagt tctgatggac 2460
cttccaagaa gtgcctcaca gttagatgca agaaacacta aagtcctcac tttcatcagc 2520
tatattgggt gtggaatatc tgctattttt tcagcagcaa ctctcctgac atatgttgct 2580
tttgagaaat tgcgaaggga ttatccctcc aaaatcttga tgaacctgag cacagccctg 2640
ctgttcctga atctcctctt cctcctagat ggctggatca cctccttcaa tgtggatgga 2700
ctttgcattg ctgttgcagt cctgttgcat ttcttccttc tggcaacctt tacctggatg 2760
gggctagaag caattcacat gtacattgct ctagttaaag tatttaacac ttacattcgc 2820
cgatacattc taaaattctg catcattggc tggggtttgc ctgccttagt ggtgtcagtt 2880
gttctagcga gcagaaacaa caatgaagtc tatggaaaag aaagttatgg gaaagaaaaa 2940
ggtgatgaat tctgttggat tcaagatcca gtcatatttt atgtgacctg tgctgggtat 3000
tttggagtca tgttttttct gaacattgcc atgttcattg tggtaatggt gcagatctgt 3060
gggaggaatg gcaagagaag caaccggacc ctgagagaag aagtgttaag gaacctgcgc 3120
agtgtggtta gcttgacctt tctgttgggc atgacatggg gttttgcatt ctttgcctgg 3180
ggacccttaa atatcccctt catgtacctc ttctccatct tcaattcatt acaaggctta 3240
tttatattca tcttccactg tgctatgaag gagaatgttc agaaacagtg gcggcggcat 3300
ctctgctgtg gtagatttcg gttagcagat aactcagatt ggagtaagac agctaccaat 3360
atcatcaaga aaagttctga taatctagga aaatctttgt cttcaagctc cattggttcc 3420
aactcaacct atcttacatc caaatctaaa tccagctcta ccacctattt caaaaggaat 3480
agccacacag acagtgcttc catggacaag tccttgtcaa aactggccca tgctgatgga 3540
gatcaaacat caatcatccc tgtccatcag gtcattgata aggtcaaggg ttattgcaat 3600
gctcattcag acaacttcta taaaaatatt atcatgtcag acaccttcag ccacagcaca 3660
aagttttaa 3669

<210> 50
<211> 1222
<212> PRT
<213> homo sapiens

<400> 50
Met Met Phe Arg Ser Asp Arg Met Trp Ser Cys His Trp Lys Trp Lys
1               5                   10                  15
Pro Ser Pro Leu Leu Phe Leu Phe Ala Leu Tyr Ile Met Cys Val Pro
            20                  25                  30
His Ser Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn

        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Asp Gly Ile Ile Tyr
    370                 375                 380
Arg Ile Ser Val Val Ile Gln Asn Ile Leu Arg His Pro Glu Val Lys
385                 390                 395                 400
Val Gln Ser Lys Val Ala Glu Trp Leu Asn Ser Thr Phe Gln Asn Trp
                405                 410                 415
Asn Tyr Thr Val Tyr Val Val Asn Ile Ser Phe His Leu Ser Ala Gly
            420                 425                 430
Glu Asp Lys Ile Lys Val Lys Arg Ser Leu Glu Asp Glu Pro Arg Leu
        435                 440                 445
Val Leu Trp Ala Leu Leu Val Tyr Asn Ala Thr Asn Asn Thr Asn Leu
    450                 455                 460
Glu Gly Lys Ile Ile Gln Gln Lys Leu Leu Lys Asn Asn Glu Ser Leu
465                 470                 475                 480
Asp Glu Gly Leu Arg Leu His Thr Val Asn Val Arg Gln Leu Gly His
                485                 490                 495
Cys Leu Ala Met Glu Glu Pro Lys Gly Tyr Tyr Trp Pro Ser Ile Gln
            500                 505                 510
Pro Ser Glu Tyr Val Leu Pro Cys Pro Asp Lys Pro Gly Phe Ser Ala
        515                 520                 525
Ser Arg Ile Cys Phe Tyr Asn Ala Thr Asn Pro Leu Val Thr Tyr Trp
    530                 535                 540
Gly Pro Val Asp Ile Ser Asn Cys Leu Lys Glu Ala Asn Glu Val Ala
545                 550                 555                 560
Asn Gln Ile Leu Asn Leu Thr Ala Asp Gly Gln Asn Leu Thr Ser Ala
                565                 570                 575
Asn Ile Thr Asn Ile Val Glu Gln Val Lys Arg Ile Val Asn Lys Glu
            580                 585                 590
Glu Asn Ile Asp Ile Thr Leu Gly Ser Thr Leu Met Asn Ile Phe Ser
        595                 600                 605
Asn Ile Leu Ser Ser Ser Asp Ser Asp Leu Leu Glu Ser Ser Ser Glu
    610                 615                 620
Ala Leu Lys Thr Ile Asp Glu Leu Ala Phe Lys Ile Asp Leu Asn Ser
625                 630                 635                 640
Thr Ser His Val Asn Ile Thr Thr Arg Asn Leu Ala Leu Ser Val Ser
                645                 650                 655
Ser Leu Leu Pro Gly Thr Asn Ala Ile Ser Asn Phe Ser Ile Gly Leu
            660                 665                 670
Pro Ser Asn Asn Glu Ser Tyr Phe Gln Met Asp Phe Glu Ser Gly Gln
        675                 680                 685
Val Asp Pro Leu Ala Ser Val Ile Leu Pro Pro Asn Leu Leu Glu Asn
    690                 695                 700
Leu Ser Pro Glu Asp Ser Val Leu Val Arg Arg Ala Gln Phe Thr Phe
705                 710                 715                 720
Phe Asn Lys Thr Gly Leu Phe Gln Asp Val Gly Pro Gln Arg Lys Thr
                725                 730                 735
Leu Val Ser Tyr Val Met Ala Cys Ser Ile Gly Asn Ile Thr Ile Gln
            740                 745                 750
Asn Leu Lys Asp Pro Val Gln Ile Lys Ile Lys His Thr Arg Thr Gln
        755                 760                 765
Glu Val His His Pro Ile Cys Ala Phe Trp Asp Leu Asn Lys Asn Lys
    770                 775                 780
Ser Phe Gly Gly Trp Asn Thr Ser Gly Cys Val Ala His Arg Asp Ser
785                 790                 795                 800
Asp Ala Ser Glu Thr Val Cys Leu Cys Asn His Phe Thr His Phe Gly
                805                 810                 815
Val Leu Met Asp Leu Pro Arg Ser Ala Ser Gln Leu Asp Ala Arg Asn

            820                 825                 830
Thr Lys Val Leu Thr Phe Ile Ser Tyr Ile Gly Cys Gly Ile Ser Ala
        835                 840                 845
Ile Phe Ser Ala Ala Thr Leu Leu Thr Tyr Val Ala Phe Glu Lys Leu
    850                 855                 860
Arg Arg Asp Tyr Pro Ser Lys Ile Leu Met Asn Leu Ser Thr Ala Leu
865                 870                 875                 880
Leu Phe Leu Asn Leu Leu Phe Leu Leu Asp Gly Trp Ile Thr Ser Phe
                885                 890                 895
Asn Val Asp Gly Leu Cys Ile Ala Val Ala Val Leu Leu His Phe Phe
            900                 905                 910
Leu Leu Ala Thr Phe Thr Trp Met Gly Leu Glu Ala Ile His Met Tyr
        915                 920                 925
Ile Ala Leu Val Lys Val Phe Asn Thr Tyr Ile Arg Arg Tyr Ile Leu
    930                 935                 940
Lys Phe Cys Ile Ile Gly Trp Gly Leu Pro Ala Leu Val Val Ser Val
945                 950                 955                 960
Val Leu Ala Ser Arg Asn Asn Asn Glu Val Tyr Gly Lys Glu Ser Tyr
                965                 970                 975
Gly Lys Glu Lys Gly Asp Glu Phe Cys Trp Ile Gln Asp Pro Val Ile
            980                 985                 990
Phe Tyr Val Thr Cys Ala Gly Tyr Phe Gly Val Met Phe Phe Leu Asn
        995                 1000                1005
Ile Ala Met Phe Ile Val Val Met Val Gln Ile Cys Gly Arg Asn Gly
    1010                1015                1020
Lys Arg Ser Asn Arg Thr Leu Arg Glu Glu Val Leu Arg Asn Leu Arg
1025                1030                1035                1040
Ser Val Val Ser Leu Thr Phe Leu Leu Gly Met Thr Trp Gly Phe Ala
                1045                1050                1055
Phe Phe Ala Trp Gly Pro Leu Asn Ile Pro Phe Met Tyr Leu Phe Ser
            1060                1065                1070
Ile Phe Asn Ser Leu Gln Gly Leu Phe Ile Phe Ile Phe His Cys Ala
        1075                1080                1085
Met Lys Glu Asn Val Gln Lys Gln Trp Arg Arg His Leu Cys Cys Gly
    1090                1095                1100
Arg Phe Arg Leu Ala Asp Asn Ser Asp Trp Ser Lys Thr Ala Thr Asn
1105                1110                1115                1120
Ile Ile Lys Lys Ser Ser Asp Asn Leu Gly Lys Ser Leu Ser Ser Ser
                1125                1130                1135
Ser Ile Gly Ser Asn Ser Thr Tyr Leu Thr Ser Lys Ser Lys Ser Ser
            1140                1145                1150
Ser Thr Thr Tyr Phe Lys Arg Asn Ser His Thr Asp Ser Ala Ser Met
        1155                1160                1165
Asp Lys Ser Leu Ser Lys Leu Ala His Ala Asp Gly Asp Gln Thr Ser
    1170                1175                1180
Ile Ile Pro Val His Gln Val Ile Asp Lys Val Lys Gly Tyr Cys Asn
1185                1190                1195                1200
Ala His Ser Asp Asn Phe Tyr Lys Asn Ile Ile Met Ser Asp Thr Phe
                1205                1210                1215
Ser His Ser Thr Lys Phe
            1220

<210> 51
<211> 3582
<212> DNA
<213> homo sapiens

<400> 51
atgatgtttc gctcagatcg aatgtggagc tgccattgga aatggaagcc cagtcctctc 60
ctgttcttat ttgctttata tatcatgtgt gttcctcact cagtgtgggg atgtgccaac 120
tgccgagtgg ttttgtccaa cccttctggg acctttactt ctccatgcta ccctaacgac 180
tacccaaaca gccaggcttg catgtggacg ctccgagccc ccaccggtta tatcattcag 240
ataacattta acgactttga cattgaagaa gctcccaatt gcatttatga ctcattatcc 300
cttgataatg gagagagcca gactaaattt tgtggagcaa ctgccaaagg cctatcattt 360
aactcaagtg cgaatgagat gcatgtgtcc ttttcaagtg actttagcat ccagaagaaa 420
ggtttcaatg ccagctacat cagagttgcc gtgtccttaa ggaatcaaaa ggtcatttta 480
ccccagacat cagatgctta ccaggtatct gttgcaaaaa gcatctctat tccagagctc 540
agtgctttca cactctgctt tgaagcaacc aaagttggcc atgaagacag tgattggaca 600

gctttctcct actcaaatgc atccttcaca caattgctca gttttggaaa ggccaagagt 660
ggctactttc tatccatttc tgattcaaaa tgtttgttga ataatgcatt acctgtcaaa 720
gaaaaagaag acatttttgc agaaagcttt gaacagctct gccttgtttg gaataattct 780
ttgggctcta ttggtgtaaa tttcaaaaga aactatgaaa cagttccatg tgattctacc 840
attagtaaag ttattcctgg gaatgggaaa ttgttgttgg gctccaatca aaatgaaatt 900
gtctctctaa aaggggacat ttataacttt cgactttgga attttaccat gaatgccaaa 960
atcctctcca acctcagctg taatgtgaaa gggaatgtag tcgactggca aaatgacttc 1020
tggaatatcc caaacctagc tctgaaagct gaaagcaacc taagctgtgg ttcctacctg 1080
atcccgctcc cagcagcaga actggccagc tgtgcagacc tggggaccct ctgtcaagat 1140
ggaattatct atagaatatc cgtagtgatt cagaacatcc ttcgtcaccc tgaggtaaaa 1200
gtacagagca aggtggcaga atggctcaat tcaaccttcc aaaattggaa ctacacggtt 1260
tatgtcgtta atatcagttt tcacctgagt gctggagagg acaagattaa agtcaagaga 1320
agccttgagg atgagccaag gttggtgctt tgggcccttc tagtttacaa tgctaccaac 1380
aatactaatt tagaaggaaa aatcattcag cagaagctcc taaaaaataa tgagtccttg 1440
gatgaaggct tgaggctaca tacagtgaat gtgagacaac tgggtcattg tcttgccatg 1500
gaggaaccca aaggctacta ctggccatct atccaacctt ctgaatacgt tcttccttgt 1560
ccagacaagc ctggcttttc tgcttctcgg atatgttttt acaatgctac caacccattg 1620
gtaacctact ggggacctgt tgatatctcc aactgtttaa aagaagcaaa tgaagttgct 1680
aaccagattt taaatttaac tgctgatggg cagaacttaa cctcagccaa tattaccaac 1740
attgtggaac aggtcaaaag aattgtgaat aaagaagaaa acattgatat aacacttggc 1800
tcaactctaa tgaatatatt ttctaatatc ttaagcagtt cagacagtga cttgcttgag 1860
tcatcttctg aagctttaaa aacaattgat gaattggcct tcaagataga cctaaatagc 1920
acatcacatg tgaatattac aactcggaac ttggctctca gcgtatcatc cctgttacca 1980
gggacaaatg caatttcaaa ttttagcatt ggtcttccaa gcaataatga atcgtatttc 2040
cagatggatt ttgagagtgg acaagtggat ccactggcat ctgtaatttt gcctccaaac 2100
ttacttgaga atttaagtcc agaagattct gtattagtta gaagagcaca gtttactttc 2160
ttcaacaaaa ctggactttt ccaggatgta ggaccccaaa gaaaaacttt agtgagttat 2220
gtgatggcgt gcagtattgg aaacattact atccagaatc tgaaggatcc tgttcaaata 2280
aaaatcaaac atacaagaac tcaggaagtg catcatccca tctgtgcctt ctgggatctg 2340
aacaaaaaca aaagttttgg aggatggaac acgtcaggat gtgttgcaca cagagattca 2400
gatgcaagtg agacagtctg cctgtgtaac cacttcacac actttggagt tctgatggac 2460
cttccaagaa gtgcctcaca gttagatgca agaaacacta aagtcctcac tttcatcagc 2520
tatattgggt gtggaatatc tgctattttt tcagcagcaa ctctcctgac atatgttgct 2580
tttgagaaat tgcgaaggga ttatccctcc aaaatcttga tgaacctgag cacagccctg 2640
ctgttcctga atctcctctt cctcctagat ggctggatca cctccttcaa tgtggatgga 2700
ctttgcattg ctgttgcagt cctgttgcat ttcttccttc tggcaacctt tacctggatg 2760
gggctagaag caattcacat gtacattgct ctagttaaag tatttaacac ttacattcgc 2820
cgatacattc taaaattctg catcattggc tggggtttgc ctgccttagt ggtgtcagtt 2880
gttctagcga gcagaaacaa caatgaagtc tatggaaaag aaagttatgg gaaagaaaaa 2940
ggtgatgaat tctgttggat tcaagatcca gtcatatttt atgtgacctg tgctgggtat 3000
tttggagtca tgttttttct gaacattgcc atgttcattg tggtaatggt gcagatctgt 3060
gggaggaatg gcaagagaag caaccggacc ctgagagaag aagtgttaag gaacctgcgc 3120
agtgtggtta gcttgacctt tctgttgggc atgacatggg gttttgcatt ctttgcctgg 3180
ggacccttaa atatcccctt catgtacctc ttctccatct tcaattcatt acaaggctta 3240
tttatattca tcttccactg tgctatgaag gagaatgttc agaaacagtg gcggcggcat 3300
ctctgctgtg gtagatttcg gttagcagat aactcagatt ggagtaagac agctaccaat 3360
atcatcaaga aaagttctga taatctagga aaatctttgt cttcaagctc cattggttcc 3420
aactcaacct atcttacatc caaatctaaa tccagctcta ccacctattt caaaaggaat 3480
agccacacag ataatgtctc ctatgagcat tccttcaaca aaagtggatc actcagacag 3540
tgcttccatg gacaagtcct tgtcaaaact ggcccatgct ga 3582

<210> 52
<211> 1193
<212> PRT
<213> homo sapiens

<400> 52
Met Met Phe Arg Ser Asp Arg Met Trp Ser Cys His Trp Lys Trp Lys
1               5                   10                  15
Pro Ser Pro Leu Leu Phe Leu Phe Ala Leu Tyr Ile Met Cys Val Pro
            20                  25                  30
His Ser Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr

                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Asp Gly Ile Ile Tyr
    370                 375                 380
Arg Ile Ser Val Val Ile Gln Asn Ile Leu Arg His Pro Glu Val Lys
385                 390                 395                 400
Val Gln Ser Lys Val Ala Glu Trp Leu Asn Ser Thr Phe Gln Asn Trp
                405                 410                 415
Asn Tyr Thr Val Tyr Val Val Asn Ile Ser Phe His Leu Ser Ala Gly
            420                 425                 430
Glu Asp Lys Ile Lys Val Lys Arg Ser Leu Glu Asp Glu Pro Arg Leu
        435                 440                 445
Val Leu Trp Ala Leu Leu Val Tyr Asn Ala Thr Asn Asn Thr Asn Leu
    450                 455                 460
Glu Gly Lys Ile Ile Gln Gln Lys Leu Leu Lys Asn Asn Glu Ser Leu
465                 470                 475                 480
Asp Glu Gly Leu Arg Leu His Thr Val Asn Val Arg Gln Leu Gly His
                485                 490                 495
Cys Leu Ala Met Glu Glu Pro Lys Gly Tyr Tyr Trp Pro Ser Ile Gln
            500                 505                 510
Pro Ser Glu Tyr Val Leu Pro Cys Pro Asp Lys Pro Gly Phe Ser Ala
        515                 520                 525
Ser Arg Ile Cys Phe Tyr Asn Ala Thr Asn Pro Leu Val Thr Tyr Trp
    530                 535                 540
Gly Pro Val Asp Ile Ser Asn Cys Leu Lys Glu Ala Asn Glu Val Ala
545                 550                 555                 560
Asn Gln Ile Leu Asn Leu Thr Ala Asp Gly Gln Asn Leu Thr Ser Ala
                565                 570                 575
Asn Ile Thr Asn Ile Val Glu Gln Val Lys Arg Ile Val Asn Lys Glu
            580                 585                 590
Glu Asn Ile Asp Ile Thr Leu Gly Ser Thr Leu Met Asn Ile Phe Ser
        595                 600                 605
Asn Ile Leu Ser Ser Ser Asp Ser Asp Leu Leu Glu Ser Ser Ser Glu
    610                 615                 620
Ala Leu Lys Thr Ile Asp Glu Leu Ala Phe Lys Ile Asp Leu Asn Ser

625                 630                 635                 640
Thr Ser His Val Asn Ile Thr Thr Arg Asn Leu Ala Leu Ser Val Ser
                645                 650                 655
Ser Leu Leu Pro Gly Thr Asn Ala Ile Ser Asn Phe Ser Ile Gly Leu
            660                 665                 670
Pro Ser Asn Asn Glu Ser Tyr Phe Gln Met Asp Phe Glu Ser Gly Gln
        675                 680                 685
Val Asp Pro Leu Ala Ser Val Ile Leu Pro Pro Asn Leu Leu Glu Asn
    690                 695                 700
Leu Ser Pro Glu Asp Ser Val Leu Val Arg Arg Ala Gln Phe Thr Phe
705                 710                 715                 720
Phe Asn Lys Thr Gly Leu Phe Gln Asp Val Gly Pro Gln Arg Lys Thr
                725                 730                 735
Leu Val Ser Tyr Val Met Ala Cys Ser Ile Gly Asn Ile Thr Ile Gln
            740                 745                 750
Asn Leu Lys Asp Pro Val Gln Ile Lys Ile Lys His Thr Arg Thr Gln
        755                 760                 765
Glu Val His His Pro Ile Cys Ala Phe Trp Asp Leu Asn Lys Asn Lys
    770                 775                 780
Ser Phe Gly Gly Trp Asn Thr Ser Gly Cys Val Ala His Arg Asp Ser
785                 790                 795                 800
Asp Ala Ser Glu Thr Val Cys Leu Cys Asn His Phe Thr His Phe Gly
                805                 810                 815
Val Leu Met Asp Leu Pro Arg Ser Ala Ser Gln Leu Asp Ala Arg Asn
            820                 825                 830
Thr Lys Val Leu Thr Phe Ile Ser Tyr Ile Gly Cys Gly Ile Ser Ala
        835                 840                 845
Ile Phe Ser Ala Ala Thr Leu Leu Thr Tyr Val Ala Phe Glu Lys Leu
    850                 855                 860
Arg Arg Asp Tyr Pro Ser Lys Ile Leu Met Asn Leu Ser Thr Ala Leu
865                 870                 875                 880
Leu Phe Leu Asn Leu Leu Phe Leu Leu Asp Gly Trp Ile Thr Ser Phe
                885                 890                 895
Asn Val Asp Gly Leu Cys Ile Ala Val Ala Val Leu Leu His Phe Phe
            900                 905                 910
Leu Leu Ala Thr Phe Thr Trp Met Gly Leu Glu Ala Ile His Met Tyr
        915                 920                 925
Ile Ala Leu Val Lys Val Phe Asn Thr Tyr Ile Arg Arg Tyr Ile Leu
    930                 935                 940
Lys Phe Cys Ile Ile Gly Trp Gly Leu Pro Ala Leu Val Val Ser Val
945                 950                 955                 960
Val Leu Ala Ser Arg Asn Asn Asn Glu Val Tyr Gly Lys Glu Ser Tyr
                965                 970                 975
Gly Lys Glu Lys Gly Asp Glu Phe Cys Trp Ile Gln Asp Pro Val Ile
            980                 985                 990
Phe Tyr Val Thr Cys Ala Gly Tyr Phe Gly Val Met Phe Phe Leu Asn
        995                 1000                1005
Ile Ala Met Phe Ile Val Val Met Val Gln Ile Cys Gly Arg Asn Gly
    1010                1015                1020
Lys Arg Ser Asn Arg Thr Leu Arg Glu Glu Val Leu Arg Asn Leu Arg
1025                1030                1035                1040
Ser Val Val Ser Leu Thr Phe Leu Leu Gly Met Thr Trp Gly Phe Ala
                1045                1050                1055
Phe Phe Ala Trp Gly Pro Leu Asn Ile Pro Phe Met Tyr Leu Phe Ser
            1060                1065                1070
Ile Phe Asn Ser Leu Gln Gly Leu Phe Ile Phe Ile Phe His Cys Ala
        1075                1080                1085
Met Lys Glu Asn Val Gln Lys Gln Trp Arg Arg His Leu Cys Cys Gly
    1090                1095                1100
Arg Phe Arg Leu Ala Asp Asn Ser Asp Trp Ser Lys Thr Ala Thr Asn
1105                1110                1115                1120
Ile Ile Lys Lys Ser Ser Asp Asn Leu Gly Lys Ser Leu Ser Ser Ser
                1125                1130                1135
Ser Ile Gly Ser Asn Ser Thr Tyr Leu Thr Ser Lys Ser Lys Ser Ser
            1140                1145                1150
Ser Thr Thr Tyr Phe Lys Arg Asn Ser His Thr Asp Asn Val Ser Tyr
        1155                1160                1165
Glu His Ser Phe Asn Lys Ser Gly Ser Leu Arg Gln Cys Phe His Gly

1170                 1175                1180
Gln Val Leu Val Lys Thr Gly Pro Cys
1185                1190

<210> 53
<211> 2073
<212> DNA
<213> homo sapiens

<400> 53
atgatgtttc gctcagatcg aatgtggagc tgccattgga aatggaagcc cagtcctctc      60
ctgttcttat ttgctttata tatcatgtgt gttcctcact cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080
atcccgctcc cagcagcaga actggccagc tgtgcagacc tggggaccct ctgtcaagat    1140
ggaattatct atagaatatc cgtagtgatt cagaacatcc ttcgtcaccc tgaggtaaaa    1200
gtacagagca aggtggcaga atggctcaat tcaaccttcc aaaattggaa ctacacggtt    1260
tatgtcgtta atatcagttt tcacctgagt gctggagagg acaagattaa agtcaagaga    1320
agccttgagg atgagccaag gttggtgctt tgggcccttc tagtttacaa tgctaccaac    1380
aatactaatt tagaaggaaa aatcattcag cagaagctcc taaaaaataa tgagtccttg    1440
gatgaaggct tgaggctaca tacagtgaat gtgagacaac tgggtcattg tcttgccatg    1500
gaggaaccca aaggctacta ctggccatct atccaacctt ctgaatacgt tcttccttgt    1560
ccagacaagc ctggcttttc tgcttctcgg atatgttttt acaatgctac caacccattg    1620
gtaacctact ggggacctgt tgatatctcc aactgtttaa aagaagcaaa tgaagttgct    1680
aaccagattt taaatttaac tgctgatggg cagaacttaa cctcagccaa tattaccaac    1740
attgtggaac aggtcaaaag aattgtgaat aaagaagaaa acattgatat aacacttggc    1800
tcaactctaa tgaatatatt ttctaatatc ttaagcagtt cagacagtga cttgcttgag    1860
tcatcttctg aagctttaaa aacaattgat gaattggcct tcaagataga cctaaatagc    1920
acatcacatg tgaatattac aactcggaac ttggctctca gcgtatcatc cctgttacca    1980
gggacaaatg caatttcaaa ttttagcatt ggtcttccaa gcaataatga atcgtatttc    2040
caggtaatga gccagtggtt tctttcattt taa                                 2073

<210> 54
<211> 690
<212> PRT
<213> homo sapiens

<400> 54
Met Met Phe Arg Ser Asp Arg Met Trp Ser Cys His Trp Lys Trp Lys
1               5                   10                  15
Pro Ser Pro Leu Leu Phe Leu Phe Ala Leu Tyr Ile Met Cys Val Pro
            20                  25                  30
His Ser Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125

Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Asp Gly Ile Ile Tyr
    370                 375                 380
Arg Ile Ser Val Val Ile Gln Asn Ile Leu Arg His Pro Glu Val Lys
385                 390                 395                 400
Val Gln Ser Lys Val Ala Glu Trp Leu Asn Ser Thr Phe Gln Asn Trp
                405                 410                 415
Asn Tyr Thr Val Tyr Val Val Asn Ile Ser Phe His Leu Ser Ala Gly
            420                 425                 430
Glu Asp Lys Ile Lys Val Lys Arg Ser Leu Glu Asp Glu Pro Arg Leu
        435                 440                 445
Val Leu Trp Ala Leu Leu Val Tyr Asn Ala Thr Asn Asn Thr Asn Leu
    450                 455                 460
Glu Gly Lys Ile Ile Gln Gln Lys Leu Leu Lys Asn Asn Glu Ser Leu
465                 470                 475                 480
Asp Glu Gly Leu Arg Leu His Thr Val Asn Val Arg Gln Leu Gly His
                485                 490                 495
Cys Leu Ala Met Glu Glu Pro Lys Gly Tyr Tyr Trp Pro Ser Ile Gln
            500                 505                 510
Pro Ser Glu Tyr Val Leu Pro Cys Pro Asp Lys Pro Gly Phe Ser Ala
        515                 520                 525
Ser Arg Ile Cys Phe Tyr Asn Ala Thr Asn Pro Leu Val Thr Tyr Trp
    530                 535                 540
Gly Pro Val Asp Ile Ser Asn Cys Leu Lys Glu Ala Asn Glu Val Ala
545                 550                 555                 560
Asn Gln Ile Leu Asn Leu Thr Ala Asp Gly Gln Asn Leu Thr Ser Ala
                565                 570                 575
Asn Ile Thr Asn Ile Val Glu Gln Val Lys Arg Ile Val Asn Lys Glu
            580                 585                 590
Glu Asn Ile Asp Ile Thr Leu Gly Ser Thr Leu Met Asn Ile Phe Ser
        595                 600                 605
Asn Ile Leu Ser Ser Ser Asp Ser Asp Leu Leu Glu Ser Ser Ser Glu
    610                 615                 620
Ala Leu Lys Thr Ile Asp Glu Leu Ala Phe Lys Ile Asp Leu Asn Ser
625                 630                 635                 640
Thr Ser His Val Asn Ile Thr Thr Arg Asn Leu Ala Leu Ser Val Ser
                645                 650                 655
Ser Leu Leu Pro Gly Thr Asn Ala Ile Ser Asn Phe Ser Ile Gly Leu
            660                 665                 670

Pro Ser Asn Asn Glu Ser Tyr Phe Gln Val Met Ser Gln Trp Phe Leu
        675                 680                 685
Ser Phe
    690

<210> 55
<211> 3255
<212> DNA
<213> homo sapiens

<400> 55
atgatgtttc gctcagatcg aatgtggagc tgccattgga aatggaagcc cagtcctctc      60
ctgttcttat ttgctttata tatcatgtgt gttcctcact cagtgtgggg atgtgccaac     120
tgccgagtgg ttttgtccaa cccttctggg acctttactt ctccatgcta ccctaacgac     180
tacccaaaca gccaggcttg catgtggacg ctccgagccc ccaccggtta tatcattcag     240
ataacattta acgactttga cattgaagaa gctcccaatt gcatttatga ctcattatcc     300
cttgataatg gagagagcca gactaaattt tgtggagcaa ctgccaaagg cctatcattt     360
aactcaagtg cgaatgagat gcatgtgtcc ttttcaagtg actttagcat ccagaagaaa     420
ggtttcaatg ccagctacat cagagttgcc gtgtccttaa ggaatcaaaa ggtcatttta     480
ccccagacat cagatgctta ccaggtatct gttgcaaaaa gcatctctat tccagagctc     540
agtgctttca cactctgctt tgaagcaacc aaagttggcc atgaagacag tgattggaca     600
gctttctcct actcaaatgc atccttcaca caattgctca gttttggaaa ggccaagagt     660
ggctactttc tatccatttc tgattcaaaa tgtttgttga ataatgcatt acctgtcaaa     720
gaaaaagaag acatttttgc agaaagcttt gaacagctct gccttgtttg gaataattct     780
ttgggctcta ttggtgtaaa tttcaaaaga aactatgaaa cagttccatg tgattctacc     840
attagtaaag ttattcctgg gaatgggaaa ttgttgttgg gctccaatca aaatgaaatt     900
gtctctctaa aaggggacat ttataacttt cgactttgga attttaccat gaatgccaaa     960
atcctctcca acctcagctg taatgtgaaa gggaatgtag tcgactggca aaatgacttc    1020
tggaatatcc caaacctagc tctgaaagct gaaagcaacc taagctgtgg ttcctacctg    1080
atcccgctcc cagcagcaga actggccagc tgtgcagacc tggggaccct ctgtcaagat    1140
ggaattatct atagaatatc cgtagtgatt cagaacatcc ttcgtcaccc tgaggtaaaa    1200
gtacagagca aggtggcaga atggctcaat tcaaccttcc aaaattggaa ctacacggtt    1260
tatgtcgtta atatcagttt tcacctgagt gctggagagg acaagattaa agtcaagaga    1320
agccttgagg atgagccaag gttggtgctt tgggcccttc tagtttacaa tgctaccaac    1380
aatactaatt tagaaggaaa aatcattcag cagaagctcc taaaaaataa tgagtccttg    1440
gatgaaggct tgaggctaca tacagtgaat gtgagacaac tgggtcattg tcttgccatg    1500
gaggaaccca aaggctacta ctggccatct atccaacctt ctgaatacgt tcttccttgt    1560
ccagacaagc ctggcttttc tgcttctcgg atatgttttt acaatgctac caacccattg    1620
gtaacctact ggggacctgt tgatatctcc aactgtttaa aagaagcaaa tgaagttgct    1680
aaccagattt taaatttaac tgctgatggg cagaacttaa cctcagccaa tattaccaac    1740
attgtggaac aggtcaaaag aattgtgaat aaagaagaaa acattgatat aacacttggc    1800
tcaactctaa tgaatatatt ttctaatatc ttaagcagtt cagacagtga cttgcttgag    1860
tcatcttctg aagctttaaa aacaattgat gaattggcct tcaagataga cctaaatagc    1920
acatcacatg tgaatattac aactcggaac ttggctctca gcgtatcatc cctgttacca    1980
gggacaaatg caatttcaaa ttttagcatt ggtcttccaa gcaataatga atcgtatttc    2040
cagatggatt ttgagagtgg acaagtggat ccactggcat ctgtaatttt gcctccaaac    2100
ttacttgaga atttaagtcc agaagattct gtattagtta gaagagcaca gtttactttc    2160
ttcaacaaaa ctggactttt ccaggatgta ggaccccaaa gaaaaacttt agtgagttat    2220
gtgatggcgt gcagtattgg aaacattact atccagaatc tgaaggatcc tgttcaaata    2280
aaaatcaaac atacaagaac tcaggaagtg catcatccca tctgtgcctt ctgggatctg    2340
aacaaaaaca aaagttttgg aggatggaac acgtcaggat gtgttgcaca cagagattca    2400
gatgcaagtg agacagtctg cctgtgtaac cacttcacac actttggagt tctgatggac    2460
cttccaagaa gtgcctcaca gttagatgca agaaacacta aagtcctcac tttcatcagc    2520
tatattgggt gtggaatatc tgctattttt tcagcagcaa ctctcctgac atatgttgct    2580
tttgagaaat tgcgaaggga ttatccctcc aaaatcttga tgaacctgag cacagccctg    2640
ctgttcctga atctcctctt cctcctagat ggctggatca cctccttcaa tgtggatgga    2700
ctttgcattg ctgttgcagt cctgttgcat ttcttccttc tggcaacctt tacctggatg    2760
gggctagaag caattcacat gtacattgct ctagttaaag tatttaacac ttacattcgc    2820
cgatacattc taaaattctg catcattggc tggggtttgc ctgccttagt ggtgtcagtt    2880
gttctagcga gcagaaacaa caatgaagtc tatggaaaag aaagttatgg gaaagaaaaa    2940
ggtgatgaat tctgttggat tcaagatcca gtcatatttt atgtgacctg tgctgggtat    3000
tttggagtca tgttttttct gaacattgcc atgttcattg tggtaatggt gcagatctgt    3060
gggaggaatg gcaagagaag caaccggacc ctgagagaag aagtgttaag gaacctgcgc    3120
agtgtggtta gcttgacctt tctgttgggc atgacatggg gttttgcatt ctttgcctgg    3180
ggacccttaa atatcccctt catgtacctc ttctccatct tcaattcatt acaaggtaag    3240
ataaattgta catga                                                     3255

<210> 56

<211> 1084
<212> PRT
<213> homo sapiens

<400> 56
Met Met Phe Arg Ser Asp Arg Met Trp Ser Cys His Trp Lys Trp Lys
1               5                   10                  15
Pro Ser Pro Leu Leu Phe Leu Phe Ala Leu Tyr Ile Met Cys Val Pro
            20                  25                  30
His Ser Val Trp Gly Cys Ala Asn Cys Arg Val Val Leu Ser Asn Pro
        35                  40                  45
Ser Gly Thr Phe Thr Ser Pro Cys Tyr Pro Asn Asp Tyr Pro Asn Ser
    50                  55                  60
Gln Ala Cys Met Trp Thr Leu Arg Ala Pro Thr Gly Tyr Ile Ile Gln
65                  70                  75                  80
Ile Thr Phe Asn Asp Phe Asp Ile Glu Glu Ala Pro Asn Cys Ile Tyr
                85                  90                  95
Asp Ser Leu Ser Leu Asp Asn Gly Glu Ser Gln Thr Lys Phe Cys Gly
            100                 105                 110
Ala Thr Ala Lys Gly Leu Ser Phe Asn Ser Ser Ala Asn Glu Met His
        115                 120                 125
Val Ser Phe Ser Ser Asp Phe Ser Ile Gln Lys Lys Gly Phe Asn Ala
    130                 135                 140
Ser Tyr Ile Arg Val Ala Val Ser Leu Arg Asn Gln Lys Val Ile Leu
145                 150                 155                 160
Pro Gln Thr Ser Asp Ala Tyr Gln Val Ser Val Ala Lys Ser Ile Ser
                165                 170                 175
Ile Pro Glu Leu Ser Ala Phe Thr Leu Cys Phe Glu Ala Thr Lys Val
            180                 185                 190
Gly His Glu Asp Ser Asp Trp Thr Ala Phe Ser Tyr Ser Asn Ala Ser
        195                 200                 205
Phe Thr Gln Leu Leu Ser Phe Gly Lys Ala Lys Ser Gly Tyr Phe Leu
    210                 215                 220
Ser Ile Ser Asp Ser Lys Cys Leu Leu Asn Asn Ala Leu Pro Val Lys
225                 230                 235                 240
Glu Lys Glu Asp Ile Phe Ala Glu Ser Phe Glu Gln Leu Cys Leu Val
                245                 250                 255
Trp Asn Asn Ser Leu Gly Ser Ile Gly Val Asn Phe Lys Arg Asn Tyr
            260                 265                 270
Glu Thr Val Pro Cys Asp Ser Thr Ile Ser Lys Val Ile Pro Gly Asn
        275                 280                 285
Gly Lys Leu Leu Leu Gly Ser Asn Gln Asn Glu Ile Val Ser Leu Lys
    290                 295                 300
Gly Asp Ile Tyr Asn Phe Arg Leu Trp Asn Phe Thr Met Asn Ala Lys
305                 310                 315                 320
Ile Leu Ser Asn Leu Ser Cys Asn Val Lys Gly Asn Val Val Asp Trp
                325                 330                 335
Gln Asn Asp Phe Trp Asn Ile Pro Asn Leu Ala Leu Lys Ala Glu Ser
            340                 345                 350
Asn Leu Ser Cys Gly Ser Tyr Leu Ile Pro Leu Pro Ala Ala Glu Leu
        355                 360                 365
Ala Ser Cys Ala Asp Leu Gly Thr Leu Cys Gln Asp Gly Ile Ile Tyr
    370                 375                 380
Arg Ile Ser Val Val Ile Gln Asn Ile Leu Arg His Pro Glu Val Lys
385                 390                 395                 400
Val Gln Ser Lys Val Ala Glu Trp Leu Asn Ser Thr Phe Gln Asn Trp
                405                 410                 415
Asn Tyr Thr Val Tyr Val Val Asn Ile Ser Phe His Leu Ser Ala Gly
            420                 425                 430
Glu Asp Lys Ile Lys Val Lys Arg Ser Leu Glu Asp Glu Pro Arg Leu
        435                 440                 445
Val Leu Trp Ala Leu Leu Val Tyr Asn Ala Thr Asn Asn Thr Asn Leu
    450                 455                 460
Glu Gly Lys Ile Ile Gln Gln Lys Leu Leu Lys Asn Asn Glu Ser Leu
465                 470                 475                 480
Asp Glu Gly Leu Arg Leu His Thr Val Asn Val Arg Gln Leu Gly His
                485                 490                 495
Cys Leu Ala Met Glu Glu Pro Lys Gly Tyr Tyr Trp Pro Ser Ile Gln

            500                 505                 510
Pro Ser Glu Tyr Val Leu Pro Cys Pro Asp Lys Pro Gly Phe Ser Ala
        515                 520                 525
Ser Arg Ile Cys Phe Tyr Asn Ala Thr Asn Pro Leu Val Thr Tyr Trp
    530                 535                 540
Gly Pro Val Asp Ile Ser Asn Cys Leu Lys Glu Ala Asn Glu Val Ala
545                 550                 555                 560
Asn Gln Ile Leu Asn Leu Thr Ala Asp Gly Gln Asn Leu Thr Ser Ala
                565                 570                 575
Asn Ile Thr Asn Ile Val Glu Gln Val Lys Arg Ile Val Asn Lys Glu
            580                 585                 590
Glu Asn Ile Asp Ile Thr Leu Gly Ser Thr Leu Met Asn Ile Phe Ser
        595                 600                 605
Asn Ile Leu Ser Ser Ser Asp Ser Asp Leu Leu Glu Ser Ser Ser Glu
    610                 615                 620
Ala Leu Lys Thr Ile Asp Glu Leu Ala Phe Lys Ile Asp Leu Asn Ser
625                 630                 635                 640
Thr Ser His Val Asn Ile Thr Thr Arg Asn Leu Ala Leu Ser Val Ser
                645                 650                 655
Ser Leu Leu Pro Gly Thr Asn Ala Ile Ser Asn Phe Ser Ile Gly Leu
            660                 665                 670
Pro Ser Asn Asn Glu Ser Tyr Phe Gln Met Asp Phe Glu Ser Gly Gln
        675                 680                 685
Val Asp Pro Leu Ala Ser Val Ile Leu Pro Pro Asn Leu Leu Glu Asn
    690                 695                 700
Leu Ser Pro Glu Asp Ser Val Leu Val Arg Arg Ala Gln Phe Thr Phe
705                 710                 715                 720
Phe Asn Lys Thr Gly Leu Phe Gln Asp Val Gly Pro Gln Arg Lys Thr
                725                 730                 735
Leu Val Ser Tyr Val Met Ala Cys Ser Ile Gly Asn Ile Thr Ile Gln
            740                 745                 750
Asn Leu Lys Asp Pro Val Gln Ile Lys Ile Lys His Thr Arg Thr Gln
        755                 760                 765
Glu Val His His Pro Ile Cys Ala Phe Trp Asp Leu Asn Lys Asn Lys
    770                 775                 780
Ser Phe Gly Gly Trp Asn Thr Ser Gly Cys Val Ala His Arg Asp Ser
785                 790                 795                 800
Asp Ala Ser Glu Thr Val Cys Leu Cys Asn His Phe Thr His Phe Gly
                805                 810                 815
Val Leu Met Asp Leu Pro Arg Ser Ala Ser Gln Leu Asp Ala Arg Asn
            820                 825                 830
Thr Lys Val Leu Thr Phe Ile Ser Tyr Ile Gly Cys Gly Ile Ser Ala
        835                 840                 845
Ile Phe Ser Ala Ala Thr Leu Leu Thr Tyr Val Ala Phe Glu Lys Leu
    850                 855                 860
Arg Arg Asp Tyr Pro Ser Lys Ile Leu Met Asn Leu Ser Thr Ala Leu
865                 870                 875                 880
Leu Phe Leu Asn Leu Leu Phe Leu Leu Asp Gly Trp Ile Thr Ser Phe
                885                 890                 895
Asn Val Asp Gly Leu Cys Ile Ala Val Ala Val Leu Leu His Phe Phe
            900                 905                 910
Leu Leu Ala Thr Phe Thr Trp Met Gly Leu Glu Ala Ile His Met Tyr
        915                 920                 925
Ile Ala Leu Val Lys Val Phe Asn Thr Tyr Ile Arg Arg Tyr Ile Leu
    930                 935                 940
Lys Phe Cys Ile Ile Gly Trp Gly Leu Pro Ala Leu Val Val Ser Val
945                 950                 955                 960
Val Leu Ala Ser Arg Asn Asn Asn Glu Val Tyr Gly Lys Glu Ser Tyr
                965                 970                 975
Gly Lys Glu Lys Gly Asp Glu Phe Cys Trp Ile Gln Asp Pro Val Ile
            980                 985                 990
Phe Tyr Val Thr Cys Ala Gly Tyr Phe Gly Val Met Phe Phe Leu Asn
        995                 1000                1005
Ile Ala Met Phe Ile Val Val Met Val Gln Ile Cys Gly Arg Asn Gly
    1010                1015                1020
Lys Arg Ser Asn Arg Thr Leu Arg Glu Glu Val Leu Arg Asn Leu Arg
1025                1030                1035                1040
Ser Val Val Ser Leu Thr Phe Leu Leu Gly Met Thr Trp Gly Phe Ala

1045                    1050                    1055
Phe Phe Ala Trp Gly Pro Leu Asn Ile Pro Phe Met Tyr Leu Phe Ser
        1060                    1065                    1070
Ile Phe Asn Ser Leu Gln Gly Lys Ile Asn Cys Thr
        1075                    1080

<210> 57
<211> 3579
<212> DNA
<213> homo sapiens

<400> 57
atgtttcgct cagatcgaat gtggagctgc cattggaaat ggaagcccag tcctctcctg      60
ttcttatttg ctttatatat catgtgtgtt cctcactcag tgtggggatg tgccaactgc     120
cgagtggttt tgtccaaccc ttctgggacc tttacttctc catgctaccc taacgactac     180
ccaaacagcc aggcttgcat gtggacgctc cgagccccca ccggttatat cattcagata     240
acatttaacg actttgacat tgaagaagct cccaattgca tttatgactc attatccctt     300
gataatggag agagccagac taaattttgt ggagcaactg ccaaaggcct atcatttaac     360
tcaagtgcga atgagatgca tgtgtccttt tcaagtgact ttagcatcca gaagaaaggt     420
ttcaatgcca gctacatcag agttgccgtg tccttaagga atcaaaaggt cattttaccc      480
cagacatcag atgcttacca ggtatctgtt gcaaaaagca tctctattcc agagctcagt     540
gctttcacac tctgctttga agcaaccaaa gttggccatg aagacagtga ttggacagct     600
ttctcctact caaatgcatc cttcacacaa ttgctcagtt ttggaaaggc caagagtggc     660
tactttctat ccatttctga ttcaaaatgt ttgttgaata atgcattacc tgtcaaagaa     720
aaagaagaca tttttgcaga aagctttgaa cagctctgcc ttgtttggaa taattctttg      780
ggctctattg gtgtaaattt caaaagaaac tatgaaacag ttccatgtga ttctaccatt     840
agtaaagtta ttcctgggaa tgggaaattg ttgttgggct ccaatcaaaa tgaaattgtc     900
tctctaaaag ggacatttta taactttcga ctttggaatt ttaccatgaa tgccaaaatc     960
ctctccaacc tcagctgtaa tgtgaaaggg aatgtagtcg actggcaaaa tgacttctgg    1020
aatatcccaa acctagctct gaaagctgaa agcaacctaa gctgtggttc ctacctgatc    1080
ccgctcccag cagcagaact ggccagctgt gcagacctgg ggaccctctg tcaagatgga    1140
attatctata gaatatccgt agtgattcag aacatccttc gtcaccctga ggtaaaagta    1200
cagagcaagg tggcagaatg gctcaattca accttccaaa attggaacta cacggtttat    1260
gtcgttaata tcagttttca cctgagtgct ggagaggaca agattaaagt caagagaagc    1320
cttgaggatg agccaaggtt ggtgctttgg gcccttctag tttacaatgc taccaacaat    1380
actaatttag aaggaaaaat cattcagcag aagctcctaa aaaataatga gtccttggat    1440
gaaggcttga ggctacatac agtgaatgtg agacaactgg gtcattgtct tgccatggag    1500
gaacccaaag gctactactg gccatctatc caaccttctg aatacgttct tccttgtcca    1560
gacaagcctg gcttttctgc ttctcggata tgtttttaca atgctaccaa cccattggta     1620
acctactggg gacctgttga tatctccaac tgtttaaaag aagcaaatga agttgctaac    1680
cagattttaa atttaactgc tgatgggcag aacttaacct cagccaatat taccaacatt    1740
gtggaacagg tcaaaagaat tgtgaataaa gaagaaaaca ttgatataac acttggctca    1800
actctaatga atatattttc taatatctta agcagttcag acagtgactt gcttgagtca    1860
tcttctgaag ctttaaaaac aattgatgaa ttggccttca agatagacct aaatagcaca    1920
tcacatgtga atattacaac tcggaacttg gctctcagcg tatcatccct gttaccaggg    1980
acaaatgcaa tttcaaattt tagcattggt cttccaagca ataatgaatc gtatttccag    2040
atggattttg agagtggaca agtggatcca ctggcatctg taattttgcc tccaaactta    2100
cttgagaatt taagtccaga agattctgta ttagttagaa gagcacagtt tactttcttc    2160
aacaaaactg gacttttcca ggatgtagga ccccaaagaa aaactttagt gagttatgtg    2220
atggcgtgca gtattggaaa cattactatc cagaatctga aggatcctgt tcaaataaaa    2280
atcaaacata caagaactca ggaagtgcat catcccatct gtgccttctg ggatctgaac    2340
aaaaacaaaa gttttggagg atggaacacg tcaggatgtg ttgcacacag agattcagat    2400
gcaagtgaga cagtctgcct gtgtaaccac ttcacacact ttggagttct gatggacctt    2460
ccaagaagtg cctcacagtt agatgcaaga aacactaaag tcctcacttt catcagctat    2520
attgggtgtg gaatatctgc tattttttca gcagcaactc tcctgacata tgttgctttt     2580
gagaaattgc gaagggatta tccctccaaa atcttgatga acctgagcac agccctgctg    2640
ttcctgaatc tcctcttcct cctagatggc tggatcacct ccttcaatgt ggatggactt    2700
tgcattgctg ttgcagtcct gttgcatttc ttccttctgg caacctttac ctggatgggg    2760
ctagaagcaa ttcacatgta cattgctcta gttaaagtat ttaacactta cattcgccga    2820
tacattctaa aattctgcat cattggctgg ggtttgcctg ccttagtggt gtcagttgtt    2880
ctagcgagca gaaacaacaa tgaagtctat ggaaaagaaa gttatgggaa agaaaaaggt    2940
gatgaattct gttggattca agatccagtc atattttatg tgacctgtgc tgggtatttt    3000
ggagtcatgt tttttctgaa cattgccatg ttcattgtgg taatggtgca gatctgtggg    3060
aggaatggca agagaagcaa ccggaccctg agagaagaag tgttaaggaa cctgcgcagt    3120
gtggttagct tgacctttct gttgggcatg acatggggtt ttgcattctt tgcctgggga    3180
cccttaaata tccccttcat gtacctcttc tccatcttca attcattaca aggcttattt    3240
atattcatct tccactgtgc tatgaaggag aatgttcaga aacagtggcg gcggcatctc    3300
tgctgtggta gatttcggtt agcagataac tcagattgga gtaagacagc taccaatatc    3360

atcaagaaaa gttctgataa tctaggaaaa tctttgtctt caagctccat tggttccaac 3420
tcaacctatc ttacatccaa atctaaatcc agctctacca cctatttcaa aaggaatagc 3480
cacacagata atgtctccta tgagcattcc ttcaacaaaa gtggatcact cagacagtgc 3540
ttccatggac aagtccttgt caaaactggc ccatgctga 3579

<210> 58
<211> 1192
<212> PRT
<213> homo sapiens

<400> 58
Met Phe Arg Ser Asp Arg Met Trp Ser Cys His Trp Lys Trp Lys Pro
1               5                   10                  15
Ser Pro Leu Leu Phe Leu Phe Ala Leu Tyr Ile Met Cys Val Pro His
            20                  25                  30
Ser Val Trp Gly Cys Ala Asn Cys Arg Val Val Leu Ser Asn Pro Ser
        35                  40                  45
Gly Thr Phe Thr Ser Pro Cys Tyr Pro Asn Asp Tyr Pro Asn Ser Gln
    50                  55                  60
Ala Cys Met Trp Thr Leu Arg Ala Pro Thr Gly Tyr Ile Ile Gln Ile
65                  70                  75                  80
Thr Phe Asn Asp Phe Asp Ile Glu Glu Ala Pro Asn Cys Ile Tyr Asp
                85                  90                  95
Ser Leu Ser Leu Asp Asn Gly Glu Ser Gln Thr Lys Phe Cys Gly Ala
            100                 105                 110
Thr Ala Lys Gly Leu Ser Phe Asn Ser Ser Ala Asn Glu Met His Val
        115                 120                 125
Ser Phe Ser Ser Asp Phe Ser Ile Gln Lys Lys Gly Phe Asn Ala Ser
    130                 135                 140
Tyr Ile Arg Val Ala Val Ser Leu Arg Asn Gln Lys Val Ile Leu Pro
145                 150                 155                 160
Gln Thr Ser Asp Ala Tyr Gln Val Ser Val Ala Lys Ser Ile Ser Ile
                165                 170                 175
Pro Glu Leu Ser Ala Phe Thr Leu Cys Phe Glu Ala Thr Lys Val Gly
            180                 185                 190
His Glu Asp Ser Asp Trp Thr Ala Phe Ser Tyr Ser Asn Ala Ser Phe
        195                 200                 205
Thr Gln Leu Leu Ser Phe Gly Lys Ala Lys Ser Gly Tyr Phe Leu Ser
    210                 215                 220
Ile Ser Asp Ser Lys Cys Leu Leu Asn Asn Ala Leu Pro Val Lys Glu
225                 230                 235                 240
Lys Glu Asp Ile Phe Ala Glu Ser Phe Glu Gln Leu Cys Leu Val Trp
                245                 250                 255
Asn Asn Ser Leu Gly Ser Ile Gly Val Asn Phe Lys Arg Asn Tyr Glu
            260                 265                 270
Thr Val Pro Cys Asp Ser Thr Ile Ser Lys Val Ile Pro Gly Asn Gly
        275                 280                 285
Lys Leu Leu Leu Gly Ser Asn Gln Asn Glu Ile Val Ser Leu Lys Gly
    290                 295                 300
Asp Ile Tyr Asn Phe Arg Leu Trp Asn Phe Thr Met Asn Ala Lys Ile
305                 310                 315                 320
Leu Ser Asn Leu Ser Cys Asn Val Lys Gly Asn Val Val Asp Trp Gln
                325                 330                 335
Asn Asp Phe Trp Asn Ile Pro Asn Leu Ala Leu Lys Ala Glu Ser Asn
            340                 345                 350
Leu Ser Cys Gly Ser Tyr Leu Ile Pro Leu Pro Ala Ala Glu Leu Ala
        355                 360                 365
Ser Cys Ala Asp Leu Gly Thr Leu Cys Gln Asp Gly Ile Ile Tyr Arg
    370                 375                 380
Ile Ser Val Val Ile Gln Asn Ile Leu Arg His Pro Glu Val Lys Val
385                 390                 395                 400
Gln Ser Lys Val Ala Glu Trp Leu Asn Ser Thr Phe Gln Asn Trp Asn
                405                 410                 415
Tyr Thr Val Tyr Val Val Asn Ile Ser Phe His Leu Ser Ala Gly Glu
            420                 425                 430
Asp Lys Ile Lys Val Lys Arg Ser Leu Glu Asp Glu Pro Arg Leu Val
        435                 440                 445
Leu Trp Ala Leu Leu Val Tyr Asn Ala Thr Asn Asn Thr Asn Leu Glu

450                 455                 460
Gly Lys Ile Ile Gln Gln Lys Leu Leu Lys Asn Asn Glu Ser Leu Asp
465                 470                 475                 480
Glu Gly Leu Arg Leu His Thr Val Asn Val Arg Gln Leu Gly His Cys
                485                 490                 495
Leu Ala Met Glu Glu Pro Lys Gly Tyr Tyr Trp Pro Ser Ile Gln Pro
            500                 505                 510
Ser Glu Tyr Val Leu Pro Cys Pro Asp Lys Pro Gly Phe Ser Ala Ser
        515                 520                 525
Arg Ile Cys Phe Tyr Asn Ala Thr Asn Pro Leu Val Thr Tyr Trp Gly
    530                 535                 540
Pro Val Asp Ile Ser Asn Cys Leu Lys Glu Ala Asn Glu Val Ala Asn
545                 550                 555                 560
Gln Ile Leu Asn Leu Thr Ala Asp Gly Gln Asn Leu Thr Ser Ala Asn
                565                 570                 575
Ile Thr Asn Ile Val Glu Gln Val Lys Arg Ile Val Asn Lys Glu Glu
            580                 585                 590
Asn Ile Asp Ile Thr Leu Gly Ser Thr Leu Met Asn Ile Phe Ser Asn
        595                 600                 605
Ile Leu Ser Ser Ser Asp Ser Asp Leu Leu Glu Ser Ser Ser Glu Ala
    610                 615                 620
Leu Lys Thr Ile Asp Glu Leu Ala Phe Lys Ile Asp Leu Asn Ser Thr
625                 630                 635                 640
Ser His Val Asn Ile Thr Thr Arg Asn Leu Ala Leu Ser Val Ser Ser
                645                 650                 655
Leu Leu Pro Gly Thr Asn Ala Ile Ser Asn Phe Ser Ile Gly Leu Pro
            660                 665                 670
Ser Asn Asn Glu Ser Tyr Phe Gln Met Asp Phe Glu Ser Gly Gln Val
        675                 680                 685
Asp Pro Leu Ala Ser Val Ile Leu Pro Pro Asn Leu Leu Glu Asn Leu
    690                 695                 700
Ser Pro Glu Asp Ser Val Leu Val Arg Arg Ala Gln Phe Thr Phe Phe
705                 710                 715                 720
Asn Lys Thr Gly Leu Phe Gln Asp Val Gly Pro Gln Arg Lys Thr Leu
                725                 730                 735
Val Ser Tyr Val Met Ala Cys Ser Ile Gly Asn Ile Thr Ile Gln Asn
            740                 745                 750
Leu Lys Asp Pro Val Gln Ile Lys Ile Lys His Thr Arg Thr Gln Glu
        755                 760                 765
Val His His Pro Ile Cys Ala Phe Trp Asp Leu Asn Lys Asn Lys Ser
    770                 775                 780
Phe Gly Gly Trp Asn Thr Ser Gly Cys Val Ala His Arg Asp Ser Asp
785                 790                 795                 800
Ala Ser Glu Thr Val Cys Leu Cys Asn His Phe Thr His Phe Gly Val
                805                 810                 815
Leu Met Asp Leu Pro Arg Ser Ala Ser Gln Leu Asp Ala Arg Asn Thr
            820                 825                 830
Lys Val Leu Thr Phe Ile Ser Tyr Ile Gly Cys Gly Ile Ser Ala Ile
        835                 840                 845
Phe Ser Ala Ala Thr Leu Leu Thr Tyr Val Ala Phe Glu Lys Leu Arg
    850                 855                 860
Arg Asp Tyr Pro Ser Lys Ile Leu Met Asn Leu Ser Thr Ala Leu Leu
865                 870                 875                 880
Phe Leu Asn Leu Leu Phe Leu Leu Asp Gly Trp Ile Thr Ser Phe Asn
                885                 890                 895
Val Asp Gly Leu Cys Ile Ala Val Ala Val Leu Leu His Phe Phe Leu
            900                 905                 910
Leu Ala Thr Phe Thr Trp Met Gly Leu Glu Ala Ile His Met Tyr Ile
        915                 920                 925
Ala Leu Val Lys Val Phe Asn Thr Tyr Ile Arg Arg Tyr Ile Leu Lys
    930                 935                 940
Phe Cys Ile Ile Gly Trp Gly Leu Pro Ala Leu Val Val Ser Val Val
945                 950                 955                 960
Leu Ala Ser Arg Asn Asn Asn Glu Val Tyr Gly Lys Glu Ser Tyr Gly
                965                 970                 975
Lys Glu Lys Gly Asp Glu Phe Cys Trp Ile Gln Asp Pro Val Ile Phe
            980                 985                 990
Tyr Val Thr Cys Ala Gly Tyr Phe Gly Val Met Phe Phe Leu Asn Ile

995                 1000                1005
Ala Met Phe Ile Val Val Met Val Gln Ile Cys Gly Arg Asn Gly Lys
    1010                1015                1020
Arg Ser Asn Arg Thr Leu Arg Glu Glu Val Leu Arg Asn Leu Arg Ser
1025                1030                1035                1040
Val Val Ser Leu Thr Phe Leu Leu Gly Met Thr Trp Gly Phe Ala Phe
                1045                1050                1055
Phe Ala Trp Gly Pro Leu Asn Ile Pro Phe Met Tyr Leu Phe Ser Ile
            1060                1065                1070
Phe Asn Ser Leu Gln Gly Leu Phe Ile Phe Ile Phe His Cys Ala Met
        1075                1080                1085
Lys Glu Asn Val Gln Lys Gln Trp Arg Arg His Leu Cys Cys Gly Arg
    1090                1095                1100
Phe Arg Leu Ala Asp Asn Ser Asp Trp Ser Lys Thr Ala Thr Asn Ile
1105                1110                1115                1120
Ile Lys Lys Ser Ser Asp Asn Leu Gly Lys Ser Leu Ser Ser Ser Ser
                1125                1130                1135
Ile Gly Ser Asn Ser Thr Tyr Leu Thr Ser Lys Ser Lys Ser Ser Ser
            1140                1145                1150
Thr Thr Tyr Phe Lys Arg Asn Ser His Thr Asp Asn Val Ser Tyr Glu
        1155                1160                1165
His Ser Phe Asn Lys Ser Gly Ser Leu Arg Gln Cys Phe His Gly Gln
    1170                1175                1180
Val Leu Val Lys Thr Gly Pro Cys
1185                1190

<210> 59
<211> 4446
<212> DNA
<213> homo sapiens

<400> 59
ttgcgccgtc gggaaagccc atgaactctc cagaaacggc gtaaaggagg gtcccgccgc      60
ggcgcagggc tggggcgcct gggttccccc tgggtggagc agcggcagca gagcgggaaa     120
gtggtggagg atgatcttgc ggccaaaggg gacctcggcg cagtaatgtc aacataatta     180
aacaaggagt aacaggcacc gctcctcagt ccaaaggcct ggccctgcca agaaggcgct     240
ctccggaatc aacacctggg ggcttggaag gatgtttcgc tcagatcgaa tgtggagctg     300
ccattggaaa tggaagccca gtcctctcct gttcttattt gctttatata tcatgtgtgt     360
tcctcactca gtgtggggat gtgccaactg ccgagtggtt ttgtccaacc cttctgggac     420
ctttacttct ccatgctacc ctaacgacta cccaaacagc caggcttgca tgtggacgct     480
ccgagccccc accggttata tcattcagat aacatttaac gactttgaca ttgaagaagc     540
tcccaattgc atttatgact cattatccct tgataatgga gagagccaga ctaaattttg     600
tggagcaact gccaaaggcc tatcatttaa ctcaagtgcg aatgagatgc atgtgtcctt     660
ttcaagtgac tttagcatcc agaagaaagg tttcaatgcc agctacatca gagttgccgt     720
gtccttaagg aatcaaaagg tcattttacc ccagacatca gatgcttacc aggtatctgt     780
tgcaaaaagc atctctattc cagagctcag tgctttcaca ctctgctttg aagcaaccaa     840
agttggccat gaagacagtg attggacagc tttctcctac tcaaatgcat ccttcacaca     900
attgctcagt tttggaaagg ccaagagtgg ctactttcta tccatttctg attcaaaatg     960
tttgttgaat aatgcattac ctgtcaaaga aaaagaagac atttttgcag aaagctttga    1020
acagctctgc cttgtttgga ataattcttt gggctctatt ggtgtaaatt tcaaaagaaa    1080
ctatgaaaca gttccatgtg attctaccat tagtaaagtt attcctggga atgggaaatt    1140
gttgttgggc tccaatcaaa atgaaattgt ctctctaaaa ggggacattt ataactttcg    1200
actttggaat tttaccatga atgccaaaat cctctccaac ctcagctgta atgtgaaagg    1260
gaatgtagtc gactggcaaa atgacttctg gaatatccca aacctagctc tgaaagctga    1320
aagcaaccta agctgtggtt cctacctgat cccgctccca gcagcagaac tggccagctg    1380
tgcagacctg ggaccctctg tcaagatgg aattatctat agaatatccg tagtgattca    1440
gaacatcctt cgtcaccctg aggtaaaagt acagagcaag gtggcagaat ggctcaattc    1500
aaccttccaa aattggaact acacggttta tgtcgttaat atcagttttc acctgagtgc    1560
tggagaggac aagattaaag tcaagagaag ccttgaggat gagccaaggt tggtgctttg    1620
ggcccttcta gtttacaatg ctaccaacaa tactaattta gaaggaaaaa tcattcagca    1680
gaagctccta aaaaataatg agtccttgga tgaaggcttg aggctacata cagtgaatgt    1740
gagacaactg ggtcattgtc ttgccatgga ggaacccaaa ggctactact ggccatctat    1800
ccaaccttct gaatacgttc ttccttgtcc agacaagcct ggcttttctg cttctcggat    1860
atgtttttac aatgctacca acccattggt aacctactgg ggacctgttg atatctccaa    1920
ctgtttaaaa gaagcaaatg aagttgctaa ccagatttta aatttaactg ctgatggcca    1980
gaacttaacc tcagccaata ttaccaacat tgtggaacag gtcaaaagaa ttgtgaataa    2040
agaagaaaac attgatataa cacttggctc aactctaatg aatatatttt ctaatatctt    2100
aagcagttca gacagtgact tgcttgagtc atcttctgaa gctttaaaaa caattgatga    2160

attggccttc aagatagacc taaatagcac atcacatgtg aatattacaa ctcggaactt 2220
ggctctcagc gtatcatccc tgttaccagg gacaaatgca atttcaaatt ttagcattgg 2280
tcttccaagc aataatgaat cgtatttcca gatggatttt gagagtggac aagtggatcc 2340
actggcatct gtaattttgc ctccaaactt acttgagaat ttaagtccag aagattctgt 2400
attagttaga agagcacagt ttactttctt caacaaaact ggacttttcc aggatgtagg 2460
accccaaaga aaaactttag tgagttatgt gatggcgtgc agtattggaa acattactat 2520
ccagaatctg aaggatcctg ttcaaataaa aatcaaacat acaagaactc aggaagtgca 2580
tcatcccatc tgtgccttct gggatctgaa caaaaacaaa agttttggag gatggaacac 2640
gtcaggatgt gttgcacaca gagattcaga tgcaagtgag acagtctgcc tgtgtaacca 2700
cttcacacac tttggagttc tgatggacct tccaagaagt gcctcacagt tagatgcaag 2760
aaacactaaa gtcctcactt tcatcagcta tattgggtgt ggaatatctg ctattttttc 2820
agcagcaact ctcctgacat atgttgcttt tgagaaattg cgaagggatt atccctccaa 2880
aatcttgatg aacctgagca cagccctgct gttcctgaat ctcctcttcc tcctagatgg 2940
ctggatcacc tccttcaatg tggatggact ttgcattgct gttgcagtcc tgttgcattt 3000
cttccttctg gcaaccttta cctggatggg gctagaagca attcacatgt acattgctct 3060
agttaaagta tttaacactt acattcgccg atacattcta aaattctgca tcattggctg 3120
gggtttgcct gccttagtgg tgtcagttgt tctagcgagc agaaacaaca atgaagtcta 3180
tggaaaagaa agttatggga aagaaaaagg tgatgaattc tgttggattc aagatccagt 3240
catattttat gtgacctgtg ctgggtattt tggagtcatg ttttttctga acattgccat 3300
gttcattgtg gtaatggtgc agatctgtgg gaggaatggc aagagaagca accggaccct 3360
gagagaagaa gtgttaagga acctgcgcag tgtggttagc ttgacctttc tgttgggcat 3420
gacatggggt tttgcattct ttgcctgggg acccttaaat atccccttca tgtacctctt 3480
ctccatcttc aattcattac aaggcttatt tatattcatc ttccactgtg ctatgaagga 3540
gaatgttcag aaacagtggc ggcggcatct ctgctgtggt agatttcggt tagcagataa 3600
ctcagattgg agtaagacag ctaccaatat catcaagaaa agttctgata atctaggaaa 3660
atctttgtct tcaagctcca ttggttccaa ctcaacctat cttacatcca aatctaaatc 3720
cagctctacc acctatttca aaaggaatag ccacacagat aatgtctcct atgagcattc 3780
cttcaacaaa agtggatcac tcagacagtg cttccatgga caagtccttg tcaaaactgg 3840
cccatgctga tggagatcaa acatcaatca tccctgtcca tcaggtcatt gataaggtca 3900
agggttattg caatgctcat tcagacaact tctataaaaa tattatcatg tcagacacct 3960
tcagccacag cacaaagttt taatgtcttt aagaaaaaga aatcaatctg cagaaatgtg 4020
aagatttgca agcagtgtaa actgcaacta gtgatgtaaa tgtgctatta cctaggtaac 4080
tgcatatata taaggaatgt attttgttaa gaaggctttt gtgaaattca gaatttttct 4140
ttttaatata tttcttccat ggaagagttg tcatcactaa aacttcagta ctgagagtaa 4200
catgactcag tagccacaga agctatgatt tgtaaaatat ataattgaat cagagtaatc 4260
ataatgcagg ggagacattc aaattagaga caagggagaa gcaatgctga ggaagaccct 4320
agatagagct cattttactc cacctaatcg ttatatctgg atatacccat tttctgcatc 4380
ttctttctca acaataaaaa atgtaactat tttgaatgcc cacaaaaaaa aaaaaaaaaa 4440
aaaaaa 4446